# EUROPEAN PATENT APPLICATION

(11) **EP 4 057 010 A2**
(43) Date of publication of application: **14.09.2022**
(21) Application number: 22160834.2
(22) Date of filing: 08.03.2022
(51) Int. Cl.: G01N 33/68, G06N 7/00, G16H 50/70

(54) **CARDIOVASCULAR DISEASE**

(30) Priority: 08.03.2021 EP 21315031
(71) Applicant: SANOFI, 75008 Paris (FR)
(72) Inventor: ALLEBRANDT, Karla, 65926 Frankfurt am Main (DE); FRAU, Francesca, 65926 Frankfurt am Main (DE)
(74) Representative: McDougall, James

(57) **Abstract**

The invention relates to cardiovascular disease, and particularly, although not exclusively, to cardiovascular disease biomarkers and their use in methods of diagnosis and prognosis. The invention also extends to diagnostic and prognostic kits utilising the biomarkers of the invention for diagnosing or prognosing cardiovascular disease.

SEQUENCE LISTING

<110>  Sanofi-Aventis Deutschland GmbH

<120>  Cardiovascular Disease

<130>  91435EP1

<160>  40

<170>  PatentIn version 3.5

<210>  1
<211>  523
<212>  PRT
<213>  Homo sapiens

<400>  1

<210>  2
<211>  10827
<212>  DNA
<213>  Homo sapiens

<400>  2
gtaccataga gttgctctga aaacagaaga tagagggagt ctcggagctc gccatctcca      60

gcgatctcta cattgggaaa aaacatggag tcagctccgg cagcccccga ccccgccgcc     120

agcgagccag gcagcagcgg cgcggacgcg gccgccggct ccagggagac cccgctgaac     180

caggaatccg cccgcaagag cgagccgcct gccccggtgc gcagacagag ctattccagc     240

accagcagag gtatctcagt aacgaagaag acacatacat ctcaaattga aattattcca     300

tgcaagatct gtggagacaa atcatcagga atccattatg gtgtcattac atgtgaaggc     360

tgcaagggct ttttcaggag aagtcagcaa agcaatgcca cctactcctg tcctcgtcag     420

aagaactgtt tgattgatcg aaccagtaga aaccgctgcc aacactgtcg attacagaaa     480

tgccttgccg tagggatgtc tcgagatgct gtaaaatttg gccgaatgtc aaaaaagcag     540

agagacagct tgtatgcaga agtacagaaa caccggatgc agcagcagca gcgcgaccac     600

cagcagcagc ctggagaggc tgagccgctg acgcccacct acaacatctc ggccaacggg     660

ctgacggaac ttcacgacga cctcagtaac tacattgacg ggcacacccc tgaggggagt     720

aaggcagact ccgccgtcag cagcttctac ctggacatac agccttcccc agaccagtca     780

ggtcttgata tcaatggaat caaaccagaa ccaatatgtg actacacacc agcatcaggc     840

ttctttccct actgttcgtt caccaacggc gagacttccc caactgtgtc catggcagaa     900

ttagaacacc ttgcacagaa tatatctaaa tcgcatctgg aaacctgcca atacttgaga     960

gaagagctcc agcagataac gtggcagacc tttttacagg aagaaattga gaactatcaa 1020

aacaagcagc gggaggtgat gtggcaattg tgtgccatca aaattacaga agctatacag 1080

tatgtggtgg agtttgccaa acgcattgat ggatttatgg aactgtgtca aaatgatcaa 1140

attgtgcttc taaaagcagg ttctctagag gtggtgttta tcagaatgtg ccgtgccttt 1200

gactctcaga acaacaccgt gtactttgat gggaagtatg ccagccccga cgtcttcaaa 1260

tccttaggtt gtgaagactt tattagcttt gtgtttgaat ttggaaagag tttatgttct 1320

atgcacctga ctgaagatga aattgcatta ttttctgcat ttgtactgat gtcagcagat 1380

cgctcatggc tgcaagaaaa ggtaaaaatt gaaaaactgc aacagaaaat tcagctagct 1440

cttcaacacg tcctacagaa gaatcaccga gaagatggaa tactaacaaa gttaatatgc 1500

aaggtgtcta ccttaagagc cttatgtgga cgacatacag aaaagctaat ggcatttaaa 1560

gcaatatacc cagacattgt gcgacttcat tttcctccat tatacaagga gttgttcact 1620

tcagaatttg agccagcaat gcaaattgat gggtaaatgt tatcacctaa gcacttctag 1680

aatgtctgaa gtacaaacat gaaaaacaaa caaaaaaatt aaccgagaca ctttatatgg 1740

ccctgcacag acctggagcg ccacacactg cacatctttt ggtgatcggg gtcaggcaaa 1800

ggaggggaaa caatgaaaac aaataaaagt tgaacttgtt tttctcatgc atatgatttc 1860

cattatgcct acagatatgg accctttttc tgtcttgact tcttgatcat tgacctctgt 1920

ttacaacagg aggagggtac taaagtcgga ggatttcctt ttcttgtagc tcactgccca 1980

cagactttct acagagtcac caatctgtca gtaacaacag agagtccagc aataatcggt 2040

gactggtgtg catagcggag gttgcggcat tactttgcac aactagctct ttgtttcatg 2100

aaggaagttt ttattttttc accgattatt gccagtccgc aggatggcat gaaaagggtc 2160

catagcagta gcaacaatag cattataata tattacaggg taaatgggca tgaagactat 2220

atatagctaa aagagatatt gtttatatat tgttttaagt aatataaaat gtagttactg 2280

gtgtagcttt tcctgttgaa ttgataaggc actttcattt tgcacctttt tctttaaatt 2340

aaatgctagc gtgttcactg tcgtgtcgca tgtgcaccag aaacacaagt ttaactgaga 2400

aggcttggaa ggtacgttgg gaggtattta tgctgctgtt tacaaattta tttttaagag 2460

actggctggt catatctaga aatcaccacg ttggattttt tttttaacat gtgaatttgg 2520

aattagaaac ggaactctcc ctaaattata ctttgctttt tggtaagttt aatgatagat 2580

gtgtttatgc ttcatacaaa gttgaatgat tgattggcgt ggtggacata taccatcatg 2640

ctcatttttt ttttttaaag ctttttaaaa tgccacctca tggaggcgag ggggaggaga 2700

agctcatttt acacaattca gtagttaaat atggactcgg tctcaacttg gaattcttat 2760

gctttgagaa caaatcaaca accagaatat ttattggaat ctagctttta ttataagaag 2820

gacccaaaga ttatatcctg agcaaatgca cactccccat gtgaggacat gaagtattta 2880

ctttgtgaat gtttatgttc ttggtataat ctaggaaccc tatgagttta tctcagagtg 2940

aactaattct agatttgttg tcaatagatg ctataattca agaatgttgc tctccatatt 3000

tgaaaaacga tggataggag ggtgagggaa gcatacaatg ttgaaccagt ttctcttatt 3060

taaatattaa atactttaag cctttaaagt gaagttgatg ctagctgcaa acatttactc 3120

ttgtatttat cttcactagg aaactgtgga ctgtaattta ttttattaaa tatttagaag 3180

attatttggc ttgtgtgttc aggtgagaaa tactgagttg tttttgttta atttcatggt 3240

tttttttttt ttaatgatcc ctagtggggg aaggggaaag gaatagtctg ataaacagat 3300

gtgcatattt taaaaacaag tgaccttttg ggaatgtagg catttagacg atgattttag 3360

tcgcactagg ggtgggattc aaactactgg tcaaagacca ttttgtacag aaaagggaac 3420

atctccgatg ggtgttaagg taggagtttc catgcagccc tttatgtctg agaaatagtc 3480

tcctctgcca ttggggtccc tgcggaatct tctacaggaa ttgcagctct tcacgtcatg 3540

ctaggttacc agcatgggct tcccagagca cttcactctg tttctaactc cactgacttt 3600

tctgacttgt ttcttgaggg acttggaaaa ggggaaggta ttattcacac agatgtgtgt 3660

atgaagccta aataacatcc aacttttttt ccaaaaacat agtaagagtt taaccataaa 3720

tagaagagta atattccttt aaatttctta caggcatgta aaattttatg tgtttataga 3780

gagatgctaa ctgtcagcat atagtattta tattggggca agaaggttaa aatcaaatct 3840

ttaatttaag taagcatagt tcctttaaag atcagtagta tttatactct gaaaagagta 3900

ccaagcttag ttcagttatt tattcatcca ttttttttct attgtttctc ctctgggggg 3960

aaatggtgtt ttgtttttgt ttgagttttg tgttttagtt ttttggtttt ggttttgttt 4020

tgtttttttg tcattcagat tcactaaatt tggggatggt tttattaaag tatattaact 4080

tctttttaac caaagctttc tgaatatgac cagcctcagg tgctagcgca ttaaagagca 4140

actaaaccta attccagtgt cgatttgtga aataataaaa gctaattgaa tttctctaag 4200

ggtgagagag aacataatga tgaacttaaa aagaaattcc ttttcccaga agggattctg 4260

catgtaccta caaacaatca tgctctaacc acagtaatag tcatgccatg gtgacattgc 4320

ttttacggta aaccaagata ggtaatatga tgcttcttcc caggtgtttc tgaaagaaaa 4380

gcagcggtgg agcttaagag ccaagtccac tgattgacat aataggatgg aattgtagac 4440

agagacatgc tccatgaaac aaggaaacaa ctgactacta tttggatcta agttgggatc 4500

tgatgttaaa caataaattc agtttaaaaa aaatatggag ctcagaaaag gatgtgaaaa 4560

actttgcatt ttcctttctt cattattaca aaaacaccta tgtgatgaca taaaatactt 4620

gggtgatatc agaacaatta tccttaatag tttttataat taaagtttcc taaacttcag 4680

tctccaatag tcttttaagg attggaacca catcactgtc agccccgctg ctacaatgcc 4740

tttgtacaat ttttttacat aaggcaaata aggcttttgt acaaagcctg aataccttct 4800

gttccaatgg tgtccaaatg gttattatat tgtgaaaaac ctggccttgg tcacaatgca 4860

aacaaaagta cagatgaaag tgctttttgg acagtttgca aattgtgtta aagctacgga 4920

ttttttttaa agtgttcagc atcttaacgt gtattaaagc tatggatttt tttttaaagt 4980

cttcagcatc ctaattcacc ctttctaact taagaaaaac atgacttaag acactgcttc 5040

taagtttggt tgttctttat agtgtggata cccagttatc tgtgagcgta tgggggtggg 5100

ctgagggtca ggtgaggaag gagtgtgtgt gtgtgtgtgt gtgtgtgtgt gtgtgtgtgt 5160

gatttgcatg tgtatgatgt gtgtgcgtcg gaccgcttct aggctactaa gtgtcaatgg 5220

aaaagaaaat gtattcaaaa tacttaaatc aaaactagaa gatggggaaa aaaagattta 5280

ttctatacaa agccttgtct ggaccacttt agagagactt ctattttttt aacccttcta 5340

taaatatttg atggcacttg aaatattcct gcaataaaat gtgatttgtg taaagaaaaa 5400

aagattttgt aatgtgaaac aaagaaagaa agtaatgtaa ttttctaaaa aaaaaaatac 5460

aaacaaacaa actttgtatt attttcttga tggaatttgt ctatctgtct ttggaaaact 5520

ttttatttca ttgaatgtgc catagtagaa atgtgtgttt ttagttttag actaaggaat 5580

agctagttgt tgtgttccga cattccaaaa tgcaaaacaa cctagtagta tctttcatga 5640

aaaggtttaa gtagtatgta agcttctctc attgttgctt ttttgcacat gttcttcatt 5700

cctctctagt gcaatatgta catagagcac ttgcgggtgt accttgatcc ctcagggaaa 5760

aatacatatt tgtacagttt tttggggttt ttttgttttt tggttttttt tgttttgcct 5820

tttgtttttg gctaaggaat gtcgatcgaa tcacttgtta ttgttgaggg gcagccagat 5880

aataatccta aagccactgt ttccaacatt gattgtttaa atcatatgtc cttccaatgc 5940

tattatttta agataataat aaaaagttat tttctgacag ttctttgtgc tgactggtga 6000

aaaacaaggg taaataagca ccttataatt gacttactgt gaatgacaat ccatcttggt 6060

atcaacgata gaagccctat cattttggag ttggggttaa gagtcagaaa caatgtgctc 6120

agggatctcc taaaactctt aaaacagggt ggccagtact actgggacaa attgtgtttt 6180

ttattattaa taataatgat aataatacta tccctccaag gcacaagtga actatataga 6240

actgcgtgtg tgtaaactct ttactctcgt ctcattttgt tgagtttaga acttgatgtg 6300

ctcgtcagtc ttgtgtttca aaacactgaa taacttccaa agcaaagtta tgccagtgtg 6360

ttcaaaagat aaattaataa tgtaccagca aagagcatca ttcaaagtat agtccttgca 6420

tattccagtt accatttctc taataattaa aatattcatg ataaatatat atatagcatc 6480

atatgttaaa aactatttca aattctacat attaaggatg aaaattttaa aatccagtaa 6540

taagaggaga gacctgccta tcagtacagt gatataggta aaagatgaaa tatgttttta 6600

aaataccagc aattaactat tgttttcatg ggttctcctc tagaagcaaa ccaaaaattc 6660

cttcatggaa aacaattctt acttctacat gtgtagttta tatctgatgc attacaagag 6720
ctaatgttta aagacaaaac aaaacctgcc tgtatgacag cagcaactcg agccaacatt 6780
tagtgttaca tgttatattt ttgaaatacc gttttgttat catattccac atattacttt 6840
ccataaagtc agagaagttc aatgtaattg ttggctctga ttcttccacc ttgggataca 6900
cattcacaag aagatttata tattttctta ctatgataga ggaacataat ctgggaaaac 6960
ttcccatgtc tgtaagatga aaaggatacc tttaccatgt tgtttttgat aataaagaat 7020
atggaaaatg gtagaaatct ctcctctcta tgtgtatttg tatatgtgtg tatacacaca 7080
cacgttgaca tttttacata acatgtgatt gccacttctt ataaagttat gatatagaac 7140
cccatgaaaa caactttata ttatagatca aataatgtgc ccagaaacgc aattgcaaca 7200
gtaaatcttg atctattggt aagagtctat gatacaggtc ttcattctat ccctaaacat 7260
aatgttagta aagaggttcc atcagattgt attatagaga ccttcctatt gctatttatt 7320
tttaagagat gagaagactg actagcaatg tctccacagt gcaattggtt tcacttctgg 7380
gtctgtctgt ctgtttgtgt aggtgagatc agtgttgtca ggctctcaga ataattttta 7440
aaagaatcca gaagcctgac ttcacaccaa gtagccatcc tagatgggcg ggggggatct 7500
ccatgttcaa ccaaaccctc agaacttaga gcaataaata cattcagtca tttatttata 7560
aatgaatgac agaatattca atccaaatag aaacaacctt ttgtcagtga tgcaacataa 7620
catggagtct tatattgaga aagtgaatat gaatatttta aatagatgcc taggaaatct 7680
gtgtttgctg tttacattta atgtactttg ccacattagc agtaccacac ttcttttact 7740
ttcatccttc taagaactaa tgaaagatag cttgctatta gctttgacat gttgcacatg 7800
ccattatgtt gttctctaag aacaactaaa ttctctccag tgttcatgtg tgattccttt 7860
ttcatcatta ttactttaat gtggatgaat actatttcta ggaacttgat tatcactgaa 7920
cagattgcat atgtaaatgc agataattct tgagcaatat agtgaaaatg atttcacaaa 7980
aaaaatccat atgtactgta tacatcttcc tgaatccaaa attctcctgt tatccacaca 8040
gtttgaaatc tcatattaaa cagtggtaaa tttttaaaca ttaggacatt agctggctgc 8100
ttctataaat gtacctgtat tgtctgcctg ctccttttgt ggagatgtgt ttttgtattg 8160
gatgtttggg ccaatgggag gcttcaagct acaacatatt ttcccacttt aaataatatt 8220
taacatatga caaaccccag acaaggcttt taatatgtat aaagaactgc agtgttaggt 8280
ggtttatttg caaactgttt tcagtgttgt ccatttttat ggcaccttta acaatattct 8340
tgtttccaaa gtacaaaaaa aataggttaa ataatctagc cataactgaa tgtcaagcaa 8400
taaaacaaat gacttttgtg catagactta aaaaatacaa attttagaca tctgcatgta 8460
aatgcaatct cttgtttact gctttcttca acttgagcaa tcgattcctt atttactatt 8520

aacttaagaa cttgtaacgg cctgtaaaca ttttctctct tactcttctt tcaaatttac 8580
ctgtccctgc ttttgagtca taatatttaa tgttgttctg cacatctcta tacagttaac 8640
tttttggctt tcattctgta tagataagaa aatgttatat tataaacagc ctactcagtg 8700
caaatattta tctgtttatc aaatccacaa tatgctgtat aataccggtt ttactatata 8760
atctatttta gacatagctg tttagaacta gagtgtgcta tttttgtgtt tttctgatgt 8820
gtggtgctag acaagttact tttgtgaaca acaaaaatta tcccttttat tcctagacaa 8880
taccaccttt gggtcttgtt aatttcactg agtataacta tatatttgta tatatataca 8940
tatatatata tatctaccta tgcccaactg gcagctgtat cagagtgctg gatttgggac 9000
atgcttttct ctttaaatac ataatatcat tatataaatt attctagagt gtatttaatt 9060
aggataaaat tacttcctta gtatggatat ttgacatcta tagggtgaat ttgtttataa 9120
atatggctat atggaaactt attagcattt actttatgtt tgctacttgg ctttacagca 9180
tatctcctaa gctgaaaaat aatttgccag gccttcaaga tcctaaagaa acttgtttaa 9240
tggagtaata tacttttttt tcttattaag gaattgtatt actggcacct aacacagttg 9300
tattcttagc tcctattata gataatgggc atttacataa aatatcctag atggcttgat 9360
ggcagaataa acctttcccc tcctacctga gtcatgagaa ggatggagac gtcctctgcc 9420
ataacatggg ccataaagca aattcgacat gggatgttct gtttcagtat gacctcaacc 9480
agttccatga actgagtgaa ggaccttcat tttcaaagtt atttaataag tagcttaatt 9540
aagcctttct acccattctc ccaagatcta ctggcattat tgaaaagcaa agtttatcaa 9600
atatctaact aaggatgtag ttaaccttat taaatattga ttagaattgt tctgtaatat 9660
tactgaattt gtaagatctt tagcaaagat ttttgagcaa tttataaatg tagagcaaat 9720
gtttctgttt actgcacttt ttgtaactga aggtgataaa ttctcaagcc atgattattg 9780
gcttccatgc actgcaatat ttatccacaa ttctagacat tttccatttt tgtggaagag 9840
ttgctgttac cttaattata aatgcaattg tgtggttaat gagagctaat gctagtagtt 9900
aaccttttaa agtggattgg ctacagttga gggagaaatc tcttttaata taaatcacat 9960
cattccttaa ctgcctctct tggaaagaga ttgaaacctt ttttttaaag cacgatttag 10020
catcctaagc ttcctgaggg tagagattgt atctttttgc gtctgcacaa tggctagcac 10080
atgtcagcat ttgacaattg ttaaatgata acaagtgtgc cccaattaaa acgtttttcc 10140
tgggttgttt tgttaaattt acaaagtaag ccaagcctta cggttaacat tctcctctac 10200
aaccaagtat taaagccaca tttaaaaaga ccacatgaaa tgctgattct aattgtgtgt 10260
aggtcttgag gattaagcac acaaatttca caaacttctg tttgagtaaa caaactcagc 10320
cttctgtaaa tatacatgca agtttggaaa cagtaatact gtacctataa atatatgctg 10380
tctgttttgt gtacagtatg taaaaactcc ttttctgcca cactaaaaat gcaagccatt 10440

tatgggaatc ctaaaactag tattgaacta aaactttgct aatgatcttt attagaggat 10500

cgtccaactt ttcacttacc ttgggttttc ttttcaattc actcttacac tagtctgctt 10560

atttccagct gtttatttta ttgagtcctg aatttaaaaa aaaaatattt tgattcattt 10620

tgtaaataca agctgtacaa aaaagagaga tttaatgttg tcttttaaat actccaattt 10680

tcattctaat atgaatgttg ttatattgta cttagaaact gtacctttaa tattacatta 10740

cctttattaa aagtgcattg aacacatcaa ttttagatgt gctttatgta ctgttatcct 10800

ataataaaac ttcagcttct aatggaa 10827

<210> 3
<211> 500
<212> DNA
<213> Homo sapiens

<220>
<221> misc_difference
<222> (1)..(500)
<223> V represent the SNP position

<400> 3
   aggcgcacct cacacggcac acaggcacat ctcacacatg gcacacatgc acacctcaca 60
   cagatggcac acatgcacac ctcacacaca cggcacgcat gcacacctca cacacacggc 120
   acgcatgcac acctcacaca cggcacacat gcacacctca cacacgacac acgggcacac 180
   ctcacacaca tggcacacgg gcacacctcc cacacacggc acacgggcac acctcccaca 240
   cacggcacac vggcacacct caaacgacac acggcacacc tcacacacaa gtctattcag 300
   ctgcaagtcc tgcctccact tgctgagaac ctgcatgact gggcaccaag gatacggcac 360
   acacacgcac ccaccccaca tacatacagt ccacacacac acaacacata tacaccacac 420
   gcaccacaga tgcacaccac acatgccaca cacacataca ctgcacacgc accctacaca 480
   caccccccac atgcttacac 500

<210> 4
<211> 638
<212> PRT
<213> Homo sapiens

<400> 4

<210>  5
<211>  4560
<212>  DNA
<213>  Homo sapiens

<400>  5
aactagcaat ggtggtacag tggatgaaaa gtgtttctct gttgatgaaa taggcggcgg      60

cggcggcagc ggcagcagca gctgctacag tggcggtggc ggcggcggct gctgctgagc     120

ccgggcggcg gcggggaccc cgggctgggg ccacgcgggc cggaggcccc ggcaccattg     180

gccccagcgc agacgcgaac ccgcgctctc tgatcagagg cgaagctcgg aggtcctaca     240

ggtatggatc tctggcagct gctgttgacc ttggcactgg caggatcaag tgatgctttt     300

tctggaagtg aggccacagc agctatcctt agcagagcac cctggagtct gcaaagtgtt     360

aatccaggcc taaagacaaa ttcttctaag gagcctaaat tcaccaagtg ccgttcacct     420

gagcgagaga ctttttcatg ccactggaca gatgaggttc atcatggtac aaagaaccta     480

ggacccatac agctgttcta taccagaagg aacactcaag aatggactca agaatggaaa     540

gaatgccctg attatgtttc tgctggggaa aacagctgtt actttaattc atcgtttacc     600

tccatctgga taccttattg tatcaagctt gcaaccagat ccacccattg ccctcaactg     660

gactttactg aacgtcagtt taactgggat tcatgcagat atccaagtga gatgggaagc     720

accacgcaat gcagatattc agaaaggatg gatggttctg gagtatgaac ttcaatacaa     780

agaagtaaat gaaactaaat ggaaaatgat ggaccctata ttgacaacat cagttccagt     840

gtactcattg aaagtggata aggaatatga agtgcgtgtg agatccaaac aacgaaactc     900

tggaaattat ggcgagttca gtgaggtgct ctatgtaaca cttcctcaga tgagccaatt     960

tacatgtgaa gaagatttct actttccatg gctcttaatt attatctttg gaatatttgg 1020

gctaacagtg atgctatttg tattcttatt ttctaaacag caaaggatta aaatgctgat 1080

tctgccccca gttccagttc caaagattaa aggaatcgat ccagatctcc tcaaggaagg 1140

aaaattagag gaggtgaaca caatcttagc cattcatgat agctataaac ccgaattcca 1200

cagtgatgac tcttgggttg aatttattga gctagatatt gatgagccag atgaaaagac 1260

tgaggaatca gacacagaca gacttctaag cagtgaccat gagaaatcac atagtaacct 1320

aggggtgaag gatggcgact ctggacgtac cagctgttgt gaacctgaca ttctggagac 1380

tgatttcaat gccaatgaca tacatgaggg tacctcagag gttgctcagc cacagaggtt 1440

aaaaggggaa gcagatctct tatgccttga ccagaagaat caaaataact caccttatca 1500

tgatgcttgc cctgctactc agcagcccag tgttatccaa gcagagaaaa acaaaccaca 1560

accacttcct actgaaggag ctgagtcaac tcaccaagct gcccatattc agctaagcaa 1620

tccaagttca ctgtcaaaca tcgactttta tgcccaggtg agcgacatta caccagcagg 1680

tagtgtggtc ctttccccgg gccaaaagaa taaggcaggg atgtcccaat gtgacatgca 1740

cccggaaatg gtctcactct gccaagaaaa cttccttatg gacaatgcct acttctgtga 1800

ggcagatgcc aaaaagtgca tccctgtggc tcctcacatc aaggttgaat cacacataca 1860

gccaagctta aaccaagagg acatttacat caccacagaa agccttacca ctgctgctgg 1920

gaggcctggg acaggagaac atgttccagg ttctgagatg cctgtcccag actatacctc 1980

cattcatata gtacagtccc cacagggcct catactcaat gcgactgcct tgcccttgcc 2040

tgacaaagag tttctctcat catgtggcta tgtgagcaca gaccaactga acaaaatcat 2100

gccttagcct ttctttggtt tcccaagagc tacgtattta atagcaaaga attgactggg 2160

gcaataacgt ttaagccaaa acaatgttta aacctttttt gggggagtga caggatgggg 2220

tatggattct aaaatgcctt ttcccaaaat gttgaaatat gatgttaaaa aaataagaag 2280

aatgcttaat cagatagata ttcctattgt gcaatgtaaa tattttaaag aattgtgtca 2340

gactgtttag tagcagtgat tgtcttaata ttgtgggtgt taatttttga tactaagcat 2400

tgaatggcta tgtttttaat gtatagtaaa tcacgctttt tgaaaaagcg aaaaaatcag 2460

gtggcttttg cggttcagga aaattgaatg caaaccatag cacaggctaa ttttttgttg 2520

tttcttaaat aagaaacttt tttatttaaa aaactaaaaa ctagaggtga gaaatttaaa 2580

ctataagcaa gaaggcaaaa atagtttgga tatgtaaaac atttattttg acataaagtt 2640

gataaagatt ttttaataat ttagacttca agcatggcta ttttatatta cactacacac 2700

tgtgtactgc agttggtatg acccctctaa ggagtgtagc aactacagtc taaagctggt 2760

ttaatgtttt ggccaatgca cctaaagaaa aacaaactcg ttttttacaa agccctttta 2820

tacctcccca gactccttca acaattctaa aatgattgta gtaatctgca ttattggaat 2880

ataattgttt tatctgaatt tttaaacaag tatttgttaa tttagaaaac tttaaagcgt 2940

ttgcacagat caacttacca ggcaccaaaa gaagtaaaag caaaaaagaa aacctttctt 3000

caccaaatct tggttgatgc caaaaaaaaa tacatgctaa gagaagtaga aatcatagct 3060

ggttcacact gaccaagata cttaagtgct gcaattgcac gcggagtgag ttttttagtg 3120

cgtgcagatg gtgagagata agatctatag cctctgcagc ggaatctgtt cacacccaac 3180

ttggttttgc tacataatta tccaggaagg gaataaggta caagaagcat tttgtaagtt 3240

gaagcaaatc gaatgaaatt aactgggtaa tgaaacaaag agttcaagaa ataagttttt 3300

gtttcacagc ctataaccag acacatactc atttttcatg ataatgaaca gaacatagac 3360

agaagaaaca aggttttcag tccccacaga taactgaaaa ttatttaaac cgctaaaaga 3420

aactttcttt ctcactaaat cttttatagg atttatttaa aatagcaaaa gaagaagttt 3480

catcattttt tacttcctct ctgagtggac tggcctcaaa gcaagcattc agaagaaaaa 3540

gaagcaacct cagtaattta gaaatcattt tgcaatccct taatatccta aacatcattc 3600

atttttgttg ttgttgttgt tgttgagaca gagtctcgct ctgtcgccag gctagagtgc 3660

ggtggcgcga tcttgactca ctgcaatctc cacctcccac aggttcaggc gattcccgtg 3720

cctcagcctc ctgagtagct gggactacag gcacgcacca ccatgccagg ctaatttttt 3780

tgtattttag cagagacggg gtttcaccat gttggccagg atggtctcga tctcctgacc 3840

tcgtgatcca cccgactcgg cctcccaaag tgctgggatt acaggtgtaa gccaccgtgc 3900

ccagccctaa acatcattct tgagagcatt gggatatctc ctgaaaaggt ttatgaaaaa 3960

gaagaatctc atctcagtga agaatacttc tcatttttta aaaaagctta aaactttgaa 4020

gttagcttta acttaaatag tatttcccat ttatcgcaga ccttttttag gaagcaagct 4080

taatggctga taattttaaa ttctctctct tgcaggaagg actatgaaaa gctagaattg 4140

agtgtttaaa gttcaacatg ttatttgtaa tagatgtttg atagattttc tgctactttg 4200

ctgctatggt tttctccaag agctacataa tttagtttca tataaagtat catcagtgta 4260

gaacctaatt caattcaaag ctgtgtgttt ggaagactat cttactattt cacaacagcc 4320

tgacaacatt tctatagcca aaaatagcta aatacctcaa tcagtctcag aatgtcattt 4380

tggtactttg gtggccacat aagccattat tcactagtat gactagttgt gtctggcagt 4440

ttatatttaa ctctctttat gtctgtggat tttttccttc aaagtttaat aaatttattt 4500

tcttggattc ctgatagtgt gcttctgtta tcaaacacca acataaaaat gatctaaacc 4560

<210> 6
<211> 233
<212> PRT
<213> Homo sapiens

<400>  6

Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210>  7

<211>   1678
<212>   DNA
<213>   Homo sapiens

<400>   7
agcagacgct ccctcagcaa ggacagcaga ggaccagcta agagggagag aagcaactac      60

agaccccccc tgaaaacaac cctcagacgc cacatcccct gacaagctgc caggcaggtt     120

ctcttcctct cacatactga cccacggctc caccctctct cccctggaaa ggacaccatg     180

agcactgaaa gcatgatccg ggacgtggag ctggccgagg aggcgctccc caagaagaca     240

ggggggcccc agggctccag gcggtgcttg ttcctcagcc tcttctcctt cctgatcgtg     300

gcaggcgcca ccacgctctt ctgcctgctg cactttggag tgatcggccc ccagagggaa     360

gagttcccca gggacctctc tctaatcagc cctctggccc aggcagtcag atcatcttct     420

cgaaccccga gtgacaagcc tgtagcccat gttgtagcaa accctcaagc tgaggggcag     480

ctccagtggc tgaaccgccg ggccaatgcc ctcctggcca atggcgtgga gctgagagat     540

aaccagctgg tggtgccatc agagggcctg tacctcatct actcccaggt cctcttcaag     600

ggccaaggct gcccctccac ccatgtgctc ctcacccaca ccatcagccg catcgccgtc     660

tcctaccaga ccaaggtcaa cctcctctct gccatcaaga gcccctgcca gagggagacc     720

ccagaggggg ctgaggccaa gccctggtat gagcccatct atctgggagg ggtcttccag     780

ctggagaagg gtgaccgact cagcgctgag atcaatcggc ccgactatct cgactttgcc     840

gagtctgggc aggtctactt tgggatcatt gccctgtgag gaggacgaac atccaacctt     900

cccaaacgcc tcccctgccc caatcccttt attaccccct ccttcagaca ccctcaacct     960

cttctggctc aaaaagagaa ttgggggctt agggtcggaa cccaagctta gaactttaag    1020

caacaagacc accacttcga aacctgggat tcaggaatgt gtggcctgca cagtgaagtg    1080

ctggcaacca ctaagaattc aaactggggc ctccagaact cactggggcc tacagctttg    1140

atccctgaca tctggaatct ggagaccagg gagcctttgg ttctggccag aatgctgcag    1200

gacttgagaa gacctcacct agaaattgac acaagtggac cttaggcctt cctctctcca    1260

gatgtttcca gacttccttg agacacggag cccagccctc cccatggagc cagctccctc    1320

tatttatgtt tgcacttgtg attatttatt atttatttat tatttattta tttacagatg    1380

aatgtattta tttgggagac cggggtatcc tgggggaccc aatgtaggag ctgccttggc    1440

tcagacatgt tttccgtgaa aacggagctg aacaataggc tgttcccatg tagccccctg    1500

gcctctgtgc cttcttttga ttatgttttt taaaatattt atctgattaa gttgtctaaa    1560

caatgctgat ttggtgacca actgtcactc attgctgagc ctctgctccc caggggagtt    1620

gtgtctgtaa tcgccctact attcagtggc gagaaataaa gtttgcttag aaaagaaa      1678

<210>   8

<211>  222
<212>  PRT
<213>  Homo sapiens

<400>  8

Met Ala Glu Lys Pro Lys Leu His Tyr Phe Asn Ala Arg Gly Arg Met
1               5                   10                  15

Glu Ser Thr Arg Trp Leu Leu Ala Ala Ala Gly Val Glu Phe Glu Glu
            20                  25                  30

Lys Phe Ile Lys Ser Ala Glu Asp Leu Asp Lys Leu Arg Asn Asp Gly
        35                  40                  45

Tyr Leu Met Phe Gln Gln Val Pro Met Val Glu Ile Asp Gly Met Lys
    50                  55                  60

Leu Val Gln Thr Arg Ala Ile Leu Asn Tyr Ile Ala Ser Lys Tyr Asn
65                  70                  75                  80

Leu Tyr Gly Lys Asp Ile Lys Glu Arg Ala Leu Ile Asp Met Tyr Ile
                85                  90                  95

Glu Gly Ile Ala Asp Leu Gly Glu Met Ile Leu Leu Leu Pro Val Cys
            100                 105                 110

Pro Pro Glu Glu Lys Asp Ala Lys Leu Ala Leu Ile Lys Glu Lys Ile
        115                 120                 125

Lys Asn Arg Tyr Phe Pro Ala Phe Glu Lys Val Leu Lys Ser His Gly
    130                 135                 140

Gln Asp Tyr Leu Val Gly Asn Lys Leu Ser Arg Ala Asp Ile His Leu
145                 150                 155                 160

Val Glu Leu Leu Tyr Tyr Val Glu Glu Leu Asp Ser Ser Leu Ile Ser
                165                 170                 175

Ser Phe Pro Leu Leu Lys Ala Leu Lys Thr Arg Ile Ser Asn Leu Pro
            180                 185                 190

Thr Val Lys Lys Phe Leu Gln Pro Gly Ser Pro Arg Lys Pro Pro Met
        195                 200                 205

Asp Glu Lys Ser Leu Glu Glu Ala Arg Lys Ile Phe Arg Phe
    210                 215                 220

<210>  9

<211>  1218
<212>  DNA
<213>  Homo sapiens

<400>  9
gtcgagccag gacggtgaca gcgtttaaca aagcttagag aaacctccag gagactgcta      60

tcatggcaga gaagcccaag ctccactact tcaatgcacg gggcagaatg gagtccaccc     120

ggtggctcct ggctgcagct ggagtagagt ttgaagagaa atttataaaa tctgcagaag     180

atttggacaa gttaagaaat gatggatatt tgatgttcca gcaagtgcca atggttgaga     240

ttgatgggat gaagctggtg cagaccagag ccattctcaa ctacattgcc agcaaataca     300

acctctatgg gaaagacata aaggagagag ccctgattga tatgtatata gaaggtatag     360

cagatttggg tgaaatgatc ctccttctgc ccgtatgtcc acctgaggaa aaagatgcca     420

agcttgcctt gatcaaagag aaaataaaaa atcgctactt ccctgccttt gaaaaagtct     480

taaagagcca tggacaagac taccttgttg gcaacaagct gagccgggct gacattcatc     540

tggtggaact tctctactac gtcgaggagc ttgactccag tcttatctcc agcttccctc     600

tgctgaaggc cctgaaaacc agaatcagca acctgcccac agtgaagaag tttctacagc     660

ctggcagccc aaggaagcct cccatggatg agaaatcttt agaagaagca aggaagattt     720

tcaggtttta ataacgcagt catggaggcc aagaacttgc aataccaatg ttctaaagtt     780

ttgcaacaat aaagtacttt acctaagtgt tgattgtgcc tgttgtgaag ctaatgaact     840

ctttcaaatt atatgctaat taaataatac aactcctatt cgctgactta gttaaaattg     900

atttgttttc attaggatct gatgtgaatt cagatttcca atcttctcct agccaaccat     960

tttcctggaa ttaaaaattc agtaaaaaag gaaactatag attatgtggt ttgtttgact    1020

tttccaagaa ttgtcccgta acatacaatt tgtcatacaa tctattaaaa tgtcaatgta    1080

gaaatgcact tctgacattt tcaggtatgc acaggagaag agttaccatc ctggataatg    1140

gcataaagac attttcttct tttcctggac agtcatttta tttctgataa aagcgttctt    1200

tcttatgcat ttgcaaaa                                                  1218

<210>  10
<211>  134
<212>  PRT
<213>  Homo sapiens

<400>  10

Met Asp Pro Gln Thr Ala Pro Ser Arg Ala Leu Leu Leu Leu Leu Phe
1               5                   10                  15

Leu His Leu Ala Phe Leu Gly Gly Arg Ser His Pro Leu Gly Ser Pro
            20                  25                  30

Gly Ser Ala Ser Asp Leu Glu Thr Ser Gly Leu Gln Glu Gln Arg Asn
        35                  40                  45

His Leu Gln Gly Lys Leu Ser Glu Leu Gln Val Glu Gln Thr Ser Leu
    50                  55                  60

Glu Pro Leu Gln Glu Ser Pro Arg Pro Thr Gly Val Trp Lys Ser Arg
65                  70                  75                  80

Glu Val Ala Thr Glu Gly Ile Arg Gly His Arg Lys Met Val Leu Tyr
                85                  90                  95

Thr Leu Arg Ala Pro Arg Ser Pro Lys Met Val Gln Gly Ser Gly Cys
            100                 105                 110

Phe Gly Arg Lys Met Asp Arg Ile Ser Ser Ser Ser Gly Leu Gly Cys
        115                 120                 125

Lys Val Leu Arg Arg His
    130

<210>  11
<211>  708
<212>  DNA
<213>  Homo sapiens

<400>  11
aggaggagca ccccgcaggc tgagggcagg tgggaagcaa acccggacgc atcgcagcag      60

cagcagcagc agcagaagca gcagcagcag cctccgcagt ccctccagag acatggatcc     120

ccagacagca ccttcccggg cgctcctgct cctgctcttc ttgcatctgg ctttcctggg     180

aggtcgttcc cacccgctgg gcagccccgg ttcagcctcg gacttggaaa cgtccgggtt     240

acaggagcag cgcaaccatt tgcagggcaa actgtcggag ctgcaggtgg agcagacatc     300

cctggagccc ctccaggaga gcccccgtcc cacaggtgtc tggaagtccc gggaggtagc     360

caccgagggc atccgtgggc accgcaaaat ggtcctctac accctgcggg caccacgaag     420

ccccaagatg gtgcaagggt ctggctgctt tgggaggaag atggaccgga tcagctcctc     480

cagtggcctg ggctgcaaag tgctgaggcg gcattaagag gaagtcctgg ctgcagacac     540

ctgcttctga ttccacaagg ggctttttcc tcaaccctgt ggccgccttt gaagtgactc     600

atttttttaa tgtatttatg tatttatttg attgttttat ataagatggt ttcttacctt     660

tgagcacaaa atttccacgg tgaaataaag tcaacattat aagcttta                 708

<210>  12
<211>  2201
<212>  PRT
<213>  Homo sapiens

<400>  12

<210> 13
<211> 8500
<212> DNA
<213> Homo sapiens

<400> 13
ggccacagcc tgcctactgt cacccgcctc tcccgcgcgc agatacacgc ccccgcctcc      60

gtgggcacaa aggcagcgct gctggggaac tcgggggaac gcgcacgtgg gaaccgccgc     120

agctccacac tccaggtact tcttccaagg acctaggtct ctcgcccatc ggaaagaaaa     180

taattctttc aagaagatca gggacaactg atttgaagtc tactctgtgc ttctaaatcc     240

ccaattctgc tgaaagtgag ataccctaga gccctagagc cccagcagca cccagccaaa     300

cccacctcca ccatgggggc catgactcag ctgttggcag gtgtctttct tgctttcctt     360

gccctcgcta ccgaaggtgg ggtcctcaag aaagtcatcc ggcacaagcg acagagtggg     420

gtgaacgcca ccctgccaga agagaaccag ccagtggtgt ttaaccacgt ttacaacatc     480

aagctgccag tgggatccca gtgttcggtg gatctggagt cagccagtgg ggagaaagac     540

ctggcaccgc cttcagagcc cagcgaaagc tttcaggagc acacagtgga tggggaaaac     600

cagattgtct tcacacatcg catcaacatc ccccgccggg cctgtggctg tgccgcagcc     660

cctgatgtta aggagctgct gagcagactg gaggagctgg agaacctggt gtcttccctg     720

agggagcaat gtactgcagg agcaggctgc tgtctccagc ctgccacagg ccgcttggac     780

accaggccct tctgtagcgg tcggggcaac ttcagcactg aaggatgtgg ctgtgtctgc     840

gaacctggct ggaaaggccc caactgctct gagcccgaat gtccaggcaa ctgtcacctt     900

cgaggccggt gcattgatgg gcagtgcatc tgtgacgacg gcttcacggg cgaggactgc     960

agccagctgg cttgccccag cgactgcaat gaccagggca agtgcgtaaa tggagtctgc    1020

atctgtttcg aaggctacgc cggggctgac tgcagccgtg aaatctgccc agtgccctgc    1080

agtgaggagc acggcacatg tgtagatggc ttgtgtgtgt gccacgatgg ctttgcaggc    1140

gatgactgca acaagcctct gtgtctcaac aattgctaca accgtggacg atgcgtggag    1200

aatgagtgcg tgtgtgatga gggtttcacg ggcgaagact gcagtgagct catctgcccc    1260

aatgactgct tcgaccgggg ccgctgcatc aatggcacct gctactgcga agaaggcttc    1320

acaggtgaag actgcgggaa acccacctgc ccacatgcct gccacaccca gggccggtgt    1380

gaggaggggc agtgtgtatg tgatgagggc tttgccggtg tggactgcag cgagaagagg    1440

tgtcctgctg actgtcacaa tcgtggccgc tgtgtagacg ggcggtgtga gtgtgatgat    1500

ggtttcactg gagctgactg tggggagctc aagtgtccca atggctgcag tggccatggc 1560

cgctgtgtca atgggcagtg tgtgtgtgat gagggctata ctggggagga ctgcagccag 1620

ctacggtgcc ccaatgactg tcacagtcgg ggccgctgtg tcgagggcaa atgtgtatgt 1680

gagcaaggct tcaagggcta tgactgcagt gacatgagct gccctaatga ctgtcaccag 1740

cacggccgct gtgtgaatgg catgtgtgtt tgtgatgacg gctacacagg ggaagactgc 1800

cgggatcgcc aatgccccag ggactgcagc aacaggggcc tctgtgtgga cggacagtgc 1860

gtctgtgagg acggcttcac cggccctgac tgtgcagaac tctcctgtcc aaatgactgc 1920

catggccagg gtcgctgtgt gaatgggcag tgcgtgtgcc atgaaggatt tatgggcaaa 1980

gactgcaagg agcaaagatg tcccagtgac tgtcatggcc agggccgctg cgtggacggc 2040

cagtgcatct gccacgaggg cttcacaggc ctggactgtg gccagcactc ctgccccagt 2100

gactgcaaca acttaggaca atgcgtctcg ggccgctgca tctgcaacga gggctacagc 2160

ggagaagact gctcagaggt gtctcctccc aaagacctcg ttgtgacaga agtgacggaa 2220

gagacggtca acctggcctg ggacaatgag atgcgggtca cagagtacct tgtcgtgtac 2280

acgcccaccc acgagggtgg tctggaaatg cagttccgtg tgcctgggga ccagacgtcc 2340

accatcatcc aggagctgga gcctggtgtg gagtacttta tccgtgtatt tgccatcctg 2400

gagaacaaga agagcattcc tgtcagcgcc agggtggcca cgtacttacc tgcacctgaa 2460

ggcctgaaat tcaagtccat caaggagaca tctgtggaag tggagtggga tcctctagac 2520

attgcttttg aaacctggga gatcatcttc cggaatatga ataaagaaga tgagggagag 2580

atcaccaaaa gcctgaggag gccagagacc tcttaccggc aaactggtct agctcctggg 2640

caagagtatg agatatctct gcacatagtg aaaaacaata cccggggccc tggcctgaag 2700

agggtgacca ccacacgctt ggatgccccc agccagatcg aggtgaaaga tgtcacagac 2760

accactgcct tgatcacctg gttcaagccc ctggctgaga tcgatggcat tgagctgacc 2820

tacggcatca aagacgtgcc aggagaccgt accaccatcg atctcacaga ggacgagaac 2880

cagtactcca tcgggaacct gaagcctgac actgagtacg aggtgtccct catctcccgc 2940

agaggtgaca tgtcaagcaa cccagccaaa gagaccttca caacaggcct cgatgctccc 3000

aggaatcttc gacgtgtttc ccagacagat aacagcatca ccctggaatg gaggaatggc 3060

aaggcagcta ttgacagtta cagaattaag tatgccccca tctctggagg ggaccacgct 3120

gaggttgatg ttccaaagag ccaacaagcc acaaccaaaa ccacactcac aggtctgagg 3180

ccgggaactg aatatgggat tggagtttct gctgtgaagg aagacaagga gagcaatcca 3240

gcgaccatca acgcagccac agagttggac acgcccaagg accttcaggt ttctgaaact 3300

gcagagacca gcctgaccct gctctggaag acaccgttgg ccaaatttga ccgctaccgc 3360

ctcaattaca gtctccccac aggccagtgg gtgggagtgc agcttccaag aaacaccact 3420

tcctatgtcc tgagaggcct ggaaccagga caggagtaca atgtcctcct gacagccgag 3480

aaaggcagac acaagagcaa gcccgcacgt gtgaaggcat ccactgaaca agcccctgag 3540

ctggaaaacc tcaccgtgac tgaggttggc tgggatggcc tcagactcaa ctggaccgca 3600

gctgaccagg cctatgagca ctttatcatt caggtgcagg aggccaacaa ggtggaggca 3660

gctcggaacc tcaccgtgcc tggcagcctt cgggctgtgg acataccggg cctcaaggct 3720

gctacgcctt atacagtctc catctatggg gtgatccagg gctatagaac accagtgctc 3780

tctgctgagg cctccacagg ggaaactccc aatttgggag aggtcgtggt ggccgaggtg 3840

ggctgggatg ccctcaaact caactggact gctccagaag gggcctatga gtactttttc 3900

attcaggtgc aggaggctga cacagtagag gcagcccaga acctcaccgt cccaggagga 3960

ctgaggtcca cagacctgcc tgggctcaaa gcagccactc attataccat caccatccgc 4020

ggggtcactc aggacttcag cacaacccct ctctctgttg aagtcttgac agaggaggtt 4080

ccagatatgg gaaacctcac agtgaccgag gttagctggg atgctctcag actgaactgg 4140

accacgccag atggaaccta tgaccagttt actattcagg tccaggaggc tgaccaggtg 4200

gaagaggctc acaatctcac ggttcctggc agcctgcgtt ccatggaaat cccaggcctc 4260

agggctggca ctccttacac agtcaccctg cacggcgagg tcaggggcca cagcactcga 4320

ccccttgctg tagaggtcgt cacagaggat ctcccacagc tgggagattt agccgtgtct 4380

gaggttggct gggatggcct cagactcaac tggaccgcag ctgacaatgc ctatgagcac 4440

tttgtcattc aggtgcagga ggtcaacaaa gtggaggcag cccagaacct cacgttgcct 4500

ggcagcctca gggctgtgga catcccgggc ctcgaggctg ccacgcctta tagagtctcc 4560

atctatgggg tgatccgggg ctatagaaca ccagtactct ctgctgaggc ctccacagcc 4620

aaagaacctg aaattggaaa cttaaatgtt tctgacataa ctcccgagag cttcaatctc 4680

tcctggatgg ctaccgatgg gatcttcgag acctttacca ttgaaattat tgattccaat 4740

aggttgctgg agactgtgga atataatatc tctggtgctg aacgaactgc ccatatctca 4800

gggctacccc ctagtactga ttttattgtc tacctctctg gacttgctcc cagcatccgg 4860

accaaaacca tcagtgccac agccacgaca gaggccctgc cccttctgga aaacctaacc 4920

atttccgaca ttaatcccta cgggttcaca gtttcctgga tggcatcgga gaatgccttt 4980

gacagctttc tagtaacggt ggtggattct gggaagctgc tggaccccca ggaattcaca 5040

ctttcaggaa cccagaggaa gctggagctt agaggcctca taactggcat tggctatgag 5100

gttatggtct ctggcttcac ccaagggcat caaaccaagc ccttgagggc tgagattgtt 5160

acagaagccg aaccggaagt tgacaacctt ctggtttcag atgccacccc agacggtttc 5220

cgtctgtcct ggacagctga tgaaggggtc ttcgacaatt ttgttctcaa aatcagagat 5280

accaaaaagc agtctgagcc actggaaata accctacttg cccccgaacg taccagggac 5340

ataacaggtc tcagagaggc tactgaatac gaaattgaac tctatggaat aagcaaagga 5400

aggcgatccc agacagtcag tgctatagca acaacagcca tgggctcccc aaaggaagtc 5460

attttctcag acatcactga aaattcggct actgtcagct ggagggcacc cacagcccaa 5520

gtggagagct tccggattac ctatgtgccc attacaggag gtacaccctc catggtaact 5580

gtggacggaa ccaagactca gaccaggctg gtgaaactca tacctggcgt ggagtacctt 5640

gtcagcatca tcgccatgaa gggctttgag gaaagtgaac ctgtctcagg gtcattcacc 5700

acagctctgg atggcccatc tggcctggtg acagccaaca tcactgactc agaagccttg 5760

gccaggtggc agccagccat tgccactgtg gacagttatg tcatctccta cacaggcgag 5820

aaagtgccag aaattacacg cacggtgtcc gggaacacag tggagtatgc tctgaccgac 5880

ctcgagcctg ccacggaata cacactgaga atctttgcag agaaagggcc ccagaagagc 5940

tcaaccatca ctgccaagtt cacaacagac ctcgattctc caagagactt gactgctact 6000

gaggttcagt cggaaactgc cctccttacc tggcgacccc cccgggcatc agtcaccggt 6060

tacctgctgg tctatgaatc agtggatggc acagtcaagg aagtcattgt gggtccagat 6120

accacctcct acagcctggc agacctgagc ccatccaccc actacacagc caagatccag 6180

gcactcaatg ggcccctgag gagcaatatg atccagacca tcttcaccac aattggactc 6240

ctgtacccct tccccaagga ctgctcccaa gcaatgctga atggagacac gacctctggc 6300

ctctacacca tttatctgaa tggtgataag gctgaggcgc tggaagtctt ctgtgacatg 6360

acctctgatg gggtggatgg gattgtgttc ctgagacgca aaaacggacg cgagaacttc 6420

taccaaaact ggaaggcata tgctgctgga tttggggacc gcagagaaga attctggctt 6480

gggctggaca acctgaacaa aatcacagcc caggggcagt acgagctccg ggtggacctg 6540

cgggaccatg gggagacagc ctttgctgtc tatgacaagt tcagcgtggg agatgccaag 6600

actcgctaca agctgaaggt ggaggggtac agtgggacag caggtgactc catggcctac 6660

cacaatggca gatccttctc cacctttgac aaggacacag attcagccat caccaactgt 6720

gctctgtcct acaaaggggc tttctggtac aggaactgtc accgtgtcaa cctgatgggg 6780

agatatgggg acaataacca cagtcagggc gttaactggt tccactggaa gggccacgaa 6840

cactcaatcc agtttgctga gatgaagctg agaccaagca acttcagaaa tcttgaaggc 6900

aggcgcaaac gggcataaat tccagggacc actgggtgag agaggaataa ggcccagagc 6960

gaggaaagga ttttaccaaa gcatcaatac aaccagccca accatcggtc cacacctggg 7020

catttggtga gagtcaaagc tgaccatgga tccctggggc caacggcaac agcatgggcc 7080

tcacctcctc tgtgatttct ttctttgcac caaagacatc agtctccaac atgtttctgt 7140

tttgttgttt gattcagcaa aaatctccca gtgacaacat cgcaatagtt ttttacttct 7200

cttaggtggc tctgggaatg ggagaggggt aggatgtaca ggggtagttt gttttagaac 7260

cagccgtatt ttacatgaag ctgtataatt aattgtcatt atttttgtta gcaaagatta 7320

aatgtgtcat tggaagccat cccttttttt acatttcata caacagaaac cagaaaagca 7380

atactgtttc cattttaagg atatgattaa tattattaat ataataatga tgatgatgat 7440

gatgaaaact aaggattttt caagagatct ttctttccaa aacatttctg gacagtacct 7500

gattgtattt tttttttaaa taaaagcaca agtacttttg agtttgttat tttgctttga 7560

attgttgagt ctgaatttca ccaaagccaa tcatttgaac aaagcgggga atgttgggat 7620

aggaaaggta agtagggata gtggtcaagt gggaggggtg gaaaggagac taaagactgg 7680

gagagaggga agcacttttt ttaaataaag ttgaacacac ttgggaaaag cttacaggcc 7740

aggcctgtaa tcccaacact ttgggaggcc aaggtgggag gatagcttaa ccccaggagt 7800

ttgagaccag cctgagcaac atagtgagaa cttgtctcta cagaaaaaaa aaaaaaaaaa 7860

aatttaatta ggcaagcgtg gtagtgcgca cctgtcgtcc cagctactca ggaggctgag 7920

gtaggaaaat cactggagcc caggagttag aggttacagt gagctatgat cacactactg 7980

cactccagcc tgggcaacag agggagaccc tgtctctaaa taaaaaaaga aaagaaaaaa 8040

aaagcttaca acttgagatt cagcatcttg ctcagtattt ccaagactaa tagattatgg 8100

tttaaaagat gcttttatac tcattttcta atgcaactcc tagaaactct atgatatagt 8160

tgaggtaagt attgttacca cacatgggct aagatcccca gaggcagact gcctgagttc 8220

aattcttggc tccaccattc ccaagttccc taacctctct atgcctcagt ttcctcttct 8280

gtaaagtagg gacactcata cttctcattt cagaacattt ttgtgaagaa taaattatgt 8340

tatccatttg aggcccttag aatggtaccc ggtgtatatt aagtgctagt acatgttagc 8400

tatcatcatt atcactttat atgagatgga ctggggttca tagaaaccca atgacttgat 8460

tgtggctact actcaataaa taatagaatt tggatttaaa 8500

<210> 14
<211> 1474
<212> PRT
<213> Homo sapiens

<400> 14

Met Gly Lys Asn Lys Leu Leu His Pro Ser Leu Val Leu Leu Leu Leu
1               5                   10                  15

Val Leu Leu Pro Thr Asp Ala Ser Val Ser Gly Lys Pro Gln Tyr Met
            20                  25                  30

Val Leu Val Pro Ser Leu Leu His Thr Glu Thr Thr Glu Lys Gly Cys

35                  40                  45

Val Leu Leu Ser Tyr Leu Asn Glu Thr Val Thr Val Ser Ala Ser Leu
    50                  55                  60

Glu Ser Val Arg Gly Asn Arg Ser Leu Phe Thr Asp Leu Glu Ala Glu
65                  70                  75                  80

Asn Asp Val Leu His Cys Val Ala Phe Ala Val Pro Lys Ser Ser Ser
                85                  90                  95

Asn Glu Glu Val Met Phe Leu Thr Val Gln Val Lys Gly Pro Thr Gln
            100                 105                 110

Glu Phe Lys Lys Arg Thr Thr Val Met Val Lys Asn Glu Asp Ser Leu
        115                 120                 125

Val Phe Val Gln Thr Asp Lys Ser Ile Tyr Lys Pro Gly Gln Thr Val
    130                 135                 140

Lys Phe Arg Val Val Ser Met Asp Glu Asn Phe His Pro Leu Asn Glu
145                 150                 155                 160

Leu Ile Pro Leu Val Tyr Ile Gln Asp Pro Lys Gly Asn Arg Ile Ala
                165                 170                 175

Gln Trp Gln Ser Phe Gln Leu Glu Gly Gly Leu Lys Gln Phe Ser Phe
            180                 185                 190

Pro Leu Ser Ser Glu Pro Phe Gln Gly Ser Tyr Lys Val Val Val Gln
        195                 200                 205

Lys Lys Ser Gly Gly Arg Thr Glu His Pro Phe Thr Val Glu Glu Phe
    210                 215                 220

Val Leu Pro Lys Phe Glu Val Gln Val Thr Val Pro Lys Ile Ile Thr
225                 230                 235                 240

Ile Leu Glu Glu Glu Met Asn Val Ser Val Cys Gly Leu Tyr Thr Tyr
                245                 250                 255

Gly Lys Pro Val Pro Gly His Val Thr Val Ser Ile Cys Arg Lys Tyr
            260                 265                 270

Ser Asp Ala Ser Asp Cys His Gly Glu Asp Ser Gln Ala Phe Cys Glu
        275                 280                 285

Lys Phe Ser Gly Gln Leu Asn Ser His Gly Cys Phe Tyr Gln Gln Val
    290                 295                 300

Lys Thr Lys Val Phe Gln Leu Lys Arg Lys Glu Tyr Glu Met Lys Leu
305                 310                 315                 320

His Thr Glu Ala Gln Ile Gln Glu Glu Gly Thr Val Val Glu Leu Thr
                325                 330                 335

Gly Arg Gln Ser Ser Glu Ile Thr Arg Thr Ile Thr Lys Leu Ser Phe
            340                 345                 350

Val Lys Val Asp Ser His Phe Arg Gln Gly Ile Pro Phe Phe Gly Gln
        355                 360                 365

Val Arg Leu Val Asp Gly Lys Gly Val Pro Ile Pro Asn Lys Val Ile
    370                 375                 380

Phe Ile Arg Gly Asn Glu Ala Asn Tyr Tyr Ser Asn Ala Thr Thr Asp
385                 390                 395                 400

Glu His Gly Leu Val Gln Phe Ser Ile Asn Thr Thr Asn Val Met Gly
                405                 410                 415

Thr Ser Leu Thr Val Arg Val Asn Tyr Lys Asp Arg Ser Pro Cys Tyr
            420                 425                 430

Gly Tyr Gln Trp Val Ser Glu Glu His Glu Glu Ala His His Thr Ala
        435                 440                 445

Tyr Leu Val Phe Ser Pro Ser Lys Ser Phe Val His Leu Glu Pro Met
    450                 455                 460

Ser His Glu Leu Pro Cys Gly His Thr Gln Thr Val Gln Ala His Tyr
465                 470                 475                 480

Ile Leu Asn Gly Gly Thr Leu Leu Gly Leu Lys Lys Leu Ser Phe Tyr
                485                 490                 495

Tyr Leu Ile Met Ala Lys Gly Gly Ile Val Arg Thr Gly Thr His Gly
            500                 505                 510

Leu Leu Val Lys Gln Glu Asp Met Lys Gly His Phe Ser Ile Ser Ile
        515                 520                 525

Pro Val Lys Ser Asp Ile Ala Pro Val Ala Arg Leu Leu Ile Tyr Ala
    530                 535                 540

Val Leu Pro Thr Gly Asp Val Ile Gly Asp Ser Ala Lys Tyr Asp Val
545                 550                 555                 560

Glu Asn Cys Leu Ala Asn Lys Val Asp Leu Ser Phe Ser Pro Ser Gln
                565                 570                 575

Ser Leu Pro Ala Ser His Ala His Leu Arg Val Thr Ala Ala Pro Gln
            580                 585                 590

Ser Val Cys Ala Leu Arg Ala Val Asp Gln Ser Val Leu Leu Met Lys
        595                 600                 605

Pro Asp Ala Glu Leu Ser Ala Ser Ser Val Tyr Asn Leu Leu Pro Glu
    610                 615                 620

Lys Asp Leu Thr Gly Phe Pro Gly Pro Leu Asn Asp Gln Asp Asn Glu
625                 630                 635                 640

Asp Cys Ile Asn Arg His Asn Val Tyr Ile Asn Gly Ile Thr Tyr Thr
                645                 650                 655

Pro Val Ser Ser Thr Asn Glu Lys Asp Met Tyr Ser Phe Leu Glu Asp
            660                 665                 670

Met Gly Leu Lys Ala Phe Thr Asn Ser Lys Ile Arg Lys Pro Lys Met
        675                 680                 685

Cys Pro Gln Leu Gln Gln Tyr Glu Met His Gly Pro Glu Gly Leu Arg
    690                 695                 700

Val Gly Phe Tyr Glu Ser Asp Val Met Gly Arg Gly His Ala Arg Leu
705                 710                 715                 720

Val His Val Glu Glu Pro His Thr Glu Thr Val Arg Lys Tyr Phe Pro
                725                 730                 735

Glu Thr Trp Ile Trp Asp Leu Val Val Val Asn Ser Ala Gly Val Ala
            740                 745                 750

Glu Val Gly Val Thr Val Pro Asp Thr Ile Thr Glu Trp Lys Ala Gly
        755                 760                 765

Ala Phe Cys Leu Ser Glu Asp Ala Gly Leu Gly Ile Ser Ser Thr Ala
    770                 775                 780

Ser Leu Arg Ala Phe Gln Pro Phe Phe Val Glu Leu Thr Met Pro Tyr
785                 790                 795                 800

Ser Val Ile Arg Gly Glu Ala Phe Thr Leu Lys Ala Thr Val Leu Asn
                805                 810                 815

Tyr Leu Pro Lys Cys Ile Arg Val Ser Val Gln Leu Glu Ala Ser Pro
            820                 825                 830

Ala Phe Leu Ala Val Pro Val Glu Lys Glu Gln Ala Pro His Cys Ile
        835                 840                 845

Cys Ala Asn Gly Arg Gln Thr Val Ser Trp Ala Val Thr Pro Lys Ser
    850                 855                 860

Leu Gly Asn Val Asn Phe Thr Val Ser Ala Glu Ala Leu Glu Ser Gln
865                 870                 875                 880

Glu Leu Cys Gly Thr Glu Val Pro Ser Val Pro Glu His Gly Arg Lys
                885                 890                 895

Asp Thr Val Ile Lys Pro Leu Leu Val Glu Pro Glu Gly Leu Glu Lys
            900                 905                 910

Glu Thr Thr Phe Asn Ser Leu Leu Cys Pro Ser Gly Gly Glu Val Ser
        915                 920                 925

Glu Glu Leu Ser Leu Lys Leu Pro Pro Asn Val Val Glu Glu Ser Ala
    930                 935                 940

Arg Ala Ser Val Ser Val Leu Gly Asp Ile Leu Gly Ser Ala Met Gln
945                 950                 955                 960

Asn Thr Gln Asn Leu Leu Gln Met Pro Tyr Gly Cys Gly Glu Gln Asn
                965                 970                 975

Met Val Leu Phe Ala Pro Asn Ile Tyr Val Leu Asp Tyr Leu Asn Glu
            980                 985                 990

Thr Gln Gln Leu Thr Pro Glu Ile Lys Ser Lys Ala Ile Gly Tyr Leu
        995                 1000                1005

Asn Thr Gly Tyr Gln Arg Gln Leu Asn Tyr Lys His Tyr Asp Gly
    1010                1015                1020

Ser Tyr Ser Thr Phe Gly Glu Arg Tyr Gly Arg Asn Gln Gly Asn
    1025                1030                1035

Thr Trp Leu Thr Ala Phe Val Leu Lys Thr Phe Ala Gln Ala Arg

1040                1045                1050

Ala Tyr Ile Phe Ile Asp Glu Ala His Ile Thr Gln Ala Leu Ile
    1055                1060                1065

Trp Leu Ser Gln Arg Gln Lys Asp Asn Gly Cys Phe Arg Ser Ser
    1070                1075                1080

Gly Ser Leu Leu Asn Asn Ala Ile Lys Gly Gly Val Glu Asp Glu
    1085                1090                1095

Val Thr Leu Ser Ala Tyr Ile Thr Ile Ala Leu Leu Glu Ile Pro
    1100                1105                1110

Leu Thr Val Thr His Pro Val Val Arg Asn Ala Leu Phe Cys Leu
    1115                1120                1125

Glu Ser Ala Trp Lys Thr Ala Gln Glu Gly Asp His Gly Ser His
    1130                1135                1140

Val Tyr Thr Lys Ala Leu Leu Ala Tyr Ala Phe Ala Leu Ala Gly
    1145                1150                1155

Asn Gln Asp Lys Arg Lys Glu Val Leu Lys Ser Leu Asn Glu Glu
    1160                1165                1170

Ala Val Lys Lys Asp Asn Ser Val His Trp Glu Arg Pro Gln Lys
    1175                1180                1185

Pro Lys Ala Pro Val Gly His Phe Tyr Glu Pro Gln Ala Pro Ser
    1190                1195                1200

Ala Glu Val Glu Met Thr Ser Tyr Val Leu Leu Ala Tyr Leu Thr
    1205                1210                1215

Ala Gln Pro Ala Pro Thr Ser Glu Asp Leu Thr Ser Ala Thr Asn
    1220                1225                1230

Ile Val Lys Trp Ile Thr Lys Gln Gln Asn Ala Gln Gly Gly Phe
    1235                1240                1245

Ser Ser Thr Gln Asp Thr Val Val Ala Leu His Ala Leu Ser Lys
    1250                1255                1260

Tyr Gly Ala Ala Thr Phe Thr Arg Thr Gly Lys Ala Ala Gln Val
    1265                1270                1275

Thr Ile Gln Ser Ser Gly Thr Phe Ser Ser Lys Phe Gln Val Asp
    1280                1285                1290

Asn Asn Asn Arg Leu Leu Leu Gln Gln Val Ser Leu Pro Glu Leu
    1295                1300                1305

Pro Gly Glu Tyr Ser Met Lys Val Thr Gly Glu Gly Cys Val Tyr
    1310                1315                1320

Leu Gln Thr Ser Leu Lys Tyr Asn Ile Leu Pro Glu Lys Glu Glu
    1325                1330                1335

Phe Pro Phe Ala Leu Gly Val Gln Thr Leu Pro Gln Thr Cys Asp
    1340                1345                1350

Glu Pro Lys Ala His Thr Ser Phe Gln Ile Ser Leu Ser Val Ser
    1355                1360                1365

Tyr Thr Gly Ser Arg Ser Ala Ser Asn Met Ala Ile Val Asp Val
    1370                1375                1380

Lys Met Val Ser Gly Phe Ile Pro Leu Lys Pro Thr Val Lys Met
    1385                1390                1395

Leu Glu Arg Ser Asn His Val Ser Arg Thr Glu Val Ser Ser Asn
    1400                1405                1410

His Val Leu Ile Tyr Leu Asp Lys Val Ser Asn Gln Thr Leu Ser
    1415                1420                1425

Leu Phe Phe Thr Val Leu Gln Asp Val Pro Val Arg Asp Leu Lys
    1430                1435                1440

Pro Ala Ile Val Lys Val Tyr Asp Tyr Tyr Glu Thr Asp Glu Phe
    1445                1450                1455

Ala Ile Ala Glu Tyr Asn Ala Pro Cys Ser Lys Asp Leu Gly Asn
    1460                1465                1470

Ala

<210> 15
<211> 4955
<212> DNA
<213> Homo sapiens

<400> 15
gaaaagctta ttagctgctg tacggtaaaa gtgagctctt acgggaatgg gaatgtagtt 60

ttagccctcc agggattcta tttagcccgc caggaattaa ccttgactat aaataggcca 120

tcaatgacct ttccagagaa tgttcagaga cctcaacttt gtttagagat cttgtgtggg 180

tggaacttcc tgtttgcaca cagagcagca taaagcccag ttgctttggg aagtgtttgg 240

gaccagatgg attgtaggga gtagggtaca atacagtctg ttctcctcca gctccttctt 300

tctgcaacat ggggaagaac aaactccttc atccaagtct ggttcttctc ctcttggtcc 360

tcctgcccac agacgcctca gtctctggaa aaccgcagta tatggttctg gtcccctccc 420

tgctccacac tgagaccact gagaagggct gtgtccttct gagctacctg aatgagacag 480

tgactgtaag tgcttccttg gagtctgtca ggggaaacag gagcctcttc actgacctgg 540

aggcggagaa tgacgtactc cactgtgtcg ccttcgctgt cccaaagtct tcatccaatg 600

aggaggtaat gttcctcact gtccaagtga aaggaccaac ccaagaattt aagaagcgga 660

ccacagtgat ggttaagaac gaggacagtc tggtctttgt ccagacagac aaatcaatct 720

acaaaccagg gcagacagtg aaatttcgtg ttgtctccat ggatgaaaac tttcaccccc 780

tgaatgagtt gattccacta gtatacattc aggatcccaa aggaaatcgc atcgcacaat 840

ggcagagttt ccagttagag ggtggcctca agcaattttc ttttcccctc tcatcagagc 900

ccttccaggg ctcctacaag gtggtggtac agaagaaatc aggtggaagg acagagcacc 960

ctttcaccgt ggaggaattt gttcttccca agtttgaagt acaagtaaca gtgccaaaga 1020

taatcaccat cttggaagaa gagatgaatg tatcagtgtg tggcctatac acatatggga 1080

agcctgtccc tggacatgtg actgtgagca tttgcagaaa gtatagtgac gcttccgact 1140

gccacggtga agattcacag gctttctgtg agaaattcag tggacagcta aacagccatg 1200

gctgcttcta tcagcaagta aaaaccaagg tcttccagct gaagaggaag gagtatgaaa 1260

tgaaacttca cactgaggcc cagatccaag aagaaggaac agtggtggaa ttgactggaa 1320

ggcagtccag tgaaatcaca agaaccataa ccaaactctc atttgtgaaa gtggactcac 1380

actttcgaca gggaattccc ttctttgggc aggtgcgcct agtagatggg aaaggcgtcc 1440

ctataccaaa taaagtcata ttcatcagag gaaatgaagc aaactattac tccaatgcta 1500

ccacggatga gcatggcctt gtacagttct ctatcaacac caccaatgtt atgggtacct 1560

ctcttactgt tagggtcaat tacaaggatc gtagtccctg ttacggctac cagtgggtgt 1620

cagaagaaca cgaagaggca catcacactg cttatcttgt gttctcccca agcaagagct 1680

ttgtccacct tgagcccatg tctcatgaac taccctgtgg ccatactcag acagtccagg 1740

cacattatat tctgaatgga ggcaccctgc tggggctgaa gaagctctcc ttctattatc 1800

tgataatggc aaagggaggc attgtccgaa ctgggactca tggactgctt gtgaagcagg 1860

aagacatgaa gggccatttt tccatctcaa tccctgtgaa gtcagacatt gctcctgtcg 1920

ctcggttgct catctatgct gttttaccta ccggggacgt gattggggat tctgcaaaat 1980

atgatgttga aaattgtctg gccaacaagg tggatttgag cttcagccca tcacaaagtc 2040

tcccagcctc acacgcccac ctgcgagtca cagcggctcc tcagtccgtc tgcgccctcc 2100

gtgctgtgga ccaaagcgtg ctgctcatga agcctgatgc tgagctctcg gcgtcctcgg 2160

tttacaacct gctaccagaa aaggacctca ctggcttccc tgggcctttg aatgaccagg 2220

acaatgaaga ctgcatcaat cgtcataatg tctatattaa tggaatcaca tatactccag 2280

tatcaagtac aaatgaaaag gatatgtaca gcttcctaga ggacatgggc ttaaaggcat 2340

tcaccaactc aaagattcgt aaacccaaaa tgtgtccaca gcttcaacag tatgaaatgc 2400

atggacctga aggtctacgt gtaggttttt atgagtcaga tgtaatggga agaggccatg 2460

cacgcctggt gcatgttgaa gagcctcaca cggagaccgt acgaaagtac ttccctgaga 2520

catggatctg ggatttggtg gtggtaaact cagcaggtgt ggctgaggta ggagtaacag 2580

tccctgacac catcaccgag tggaaggcag gggccttctg cctgtctgaa gatgctggac 2640

ttggtatctc ttccactgcc tctctccgag ccttccagcc cttctttgtg gagctcacaa 2700

tgccttactc tgtgattcgt ggagaggcct tcacactcaa ggccacggtc ctaaactacc 2760

ttcccaaatg catccgggtc agtgtgcagc tggaagcctc tcccgccttc ctagctgtcc 2820

cagtggagaa ggaacaagcg cctcactgca tctgtgcaaa cgggcggcaa actgtgtcct 2880

gggcagtaac cccaaagtca ttaggaaatg tgaatttcac tgtgagcgca gaggcactag 2940

agtctcaaga gctgtgtggg actgaggtgc cttcagttcc tgaacacgga aggaaagaca 3000

cagtcatcaa gcctctgttg gttgaacctg aaggactaga gaaggaaaca acattcaact 3060

ccctactttg tccatcaggt ggtgaggttt ctgaagaatt atccctgaaa ctgccaccaa 3120

atgtggtaga agaatctgcc cgagcttctg tctcagtttt gggagacata ttaggctctg 3180

ccatgcaaaa cacacaaaat cttctccaga tgccctatgg ctgtggagag cagaatatgg 3240

tcctctttgc tcctaacatc tatgtactgg attatctaaa tgaaacacag cagcttactc 3300

cagagatcaa gtccaaggcc attggctatc tcaacactgg ttaccagaga cagttgaact 3360

acaaacacta tgatggctcc tacagcacct ttggggagcg atatggcagg aaccaggcca 3420

acacctggct cacagccttt gttctgaaga cttttgccca agctcgagcc tacatcttca 3480

tcgatgaagc acacattacc caagccctca tatggctctc ccagaggcag aaggacaatg 3540

gctgtttcag gagctctggg tcactgctca acaatgccat aaaggaggga gtagaagatg 3600

aagtgaccct ctccgcctat atcaccatcg cccttctgga gattcctctc acagtcactc 3660

accctgttgt ccgcaatgcc ctgttttgcc tggagtcagc ctggaagaca gcacaagaag 3720

gggaccatgg cagccatgta tataccaaag cactgctggc ctatgctttt gccctggcag 3780

gtaaccagga caagaggaag gaagtactca agtcacttaa tgaggaagct gtgaagaaag 3840

acaactctgt ccattgggag cgccctcaga aacccaaggc accagtgggg catttttacg 3900

aaccccaggc tccctctgct gaggtggaga tgacatccta tgtgctcctc gcttatctca 3960

cggcccagcc agccccaacc tcggaggacc tgacctctgc aaccaacatc gtgaagtgga 4020

tcacgaagca gcagaatgcc cagggcggtt tctcctccac ccaggacaca gtggtggctc 4080

tccatgctct gtccaaatat ggagcagcca catttaccag gactgggaag gctgcacagg 4140

tgactatcca gtcttcaggg acattttcca gcaaattcca agtggacaac aacaaccgcc 4200

tgttactgca gcaggtctca ttgccagagc tgcctgggga atacagcatg aaagtgacag 4260

gagaaggatg tgtctacctc cagacatcct tgaaatacaa tattctccca gaaaaggaag 4320

agttcccctt tgctttagga gtgcagactc tgcctcaaac ttgtgatgaa cccaaagccc 4380

acaccagctt ccaaatctcc ctaagtgtca gttacacagg gagccgctct gcctccaaca 4440

tggcgatcgt tgatgtgaag atggtctctg gcttcattcc cctgaagcca acagtgaaaa 4500

tgcttgaaag atctaaccat gtgagccgga cagaagtcag cagcaaccat gtcttgattt 4560

accttgataa ggtgtcaaat cagacactga gcttgttctt cacggttctg caagatgtcc 4620

cagtaagaga tctgaaacca gccatagtga aagtctatga ttactacgag acggatgagt 4680

ttgcaattgc tgagtacaat gctccttgca gcaaagatct tggaaatgct tgaagaccac 4740

aaggctgaaa agtgctttgc tggagtcctg ttctcagagc tccacagaag acacgtgttt 4800

ttgtatcttt aaagacttga tgaataaaca ctttttctgg tcaaagacca caaggctgaa 4860

aagtgctttg ctggagtcct gttctcagag ctccacagaa gacacgtgtt tttgtatctt 4920

taaagacttg atgaataaac actttttctg gtcaa 4955

<210> 16
<211> 325
<212> PRT
<213> Homo sapiens

<400> 16

Met Leu Pro Arg Val Gly Cys Pro Ala Leu Pro Leu Pro Pro Pro Pro
1               5                   10                  15

Leu Leu Pro Leu Leu Leu Leu Leu Leu Gly Ala Ser Gly Gly Gly Gly
            20                  25                  30

Gly Ala Arg Ala Glu Val Leu Phe Arg Cys Pro Pro Cys Thr Pro Glu
        35                  40                  45

Arg Leu Ala Ala Cys Gly Pro Pro Pro Val Ala Pro Pro Ala Ala Val
    50                  55                  60

Ala Ala Val Ala Gly Gly Ala Arg Met Pro Cys Ala Glu Leu Val Arg
65                  70                  75                  80

Glu Pro Gly Cys Gly Cys Cys Ser Val Cys Ala Arg Leu Glu Gly Glu
                85                  90                  95

Ala Cys Gly Val Tyr Thr Pro Arg Cys Gly Gln Gly Leu Arg Cys Tyr
            100                 105                 110

Pro His Pro Gly Ser Glu Leu Pro Leu Gln Ala Leu Val Met Gly Glu
        115                 120                 125

Gly Thr Cys Glu Lys Arg Arg Asp Ala Glu Tyr Gly Ala Ser Pro Glu
    130                 135                 140

Gln Val Ala Asp Asn Gly Asp Asp His Ser Glu Gly Gly Leu Val Glu
145                 150                 155                 160

Asn His Val Asp Ser Thr Met Asn Met Leu Gly Gly Gly Gly Ser Ala
                165                 170                 175

Gly Arg Lys Pro Leu Lys Ser Gly Met Lys Glu Leu Ala Val Phe Arg
            180                 185                 190

Glu Lys Val Thr Glu Gln His Arg Gln Met Gly Lys Gly Gly Lys His
        195                 200                 205

His Leu Gly Leu Glu Glu Pro Lys Lys Leu Arg Pro Pro Pro Ala Arg
    210                 215                 220

Thr Pro Cys Gln Gln Glu Leu Asp Gln Val Leu Glu Arg Ile Ser Thr
225                 230                 235                 240

Met Arg Leu Pro Asp Glu Arg Gly Pro Leu Glu His Leu Tyr Ser Leu
                245                 250                 255

His Ile Pro Asn Cys Asp Lys His Gly Leu Tyr Asn Leu Lys Gln Cys
            260                 265                 270

Lys Met Ser Leu Asn Gly Gln Arg Gly Glu Cys Trp Cys Val Asn Pro
        275                 280                 285

Asn Thr Gly Lys Leu Ile Gln Gly Ala Pro Thr Ile Arg Gly Asp Pro
    290                 295                 300

Glu Cys His Leu Phe Tyr Asn Glu Gln Gln Glu Ala Arg Gly Val His
305                 310                 315                 320

Thr Gln Arg Met Gln
                325

<210>  17
<211>  1414
<212>  DNA
<213>  Homo sapiens

<400>  17
gaggaagaag cggaggaggc ggctcccgcg ctcgcagggc cgtgccacct gcccgcccgc      60

ccgctcgctc gctcgcccgc cgcgccgcgc tgccgaccgc cagcatgctg ccgagagtgg     120

gctgccccgc gctgccgctg ccgccgccgc cgctgctgcc gctgctgctg ctgctactgg     180

gcgcgagtgg cggcggcggc ggggcgcgcg cggaggtgct gttccgctgc ccgccctgca     240

cacccgagcg cctggccgcc tgcgggcccc cgccggttgc gccgcccgcc gcggtggccg     300

cagtggccgg aggcgcccgc atgccatgcg cggagctcgt ccgggagccg ggctgcggct     360

gctgctcggt gtgcgcccgg ctggagggcg aggcgtgcgg cgtctacacc ccgcgctgcg     420

gccaggggct gcgctgctat ccccacccgg gctccgagct gcccctgcag gcgctggtca     480

tgggcgaggg cacttgtgag aagcgccggg acgccgagta tggcgccagc ccggagcagg     540

ttgcagacaa tggcgatgac cactcagaag gaggcctggt ggagaaccac gtggacagca     600

ccatgaacat gttgggcggg ggaggcagtg ctggccggaa gcccctcaag tcgggtatga     660

aggagctggc cgtgttccgg gagaaggtca ctgagcagca ccggcagatg ggcaagggtg     720

gcaagcatca ccttggcctg gaggagccca agaagctgcg accaccccct gccaggactc     780

cctgccaaca ggaactggac caggtcctgg agcggatctc caccatgcgc cttccggatg     840

agcggggccc tctggagcac ctctactccc tgcacatccc caactgtgac aagcatggcc     900

tgtacaacct caaacagtgc aagatgtctc tgaacgggca gcgtggggag tgctggtgtg     960

tgaaccccaa caccgggaag ctgatccagg gagcccccac catccggggg gaccccgagt    1020

gtcatctctt ctacaatgag cagcaggagg ctcgcggggt gcacacccag cggatgcagt    1080

agaccgcagc cagccggtgc ctggcgcccc tgccccccgc ccctctccaa acaccggcag    1140

aaaacggaga gtgcttgggt ggtgggtgct ggaggatttt ccagttctga cacacgtatt    1200

tatatttgga aagagaccag caccgagctc ggcacctccc cggcctctct cttcccagct    1260

gcagatgcca cacctgctcc ttcttgcttt ccccggggga ggaagggggt tgtggtcggg    1320

gagctggggt acaggtttgg ggagggggaa gagaaatttt tatttttgaa cccctgtgtc    1380

ccttttgcat aagattaaag gaaggaaaag taaa                                1414

<210>  18
<211>  4563
<212>  PRT

<213>  Homo sapiens

<400>  18

Met Asp Pro Pro Arg Pro Ala Leu Leu Ala Leu Leu Ala Leu Pro Ala
1               5                   10                  15

Leu Leu Leu Leu Leu Leu Ala Gly Ala Arg Ala Glu Glu Glu Met Leu
            20                  25                  30

Glu Asn Val Ser Leu Val Cys Pro Lys Asp Ala Thr Arg Phe Lys His
        35                  40                  45

Leu Arg Lys Tyr Thr Tyr Asn Tyr Glu Ala Glu Ser Ser Ser Gly Val
    50                  55                  60

Pro Gly Thr Ala Asp Ser Arg Ser Ala Thr Arg Ile Asn Cys Lys Val
65                  70                  75                  80

Glu Leu Glu Val Pro Gln Leu Cys Ser Phe Ile Leu Lys Thr Ser Gln
                85                  90                  95

Cys Thr Leu Lys Glu Val Tyr Gly Phe Asn Pro Glu Gly Lys Ala Leu
            100                 105                 110

Leu Lys Lys Thr Lys Asn Ser Glu Glu Phe Ala Ala Ala Met Ser Arg
        115                 120                 125

Tyr Glu Leu Lys Leu Ala Ile Pro Glu Gly Lys Gln Val Phe Leu Tyr
    130                 135                 140

Pro Glu Lys Asp Glu Pro Thr Tyr Ile Leu Asn Ile Lys Arg Gly Ile
145                 150                 155                 160

Ile Ser Ala Leu Leu Val Pro Pro Glu Thr Glu Glu Ala Lys Gln Val
                165                 170                 175

Leu Phe Leu Asp Thr Val Tyr Gly Asn Cys Ser Thr His Phe Thr Val
            180                 185                 190

Lys Thr Arg Lys Gly Asn Val Ala Thr Glu Ile Ser Thr Glu Arg Asp
        195                 200                 205

Leu Gly Gln Cys Asp Arg Phe Lys Pro Ile Arg Thr Gly Ile Ser Pro
    210                 215                 220

Leu Ala Leu Ile Lys Gly Met Thr Arg Pro Leu Ser Thr Leu Ile Ser
225                 230                 235                 240

Ser Ser Gln Ser Cys Gln Tyr Thr Leu Asp Ala Lys Arg Lys His Val
                245                 250                 255

Ala Glu Ala Ile Cys Lys Glu Gln His Leu Phe Leu Pro Phe Ser Tyr
            260                 265                 270

Lys Asn Lys Tyr Gly Met Val Ala Gln Val Thr Gln Thr Leu Lys Leu
        275                 280                 285

Glu Asp Thr Pro Lys Ile Asn Ser Arg Phe Phe Gly Glu Gly Thr Lys
    290                 295                 300

Lys Met Gly Leu Ala Phe Glu Ser Thr Lys Ser Thr Ser Pro Pro Lys
305                 310                 315                 320

Gln Ala Glu Ala Val Leu Lys Thr Leu Gln Glu Leu Lys Lys Leu Thr
                325                 330                 335

Ile Ser Glu Gln Asn Ile Gln Arg Ala Asn Leu Phe Asn Lys Leu Val
            340                 345                 350

Thr Glu Leu Arg Gly Leu Ser Asp Glu Ala Val Thr Ser Leu Leu Pro
        355                 360                 365

Gln Leu Ile Glu Val Ser Ser Pro Ile Thr Leu Gln Ala Leu Val Gln
    370                 375                 380

Cys Gly Gln Pro Gln Cys Ser Thr His Ile Leu Gln Trp Leu Lys Arg
385                 390                 395                 400

Val His Ala Asn Pro Leu Leu Ile Asp Val Val Thr Tyr Leu Val Ala
                405                 410                 415

Leu Ile Pro Glu Pro Ser Ala Gln Gln Leu Arg Glu Ile Phe Asn Met
            420                 425                 430

Ala Arg Asp Gln Arg Ser Arg Ala Thr Leu Tyr Ala Leu Ser His Ala
        435                 440                 445

Val Asn Asn Tyr His Lys Thr Asn Pro Thr Gly Thr Gln Glu Leu Leu
    450                 455                 460

Asp Ile Ala Asn Tyr Leu Met Glu Gln Ile Gln Asp Asp Cys Thr Gly
465                 470                 475                 480

Asp Glu Asp Tyr Thr Tyr Leu Ile Leu Arg Val Ile Gly Asn Met Gly
                485                 490                 495

Gln Thr Met Glu Gln Leu Thr Pro Glu Leu Lys Ser Ser Ile Leu Lys
            500                 505                 510

Cys Val Gln Ser Thr Lys Pro Ser Leu Met Ile Gln Lys Ala Ala Ile
        515                 520                 525

Gln Ala Leu Arg Lys Met Glu Pro Lys Asp Lys Asp Gln Glu Val Leu
    530                 535                 540

Leu Gln Thr Phe Leu Asp Asp Ala Ser Pro Gly Asp Lys Arg Leu Ala
545                 550                 555                 560

Ala Tyr Leu Met Leu Met Arg Ser Pro Ser Gln Ala Asp Ile Asn Lys
                565                 570                 575

Ile Val Gln Ile Leu Pro Trp Glu Gln Asn Glu Gln Val Lys Asn Phe
            580                 585                 590

Val Ala Ser His Ile Ala Asn Ile Leu Asn Ser Glu Glu Leu Asp Ile
        595                 600                 605

Gln Asp Leu Lys Lys Leu Val Lys Glu Ala Leu Lys Glu Ser Gln Leu
    610                 615                 620

Pro Thr Val Met Asp Phe Arg Lys Phe Ser Arg Asn Tyr Gln Leu Tyr
625                 630                 635                 640

Lys Ser Val Ser Leu Pro Ser Leu Asp Pro Ala Ser Ala Lys Ile Glu
                645                 650                 655

Gly Asn Leu Ile Phe Asp Pro Asn Asn Tyr Leu Pro Lys Glu Ser Met
            660                 665                 670

Leu Lys Thr Thr Leu Thr Ala Phe Gly Phe Ala Ser Ala Asp Leu Ile
        675                 680                 685

Glu Ile Gly Leu Glu Gly Lys Gly Phe Glu Pro Thr Leu Glu Ala Leu
    690                 695                 700

Phe Gly Lys Gln Gly Phe Phe Pro Asp Ser Val Asn Lys Ala Leu Tyr
705                 710                 715                 720

Trp Val Asn Gly Gln Val Pro Asp Gly Val Ser Lys Val Leu Val Asp
                725                 730                 735

His Phe Gly Tyr Thr Lys Asp Asp Lys His Glu Gln Asp Met Val Asn

740                 745                 750

Gly Ile Met Leu Ser Val Glu Lys Leu Ile Lys Asp Leu Lys Ser Lys
        755                 760                 765

Glu Val Pro Glu Ala Arg Ala Tyr Leu Arg Ile Leu Gly Glu Glu Leu
    770                 775                 780

Gly Phe Ala Ser Leu His Asp Leu Gln Leu Leu Gly Lys Leu Leu Leu
785                 790                 795                 800

Met Gly Ala Arg Thr Leu Gln Gly Ile Pro Gln Met Ile Gly Glu Val
                805                 810                 815

Ile Arg Lys Gly Ser Lys Asn Asp Phe Phe Leu His Tyr Ile Phe Met
            820                 825                 830

Glu Asn Ala Phe Glu Leu Pro Thr Gly Ala Gly Leu Gln Leu Gln Ile
        835                 840                 845

Ser Ser Ser Gly Val Ile Ala Pro Gly Ala Lys Ala Gly Val Lys Leu
    850                 855                 860

Glu Val Ala Asn Met Gln Ala Glu Leu Val Ala Lys Pro Ser Val Ser
865                 870                 875                 880

Val Glu Phe Val Thr Asn Met Gly Ile Ile Ile Pro Asp Phe Ala Arg
                885                 890                 895

Ser Gly Val Gln Met Asn Thr Asn Phe Phe His Glu Ser Gly Leu Glu
            900                 905                 910

Ala His Val Ala Leu Lys Ala Gly Lys Leu Lys Phe Ile Ile Pro Ser
        915                 920                 925

Pro Lys Arg Pro Val Lys Leu Leu Ser Gly Gly Asn Thr Leu His Leu
    930                 935                 940

Val Ser Thr Thr Lys Thr Glu Val Ile Pro Pro Leu Ile Glu Asn Arg
945                 950                 955                 960

Gln Ser Trp Ser Val Cys Lys Gln Val Phe Pro Gly Leu Asn Tyr Cys
                965                 970                 975

Thr Ser Gly Ala Tyr Ser Asn Ala Ser Ser Thr Asp Ser Ala Ser Tyr
            980                 985                 990

Tyr Pro Leu Thr Gly Asp Thr Arg Leu Glu Leu Glu Leu Arg Pro Thr
        995                 1000                1005

Gly Glu Ile Glu Gln Tyr Ser Val Ser Ala Thr Tyr Glu Leu Gln
    1010                1015                1020

Arg Glu Asp Arg Ala Leu Val Asp Thr Leu Lys Phe Val Thr Gln
    1025                1030                1035

Ala Glu Gly Ala Lys Gln Thr Glu Ala Thr Met Thr Phe Lys Tyr
    1040                1045                1050

Asn Arg Gln Ser Met Thr Leu Ser Ser Glu Val Gln Ile Pro Asp
    1055                1060                1065

Phe Asp Val Asp Leu Gly Thr Ile Leu Arg Val Asn Asp Glu Ser
    1070                1075                1080

Thr Glu Gly Lys Thr Ser Tyr Arg Leu Thr Leu Asp Ile Gln Asn
    1085                1090                1095

Lys Lys Ile Thr Glu Val Ala Leu Met Gly His Leu Ser Cys Asp
    1100                1105                1110

Thr Lys Glu Glu Arg Lys Ile Lys Gly Val Ile Ser Ile Pro Arg
    1115                1120                1125

Leu Gln Ala Glu Ala Arg Ser Glu Ile Leu Ala His Trp Ser Pro
    1130                1135                1140

Ala Lys Leu Leu Leu Gln Met Asp Ser Ser Ala Thr Ala Tyr Gly
    1145                1150                1155

Ser Thr Val Ser Lys Arg Val Ala Trp His Tyr Asp Glu Glu Lys
    1160                1165                1170

Ile Glu Phe Glu Trp Asn Thr Gly Thr Asn Val Asp Thr Lys Lys
    1175                1180                1185

Met Thr Ser Asn Phe Pro Val Asp Leu Ser Asp Tyr Pro Lys Ser
    1190                1195                1200

Leu His Met Tyr Ala Asn Arg Leu Leu Asp His Arg Val Pro Gln
    1205                1210                1215

Thr Asp Met Thr Phe Arg His Val Gly Ser Lys Leu Ile Val Ala
    1220                1225                1230

Met Ser Ser Trp Leu Gln Lys Ala Ser Gly Ser Leu Pro Tyr Thr
    1235                1240                1245

Gln Thr Leu Gln Asp His Leu Asn Ser Leu Lys Glu Phe Asn Leu
    1250                1255                1260

Gln Asn Met Gly Leu Pro Asp Phe His Ile Pro Glu Asn Leu Phe
    1265                1270                1275

Leu Lys Ser Asp Gly Arg Val Lys Tyr Thr Leu Asn Lys Asn Ser
    1280                1285                1290

Leu Lys Ile Glu Ile Pro Leu Pro Phe Gly Gly Lys Ser Ser Arg
    1295                1300                1305

Asp Leu Lys Met Leu Glu Thr Val Arg Thr Pro Ala Leu His Phe
    1310                1315                1320

Lys Ser Val Gly Phe His Leu Pro Ser Arg Glu Phe Gln Val Pro
    1325                1330                1335

Thr Phe Thr Ile Pro Lys Leu Tyr Gln Leu Gln Val Pro Leu Leu
    1340                1345                1350

Gly Val Leu Asp Leu Ser Thr Asn Val Tyr Ser Asn Leu Tyr Asn
    1355                1360                1365

Trp Ser Ala Ser Tyr Ser Gly Gly Asn Thr Ser Thr Asp His Phe
    1370                1375                1380

Ser Leu Arg Ala Arg Tyr His Met Lys Ala Asp Ser Val Val Asp
    1385                1390                1395

Leu Leu Ser Tyr Asn Val Gln Gly Ser Gly Glu Thr Thr Tyr Asp
    1400                1405                1410

His Lys Asn Thr Phe Thr Leu Ser Cys Asp Gly Ser Leu Arg His
    1415                1420                1425

Lys Phe Leu Asp Ser Asn Ile Lys Phe Ser His Val Glu Lys Leu
    1430                1435                1440

Gly Asn Asn Pro Val Ser Lys Gly Leu Leu Ile Phe Asp Ala Ser
    1445                1450                1455

Ser Ser Trp Gly Pro Gln Met Ser Ala Ser Val His Leu Asp Ser
    1460                1465                1470

Lys Lys Lys Gln His Leu Phe Val Lys Glu Val Lys Ile Asp Gly
    1475                1480                1485

Gln Phe Arg Val Ser Ser Phe Tyr Ala Lys Gly Thr Tyr Gly Leu
    1490                1495                1500

Ser Cys Gln Arg Asp Pro Asn Thr Gly Arg Leu Asn Gly Glu Ser
    1505                1510                1515

Asn Leu Arg Phe Asn Ser Ser Tyr Leu Gln Gly Thr Asn Gln Ile
    1520                1525                1530

Thr Gly Arg Tyr Glu Asp Gly Thr Leu Ser Leu Thr Ser Thr Ser
    1535                1540                1545

Asp Leu Gln Ser Gly Ile Ile Lys Asn Thr Ala Ser Leu Lys Tyr
    1550                1555                1560

Glu Asn Tyr Glu Leu Thr Leu Lys Ser Asp Thr Asn Gly Lys Tyr
    1565                1570                1575

Lys Asn Phe Ala Thr Ser Asn Lys Met Asp Met Thr Phe Ser Lys
    1580                1585                1590

Gln Asn Ala Leu Leu Arg Ser Glu Tyr Gln Ala Asp Tyr Glu Ser
    1595                1600                1605

Leu Arg Phe Phe Ser Leu Leu Ser Gly Ser Leu Asn Ser His Gly
    1610                1615                1620

Leu Glu Leu Asn Ala Asp Ile Leu Gly Thr Asp Lys Ile Asn Ser
    1625                1630                1635

Gly Ala His Lys Ala Thr Leu Arg Ile Gly Gln Asp Gly Ile Ser
    1640                1645                1650

Thr Ser Ala Thr Thr Asn Leu Lys Cys Ser Leu Leu Val Leu Glu
    1655                1660                1665

Asn Glu Leu Asn Ala Glu Leu Gly Leu Ser Gly Ala Ser Met Lys
    1670                1675                1680

Leu Thr Thr Asn Gly Arg Phe Arg Glu His Asn Ala Lys Phe Ser
    1685                1690                1695

Leu Asp Gly Lys Ala Ala Leu Thr Glu Leu Ser Leu Gly Ser Ala

1700                1705                1710

Tyr Gln Ala Met Ile Leu Gly Val Asp Ser Lys Asn Ile Phe Asn
    1715                1720                1725

Phe Lys Val Ser Gln Glu Gly Leu Lys Leu Ser Asn Asp Met Met
    1730                1735                1740

Gly Ser Tyr Ala Glu Met Lys Phe Asp His Thr Asn Ser Leu Asn
    1745                1750                1755

Ile Ala Gly Leu Ser Leu Asp Phe Ser Ser Lys Leu Asp Asn Ile
    1760                1765                1770

Tyr Ser Ser Asp Lys Phe Tyr Lys Gln Thr Val Asn Leu Gln Leu
    1775                1780                1785

Gln Pro Tyr Ser Leu Val Thr Thr Leu Asn Ser Asp Leu Lys Tyr
    1790                1795                1800

Asn Ala Leu Asp Leu Thr Asn Asn Gly Lys Leu Arg Leu Glu Pro
    1805                1810                1815

Leu Lys Leu His Val Ala Gly Asn Leu Lys Gly Ala Tyr Gln Asn
    1820                1825                1830

Asn Glu Ile Lys His Ile Tyr Ala Ile Ser Ser Ala Ala Leu Ser
    1835                1840                1845

Ala Ser Tyr Lys Ala Asp Thr Val Ala Lys Val Gln Gly Val Glu
    1850                1855                1860

Phe Ser His Arg Leu Asn Thr Asp Ile Ala Gly Leu Ala Ser Ala
    1865                1870                1875

Ile Asp Met Ser Thr Asn Tyr Asn Ser Asp Ser Leu His Phe Ser
    1880                1885                1890

Asn Val Phe Arg Ser Val Met Ala Pro Phe Thr Met Thr Ile Asp
    1895                1900                1905

Ala His Thr Asn Gly Asn Gly Lys Leu Ala Leu Trp Gly Glu His
    1910                1915                1920

Thr Gly Gln Leu Tyr Ser Lys Phe Leu Leu Lys Ala Glu Pro Leu
    1925                1930                1935

Ala Phe Thr Phe Ser His Asp Tyr Lys Gly Ser Thr Ser His His
    1940                1945                1950

Leu Val Ser Arg Lys Ser Ile Ser Ala Ala Leu Glu His Lys Val
    1955                1960                1965

Ser Ala Leu Leu Thr Pro Ala Glu Gln Thr Gly Thr Trp Lys Leu
    1970                1975                1980

Lys Thr Gln Phe Asn Asn Asn Glu Tyr Ser Gln Asp Leu Asp Ala
    1985                1990                1995

Tyr Asn Thr Lys Asp Lys Ile Gly Val Glu Leu Thr Gly Arg Thr
    2000                2005                2010

Leu Ala Asp Leu Thr Leu Leu Asp Ser Pro Ile Lys Val Pro Leu
    2015                2020                2025

Leu Leu Ser Glu Pro Ile Asn Ile Ile Asp Ala Leu Glu Met Arg
    2030                2035                2040

Asp Ala Val Glu Lys Pro Gln Glu Phe Thr Ile Val Ala Phe Val
    2045                2050                2055

Lys Tyr Asp Lys Asn Gln Asp Val His Ser Ile Asn Leu Pro Phe
    2060                2065                2070

Phe Glu Thr Leu Gln Glu Tyr Phe Glu Arg Asn Arg Gln Thr Ile
    2075                2080                2085

Ile Val Val Leu Glu Asn Val Gln Arg Asn Leu Lys His Ile Asn
    2090                2095                2100

Ile Asp Gln Phe Val Arg Lys Tyr Arg Ala Ala Leu Gly Lys Leu
    2105                2110                2115

Pro Gln Gln Ala Asn Asp Tyr Leu Asn Ser Phe Asn Trp Glu Arg
    2120                2125                2130

Gln Val Ser His Ala Lys Glu Lys Leu Thr Ala Leu Thr Lys Lys
    2135                2140                2145

Tyr Arg Ile Thr Glu Asn Asp Ile Gln Ile Ala Leu Asp Asp Ala
    2150                2155                2160

Lys Ile Asn Phe Asn Glu Lys Leu Ser Gln Leu Gln Thr Tyr Met
    2165                2170                2175

Ile Gln Phe Asp Gln Tyr Ile Lys Asp Ser Tyr Asp Leu His Asp
    2180                2185                2190

Leu Lys Ile Ala Ile Ala Asn Ile Ile Asp Glu Ile Ile Glu Lys
    2195                2200                2205

Leu Lys Ser Leu Asp Glu His Tyr His Ile Arg Val Asn Leu Val
    2210                2215                2220

Lys Thr Ile His Asp Leu His Leu Phe Ile Glu Asn Ile Asp Phe
    2225                2230                2235

Asn Lys Ser Gly Ser Ser Thr Ala Ser Trp Ile Gln Asn Val Asp
    2240                2245                2250

Thr Lys Tyr Gln Ile Arg Ile Gln Ile Gln Glu Lys Leu Gln Gln
    2255                2260                2265

Leu Lys Arg His Ile Gln Asn Ile Asp Ile Gln His Leu Ala Gly
    2270                2275                2280

Lys Leu Lys Gln His Ile Glu Ala Ile Asp Val Arg Val Leu Leu
    2285                2290                2295

Asp Gln Leu Gly Thr Thr Ile Ser Phe Glu Arg Ile Asn Asp Ile
    2300                2305                2310

Leu Glu His Val Lys His Phe Val Ile Asn Leu Ile Gly Asp Phe
    2315                2320                2325

Glu Val Ala Glu Lys Ile Asn Ala Phe Arg Ala Lys Val His Glu
    2330                2335                2340

Leu Ile Glu Arg Tyr Glu Val Asp Gln Gln Ile Gln Val Leu Met
    2345                2350                2355

Asp Lys Leu Val Glu Leu Ala His Gln Tyr Lys Leu Lys Glu Thr
    2360                2365                2370

Ile Gln Lys Leu Ser Asn Val Leu Gln Gln Val Lys Ile Lys Asp
    2375                2380                2385

Tyr Phe Glu Lys Leu Val Gly Phe Ile Asp Asp Ala Val Lys Lys
    2390                2395                2400

Leu Asn Glu Leu Ser Phe Lys Thr Phe Ile Glu Asp Val Asn Lys
    2405                2410                2415

Phe Leu Asp Met Leu Ile Lys Lys Leu Lys Ser Phe Asp Tyr His
    2420                2425                2430

Gln Phe Val Asp Glu Thr Asn Asp Lys Ile Arg Glu Val Thr Gln
    2435                2440                2445

Arg Leu Asn Gly Glu Ile Gln Ala Leu Glu Leu Pro Gln Lys Ala
    2450                2455                2460

Glu Ala Leu Lys Leu Phe Leu Glu Glu Thr Lys Ala Thr Val Ala
    2465                2470                2475

Val Tyr Leu Glu Ser Leu Gln Asp Thr Lys Ile Thr Leu Ile Ile
    2480                2485                2490

Asn Trp Leu Gln Glu Ala Leu Ser Ser Ala Ser Leu Ala His Met
    2495                2500                2505

Lys Ala Lys Phe Arg Glu Thr Leu Glu Asp Thr Arg Asp Arg Met
    2510                2515                2520

Tyr Gln Met Asp Ile Gln Gln Glu Leu Gln Arg Tyr Leu Ser Leu
    2525                2530                2535

Val Gly Gln Val Tyr Ser Thr Leu Val Thr Tyr Ile Ser Asp Trp
    2540                2545                2550

Trp Thr Leu Ala Ala Lys Asn Leu Thr Asp Phe Ala Glu Gln Tyr
    2555                2560                2565

Ser Ile Gln Asp Trp Ala Lys Arg Met Lys Ala Leu Val Glu Gln
    2570                2575                2580

Gly Phe Thr Val Pro Glu Ile Lys Thr Ile Leu Gly Thr Met Pro
    2585                2590                2595

Ala Phe Glu Val Ser Leu Gln Ala Leu Gln Lys Ala Thr Phe Gln
    2600                2605                2610

Thr Pro Asp Phe Ile Val Pro Leu Thr Asp Leu Arg Ile Pro Ser
    2615                2620                2625

Val Gln Ile Asn Phe Lys Asp Leu Lys Asn Ile Lys Ile Pro Ser
    2630                2635                2640

Arg Phe Ser Thr Pro Glu Phe Thr Ile Leu Asn Thr Phe His Ile

2645                2650                2655

Pro Ser Phe Thr Ile Asp Phe Val Glu Met Lys Val Lys Ile Ile
2660                2665                2670

Arg Thr Ile Asp Gln Met Leu Asn Ser Glu Leu Gln Trp Pro Val
2675                2680                2685

Pro Asp Ile Tyr Leu Arg Asp Leu Lys Val Glu Asp Ile Pro Leu
2690                2695                2700

Ala Arg Ile Thr Leu Pro Asp Phe Arg Leu Pro Glu Ile Ala Ile
2705                2710                2715

Pro Glu Phe Ile Ile Pro Thr Leu Asn Leu Asn Asp Phe Gln Val
2720                2725                2730

Pro Asp Leu His Ile Pro Glu Phe Gln Leu Pro His Ile Ser His
2735                2740                2745

Thr Ile Glu Val Pro Thr Phe Gly Lys Leu Tyr Ser Ile Leu Lys
2750                2755                2760

Ile Gln Ser Pro Leu Phe Thr Leu Asp Ala Asn Ala Asp Ile Gly
2765                2770                2775

Asn Gly Thr Thr Ser Ala Asn Glu Ala Gly Ile Ala Ala Ser Ile
2780                2785                2790

Thr Ala Lys Gly Glu Ser Lys Leu Glu Val Leu Asn Phe Asp Phe
2795                2800                2805

Gln Ala Asn Ala Gln Leu Ser Asn Pro Lys Ile Asn Pro Leu Ala
2810                2815                2820

Leu Lys Glu Ser Val Lys Phe Ser Ser Lys Tyr Leu Arg Thr Glu
2825                2830                2835

His Gly Ser Glu Met Leu Phe Phe Gly Asn Ala Ile Glu Gly Lys
2840                2845                2850

Ser Asn Thr Val Ala Ser Leu His Thr Glu Lys Asn Thr Leu Glu
2855                2860                2865

Leu Ser Asn Gly Val Ile Val Lys Ile Asn Asn Gln Leu Thr Leu
2870                2875                2880

Asp Ser Asn Thr Lys Tyr Phe His Lys Leu Asn Ile Pro Lys Leu
    2885                2890                2895

Asp Phe Ser Ser Gln Ala Asp Leu Arg Asn Glu Ile Lys Thr Leu
    2900                2905                2910

Leu Lys Ala Gly His Ile Ala Trp Thr Ser Ser Gly Lys Gly Ser
    2915                2920                2925

Trp Lys Trp Ala Cys Pro Arg Phe Ser Asp Glu Gly Thr His Glu
    2930                2935                2940

Ser Gln Ile Ser Phe Thr Ile Glu Gly Pro Leu Thr Ser Phe Gly
    2945                2950                2955

Leu Ser Asn Lys Ile Asn Ser Lys His Leu Arg Val Asn Gln Asn
    2960                2965                2970

Leu Val Tyr Glu Ser Gly Ser Leu Asn Phe Ser Lys Leu Glu Ile
    2975                2980                2985

Gln Ser Gln Val Asp Ser Gln His Val Gly His Ser Val Leu Thr
    2990                2995                3000

Ala Lys Gly Met Ala Leu Phe Gly Glu Gly Lys Ala Glu Phe Thr
    3005                3010                3015

Gly Arg His Asp Ala His Leu Asn Gly Lys Val Ile Gly Thr Leu
    3020                3025                3030

Lys Asn Ser Leu Phe Phe Ser Ala Gln Pro Phe Glu Ile Thr Ala
    3035                3040                3045

Ser Thr Asn Asn Glu Gly Asn Leu Lys Val Arg Phe Pro Leu Arg
    3050                3055                3060

Leu Thr Gly Lys Ile Asp Phe Leu Asn Asn Tyr Ala Leu Phe Leu
    3065                3070                3075

Ser Pro Ser Ala Gln Gln Ala Ser Trp Gln Val Ser Ala Arg Phe
    3080                3085                3090

Asn Gln Tyr Lys Tyr Asn Gln Asn Phe Ser Ala Gly Asn Asn Glu
    3095                3100                3105

Asn Ile Met Glu Ala His Val Gly Ile Asn Gly Glu Ala Asn Leu
    3110                3115                3120

Asp Phe Leu Asn Ile Pro Leu Thr Ile Pro Glu Met Arg Leu Pro
    3125                3130                3135

Tyr Thr Ile Ile Thr Thr Pro Pro Leu Lys Asp Phe Ser Leu Trp
    3140                3145                3150

Glu Lys Thr Gly Leu Lys Glu Phe Leu Lys Thr Thr Lys Gln Ser
    3155                3160                3165

Phe Asp Leu Ser Val Lys Ala Gln Tyr Lys Lys Asn Lys His Arg
    3170                3175                3180

His Ser Ile Thr Asn Pro Leu Ala Val Leu Cys Glu Phe Ile Ser
    3185                3190                3195

Gln Ser Ile Lys Ser Phe Asp Arg His Phe Glu Lys Asn Arg Asn
    3200                3205                3210

Asn Ala Leu Asp Phe Val Thr Lys Ser Tyr Asn Glu Thr Lys Ile
    3215                3220                3225

Lys Phe Asp Lys Tyr Lys Ala Glu Lys Ser His Asp Glu Leu Pro
    3230                3235                3240

Arg Thr Phe Gln Ile Pro Gly Tyr Thr Val Pro Val Val Asn Val
    3245                3250                3255

Glu Val Ser Pro Phe Thr Ile Glu Met Ser Ala Phe Gly Tyr Val
    3260                3265                3270

Phe Pro Lys Ala Val Ser Met Pro Ser Phe Ser Ile Leu Gly Ser
    3275                3280                3285

Asp Val Arg Val Pro Ser Tyr Thr Leu Ile Leu Pro Ser Leu Glu
    3290                3295                3300

Leu Pro Val Leu His Val Pro Arg Asn Leu Lys Leu Ser Leu Pro
    3305                3310                3315

Asp Phe Lys Glu Leu Cys Thr Ile Ser His Ile Phe Ile Pro Ala
    3320                3325                3330

Met Gly Asn Ile Thr Tyr Asp Phe Ser Phe Lys Ser Ser Val Ile
    3335                3340                3345

Thr Leu Asn Thr Asn Ala Glu Leu Phe Asn Gln Ser Asp Ile Val
    3350                3355                3360

Ala His Leu Leu Ser Ser Ser Ser Ser Val Ile Asp Ala Leu Gln
    3365                3370                3375

Tyr Lys Leu Glu Gly Thr Thr Arg Leu Thr Arg Lys Arg Gly Leu
    3380                3385                3390

Lys Leu Ala Thr Ala Leu Ser Leu Ser Asn Lys Phe Val Glu Gly
    3395                3400                3405

Ser His Asn Ser Thr Val Ser Leu Thr Thr Lys Asn Met Glu Val
    3410                3415                3420

Ser Val Ala Thr Thr Thr Lys Ala Gln Ile Pro Ile Leu Arg Met
    3425                3430                3435

Asn Phe Lys Gln Glu Leu Asn Gly Asn Thr Lys Ser Lys Pro Thr
    3440                3445                3450

Val Ser Ser Ser Met Glu Phe Lys Tyr Asp Phe Asn Ser Ser Met
    3455                3460                3465

Leu Tyr Ser Thr Ala Lys Gly Ala Val Asp His Lys Leu Ser Leu
    3470                3475                3480

Glu Ser Leu Thr Ser Tyr Phe Ser Ile Glu Ser Ser Thr Lys Gly
    3485                3490                3495

Asp Val Lys Gly Ser Val Leu Ser Arg Glu Tyr Ser Gly Thr Ile
    3500                3505                3510

Ala Ser Glu Ala Asn Thr Tyr Leu Asn Ser Lys Ser Thr Arg Ser
    3515                3520                3525

Ser Val Lys Leu Gln Gly Thr Ser Lys Ile Asp Asp Ile Trp Asn
    3530                3535                3540

Leu Glu Val Lys Glu Asn Phe Ala Gly Glu Ala Thr Leu Gln Arg
    3545                3550                3555

Ile Tyr Ser Leu Trp Glu His Ser Thr Lys Asn His Leu Gln Leu
    3560                3565                3570

Glu Gly Leu Phe Phe Thr Asn Gly Glu His Thr Ser Lys Ala Thr
    3575                3580                3585

Leu Glu Leu Ser Pro Trp Gln Met Ser Ala Leu Val Gln Val His

3590                3595                3600

Ala Ser Gln Pro Ser Ser Phe His Asp Phe Pro Asp Leu Gly Gln
    3605                3610                3615

Glu Val Ala Leu Asn Ala Asn Thr Lys Asn Gln Lys Ile Arg Trp
    3620                3625                3630

Lys Asn Glu Val Arg Ile His Ser Gly Ser Phe Gln Ser Gln Val
    3635                3640                3645

Glu Leu Ser Asn Asp Gln Glu Lys Ala His Leu Asp Ile Ala Gly
    3650                3655                3660

Ser Leu Glu Gly His Leu Arg Phe Leu Lys Asn Ile Ile Leu Pro
    3665                3670                3675

Val Tyr Asp Lys Ser Leu Trp Asp Phe Leu Lys Leu Asp Val Thr
    3680                3685                3690

Thr Ser Ile Gly Arg Arg Gln His Leu Arg Val Ser Thr Ala Phe
    3695                3700                3705

Val Tyr Thr Lys Asn Pro Asn Gly Tyr Ser Phe Ser Ile Pro Val
    3710                3715                3720

Lys Val Leu Ala Asp Lys Phe Ile Ile Pro Gly Leu Lys Leu Asn
    3725                3730                3735

Asp Leu Asn Ser Val Leu Val Met Pro Thr Phe His Val Pro Phe
    3740                3745                3750

Thr Asp Leu Gln Val Pro Ser Cys Lys Leu Asp Phe Arg Glu Ile
    3755                3760                3765

Gln Ile Tyr Lys Lys Leu Arg Thr Ser Ser Phe Ala Leu Asn Leu
    3770                3775                3780

Pro Thr Leu Pro Glu Val Lys Phe Pro Glu Val Asp Val Leu Thr
    3785                3790                3795

Lys Tyr Ser Gln Pro Glu Asp Ser Leu Ile Pro Phe Phe Glu Ile
    3800                3805                3810

Thr Val Pro Glu Ser Gln Leu Thr Val Ser Gln Phe Thr Leu Pro
    3815                3820                3825

Lys Ser Val Ser Asp Gly Ile Ala Ala Leu Asp Leu Asn Ala Val
    3830                3835                3840

Ala Asn Lys Ile Ala Asp Phe Glu Leu Pro Thr Ile Ile Val Pro
    3845                3850                3855

Glu Gln Thr Ile Glu Ile Pro Ser Ile Lys Phe Ser Val Pro Ala
    3860                3865                3870

Gly Ile Val Ile Pro Ser Phe Gln Ala Leu Thr Ala Arg Phe Glu
    3875                3880                3885

Val Asp Ser Pro Val Tyr Asn Ala Thr Trp Ser Ala Ser Leu Lys
    3890                3895                3900

Asn Lys Ala Asp Tyr Val Glu Thr Val Leu Asp Ser Thr Cys Ser
    3905                3910                3915

Ser Thr Val Gln Phe Leu Glu Tyr Glu Leu Asn Val Leu Gly Thr
    3920                3925                3930

His Lys Ile Glu Asp Gly Thr Leu Ala Ser Lys Thr Lys Gly Thr
    3935                3940                3945

Phe Ala His Arg Asp Phe Ser Ala Glu Tyr Glu Glu Asp Gly Lys
    3950                3955                3960

Tyr Glu Gly Leu Gln Glu Trp Glu Gly Lys Ala His Leu Asn Ile
    3965                3970                3975

Lys Ser Pro Ala Phe Thr Asp Leu His Leu Arg Tyr Gln Lys Asp
    3980                3985                3990

Lys Lys Gly Ile Ser Thr Ser Ala Ala Ser Pro Ala Val Gly Thr
    3995                4000                4005

Val Gly Met Asp Met Asp Glu Asp Asp Asp Phe Ser Lys Trp Asn
    4010                4015                4020

Phe Tyr Tyr Ser Pro Gln Ser Ser Pro Asp Lys Lys Leu Thr Ile
    4025                4030                4035

Phe Lys Thr Glu Leu Arg Val Arg Glu Ser Asp Glu Glu Thr Gln
    4040                4045                4050

Ile Lys Val Asn Trp Glu Glu Glu Ala Ala Ser Gly Leu Leu Thr
    4055                4060                4065

Ser Leu Lys Asp Asn Val Pro Lys Ala Thr Gly Val Leu Tyr Asp
    4070                4075                4080

Tyr Val Asn Lys Tyr His Trp Glu His Thr Gly Leu Thr Leu Arg
    4085                4090                4095

Glu Val Ser Ser Lys Leu Arg Arg Asn Leu Gln Asn Asn Ala Glu
    4100                4105                4110

Trp Val Tyr Gln Gly Ala Ile Arg Gln Ile Asp Asp Ile Asp Val
    4115                4120                4125

Arg Phe Gln Lys Ala Ala Ser Gly Thr Thr Gly Thr Tyr Gln Glu
    4130                4135                4140

Trp Lys Asp Lys Ala Gln Asn Leu Tyr Gln Glu Leu Leu Thr Gln
    4145                4150                4155

Glu Gly Gln Ala Ser Phe Gln Gly Leu Lys Asp Asn Val Phe Asp
    4160                4165                4170

Gly Leu Val Arg Val Thr Gln Glu Phe His Met Lys Val Lys His
    4175                4180                4185

Leu Ile Asp Ser Leu Ile Asp Phe Leu Asn Phe Pro Arg Phe Gln
    4190                4195                4200

Phe Pro Gly Lys Pro Gly Ile Tyr Thr Arg Glu Glu Leu Cys Thr
    4205                4210                4215

Met Phe Ile Arg Glu Val Gly Thr Val Leu Ser Gln Val Tyr Ser
    4220                4225                4230

Lys Val His Asn Gly Ser Glu Ile Leu Phe Ser Tyr Phe Gln Asp
    4235                4240                4245

Leu Val Ile Thr Leu Pro Phe Glu Leu Arg Lys His Lys Leu Ile
    4250                4255                4260

Asp Val Ile Ser Met Tyr Arg Glu Leu Leu Lys Asp Leu Ser Lys
    4265                4270                4275

Glu Ala Gln Glu Val Phe Lys Ala Ile Gln Ser Leu Lys Thr Thr
    4280                4285                4290

Glu Val Leu Arg Asn Leu Gln Asp Leu Leu Gln Phe Ile Phe Gln
    4295                4300                4305

Leu Ile Glu Asp Asn Ile Lys Gln Leu Lys Glu Met Lys Phe Thr
    4310                4315                4320

Tyr Leu Ile Asn Tyr Ile Gln Asp Glu Ile Asn Thr Ile Phe Ser
    4325                4330                4335

Asp Tyr Ile Pro Tyr Val Phe Lys Leu Leu Lys Glu Asn Leu Cys
    4340                4345                4350

Leu Asn Leu His Lys Phe Asn Glu Phe Ile Gln Asn Glu Leu Gln
    4355                4360                4365

Glu Ala Ser Gln Glu Leu Gln Gln Ile His Gln Tyr Ile Met Ala
    4370                4375                4380

Leu Arg Glu Glu Tyr Phe Asp Pro Ser Ile Val Gly Trp Thr Val
    4385                4390                4395

Lys Tyr Tyr Glu Leu Glu Glu Lys Ile Val Ser Leu Ile Lys Asn
    4400                4405                4410

Leu Leu Val Ala Leu Lys Asp Phe His Ser Glu Tyr Ile Val Ser
    4415                4420                4425

Ala Ser Asn Phe Thr Ser Gln Leu Ser Ser Gln Val Glu Gln Phe
    4430                4435                4440

Leu His Arg Asn Ile Gln Glu Tyr Leu Ser Ile Leu Thr Asp Pro
    4445                4450                4455

Asp Gly Lys Gly Lys Glu Lys Ile Ala Glu Leu Ser Ala Thr Ala
    4460                4465                4470

Gln Glu Ile Ile Lys Ser Gln Ala Ile Ala Thr Lys Lys Ile Ile
    4475                4480                4485

Ser Asp Tyr His Gln Gln Phe Arg Tyr Lys Leu Gln Asp Phe Ser
    4490                4495                4500

Asp Gln Leu Ser Asp Tyr Tyr Glu Lys Phe Ile Ala Glu Ser Lys
    4505                4510                4515

Arg Leu Ile Asp Leu Ser Ile Gln Asn Tyr His Thr Phe Leu Ile
    4520                4525                4530

Tyr Ile Thr Glu Leu Leu Lys Lys Leu Gln Ser Thr Thr Val Met

4535                4540                4545

Asn Pro Tyr Met Lys Leu Ala Pro Gly Glu Leu Thr Ile Ile Leu
    4550                4555                4560

<210>  19
<211>  1957
<212>  DNA
<213>  Homo sapiens

<400>  19
attcccaccg ggacctgcgg ggctgagtgc ccttctcggt tgctgccgct gaggagcccg      60

cccagccagc cagggccgcg aggccgaggc caggccgcag cccaggagcc gccccaccgc     120

agctggcgat ggacccgccg aggcccgcgc tgctggcgct gctggcgctg cctgcgctgc     180

tgctgctgct gctggcgggc gccagggccg aagaggaaat gctggaaaat gtcagcctgg     240

tctgtccaaa agatgcgacc cgattcaagc acctccggaa gtacacatac aactatgagg     300

ctgagagttc cagtggagtc cctgggactg ctgattcaag aagtgccacc aggatcaact     360

gcaaggttga gctggaggtt ccccagctct gcagcttcat cctgaagacc agccagtgca     420

ccctgaaaga ggtgtatggc ttcaaccctg agggcaaagc cttgctgaag aaaaccaaga     480

actctgagga gtttgctgca gccatgtcca ggtatgagct caagctggcc attccagaag     540

ggaagcaggt tttcctttac ccggagaaag atgaacctac ttacatcctg aacatcaaga     600

ggggcatcat ttctgccctc ctggttcccc cagagacaga agaagccaag caagtgttgt     660

ttctggatac cgtgtatgga aactgctcca ctcactttac cgtcaagacg aggaagggca     720

atgtggcaac agaaatatcc actgaaagag acctggggca gtgtgatcgc ttcaagccca     780

tccgcacagg catcagccca cttgctctca tcaaaggcat gacccgcccc ttgtcaactc     840

tgatcagcag cagccagtcc tgtcagtaca cactggacgc taagaggaag catgtggcag     900

aagccatctg caaggagcaa cacctcttcc tgcctttctc ctacaagaat aagtatggga     960

tggtagcaca agtgacacag actttgaaac ttgaagacac accaaagatc aacagccgct    1020

tctttggtga aggtactaag aagatgggcc tcgcatttga gagcaccaaa tccacatcac    1080

ctccaaagca ggccgaagct gttttgaaga ctctccagga actgaaaaaa ctaaccatct    1140

ctgagcaaaa tatccagaga gctaatctct tcaataagct ggttactgag ctgagaggcc    1200

tcagtgatga agcagtcaca tctctcttgc cacagctgat tgaggtgtcc agccccatca    1260

ctttacaagc cttggttcag tgtggacagc ctcagtgctc cactcacatc ctccagtggc    1320

tgaaacgtgt gcatgccaac ccccttctga tagatgtggt cacctacctg gtggccctga    1380

tccccgagcc ctcagcacag cagctgcgag agatcttcaa catggcgagg gatcagcgca    1440

gccgagccac cttgtatgcg ctgagccacg cggtcaacaa ctatcataag acaaacccta    1500

cagggaccca ggagctgctg gacattgcta attacctgat ggaacagatt caagatgact 1560

gcactgggga tgaagattac acctatttga ttctgcgggt cattggaaat atgggccaaa 1620

ccatggagca gttaactcca gaactcaagt cttcaatcct gaaatgtgtc caaagtacaa 1680

agccatcact gatgatccag aaagctgcca tccaggctct gcggaaaatg gagcctaaag 1740

acaaggacca ggaggttctt cttcagactt tccttgatga tgcttctccg ggagataagc 1800

gactggctgc ctatcttatg ttgatgagga gtccttcaca ggcagatatt aacaaaattg 1860

tccaaattct accatgggaa cagaatgagc aagtgaagaa ctttgtggct tcccatattg 1920

ccaatatctt gaactcagaa gaattggata tccaaga 1957

<210> 20
<211> 381
<212> PRT
<213> Homo sapiens

<220>
<221> MOD_RES
<222> (1)..(381)
<223> X= Selenocysteine

<400> 20

Met Trp Arg Ser Leu Gly Leu Ala Leu Ala Leu Cys Leu Leu Pro Ser
1               5                   10                  15

Gly Gly Thr Glu Ser Gln Asp Gln Ser Ser Leu Cys Lys Gln Pro Pro
            20                  25                  30

Ala Trp Ser Ile Arg Asp Gln Asp Pro Met Leu Asn Ser Asn Gly Ser
        35                  40                  45

Val Thr Val Val Ala Leu Leu Gln Ala Ser Xaa Tyr Leu Cys Ile Leu
    50                  55                  60

Gln Ala Ser Lys Leu Glu Asp Leu Arg Val Lys Leu Lys Lys Glu Gly
65                  70                  75                  80

Tyr Ser Asn Ile Ser Tyr Ile Val Val Asn His Gln Gly Ile Ser Ser
                85                  90                  95

Arg Leu Lys Tyr Thr His Leu Lys Asn Lys Val Ser Glu His Ile Pro
            100                 105                 110

Val Tyr Gln Gln Glu Glu Asn Gln Thr Asp Val Trp Thr Leu Leu Asn
        115                 120                 125

Gly Ser Lys Asp Asp Phe Leu Ile Tyr Asp Arg Cys Gly Arg Leu Val

130                 135                 140

Tyr His Leu Gly Leu Pro Phe Ser Phe Leu Thr Phe Pro Tyr Val Glu
145                 150                 155                 160

Glu Ala Ile Lys Ile Ala Tyr Cys Glu Lys Lys Cys Gly Asn Cys Ser
                165                 170                 175

Leu Thr Thr Leu Lys Asp Glu Asp Phe Cys Lys Arg Val Ser Leu Ala
            180                 185                 190

Thr Val Asp Lys Thr Val Glu Thr Pro Ser Pro His Tyr His His Glu
        195                 200                 205

His His His Asn His Gly His Gln His Leu Gly Ser Ser Glu Leu Ser
    210                 215                 220

Glu Asn Gln Gln Pro Gly Ala Pro Asn Ala Pro Thr His Pro Ala Pro
225                 230                 235                 240

Pro Gly Leu His His His His Lys His Lys Gly Gln His Arg Gln Gly
                245                 250                 255

His Pro Glu Asn Arg Asp Met Pro Ala Ser Glu Asp Leu Gln Asp Leu
            260                 265                 270

Gln Lys Lys Leu Cys Arg Lys Arg Cys Ile Asn Gln Leu Leu Cys Lys
        275                 280                 285

Leu Pro Thr Asp Ser Glu Leu Ala Pro Arg Ser Xaa Cys Cys His Cys
    290                 295                 300

Arg His Leu Ile Phe Glu Lys Thr Gly Ser Ala Ile Thr Xaa Gln Cys
305                 310                 315                 320

Lys Glu Asn Leu Pro Ser Leu Cys Ser Xaa Gln Gly Leu Arg Ala Glu
                325                 330                 335

Glu Asn Ile Thr Glu Ser Cys Gln Xaa Arg Leu Pro Pro Ala Ala Xaa
            340                 345                 350

Gln Ile Ser Gln Gln Leu Ile Pro Thr Glu Ala Ser Ala Ser Xaa Arg
        355                 360                 365

Xaa Lys Asn Gln Ala Lys Lys Xaa Glu Xaa Pro Ser Asn
    370                 375                 380

<210>  21
<211>  2204
<212>  DNA
<213>  Homo sapiens

<400>  21
acagacaggc aggtgcagga gacggggtga gcgcttttgg gctctagccc catggcagca      60

tctaggggtg tgttacaatt aatgctcttt tagcagttgc tgttcgcgga tggctaagtg     120

ttaaaccagc tcagtggaga gtcagggtgg cagcctttta cgccctgtcc tcttggtacc     180

cgggtctttg tctagcatcc aggaagaatc aggtcctaca gacttgaagg atggtgaata     240

cagagatttt attgaatgat ggaggaggct ttcagcagga tggatgggga gctggaaagc     300

ggatggagtg agaagataat cttcccctgg ggtttggcca tcccatggct gatctctctg     360

acagtcccca gccgaactcc tctcgatgtt cagacactct ttctcctctc tcctctgcca     420

cactgctctg ctgctcttct gctcatggag cctgaggaca accccagcaa tgtggagaag     480

cctggggctt gccctggctc tctgtctcct cccatcggga ggaacagaga gccaggacca     540

aagctcctta tgtaagcaac ccccagcctg gagcataaga gatcaagatc caatgctaaa     600

ctccaatggt tcagtgactg tggttgctct tcttcaagcc agctgatacc tgtgcatact     660

gcaggcatct aaattagaag acctgcgagt aaaactgaag aaagaaggat attctaatat     720

ttcttatatt gttgttaatc atcaaggaat ctcttctcga ttaaaataca cacatcttaa     780

gaataaggtt tcagagcata ttcctgttta tcaacaagaa gaaaaccaaa cagatgtctg     840

gactctttta aatggaagca aagatgactt cctcatatat gatagatgtg gccgtcttgt     900

atatcatctt ggtttgcctt tttccttcct aactttccca tatgtagaag aagccattaa     960

gattgcttac tgtgaaaaga aatgtggaaa ctgctctctc acgactctca aagatgaaga    1020

cttttgtaaa cgtgtatctt tggctactgt ggataaaaca gttgaaactc catcgcctca    1080

ttaccatcat gagcatcatc acaatcatgg acatcagcac cttggcagca gtgagctttc    1140

agagaatcag caaccaggag caccaaatgc tcctactcat cctgctcctc caggccttca    1200

tcaccaccat aagcacaagg gtcagcatag gcagggtcac ccagagaacc gagatatgcc    1260

agcaagtgaa gatttacaag atttacaaaa gaagctctgt cgaaagagat gtataaatca    1320

attactctgt aaattgccca cagattcaga gttggctcct aggagctgat gctgccattg    1380

tcgacatctg atatttgaaa aaacagggtc tgcaatcacc tgacagtgta aagaaaacct    1440

cccatcttta tgtagctgac agggacttcg ggcagaggag aacataactg aatcttgtca    1500

gtgacgtttg cctccagctg cctgacaaat aagtcagcag cttataccca cagaagccag    1560

tgccagttga cgctgaaaga atcaggcaaa aaagtgagaa tgaccttcaa actaaatatt    1620

taaaatagga catactcccc aatttagtct agacacaatt tcatttccag catttttata    1680

aactaccaaa ttagtgaacc aaaaatagaa attagatttg tgcaaacatg gagaaatcta    1740

ctgaattggc ttccagattt taaattttat gtcatagaaa tattgactca aaccatattt 1800

tttatgatgg agcaactgaa aggtgattgc agcttttggt taatatgtct ttttttttct 1860

ttttccagtg ttctatttgc tttaatgaga atagaaacgt aaactatgac ctaggggttt 1920

ctgttggata attagcagtt tagaatggag gaagaacaac aaagacatgc tttccatttt 1980

tttctttact tatctctcaa aacaatatta ctttgtcttt tcaatcttct acttttaact 2040

aataaaataa gtggattttg tattttaaga tccagaaata cttaacacgt gaatattttg 2100

ctaaaaaagc atatataact attttaaata tccatttatc ttttgtatat ctaagactca 2160

tcctgatttt tactatcaca catgaataaa gcctttgtat cttt 2204

<210> 22
<211> 94
<212> PRT
<213> Homo sapiens

<400> 22

Met Lys Arg Val Leu Ser Cys Val Pro Glu Pro Thr Val Val Met Ala
1               5                   10                  15

Ala Arg Ala Leu Cys Met Leu Gly Leu Val Leu Ala Leu Leu Ser Ser
            20                  25                  30

Ser Ser Ala Glu Glu Tyr Val Gly Leu Ser Ala Asn Gln Cys Ala Val
        35                  40                  45

Pro Ala Lys Asp Arg Val Asp Cys Gly Tyr Pro His Val Thr Pro Lys
    50                  55                  60

Glu Cys Asn Asn Arg Gly Cys Cys Phe Asp Ser Arg Ile Pro Gly Val
65                  70                  75                  80

Pro Trp Cys Phe Lys Pro Leu Gln Glu Ala Glu Cys Thr Phe
                85                  90

<210> 23
<211> 1109
<212> DNA
<213> Homo sapiens

<400> 23
cgctccccag tagaggaccc ggaaccagaa ctggaatccg cccttaccgc ttgctgccaa 60

aacagtgggg gctgaactga cctctcccct ttgggagaga aaaactgtct gggagcttga 120

caaaggcatg caggagagaa caggagcagc cacagccagg agggagagcc ttccccaagc 180

aaacaatcca gagcagctgt gcaaacaacg gtgcataaat gaggcctcct ggaccatgaa 240

gcgagtcctg agctgcgtcc cggagcccac ggtggtcatg gctgccagag cgctctgcat 300

gctggggctg gtcctggcct tgctgtcctc cagctctgct gaggagtacg tgggcctgtc 360

tgcaaaccag tgtgccgtgc cagccaagga cagggtggac tgcggctacc cccatgtcac 420

ccccaaggag tgcaacaacc ggggctgctg ctttgactcc aggatccctg gagtgccttg 480

gtgtttcaag cccctgcagg aagcagaatg caccttctga ggcacctcca gctgcccccg 540

gccgggggat gcgaggctcg gagcaccctt gcccggctgt gattgctgcc aggcactgtt 600

catctcagct tttctgtccc tttgctcccg gcaagcgctt ctgctgaaag ttcatatctg 660

gagcctgatg tcttaacgaa taaaggtccc atgctccacc cgaggacagt tcttcgtgcc 720

tgagactttc tgaggttgtg ctttatttct gctgcgtcgt gggagagggc gggagggtgt 780

caggggagag tctgcccagg cctcaagggc aggaaaagac tccctaagga gctgcagtgc 840

atgcaaggat attttgaatc cagactggca cccacgtcac aggaaagcct aggaacactg 900

taagtgccgc ttcctcggga aagcagaaaa aatacatttc aggtagaagt tttcaaaaat 960

cacaagtctt tcttggtgaa gacagcaagc caataaaact gtcttccaaa gtggtccttt 1020

atttcacaac cactctcgct actgttcaat acttgtacta ttcctgggtt ttgtttcttt 1080

gtacagtaaa cattatgaac aaacaggca 1109

<210> 24
<211> 212
<212> PRT
<213> Homo sapiens

<400> 24

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu

            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210

<210> 25
<211> 1526
<212> DNA
<213> Homo sapiens

<400> 25
   aggtaacacc atgtttggta aataagtgtt ttggtgttgt gcaagggtct ggtttcagcc 60
   tgaagccatc tcagagctgt ctgggtctct ggagactgga gggacaacct agtctagagc 120
   ccatttgcat gagaccaagg atcctcctgc aagagacacc atcctgaggg aagagggctt 180
   ctgaaccagc ttgacccaat aagaaattct tgggtgccga cgcggaagca gattcagagc 240
   ctagagccgt gcctgcgtcc gtagtttcct tctagcttct tttgatttca aatcaagact 300
   tacagggaga gggagcgata aacacaaact ctgcaagatg ccacaaggtc ctcctttgac 360
   atccccaaca aagaggactg gagatgtctg aggctcattc tgccctcgag cccaccggga 420
   acgaaagaga agctctatct cccctccagg agcccagcta tgaactcctt ctccacaagc 480
   gccttcggtc cagttgcctt ctccctgggg ctgctcctgg tgttgcctgc tgccttccct 540
   gccccagtac ccccaggaga agattccaaa gatgtagccg ccccacacag acagccactc 600
   acctcttcag aacgaattga caaacaaatt cggtacatcc tcgacggcat ctcagccctg 660
   agaaaggaga catgtaacaa gagtaacatg tgtgaaagca gcaaagaggc actggcagaa 720
   aacaacctga accttccaaa gatggctgaa aaagatggat gcttccaatc tggattcaat 780
   gaggagactt gcctggtgaa aatcatcact ggtcttttgg agtttgaggt atacctagag 840
   tacctccaga acagatttga gagtagtgag gaacaagcca gagctgtgca gatgagtaca 900
   aaagtcctga tccagttcct gcagaaaaag gcaaagaatc tagatgcaat aaccacccct 960
   gacccaacca caaatgccag cctgctgacg aagctgcagg cacagaacca gtggctgcag 1020
   gacatgacaa ctcatctcat tctgcgcagc tttaaggagt tcctgcagtc cagcctgagg 1080
   gctcttcggc aaatgtagca tgggcacctc agattgttgt tgttaatggg cattccttct 1140
   tctggtcaga aacctgtcca ctgggcacag aacttatgtt gttctctatg gagaactaaa 1200
   agtatgagcg ttaggacact attttaatta tttttaattt attaatattt aaatatgtga 1260
   agctgagtta atttatgtaa gtcatattta tatttttaag aagtaccact tgaaacattt 1320
   tatgtattag ttttgaaata ataatggaaa gtggctatgc agtttgaata tcctttgttt 1380
   cagagccaga tcatttcttg gaaagtgtag gcttacctca aataaatggc taacttatac 1440
   atatttttaa agaaatattt atattgtatt tatataatgt ataaatggtt tttataccaa 1500
   taaatggcat tttaaaaaat tcagca 1526

<210> 26
<211> 383
<212> PRT
<213> Homo sapiens

<400> 26

Met Gly Val Lys Ala Ser Gln Thr Gly Phe Val Val Leu Val Leu Leu
1               5                   10                  15

Gln Cys Cys Ser Ala Tyr Lys Leu Val Cys Tyr Tyr Thr Ser Trp Ser
            20                  25                  30

Gln Tyr Arg Glu Gly Asp Gly Ser Cys Phe Pro Asp Ala Leu Asp Arg
        35                  40                  45

Phe Leu Cys Thr His Ile Ile Tyr Ser Phe Ala Asn Ile Ser Asn Asp
    50                  55                  60

His Ile Asp Thr Trp Glu Trp Asn Asp Val Thr Leu Tyr Gly Met Leu
65                  70                  75                  80

Asn Thr Leu Lys Asn Arg Asn Pro Asn Leu Lys Thr Leu Leu Ser Val
                85                  90                  95

Gly Gly Trp Asn Phe Gly Ser Gln Arg Phe Ser Lys Ile Ala Ser Asn
            100                 105                 110

Thr Gln Ser Arg Arg Thr Phe Ile Lys Ser Val Pro Pro Phe Leu Arg

        115                 120                 125

Thr His Gly Phe Asp Gly Leu Asp Leu Ala Trp Leu Tyr Pro Gly Arg
    130                 135                 140

Arg Asp Lys Gln His Phe Thr Thr Leu Ile Lys Glu Met Lys Ala Glu
145                 150                 155                 160

Phe Ile Lys Glu Ala Gln Pro Gly Lys Lys Gln Leu Leu Leu Ser Ala
                165                 170                 175

Ala Leu Ser Ala Gly Lys Val Thr Ile Asp Ser Ser Tyr Asp Ile Ala
            180                 185                 190

Lys Ile Ser Gln His Leu Asp Phe Ile Ser Ile Met Thr Tyr Asp Phe
        195                 200                 205

His Gly Ala Trp Arg Gly Thr Thr Gly His His Ser Pro Leu Phe Arg
    210                 215                 220

Gly Gln Glu Asp Ala Ser Pro Asp Arg Phe Ser Asn Thr Asp Tyr Ala
225                 230                 235                 240

Val Gly Tyr Met Leu Arg Leu Gly Ala Pro Ala Ser Lys Leu Val Met
                245                 250                 255

Gly Ile Pro Thr Phe Gly Arg Ser Phe Thr Leu Ala Ser Ser Glu Thr
            260                 265                 270

Gly Val Gly Ala Pro Ile Ser Gly Pro Gly Ile Pro Gly Arg Phe Thr
        275                 280                 285

Lys Glu Ala Gly Thr Leu Ala Tyr Tyr Glu Ile Cys Asp Phe Leu Arg
    290                 295                 300

Gly Ala Thr Val His Arg Ile Leu Gly Gln Gln Val Pro Tyr Ala Thr
305                 310                 315                 320

Lys Gly Asn Gln Trp Val Gly Tyr Asp Asp Gln Glu Ser Val Lys Ser
                325                 330                 335

Lys Val Gln Tyr Leu Lys Asp Arg Gln Leu Ala Gly Ala Met Val Trp
            340                 345                 350

Ala Leu Asp Leu Asp Asp Phe Gln Gly Ser Phe Cys Gly Gln Asp Leu
        355                 360                 365

Arg Phe Pro Leu Thr Asn Ala Ile Lys Asp Ala Leu Ala Ala Thr
    370                 375                 380

<210>  27
<211>  16143
<212>  DNA
<213>  Homo sapiens

<400>  27
gaggccctgt ctaggtagct ggcaccagga gccgtgggca agggaagagg ccacaccctg      60

ccctgctctg ctgcagccag aatgggtgtg aaggcgtctc aaacaggctt tgtggtcctg     120

gtgctgctcc agtgctgctc tgcatacaaa ctggtctgct actacaccag ctggtcccag     180

taccgggaag gcgatgggag ctgcttccca gatgcccttg accgcttcct ctgtacccac     240

atcatctaca gctttgccaa tataagcaac gatcacatcg acacctggga gtggaatgat     300

gtgacgctct acggcatgct caacacactc aagaacagga accccaacct gaagactctc     360

ttgtctgtcg gaggatggaa ctttgggtct caaagatttt ccaagatagc ctccaacacc     420

cagagtcgcc ggactttcat caagtcagta ccgccatttc tgcgcaccca tggctttgat     480

gggctggacc ttgcctggct ctaccctgga cggagagaca aacagcattt taccacccta     540

atcaaggaaa tgaaggccga atttataaag gaagcccagc cagggaaaaa gcagctcctg     600

ctcagcgcag cactgtctgc ggggaaggtc accattgaca gcagctatga cattgccaag     660

atatcccaac acctggattt cattagcatc atgacctacg attttcatgg agcctggcgt     720

gggaccacag gccatcacag tcccctgttc cgaggtcagg aggatgcaag tcctgacaga     780

ttcagcaaca ctgactatgc tgtggggtac atgttgaggc tgggggctcc tgccagtaag     840

ctggtgatgg gcatccccac cttcgggagg agcttcactc tggcttcttc tgagactggt     900

gttggagccc caatctcagg accgggaatt ccaggccggt tcaccaagga ggcagggacc     960

cttgcctact atgagatctg tgacttcctc cgcggagcca cagtccatag aatcctcggc    1020

cagcaggtcc cctatgccac caagggcaac cagtgggtag gatacgacga ccaggaaagc    1080

gtcaaaagca aggtgcagta cctgaaggac aggcagctgg cgggcgccat ggtatgggcc    1140

ctggacctgg atgacttcca gggctccttc tgtggccagg atctgcgctt ccctctcacc    1200

aatgccatca aggatgcact cgctgcaacg tagccctctg ttctgcacac agcacggggg    1260

ccaaggatgc cccgtccccc tctggctcca gctggccggg agcctgatca cctgccctgc    1320

tgagtcccag gctgagcctc agtctccctc ccttggggcc tatgcagagg tccacaacac    1380

acagatttga gctcagccct ggtgggcaga gaggtaggga tggggctgtg gggatagtga    1440

ggcatcgcaa tgtaagactc gggattagta cacacttgtt gattaatgga aatgtttaca    1500

gatccccaag cctggcaagg gaatttcttc aactccctgc cccccagccc tccttatcaa    1560

aggacaccat tttggcaagc tctatcacca aggagccaaa catcctacaa gacacagtga    1620

ccatactaat tataccccct gcaaagccca gcttgaaacc ttcacttagg aacgtaatcg 1680

tgtcccctat cctacttccc cttcctaatt ccacagctgc tcaataaagt acaagagctt 1740

aacagtggta tctgggctag ccaaggttaa tccatcagag ttgtgggttt tcaggcccag 1800

acagcccgca gagccatctg cctgctgggt gagggactaa gggagtgggc agagggggag 1860

gagaagcaga gccaggggag ggactgaggc tgcaaccagg aggtgggggt gggggaggtg 1920

ggtctcagtt gcttggggga gggagcaggg cggaagggca ggatgcactt gcaggggtct 1980

catcctggat ttctcttcag gtgagtaatc cctccacctc cacttttaag tccagaggcg 2040

tggcgagggc acagggcagg tgtggaggag gtcttagctc caagggaaca ctttgccagg 2100

ttcttctgtg cttccaatga cttcgaaata gtcacgtttg ctaaactgga gtgaggagta 2160

tatggatgtt tattttgcta tactctttgg tatatgtgaa aattctcaaa ataagaaact 2220

ttaaaaggtc ctggttcagt gaaggctttg actaacagcc cctgggagcc gctagtacag 2280

ttgcccagta gctgcttgga tgagtgatgc ccacattgtt tgcagcagga gaaacagaat 2340

tagaggcgag gaaggatttc ctggtccatt gtgatattgt gccccagcct gggggacatg 2400

ccgagggagc acaggactgc cattccgggt gggttacaag ttagaggcac tctcaccagg 2460

aggcagagaa aggtgggcca gagtccctca tggatgaagg acctcactgc aggtcactaa 2520

aggtgccagc ctgaaagcca gggccatctg tgacacaggc tatcttgggc cttcccctcc 2580

cacaaagcca ctgcttccag gaagcctgct gtgctggcac caagtcccat ttcatcctta 2640

ttccccagct cctacccttc ccccacacag tgcctgtagc gtattcatcc cctgcattgg 2700

atcttttcta agcattttca gaataggcct tattttcacc agtcagggaa ctccccagag 2760

gcagggggac ctgaaccagc tcctttcatt taagaattct gactttgctc acaatctgca 2820

ccccactcct ccctacccaa ccccacaccc cattggtgct cagctctggg ctcagcctga 2880

ggtctcttgc cgaatcctgg cccgggcccc atcccagact tctccttcag gttgggccag 2940

ggctggccag gagaggaggg accagctggg ctggtcccag ggcagggcaa ccccacccag 3000

gaggaagggc gaggccccac cctgctcacc tatgtttgga gagttagagg ggcttggctt 3060

aaatgggtgg aggggatgac tgtaggtgct gggcaatatt tggggtgatg agagactgta 3120

gacggaagcg cagctgagcc cctctgggag aggggaaccc tctcctgccc ctaacgtgct 3180

ctccagggag catggaaaat gagcaagact ctactttgcc atactctgtc ttctccactg 3240

gggaaaacaa aaatgggcag cagacctcag agtaactccc atgaagcctg tgacccctca 3300

gagatccacc actatgaatc tctatgggat tcccatgatg tcctatggga ggacatcagc 3360

ggcctaaccc agcctctcac ccaaagcagg aaaccttcta tggcctctca gacatggggc 3420

cacccagtgt catgacaatg tcattccact cctgccctcc ccacctccct gtgctccaca 3480

ggtaggagcc tctccccagg ggcaggacgg cagaggatga tggcatagga gtgaggagct 3540

tgggtctccc gcatccactg tatggatgtt ccaggggctc cagactagat aggtacaagg 3600

cctacctgtt tgtcaagggc ctgcctgatg tgtagcaaag aaagcagagc ccagagagag 3660

agcaggactt gccaagagtc acacagcaag ttaacagtgg atctcccaat tccctgccaa 3720

tgctctattt actacctcac aggcccgaaa atatgggact tctggggcta ccaccattag 3780

ggctggaaag agagagatgg aaaccaatgg ggacattgag aagtggggag accctgggag 3840

gagtctttgg attaggtggg gttggagcag ggcttgaaat gggggtggtt atggccatcg 3900

ttggcaccca tgtgctgggt actctttctg tgtccagcac cctgtctccc catgatccca 3960

tgttgtcctc acaacagcaa ggtgagctgt tatatttgtc cccattttac agatgatgaa 4020

actgagactc aggttataac ctttcacagt ggctggctag taagtgatag agccagactt 4080

ggaaacctgg aatgcctggc tctaaggcac atgcatgcct ggaggcgacc cctgtcccaa 4140

tcatgccctc ccagagctgt gtggcctcag gatcccagct ctgcaggtcc tggaaacccc 4200

accagaggcc caaggcacct agcatatcag tgctgagcat gctacagggc tgattttggt 4260

cctaggtgct ggtcaggtca gggacaggga gggaggtggg caggcatgag ggaatggggt 4320

gggctagaag ccggcgtcag ctgctgtcct cctgaggaca ggtaaagagg gacttcagcc 4380

tcagggcagt gtcctgggac cctgtgccct gaagatctca catagcaagg aaggcttctt 4440

gtgaccatgg tgggaggtgg gaatggggtt ctaagaggtg gaaggttgtg actgagcaga 4500

gcacccactt ataactaccc acttagtgca ttgcccattg cccacccttc aatcccatac 4560

tgatgccacc catacccagc atgcactgtg tccagcagct ctcagctctg cctgggggca 4620

gcctaagtat ctccagttac caggggcaga aggagagtct aaacaaattg ttcacaatac 4680

cagcagcaat cactttaact ttgggatttc atgcaccagc tagaacaaag cacagaccga 4740

aaggcaaatg tctcccaaag tcatctgtgg gccaataggg gtcacccact gtccgatgct 4800

ccctccagga cagggagtaa ttgagtgctg atgggtgtgc atgggtttgg ggagaagatt 4860

agtcagtttt ttaggaaaag gatgggtgac ttgaggatgg gaccactgat gagccacttt 4920

atcacttcca ctaggcccca ggcaggttgg ggagtacagc aaatgggttg cgcagagacc 4980

tagtccgccc tcgatgagtc tacctctcat gccacttggg accctttctc tcacctgcct 5040

gtctcccatc taggtgagtc tcctgcccca ggcagcctcc ccagaacctc tgccaacact 5100

cctgtttccc cgccaccccc agccctagca ctccatgtag actgaggaca ctgggcttcc 5160

agtcccagtt cttccaaagc cttgatgtgt gaccttgagc aagtcacttc cctgccctgt 5220

gcctcagttt ccccatctgt caaatgaggc ataacaatcc ttgcctttcc tttttcacct 5280

gggctgttgg ctgagcagtt gagagagctg ctatgaaagt attttggaaa gggaagtgga 5340

aaaagctatt caaaccctcc agctattgca gagtattttc tccacaccag gcagagcaga 5400

gtgctgggcc ctggagaggc cacacagcag cctctttcta gagtgctggg gagtcctcaa 5460

ggctctctca tacacacagg cctccccctt tcctgttggc cccacgcctc atcctcaccc 5520

cacccctcat gcctgtgagg aacaaggaac agccaagcag cctcatctct cctgagagca 5580

gagccatggg tctcgggaaa cccaggagta aggaacaaag actcttcaaa atgacttcag 5640

agctttcttt aggatcccag ggaggtgtaa actcagtgct taattaaatg gattctttag 5700

agggtgggaa acaggtggat gtcaaccatt tgccccacat acccgtattc atgcaatcca 5760

cccccagtgg gctcaccggt gcgggtgtgc aaaacctcct cccaccccag tcatcttagg 5820

ttcaggtcat cctttggtcc tgctctttcc ccgccaggct gtctgttgat gctactttta 5880

atctgctttc actaagataa gcctggcaga agtggtgggg gtaaggtggg ctgtaggcca 5940

gctcccaaat gtgtgctggg cataacagaa gacccattct tgactgaagt gcccttgtgg 6000

gaccctgagc ccgtgccctg gagtggcaca gggaggtgtg ccagcaatgg ggaccgaggt 6060

cactggggac tgctggggtt cgggctagtg gcctgtctgg ctgctgtctg cttccctcgt 6120

tcacatcctg ctggagcctt aaataggagc ccaaaagctt ttctttctta cttttttttg 6180

agacaaaatc tcactctgtt gcccaggccg gagtgcagtg gcacaacctc tgccgcctgg 6240

gttcaagcga ttctcctgcc tcagcctccc gagtagctgg gattacaggt gcctgccacc 6300

atgcctggct aattttcgta gttagagtag agacggggtt tcaccatctt ggccaggctg 6360

gtcttgaact cctcacctca tgatccacct gcctcggcct cccaaagtgc tgggattcca 6420

ggtatgagcc accacgcccg gcctaaagct tttctattaa taatttcctg cctcaccctc 6480

catccccttc ctcctcaggt gagttcccag aaggagggag ggccaggagg gggccgcaag 6540

acctgccatc tgccagtgct cacacaccaa tctctctagc cctcagtaca gtcctgcaag 6600

aggggggtca tgaggcccat tccacagata aggaaactga ggcccaaagt ctgggcatgc 6660

tgcgttgctc tgggaaggtg atctgcaggg taaatggagt gagggcaggg ggccgaatgg 6720

ggagaggctg ggagccgagg aggtaggagt cattgtgccc tcagagccaa ccacctgatt 6780

tctgcatctg tcaaatagta atagcccctt cctatgcctc aaaggatttt tttcaagaat 6840

gaaactgtaa aattcacttt aaagtgacat gatccgttcc cggagggaca ggggaatccc 6900

cagtgcacca tacaccaata acccctgcta aggcagcagt attaattgct caaccttccg 6960

cacctgtgca ctaactgctc acgttgttcc caccctaccc cacccaggta atggagttgg 7020

ggagaaggta ggattccccc acaccaccag cagccctggg gaaggtgatt ccccaccacg 7080

ttcttgcttt ttctcctttg ggaataaaga aaatgtccgg ttgccccagc atgcctgagg 7140

aagtggaagg gagaggttag gacatttgtt gctgaaaatc tccagcaggt acaggcacat 7200

gggcctgcac cactaggcac ctgggatagc cctctggcta tggggctgag gtcttccttc 7260

cagcccagga aagagcagag gtcaagaggc agattttttg tttcactcta gcctctgcta 7320

ctctgtgtgg ccttgggcct gtcctcagtg tcaaccagca ggcctcacat ctctgtttaa 7380

atggaagaag ctaagcaggg ccaaggcagc caccatctct gggtcatttg cctctggttt 7440

gtataaactt gtgtgatgca tgcagcctgc agaccctgca gagagtgagg ctgcaggatg 7500

gagcaggagc taaaagagat ttggagagtg gcgtctcctg gtgacctgca aggtctcggc 7560

acgactcccc acactgcctt ttccctgtta tctgctcagg taggtttccc caaggccaca 7620

cctcaggaca aagagaaaga aggaggcccc gctcccagca gccagattcc tgtcccttgc 7680

actgaggtct gggctgggct cacagagcac atgtgccctg tacacactct gggtcaggga 7740

acccagccct gctcctctgg gcctcccctg ccagccctct gttctgcaca cagcacgggg 7800

gccaaggatg ccccgtcccc ctctggctcc agctggccgg gagcctgatc acctgccctg 7860

ctgagtccca ggctgagcct cagtctccct cccttggggc ctatgcagag gtccacaaca 7920

cacagatttg agctcagccc tggtgggcag agaggtaggg atggggctgt ggggatagtg 7980

aggcatcgca atgtaagact cgggattagt acacacttgt tgattaatgg aaatgtttac 8040

agatccccaa gcctggcaag ggaatttctt caactccctg ccccccagcc ctccttatca 8100

aaggacacca ttttggcaag ctctatcacc aaggagccaa acatcctaca agacacagtg 8160

accatactaa ttataccccc tgcaaagccc agcttgaaac cttcacttag gaacgtaatc 8220

gtgtccccta tcctacttcc ccttcctaat tccacagctg ctcaataaag tacaagagct 8280

taacagtgga ggccctgtct aggtagctgg caccaggagc cgtgggcaag ggaagaggcc 8340

acaccctgcc ctgctctgct gcagccagag aggccctgtc taggtagctg gcaccaggag 8400

ccgtgggcaa gggaagaggc cacaccctgc cctgctctgc tgcagccaga atgggtgtga 8460

aggcgtctca aacaggtatc tgggctagcc aaggttaatc catcagagtt gtgggttttc 8520

aggcccagac agcccgcaga gccatctgcc tgctgggtga gggactaagg gagtgggcag 8580

agggggagga gaagcagagc caggggaggg actgaggctg caaccaggag gtgggggtgg 8640

gggaggtggg tctcagttgc ttgggggagg gagcagggcg gaagggcagg atgcacttgc 8700

aggggtctca tcctggattt ctcttcaggc tttgtggtcc tggtgctgct ccagtgctgt 8760

gagtaatccc tccacctcca cttttaagtc cagaggcgtg gcgagggcac agggcaggtg 8820

tggaggaggt cttagctcca agggaacact ttgccaggtt cttctgtgct tccaatgact 8880

tcgaaatagt cacgtttgct aaactggagt gaggagtata tggatgttta ttttgctata 8940

ctctttggta tatgtgaaaa ttctcaaaat aagaaacttt aaaaggtcct ggttcagtga 9000

aggctttgac taacagcccc tgggagccgc tagtacagtt gcccagtagc tgcttggatg 9060

agtgatgccc acattgtttg cagcaggaga aacagaatta gaggcgagga aggatttcct 9120

ggtccattgt gatattgtgc cccagcctgg gggacatgcc gagggagcac aggactgcca 9180

ttccgggtgg gttacaagtt agaggcactc tcaccaggag gcagagaaag gtgggccaga 9240

gtccctcatg gatgaaggac ctcactgcag gtcactaaag gtgccagcct gaaagccagg 9300

gccatctgtg acacaggcta tcttgggcct tcccctccca caaagccact gcttccagga 9360

agcctgctgt gctggcacca agtcccattt catccttatt ccccagctcc tacccttccc 9420

ccacacagtg cctgtagcgt attcatcccc tgcattggat cttttctaag cattttcaga 9480

ataggcctta ttttcaccag tcagggaact ccccagaggc agggggacct gaaccagctc 9540

ctttcattta agaattctga ctttgctcac aatctgcacc ccactcctcc ctacccaacc 9600

ccacacccca ttggtgctca gctctgggct cagcctgagg tctcttgccg aatcctggcc 9660

cgggccccat cccagacttc tccttcaggc tctgcataca aactggtctg ctactacacc 9720

agctggtccc agtaccggga aggcgatggg agctgcttcc cagatgccct tgaccgcttc 9780

ctctgtaccc acatcatcta cagctttgcc aatataagca acgatcacat cgacacctgg 9840

gagtggaatg atgtgacgct ctacggcatg ctcaacacac tcaagaacag gttgggccag 9900

ggctggccag gagaggaggg accagctggg ctggtcccag ggcagggcaa ccccacccag 9960

gaggaagggc gaggccccac cctgctcacc tatgtttgga gagttagagg ggcttggctt 10020

aaatgggtgg aggggatgac tgtaggtgct gggcaatatt tggggtgatg agagactgta 10080

gacggaagcg cagctgagcc cctctgggag aggggaaccc tctcctgccc ctaacgtgct 10140

ctccagggag catggaaaat gagcaagact ctactttgcc atactctgtc ttctccactg 10200

gggaaaacaa aaatgggcag cagacctcag agtaactccc atgaagcctg tgacccctca 10260

gagatccacc actatgaatc tctatgggat tcccatgatg tcctatggga ggacatcagc 10320

ggcctaaccc agcctctcac ccaaagcagg aaaccttcta tggcctctca gacatggggc 10380

cacccagtgt catgacaatg tcattccact cctgccctcc ccacctccct gtgctccaca 10440

ggaaccccaa cctgaagact ctcttgtctg tcggaggatg gaactttggg tctcaaaggt 10500

aggagcctct ccccaggggc aggacggcag aggatgatgg cataggagtg aggagcttgg 10560

gtctcccgca tccactgtat ggatgttcca ggggctccag actagatagg tacaaggcct 10620

acctgtttgt caagggcctg cctgatgtgt agcaaagaaa gcagagccca gagagagagc 10680

aggacttgcc aagagtcaca cagcaagtta acagtggatc tcccaattcc ctgccaatgc 10740

tctatttact acctcacagg cccgaaaata tgggacttct ggggctacca ccattagggc 10800

tggaaagaga gagatggaaa ccaatgggga cattgagaag tggggagacc ctgggaggag 10860

tctttggatt aggtggggtt ggagcagggc ttgaaatggg ggtggttatg gccatcgttg 10920

gcacccatgt gctgggtact ctttctgtgt ccagcaccct gtctccccat gatcccatgt 10980

tgtcctcaca acagcaaggt gagctgttat atttgtcccc attttacaga tgatgaaact 11040

gagactcagg ttataacctt tcacagtggc tggctagtaa gtgatagagc cagacttgga 11100
aacctggaat gcctggctct aaggcacatg catgcctgga ggcgacccct gtcccaatca 11160
tgccctccca gagctgtgtg gcctcaggat cccagctctg caggtcctgg aaaccccacc 11220
agaggcccaa ggcacctagc atatcagtgc tgagcatgct acagggctga ttttggtcct 11280
agattttcca agatagcctc caacacccag agtcgccgga ctttcatcaa gtcagtaccg 11340
ccatttctgc gcacccatgg ctttgatggg ctggaccttg cctggctcta ccctggacgg 11400
agagacaaac agcattttac caccctaatc aaggtgctgg tcaggtcagg gacagggagg 11460
gaggtgggca ggcatgaggg aatggggtgg gctagaagcc ggcgtcagct gctgtcctcc 11520
tgaggacagg taaagaggga cttcagcctc agggcagtgt cctgggaccc tgtgccctga 11580
agatctcaca tagcaaggaa ggcttcttgt gaccatggtg ggaggtggga atggggttct 11640
aagaggtgga aggttgtgac tgagcagagc acccacttat aactacccac ttagtgcatt 11700
gcccattgcc cacccttcaa tcccatactg atgccaccca tacccagcat gcactgtgtc 11760
cagcagctct cagctctgcc tgggggcagc ctaagtatct ccagttacca ggggcagaag 11820
gagagtctaa acaaattgtt cacaatacca gcagcaatca ctttaacttt gggatttcat 11880
gcaccagcta gaacaaagca cagaccgaaa ggcaaatgtc tcccaaagtc atctgtgggc 11940
caataggggt cacccactgt ccgatgctcc ctccaggaca gggagtaatt gagtgctgat 12000
gggtgtgcat gggtttgggg agaagattag tcagtttttt aggaaaagga tgggtgactt 12060
gaggatggga ccactgatga gccactttat cacttccact aggccccagg caggttgggg 12120
agtacagcaa atgggttgcg cagagaccta gtccgccctc gatgagtcta cctctcatgc 12180
cacttgggac cctttctctc acctgcctgt ctcccatcta ggaaatgaag gccgaattta 12240
taaaggaagc ccagccaggg aaaaagcagc tcctgctcag cgcagcactg tctgcgggga 12300
aggtcaccat tgacagcagc tatgacattg ccaagatatc ccagtgagtc tcctgcccca 12360
ggcagcctcc ccagaacctc tgccaacact cctgtttccc cgccaccccc agccctagca 12420
ctccatgtag actgaggaca ctgggcttcc agtcccagtt cttccaaagc cttgatgtgt 12480
gaccttgagc aagtcacttc cctgccctgt gcctcagttt ccccatctgt caaatgaggc 12540
ataacaatcc ttgcctttcc tttttcacct gggctgttgg ctgagcagtt gagagagctg 12600
ctatgaaagt attttggaaa gggaagtgga aaaagctatt caaaccctcc agctattgca 12660
gagtattttc tccacaccag gcagagcaga gtgctgggcc ctggagaggc cacacagcag 12720
cctctttcta gagtgctggg gagtcctcaa ggctctctca tacacacagg cctccccctt 12780
tcctgttggc cccacgcctc atcctcaccc cacccctcat gcctgtgagg aacaaggaac 12840
agccaagcag cctcatctct cctgagagca gagccatggg tctcgggaaa cccaggagta 12900
aggaacaaag actcttcaaa atgacttcag agctttcttt aggatcccag ggaggtgtaa 12960

actcagtgct taattaaatg gattctttag agggtgggaa acaggtggat gtcaaccatt 13020

tgccccacat acccgtattc atgcaatcca cccccagtgg gctcaccggt gcgggtgtgc 13080

aaaacctcct cccaccccag tcatcttagg ttcaggtcat cctttggtcc tgctctttcc 13140

ccgccaggct gtctgttgat gctactttta atctgctttc actaagataa gcctggcaga 13200

agtggtgggg gtaaggtggg ctgtaggcca gctcccaaat gtgtgctggg cataacagaa 13260

gacccattct tgactgaagt gcccttgtgg gaccctgagc ccgtgccctg gagtggcaca 13320

gggaggtgtg ccagcaatgg ggaccgaggt cactggggac tgctggggtt cgggctagtg 13380

gcctgtctgg ctgctgtctg cttccctcgt tcacatcctg ctggagcctt aaataggagc 13440

ccaaaagctt ttctttctta cttttttttg agacaaaatc tcactctgtt gcccaggccg 13500

gagtgcagtg gcacaacctc tgccgcctgg gttcaagcga ttctcctgcc tcagcctccc 13560

gagtagctgg gattacaggt gcctgccacc atgcctggct aattttcgta gttagagtag 13620

agacggggtt tcaccatctt ggccaggctg gtcttgaact cctcacctca tgatccacct 13680

gcctcggcct cccaaagtgc tgggattcca ggtatgagcc accacgcccg gcctaaagct 13740

tttctattaa taatttcctg cctcaccctc catccccttc ctcctcagac acctggattt 13800

cattagcatc atgacctacg attttcatgg agcctggcgt gggaccacag gccatcacag 13860

tcccctgttc cgaggtcagg aggatgcaag tcctgacaga ttcagcaaca ctgtgagttc 13920

ccagaaggag ggagggccag gagggggccg caagacctgc catctgccag tgctcacaca 13980

ccaatctctc tagccctcag tacagtcctg caagaggggg gtcatgaggc ccattccaca 14040

gataaggaaa ctgaggccca aagtctgggc atgctgcgtt gctctgggaa ggtgatctgc 14100

agggtaaatg gagtgagggc agggggccga atggggagag gctgggagcc gaggaggtag 14160

gagtcattgt gccctcagag ccaaccacct gatttctgca tctgtcaaat agtaatagcc 14220

ccttcctatg cctcaaagga tttttttcaa gaatgaaact gtaaaattca ctttaaagtg 14280

acatgatccg ttcccggagg gacaggggaa tccccagtgc accatacacc aataacccct 14340

gctaaggcag cagtattaat tgctcaacct tccgcacctg tgcactaact gctcacgttg 14400

ttcccaccct accccaccca ggactatgct gtggggtaca tgttgaggct gggggctcct 14460

gccagtaagc tggtgatggg catccccacc ttcgggagga gcttcactct ggcttcttct 14520

gagactggtg ttggagcccc aatctcagga ccgggaattc caggccggtt caccaaggag 14580

gcagggaccc ttgcctacta tgaggtaatg gagttgggga gaaggtagga ttcccccaca 14640

ccaccagcag ccctggggaa ggtgattccc caccacgttc ttgctttttc tcctttggga 14700

ataaagaaaa tgtccggttg ccccagcatg cctgaggaag tggaagggag aggttaggac 14760

atttgttgct gaaaatctcc agcaggtaca ggcacatggg cctgcaccac taggcacctg 14820

ggatagccct ctggctatgg ggctgaggtc ttccttccag cccaggaaag agcagaggtc 14880

aagaggcaga ttttttgttt cactctagcc tctgctactc tgtgtggcct tgggcctgtc 14940

ctcagtgtca accagcaggc ctcacatctc tgtttaaatg gaagaagcta agcagggcca 15000

aggcagccac catctctggg tcatttgcct ctggtttgta taaacttgtg tgatgcatgc 15060

agcctgcaga ccctgcagag agtgaggctg caggatggag caggagctaa aagagatttg 15120

gagagtggcg tctcctggtg acctgcaagg tctcggcacg actccccaca ctgccttttc 15180

cctgttatct gctcagatct gtgacttcct ccgcggagcc acagtccata gaatcctcgg 15240

ccagcaggtc ccctatgcca ccaagggcaa ccagtgggta ggatacgacg accaggaaag 15300

cgtcaaaagc aaggtaggtt tccccaaggc cacacctcag gacaaagaga aagaaggagg 15360

ccccgctccc agcagccaga ttcctgtccc ttgcactgag gtctgggctg ggctcacaga 15420

gcacatgtgc cctgtacaca ctctgggtca gggaacccag ccctgctcct ctgggcctcc 15480

cctgccaggt gcagtacctg aaggacaggc agctggcggg cgccatggta tgggccctgg 15540

acctggatga cttccagggc tccttctgtg gccaggatct gcgcttccct ctcaccaatg 15600

ccatcaagga tgcactcgct gcaacgtagc cctctgttct gcacacagca cgggggccaa 15660

ggatgccccg tccccctctg gctccagctg gccgggagcc tgatcacctg ccctgctgag 15720

tcccaggctg agcctcagtc tccctccctt ggggcctatg cagaggtcca caacacacag 15780

atttgagctc agccctggtg ggcagagagg tagggatggg gctgtgggga tagtgaggca 15840

tcgcaatgta agactcggga ttagtacaca cttgttgatt aatggaaatg tttacagatc 15900

cccaagcctg gcaagggaat ttcttcaact ccctgccccc cagccctcct tatcaaagga 15960

caccattttg gcaagctcta tcaccaagga gccaaacatc ctacaagaca cagtgaccat 16020

actaattata ccccctgcaa agcccagctt gaaaccttca cttaggaacg taatcgtgtc 16080

ccctatccta cttccccttc ctaattccac agctgctcaa taaagtacaa gagcttaaca 16140

gtg 16143

<210> 28
<211> 1408
<212> PRT
<213> Homo sapiens

<400> 28

<210>  29
<211>  6876
<212>  DNA
<213>  Homo sapiens

<400>  29
agacacgtgc tggggcgggc aggcgagcgc ctcagtctgg tcgcctggcg gtgcctccgg      60

ccccaacgcg cccgggccgc cgcgggccgc gcgcgccgat gcccggctga gtcactggca     120

gggcagcgcg cgtgtgggaa ggggcggagg gagtgcggcc ggcgggcggg cggggcgctg     180

ggctcagccc ggccgcaggt gacccggagg ccctcgccgc ccgcggcgcc ccgagcgctt     240

tgtgagcaga tgcggagccg agtggagggc gcgagccaga tgcggggcga cagctgactt     300

gctgagagga ggcggggagg cgcggagcgc gcgtgtggtc cttgcgccgc tgacttctcc     360

actggttcct gggcaccgaa agataaacct ctcataatga aggcccccgc tgtgcttgca     420

cctggcatcc tcgtgctcct gtttaccttg gtgcagagga gcaatgggga gtgtaaagag     480

gcactagcaa agtccgagat gaatgtgaat atgaagtatc agcttcccaa cttcaccgcg     540

gaaacaccca tccagaatgt cattctacat gagcatcaca ttttccttgg tgccactaac     600

tacatttatg ttttaaatga ggaagacctt cagaaggttg ctgagtacaa gactgggcct     660

gtgctggaac acccagattg tttcccatgt caggactgca gcagcaaagc caatttatca 720

ggaggtgttt ggaaagataa catcaacatg gctctagttg tcgacaccta ctatgatgat 780

caactcatta gctgtggcag cgtcaacaga gggacctgcc agcgacatgt ctttccccac 840

aatcatactg ctgacataca gtcggaggtt cactgcatat tctccccaca gatagaagag 900

cccagccagt gtcctgactg tgtggtgagc gccctgggag ccaaagtcct ttcatctgta 960

aaggaccggt tcatcaactt ctttgtaggc aataccataa attcttctta tttcccagat 1020

catccattgc attcgatatc agtgagaagg ctaaaggaaa cgaaagatgg ttttatgttt 1080

ttgacggacc agtcctacat tgatgtttta cctgagttca gagattctta ccccattaag 1140

tatgtccatg cctttgaaag caacaatttt atttacttct tgacggtcca aagggaaact 1200

ctagatgctc agacttttca cacaagaata atcaggttct gttccataaa ctctggattg 1260

cattcctaca tggaaatgcc tctggagtgt attctcacag aaaagagaaa aaagagatcc 1320

acaaagaagg aagtgtttaa tatacttcag gctgcgtatg tcagcaagcc tggggcccag 1380

cttgctagac aaataggagc cagcctgaat gatgacattc ttttcggggt gttcgcacaa 1440

agcaagccag attctgccga accaatggat cgatctgcca tgtgtgcatt ccctatcaaa 1500

tatgtcaacg acttcttcaa caagatcgtc aacaaaaaca atgtgagatg tctccagcat 1560

ttttacggac ccaatcatga gcactgcttt aataggacac ttctgagaaa ttcatcaggc 1620

tgtgaagcgc gccgtgatga atatcgaaca gagtttacca cagctttgca gcgcgttgac 1680

ttattcatgg gtcaattcag cgaagtcctc ttaacatcta tatccacctt cattaaagga 1740

gacctcacca tagctaatct tgggacatca gagggtcgct tcatgcaggt tgtggtttct 1800

cgatcaggac catcaacccc tcatgtgaat tttctcctgg actcccatcc agtgtctcca 1860

gaagtgattg tggagcatac attaaaccaa aatggctaca cactggttat cactgggaag 1920

aagatcacga agatcccatt gaatggcttg ggctgcagac atttccagtc ctgcagtcaa 1980

tgcctctctg ccccaccctt tgttcagtgt ggctggtgcc acgacaaatg tgtgcgatcg 2040

gaggaatgcc tgagcgggac atggactcaa cagatctgtc tgcctgcaat ctacaaggtt 2100

ttcccaaata gtgcacccct tgaaggaggg acaaggctga ccatatgtgg ctgggacttt 2160

ggatttcgga ggaataataa atttgattta aagaaaacta gagttctcct tggaaatgag 2220

agctgcacct tgactttaag tgagagcacg atgaatacat tgaaatgcac agttggtcct 2280

gccatgaata agcatttcaa tatgtccata attatttcaa atggccacgg gacaacacaa 2340

tacagtacat tctcctatgt ggatcctgta ataacaagta tttcgccgaa atacggtcct 2400

atggctggtg gcactttact tactttaact ggaaattacc taaacagtgg gaattctaga 2460

cacatttcaa ttggtggaaa aacatgtact ttaaaaagtg tgtcaaacag tattcttgaa 2520

tgttataccc cagcccaaac catttcaact gagtttgctg ttaaattgaa aattgactta 2580

gccaaccgag agacaagcat cttcagttac cgtgaagatc ccattgtcta tgaaattcat 2640

ccaaccaaat cttttattag tacttggtgg aaagaacctc tcaacattgt cagttttcta 2700

ttttgctttg ccagtggtgg gagcacaata acaggtgttg ggaaaaacct gaattcagtt 2760

agtgtcccga gaatggtcat aaatgtgcat gaagcaggaa ggaactttac agtggcatgt 2820

caacatcgct ctaattcaga gataatctgt tgtaccactc cttccctgca acagctgaat 2880

ctgcaactcc ccctgaaaac caaagccttt ttcatgttag atgggatcct ttccaaatac 2940

tttgatctca tttatgtaca taatcctgtg tttaagcctt ttgaaaagcc agtgatgatc 3000

tcaatgggca atgaaaatgt actggaaatt aagggaaatg atattgaccc tgaagcagtt 3060

aaaggtgaag tgttaaaagt tggaaataag agctgtgaga atatacactt acattctgaa 3120

gccgttttat gcacggtccc caatgacctg ctgaaattga acagcgagct aaatatagag 3180

tggaagcaag caatttcttc aaccgtcctt ggaaaagtaa tagttcaacc agatcagaat 3240

ttcacaggat tgattgctgg tgttgtctca atatcaacag cactgttatt actacttggg 3300

tttttcctgt ggctgaaaaa gagaaagcaa attaaagatc tgggcagtga attagttcgc 3360

tacgatgcaa gagtacacac tcctcatttg gataggcttg taagtgcccg aagtgtaagc 3420

ccaactacag aaatggtttc aaatgaatct gtagactacc gagctacttt tccagaagat 3480

cagtttccta attcatctca gaacggttca tgccgacaag tgcagtatcc tctgacagac 3540

atgtccccca tcctaactag tggggactct gatatatcca gtccattact gcaaaatact 3600

gtccacattg acctcagtgc tctaaatcca gagctggtcc aggcagtgca gcatgtagtg 3660

attgggccca gtagcctgat tgtgcatttc aatgaagtca taggaagagg gcattttggt 3720

tgtgtatatc atgggacttt gttggacaat gatggcaaga aaattcactg tgctgtgaaa 3780

tccttgaaca gaatcactga cataggagaa gtttcccaat ttctgaccga gggaatcatc 3840

atgaaagatt ttagtcatcc caatgtcctc tcgctcctgg gaatctgcct gcgaagtgaa 3900

gggtctccgc tggtggtcct accatacatg aaacatggag atcttcgaaa tttcattcga 3960

aatgagactc ataatccaac tgtaaaagat cttattggct ttggtcttca agtagccaaa 4020

ggcatgaaat atcttgcaag caaaaagttt gtccacagag acttggctgc aagaaactgt 4080

atgctggatg aaaaattcac agtcaaggtt gctgattttg gtcttgccag agacatgtat 4140

gataaagaat actatagtgt acacaacaaa acaggtgcaa agctgccagt gaagtggatg 4200

gctttggaaa gtctgcaaac tcaaaagttt accaccaagt cagatgtgtg gtcctttggc 4260

gtgctcctct gggagctgat gacaagagga gccccacctt atcctgacgt aaacaccttt 4320

gatataactg tttacttgtt gcaagggaga agactcctac aacccgaata ctgcccagac 4380

cccttatatg aagtaatgct aaaatgctgg caccctaaag ccgaaatgcg cccatccttt 4440

tctgaactgg tgtcccggat atcagcgatc ttctctactt tcattgggga gcactatgtc 4500

catgtgaacg ctacttatgt gaacgtaaaa tgtgtcgctc cgtatccttc tctgttgtca 4560

tcagaagata acgctgatga tgaggtggac acacgaccag cctccttctg ggagacatca 4620

tagtgctagt actatgtcaa agcaacagtc cacactttgt ccaatggttt tttcactgcc 4680

tgacctttaa aaggccatcg atattctttg ctcttgccaa aattgcacta ttataggact 4740

tgtattgtta tttaaattac tggattctaa ggaatttctt atctgacaga gcatcagaac 4800

cagaggcttg gtcccacagg ccacggacca atggcctgca gccgtgacaa cactcctgtc 4860

atattggagt ccaaaacttg aattctgggt tgaatttttt aaaaatcagg taccacttga 4920

tttcatatgg gaaattgaag caggaaatat tgagggcttc ttgatcacag aaaactcaga 4980

agagatagta atgctcagga caggagcggc agccccagaa caggccactc atttagaatt 5040

ctagtgtttc aaaacacttt tgtgtgttgt atggtcaata acatttttca ttactgatgg 5100

tgtcattcac ccattaggta aacattccct tttaaatgtt tgtttgtttt ttgagacagg 5160

atctcactct gttgccaggg ctgtagtgca gtggtgtgat catagctcac tgcaacctcc 5220

acctcccagg ctcaagcctc ccgaatagct gggactacag gcgcacacca ccatccccgg 5280

ctaatttttg tattttttgt agagacgggg ttttgccatg ttgccaaggc tggtttcaaa 5340

ctcctggact caagaaatcc acccacctca gcctcccaaa gtgctaggat tacaggcatg 5400

agccactgcg cccagccctt ataaattttt gtatagacat tcctttggtt ggaagaatat 5460

ttataggcaa tacagtcaaa gtttcaaaat agcatcacac aaaacatgtt tataaatgaa 5520

caggatgtaa tgtacataga tgacattaag aaaatttgta tgaaataatt tagtcatcat 5580

gaaatattta gttgtcatat aaaaacccac tgtttgagaa tgatgctact ctgatctaat 5640

gaatgtgaac atgtagatgt tttgtgtgta tttttttaaa tgaaaactca aaataagaca 5700

agtaatttgt tgataaatat ttttaaagat aactcagcat gtttgtaaag caggatacat 5760

tttactaaaa ggttcattgg ttccaatcac agctcatagg tagagcaaag aaagggtgga 5820

tggattgaaa agattagcct ctgtctcggt ggcaggttcc cacctcgcaa gcaattggaa 5880

acaaaacttt tggggagttt tattttgcat tagggtgtgt tttatgttaa gcaaaacata 5940

ctttagaaac aaatgaaaaa ggcaattgaa aatcccagct atttcaccta gatggaatag 6000

ccaccctgag cagaactttg tgatgcttca ttctgtggaa ttttgtgctt gctactgtat 6060

agtgcatgtg gtgtaggtta ctctaactgg ttttgtcgac gtaaacattt aaagtgttat 6120

attttttata aaaatgttta tttttaatga tatgagaaaa attttgttag gccacaaaaa 6180

cactgcactg tgaacatttt agaaaaggta tgtcagactg ggattaatga cagcatgatt 6240

ttcaatgact gtaaattgcg ataaggaaat gtactgattg ccaatacacc ccaccctcat 6300

tacatcatca ggacttgaag ccaagggtta acccagcaag ctacaaagag ggtgtgtcac 6360

actgaaactc aatagttgag tttggctgtt gttgcaggaa aatgattata actaaaagct 6420

ctctgatagt gcagagactt accagaagac acaaggaatt gtactgaaga gctattacaa 6480

tccaaatatt gccgtttcat aaatgtaata agtaatacta attcacagag tattgtaaat 6540

ggtggatgac aaaagaaaat ctgctctgtg gaaagaaaga actgtctcta ccagggtcaa 6600

gagcatgaac gcatcaatag aaagaactcg gggaaacatc ccatcaacag gactacacac 6660

ttgtatatac attcttgaga acactgcaat gtgaaaatca cgtttgctat ttataaactt 6720

gtccttagat taatgtgtct ggacagattg tgggagtaag tgattcttct aagaattaga 6780

tacttgtcac tgcctatacc tgcagctgaa ctgaatggta cttcgtatgt taatagttgt 6840

tctgataaat catgcaatta aagtaaagtg atgcaa 6876

<210> 30
<211> 308
<212> PRT
<213> Homo sapiens

<400> 30

Met Pro Gly Gln Glu Leu Arg Thr Val Asn Gly Ser Gln Met Leu Leu
1               5                   10                  15

Val Leu Leu Val Leu Ser Trp Leu Pro His Gly Gly Ala Leu Ser Leu
            20                  25                  30

Ala Glu Ala Ser Arg Ala Ser Phe Pro Gly Pro Ser Glu Leu His Ser
        35                  40                  45

Glu Asp Ser Arg Phe Arg Glu Leu Arg Lys Arg Tyr Glu Asp Leu Leu
    50                  55                  60

Thr Arg Leu Arg Ala Asn Gln Ser Trp Glu Asp Ser Asn Thr Asp Leu
65                  70                  75                  80

Val Pro Ala Pro Ala Val Arg Ile Leu Thr Pro Glu Val Arg Leu Gly
                85                  90                  95

Ser Gly Gly His Leu His Leu Arg Ile Ser Arg Ala Ala Leu Pro Glu
            100                 105                 110

Gly Leu Pro Glu Ala Ser Arg Leu His Arg Ala Leu Phe Arg Leu Ser
        115                 120                 125

Pro Thr Ala Ser Arg Ser Trp Asp Val Thr Arg Pro Leu Arg Arg Gln
    130                 135                 140

Leu Ser Leu Ala Arg Pro Gln Ala Pro Ala Leu His Leu Arg Leu Ser
145                 150                 155                 160

Pro Pro Pro Ser Gln Ser Asp Gln Leu Leu Ala Glu Ser Ser Ser Ala
                165                 170                 175

Arg Pro Gln Leu Glu Leu His Leu Arg Pro Gln Ala Ala Arg Gly Arg
            180                 185                 190

Arg Arg Ala Arg Ala Arg Asn Gly Asp His Cys Pro Leu Gly Pro Gly
        195                 200                 205

Arg Cys Cys Arg Leu His Thr Val Arg Ala Ser Leu Glu Asp Leu Gly
    210                 215                 220

Trp Ala Asp Trp Val Leu Ser Pro Arg Glu Val Gln Val Thr Met Cys
225                 230                 235                 240

Ile Gly Ala Cys Pro Ser Gln Phe Arg Ala Ala Asn Met His Ala Gln
                245                 250                 255

Ile Lys Thr Ser Leu His Arg Leu Lys Pro Asp Thr Val Pro Ala Pro
            260                 265                 270

Cys Cys Val Pro Ala Ser Tyr Asn Pro Met Val Leu Ile Gln Lys Thr
        275                 280                 285

Asp Thr Gly Val Ser Leu Gln Thr Tyr Asp Asp Leu Leu Ala Lys Asp
    290                 295                 300

Cys His Cys Ile
305

<210>  31
<211>  1200
<212>  DNA
<213>  Homo sapiens

<400>  31
agtcccagct cagagccgca acctgcacag ccatgcccgg gcaagaactc aggacggtga      60

atggctctca gatgctcctg gtgttgctgg tgctctcgtg gctgccgcat gggggcgccc     120

tgtctctggc cgaggcgagc cgcgcaagtt tcccgggacc ctcagagttg cactccgaag     180

actccagatt ccgagagttg cggaaacgct acgaggacct gctaaccagg ctgcgggcca     240

accagagctg ggaagattcg aacaccgacc tcgtcccggc ccctgcagtc cggatactca     300

cgccagaagt gcggctggga tccggcggcc acctgcacct gcgtatctct cgggccgccc     360

ttcccgaggg gctccccgag gcctcccgcc ttcaccgggc tctgttccgg ctgtccccga 420

cggcgtcaag gtcgtgggac gtgacacgac cgctgcggcg tcagctcagc cttgcaagac 480

cccaggcgcc cgcgctgcac ctgcgactgt cgccgccgcc gtcgcagtcg gaccaactgc 540

tggcagaatc ttcgtccgca cggccccagc tggagttgca cttgcggccg caagccgcca 600

gggggcgccg cagagcgcgt gcgcgcaacg gggaccactg tccgctcggg cccgggcgtt 660

gctgccgtct gcacacggtc cgcgcgtcgc tggaagacct gggctgggcc gattgggtgc 720

tgtcgccacg ggaggtgcaa gtgaccatgt gcatcggcgc gtgcccgagc cagttccggg 780

cggcaaacat gcacgcgcag atcaagacga gcctgcaccg cctgaagccc gacacggtgc 840

cagcgccctg ctgcgtgccc gccagctaca atcccatggt gctcattcaa aagaccgaca 900

ccggggtgtc gctccagacc tatgatgact tgttagccaa agactgccac tgcatatgag 960

cagtcctggt ccttccactg tgcacctgcg cggaggacgc gacctcagtt gtcctgccct 1020

gtggaatggg ctcaaggttc ctgagacacc cgattcctgc ccaaacagct gtatttatat 1080

aagtctgtta tttattatta atttattggg gtgaccttct tggggactcg ggggctggtc 1140

tgatggaact gtgtatttat ttaaaactct ggtgataaaa ataaagctgt ctgaactgtt 1200

<210> 32
<211> 93
<212> PRT
<213> Homo sapiens

<400> 32

Met Asp Arg Leu Gln Thr Ala Leu Leu Val Val Leu Val Leu Leu Ala
1               5                   10                  15

Val Ala Leu Gln Ala Thr Glu Ala Gly Pro Tyr Gly Ala Asn Met Glu
            20                  25                  30

Asp Ser Val Cys Cys Arg Asp Tyr Val Arg Tyr Arg Leu Pro Leu Arg
        35                  40                  45

Val Val Lys His Phe Tyr Trp Thr Ser Asp Ser Cys Pro Arg Pro Gly
    50                  55                  60

Val Val Leu Leu Thr Phe Arg Asp Lys Glu Ile Cys Ala Asp Pro Arg
65                  70                  75                  80

Val Pro Trp Val Lys Met Ile Leu Asn Lys Leu Ser Gln
                85                  90

<210> 33
<211> 2917
<212> DNA

<213>  Homo sapiens

<400>  33
gcagacacct gggctgagac atacaggaca gagcatggat cgcctacaga ctgcactcct      60

ggttgtcctc gtcctccttg ctgtggcgct tcaagcaact gaggcaggcc cctacggcgc     120

caacatggaa gacagcgtct gctgccgtga ttacgtccgt taccgtctgc ccctgcgcgt     180

ggtgaaacac ttctactgga cctcagactc ctgcccgagg cctggcgtgg tgttgctaac     240

cttcagggat aaggagatct gtgccgatcc cagagtgccc tgggtgaaga tgattctcaa     300

taagctgagc caatgaagag cctactctga tgaccgtggc cttggctcct ccaggaaggc     360

tcaggagccc tacctccctg ccattatagc tgctccccgc cagaagcctg tgccaactct     420

ctgcattccc tgatctccat ccctgtggct gtcacccttg gtcacctccg tgctgtcact     480

gccatctccc ccctgacccc tctaacccat cctctgcctc cctccctgca gtcagagggt     540

cctgttccca tcagcgattc ccctgcttaa acccttccat gactccccac tgccctaagc     600

tgaggtcagt ctcccaagcc tggcatgtgg ccctctggat ctgggttcca tctctgtctc     660

cagcctgccc acttcccttc atgaatgttg ggttctagct ccctgttctc caaacccata     720

ctacacatcc cacttctggg tctttgcctg ggatgttgct gacacccaga aagtcccacc     780

acctgcacat gtgtagcccc accagccctc caaggcattg ctcgcccaag cagctggtaa     840

ttccatttca tgtattagat gtcccctggc cctctgtccc ctcttaataa ccctagtcac     900

agtctccgca gattcttggg atttgggggt tttctccccc acctctccac tagttggacc     960

aaggtttcta gctaagttac tctagtctcc aagcctctag catagagcac tgcagacagg    1020

ccctggctca gaatcagagc ccagaaagtg gctgcagaca aaatcaataa aactaatgtc    1080

cctcccctct ccctgccaaa aggcagttac atatcaatac agagactcaa ggtcactaga    1140

aatgggccag ctgggtcaat gtgaagcccc aaatttgccc agattcacct ttcttccccc    1200

actccctttt tttttttttt tttgagatgg agtttcgctc ttgtcaccca cgctggagtg    1260

caatggtgtg gtcttggctt attgaagcct ctgcctcctg ggttcaagtg attctcttgc    1320

ctcagcctcc tgagtagctg ggattacagg ttcctgctac cacgcccagc taatttttgt    1380

atttttagta gagacgaggc ttcaccatgt tggccaggct ggtctcgaac tcctgtcctc    1440

aggtaatccg cccacctcag cctcccaaag tgctgggatt acaggcgtga gccacagtgc    1500

ctggcctctt ccctctcccc accccccccc caactttttt ttttttttat ggcagggtct    1560

cactctgtcg cccaggctgg agtgcagtgg cgtgatctcg gctcactaca acctcgacct    1620

cctgggttca agcgattctc ccaccccagc ctcccaagta gctgggatta caggtgtgtg    1680

ccactacggc tggctaattt ttgtattttt agtagagaca ggtttcacca tattggccag    1740

gctggtcttg aactcctgac ctcaagtgat ccaccttcct tgtgctccca aagtgctgag    1800

attacaggcg tgagctatca cacccagcct cccccttttt ttcctaatag gagactcctg 1860

tacctttctt cgttttacct atgtgtcgtg tctgcttaca tttccttctc ccctcaggct 1920

ttttttgggt ggtcctccaa cctccaatac ccaggcctgg cctcttcaga gtacccccca 1980

ttccactttc cctgcctcct tccttaaata gctgacaatc aaattcatgc tatggtgtga 2040

aagactacct ttgacttggt attataagct ggagttatat atgtatttga aaacagagta 2100

aatacttaag aggccaaata gatgaatgga agaattttag gaactgtgag agggggacaa 2160

ggtggagctt tcctggccct gggaggaagc tggctgtggt agcgtagcgc tctctctctc 2220

tgtctgtggc aggaggcaaa gagtagggtg taattgagtg aaggaatcct gggtagagac 2280

cattctcagg tggttgggcc aggctaaaga ctgggatttg ggtctatcta tgcctttctg 2340

gctgattttt gtagagacgg ggttttgcca tgttacccag gctggtctca aactcctggg 2400

ctcaagcgat cctcctggct cagcctccca aagtgctggg attacaggcg tgagtcactg 2460

cgcctggctt cctcttcctc ttgagaaata ttcttttcat acagcaagta tgggacagca 2520

gtgtcccagg taaaggacat aaatgttaca agtgtctggt cctttctgag ggaggctggt 2580

gccgctctgc agggtatttg aacctgtgga attggaggag gccatttcac tccctgaacc 2640

cagcctgaca aatcacagtg agaatgttca ccttataggc ttgctgtggg gctcaggttg 2700

aaagtgtggg gagtgacact gcctaggcat ccagctcagt gtcatccagg gcctgtgtcc 2760

ctcccgaacc cagggtcaac ctgcctacca caggcactag aaggacgaat ctgcctactg 2820

cccatgaacg gggccctcaa gcgtcctggg atctccttct ccctcctgtc ctgtccttgc 2880

ccctcaggac tgctggaaaa taaatccttt aaaatag 2917

<210> 34
<211> 401
<212> PRT
<213> Homo sapiens

<400> 34

Met Asn Asn Leu Leu Cys Cys Ala Leu Val Phe Leu Asp Ile Ser Ile
1               5                   10                  15

Lys Trp Thr Thr Gln Glu Thr Phe Pro Pro Lys Tyr Leu His Tyr Asp
            20                  25                  30

Glu Glu Thr Ser His Gln Leu Leu Cys Asp Lys Cys Pro Pro Gly Thr
        35                  40                  45

Tyr Leu Lys Gln His Cys Thr Ala Lys Trp Lys Thr Val Cys Ala Pro
    50                  55                  60

Cys Pro Asp His Tyr Tyr Thr Asp Ser Trp His Thr Ser Asp Glu Cys

65                  70                  75                  80

Leu Tyr Cys Ser Pro Val Cys Lys Glu Leu Gln Tyr Val Lys Gln Glu
                85                  90                  95

Cys Asn Arg Thr His Asn Arg Val Cys Glu Cys Lys Glu Gly Arg Tyr
            100                 105                 110

Leu Glu Ile Glu Phe Cys Leu Lys His Arg Ser Cys Pro Pro Gly Phe
        115                 120                 125

Gly Val Val Gln Ala Gly Thr Pro Glu Arg Asn Thr Val Cys Lys Arg
    130                 135                 140

Cys Pro Asp Gly Phe Phe Ser Asn Glu Thr Ser Ser Lys Ala Pro Cys
145                 150                 155                 160

Arg Lys His Thr Asn Cys Ser Val Phe Gly Leu Leu Leu Thr Gln Lys
                165                 170                 175

Gly Asn Ala Thr His Asp Asn Ile Cys Ser Gly Asn Ser Glu Ser Thr
            180                 185                 190

Gln Lys Cys Gly Ile Asp Val Thr Leu Cys Glu Glu Ala Phe Phe Arg
        195                 200                 205

Phe Ala Val Pro Thr Lys Phe Thr Pro Asn Trp Leu Ser Val Leu Val
    210                 215                 220

Asp Asn Leu Pro Gly Thr Lys Val Asn Ala Glu Ser Val Glu Arg Ile
225                 230                 235                 240

Lys Arg Gln His Ser Ser Gln Glu Gln Thr Phe Gln Leu Leu Lys Leu
                245                 250                 255

Trp Lys His Gln Asn Lys Asp Gln Asp Ile Val Lys Lys Ile Ile Gln
            260                 265                 270

Asp Ile Asp Leu Cys Glu Asn Ser Val Gln Arg His Ile Gly His Ala
        275                 280                 285

Asn Leu Thr Phe Glu Gln Leu Arg Ser Leu Met Glu Ser Leu Pro Gly
    290                 295                 300

Lys Lys Val Gly Ala Glu Asp Ile Glu Lys Thr Ile Lys Ala Cys Lys
305                 310                 315                 320

Pro Ser Asp Gln Ile Leu Lys Leu Leu Ser Leu Trp Arg Ile Lys Asn
                325                 330                 335

Gly Asp Gln Asp Thr Leu Lys Gly Leu Met His Ala Leu Lys His Ser
            340                 345                 350

Lys Thr Tyr His Phe Pro Lys Thr Val Thr Gln Ser Leu Lys Lys Thr
        355                 360                 365

Ile Arg Phe Leu His Ser Phe Thr Met Tyr Lys Leu Tyr Gln Lys Leu
    370                 375                 380

Phe Leu Glu Met Ile Gly Asn Gln Val Gln Ser Val Lys Ile Ser Cys
385                 390                 395                 400

Leu

<210>  35
<211>  2087
<212>  DNA
<213>  Homo sapiens

<400>  35
ggagacgcac cggagcgctc gcccagccgc cgcctccaag cccctgaggt ttccggggac      60

cacaatgaac aacttgctgt gctgcgcgct cgtgtttctg gacatctcca ttaagtggac     120

cacccaggaa acgtttcctc caaagtacct tcattatgac gaagaaacct ctcatcagct     180

gttgtgtgac aaatgtcctc ctggtaccta cctaaaacaa cactgtacag caaagtggaa     240

gaccgtgtgc gccccttgcc ctgaccacta ctacacagac agctggcaca ccagtgacga     300

gtgtctatac tgcagccccg tgtgcaagga gctgcagtac gtcaagcagg agtgcaatcg     360

cacccacaac cgcgtgtgcg aatgcaagga agggcgctac cttgagatag agttctgctt     420

gaaacatagg agctgccctc ctggatttgg agtggtgcaa gctggaaccc cagagcgaaa     480

tacagtttgc aaaagatgtc cagatgggtt cttctcaaat gagacgtcat ctaaagcacc     540

ctgtagaaaa cacacaaatt gcagtgtctt tggtctcctg ctaactcaga aaggaaatgc     600

aacacacgac aacatatgtt ccggaaacag tgaatcaact caaaaatgtg gaatagatgt     660

taccctgtgt gaggaggcat tcttcaggtt tgctgttcct acaaagttta cgcctaactg     720

gcttagtgtc ttggtagaca atttgcctgg caccaaagta aacgcagaga gtgtagagag     780

gataaaacgg caacacagct cacaagaaca gactttccag ctgctgaagt tatggaaaca     840

tcaaaacaaa gaccaagata tagtcaagaa gatcatccaa gatattgacc tctgtgaaaa     900

cagcgtgcag cggcacattg gacatgctaa cctcaccttc gagcagcttc gtagcttgat     960

ggaaagctta ccgggaaaga aagtgggagc agaagacatt gaaaaaacaa taaaggcatg    1020

caaacccagt gaccagatcc tgaagctgct cagtttgtgg cgaataaaaa atggcgacca 1080

agacaccttg aagggcctaa tgcacgcact aaagcactca aagacgtacc actttcccaa 1140

aactgtcact cagagtctaa agaagaccat caggttcctt cacagcttca caatgtacaa 1200

attgtatcag aagttatttt tagaaatgat aggtaaccag gtccaatcag taaaaataag 1260

ctgcttataa ctggaaatgg ccattgagct gtttcctcac aattggcgag atcccatgga 1320

tgagtaaact gtttctcagg cacttgaggc tttcagtgat atctttctca ttaccagtga 1380

ctaattttgc cacagggtac taaaagaaac tatgatgtgg agaaaggact aacatctcct 1440

ccaataaacc ccaaatggtt aatccaactg tcagatctgg atcgttatct actgactata 1500

ttttccctta ttactgcttg cagtaattca actggaaatt aaaaaaaaaa aactagactc 1560

cattgtgcct tactaaatat gggaatgtct aacttaaata gctttgagat ttcagctatg 1620

ctagaggctt ttattagaaa gccatatttt tttctgtaaa agttactaat atatctgtaa 1680

cactattaca gtattgctat ttatattcat tcagatataa gatttgtaca tattatcatc 1740

ctataaagaa acggtatgac ttaattttag aaagaaaatt atattctgtt tattatgaca 1800

aatgaaagag aaaatatata tttttaatgg aaagtttgta gcatttttct aataggtact 1860

gccatatttt tctgtgtgga gtatttttat aattttatct gtataagctg taatatcatt 1920

ttatagaaaa tgcattattt agtcaattgt ttaatgttgg aaaacatatg aaatataaat 1980

tatctgaata ttagatgctc tgagaaattg aatgtacctt atttaaaaga ttttatggtt 2040

ttataactat ataaatgaca ttattaaagt tttcaaatta tttttta 2087

<210> 36
<211> 496
<212> PRT
<213> Homo sapiens

<400> 36

Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
        35                  40                  45

Glu Met Asp Asn Cys Arg Ser Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80

Gln Val Leu Glu Asn Ile Met Glu Asn Asn Thr Gln Trp Leu Met Lys
                85                  90                  95

Leu Glu Asn Tyr Ile Gln Asp Asn Met Lys Lys Glu Met Val Glu Ile
            100                 105                 110

Gln Gln Asn Ala Val Gln Asn Gln Thr Ala Val Met Ile Glu Ile Gly
        115                 120                 125

Thr Asn Leu Leu Asn Gln Thr Ala Glu Gln Thr Arg Lys Leu Thr Asp
    130                 135                 140

Val Glu Ala Gln Val Leu Asn Gln Thr Thr Arg Leu Glu Leu Gln Leu
145                 150                 155                 160

Leu Glu His Ser Leu Ser Thr Asn Lys Leu Glu Lys Gln Ile Leu Asp
                165                 170                 175

Gln Thr Ser Glu Ile Asn Lys Leu Gln Asp Lys Asn Ser Phe Leu Glu
            180                 185                 190

Lys Lys Val Leu Ala Met Glu Asp Lys His Ile Ile Gln Leu Gln Ser
        195                 200                 205

Ile Lys Glu Glu Lys Asp Gln Leu Gln Val Leu Val Ser Lys Gln Asn
    210                 215                 220

Ser Ile Ile Glu Glu Leu Glu Lys Lys Ile Val Thr Ala Thr Val Asn
225                 230                 235                 240

Asn Ser Val Leu Gln Lys Gln Gln His Asp Leu Met Glu Thr Val Asn
                245                 250                 255

Asn Leu Leu Thr Met Met Ser Thr Ser Asn Ser Ala Lys Asp Pro Thr
            260                 265                 270

Val Ala Lys Glu Glu Gln Ile Ser Phe Arg Asp Cys Ala Glu Val Phe
        275                 280                 285

Lys Ser Gly His Thr Thr Asn Gly Ile Tyr Thr Leu Thr Phe Pro Asn
    290                 295                 300

Ser Thr Glu Glu Ile Lys Ala Tyr Cys Asp Met Glu Ala Gly Gly Gly
305                 310                 315                 320

Gly Trp Thr Ile Ile Gln Arg Arg Glu Asp Gly Ser Val Asp Phe Gln

325                 330                 335

Arg Thr Trp Lys Glu Tyr Lys Val Gly Phe Gly Asn Pro Ser Gly Glu
            340                 345                 350

Tyr Trp Leu Gly Asn Glu Phe Val Ser Gln Leu Thr Asn Gln Gln Arg
        355                 360                 365

Tyr Val Leu Lys Ile His Leu Lys Asp Trp Glu Gly Asn Glu Ala Tyr
    370                 375                 380

Ser Leu Tyr Glu His Phe Tyr Leu Ser Ser Glu Glu Leu Asn Tyr Arg
385                 390                 395                 400

Ile His Leu Lys Gly Leu Thr Gly Thr Ala Gly Lys Ile Ser Ser Ile
                405                 410                 415

Ser Gln Pro Gly Asn Asp Phe Ser Thr Lys Asp Gly Asp Asn Asp Lys
            420                 425                 430

Cys Ile Cys Lys Cys Ser Gln Met Leu Thr Gly Gly Trp Trp Phe Asp
        435                 440                 445

Ala Cys Gly Pro Ser Asn Leu Asn Gly Met Tyr Tyr Pro Gln Arg Gln
    450                 455                 460

Asn Thr Asn Lys Phe Asn Gly Ile Lys Trp Tyr Tyr Trp Lys Gly Ser
465                 470                 475                 480

Gly Tyr Ser Leu Lys Ala Thr Thr Met Met Ile Arg Pro Ala Asp Phe
                485                 490                 495

<210>  37
<211>  5416
<212>  DNA
<213>  Homo sapiens

<400>  37
ctggttggag ggcaggcatt ctgctctgat ttttcctgtt gcctggctag tgacccccta      60

caggaagata acggctaagc caggagggcg gagcagccca ctacacatgt ctggctgctc     120

ttatcaactt atcatataag gaaaggaaag tgattgattc ggatactgac actgtaggat     180

ctggggagag aggaacaaag gaccgtgaaa gctgctctgt aaaagctgac acagccctcc     240

caagtgagca ggactgttct tcccactgca atctgacagt ttactgcatg cctggagaga     300

acacagcagt aaaaaccagg tttgctactg gaaaaagagg aaagagaaga ctttcattga     360

cggacccagc catggcagcg tagcagccct gcgttttaga cggcagcagc tcgggactct     420

ggacgtgtgt ttgccctcaa gtttgctaag ctgctggttt attactgaag aaagaatgtg 480

gcagattgtt ttctttactc tgagctgtga tcttgtcttg gccgcagcct ataacaactt 540

tcggaagagc atggacagca taggaaagaa gcaatatcag gtccagcatg ggtcctgcag 600

ctacactttc ctcctgccag agatggacaa ctgccgctct tcctccagcc cctacgtgtc 660

caatgctgtg cagagggacg cgccgctcga atacgatgac tcggtgcaga ggctgcaagt 720

gctggagaac atcatggaaa acaacactca gtggctaatg aagcttgaga attatatcca 780

ggacaacatg aagaaagaaa tggtagagat acagcagaat gcagtacaga accagacggc 840

tgtgatgata gaaataggga caaacctgtt gaaccaaaca gcggagcaaa cgcggaagtt 900

aactgatgtg gaagcccaag tattaaatca gaccacgaga cttgaacttc agctcttgga 960

acactccctc tcgacaaaca aattggaaaa acagattttg gaccagacca gtgaaataaa 1020

caaattgcaa gataagaaca gtttcctaga aaagaaggtg ctagctatgg aagacaagca 1080

catcatccaa ctacagtcaa taaaagaaga gaaagatcag ctacaggtgt tagtatccaa 1140

gcaaaattcc atcattgaag aactagaaaa aaaaatagtg actgccacgg tgaataattc 1200

agttcttcag aagcagcaac atgatctcat ggagacagtt aataacttac tgactatgat 1260

gtccacatca aactcagcta aggaccccac tgttgctaaa gaagaacaaa tcagcttcag 1320

agactgtgct gaagtattca aatcaggaca caccacgaat ggcatctaca cgttaacatt 1380

ccctaattct acagaagaga tcaaggccta ctgtgacatg gaagctggag gaggcgggtg 1440

gacaattatt cagcgacgtg aggatggcag cgttgatttt cagaggactt ggaaagaata 1500

taaagtggga tttggtaacc cttcaggaga atattggctg ggaaatgagt ttgtttcgca 1560

actgactaat cagcaacgct atgtgcttaa aatacacctt aaagactggg aagggaatga 1620

ggcttactca ttgtatgaac atttctatct ctcaagtgaa gaactcaatt ataggattca 1680

ccttaaagga cttacaggga cagccggcaa aataagcagc atcagccaac caggaaatga 1740

ttttagcaca aaggatggag acaacgacaa atgtatttgc aaatgttcac aaatgctaac 1800

aggaggctgg tggtttgatg catgtggtcc ttccaacttg aacggaatgt actatccaca 1860

gaggcagaac acaaataagt tcaacggcat taaatggtac tactggaaag gctcaggcta 1920

ttcgctcaag gccacaacca tgatgatccg accagcagat ttctaaacat cccagtccac 1980

ctgaggaact gtctcgaact attttcaaag acttaagccc agtgcactga aagtcacggc 2040

tgcgcactgt gtcctcttcc accacagagg gcgtgtgctc ggtgctgacg ggacccacat 2100

gctccagatt agagcctgta aactttatca cttaaacttg catcacttaa cggaccaaag 2160

caagacccta aacatccata attgtgatta gacagaacac ctatgcaaag atgaacccga 2220

ggctgagaat cagactgaca gtttacagac gctgctgtca caaccaagaa tgttatgtgc 2280

aagtttatca gtaaataact ggaaaacaga acacttatgt tatacaatac agatcatctt 2340

ggaactgcat tcttctgagc actgtttata cactgtgtaa atacccatat gtcctgaatt 2400

caccatcact atcacaatta aaaggaagaa aaaaactctc taagccataa aaagacatat 2460

tcagggatat tctgagaagg ggttactaga agtttaatat ttggaaaaac agttagtgca 2520

tttttactcc atctcttagg tgctttaaat ttttatttca aaaacagcgt atttacattt 2580

atgttgacag cttagttata agttaatgct caaatacgta tttcaaattt atatggtaga 2640

aacttccaga atctctgaaa ttatcaacag aaacgtgcca ttttagttta tatgcagacc 2700

gtactatttt tttctgcctg attgttaaat atgaaggtat ttttagtaat taaatataac 2760

ttattagggg atatgcctat gtttaacttt tatgataata tttacaattt tataatttgt 2820

ttccaaaaga cctaattgtg ccttgtgata aggaaacttc ttacttttaa tgatgaggaa 2880

aattatacat ttcattctat gacaaagaaa ctttactatc ttctcactat tctaaaacag 2940

aggtctgttt tctttcctag taagatatat ttttatagaa ctagactaca atttaatttc 3000

tggttgagaa aagccttcta tttaagaaat ttacaaagct atatgtctca agattcaccc 3060

ttaaatttac ttaaggaaaa aaataattga cactagtaag tttttttatg tcaatcagca 3120

aactgaaaaa aaaaaaaggg tttcaaagtg caaaaacaaa atctgatgtt cataatatat 3180

ttaaatattt accaaaaatt tgagaacaca gggctgggcg cagtggctca cacctataat 3240

cccagtacat tggtaggcaa ggtgggcaga tcacctgagg tcaggagttc aagaccagcc 3300

tggacaacat ggtgaaaccc tgtctctact aaataataca aaaattagcc aggcgtgctg 3360

gcgggcacct gtaatcccag ctactcggga ggctgaggca gggagaattg cttgcaccag 3420

ggaggtagag gttgcagtga gccaagatcg caccactgca ctccagccgg ggcaacagag 3480

caagactcca tctcaaaaaa aaaaaaaaaa aaagaaagaa aagaaaattt gagaacacag 3540

ctttatactc gggactacaa aaccataaac tcctggagtt ttaactcctt ttgaaatttt 3600

catagtacaa ttaatactaa tgaacatttg tgtaaagctt tataatttaa aggcaatttc 3660

tcatatattc ttttctgaat catttgcaag gaagttcaga gtccagtctg taactagcat 3720

ctactatatg tctgtcttca ccttacagtg ttctaccatt atttttcttt tattccattt 3780

caaaatctaa tttattttac cccaacttct ccccaccact tgacgtagtt ttagaacaca 3840

caggtgttgc tacatatttg gagtcaatga tggactctgg caaagtcaag gctctgtttt 3900

atttccacca aggtgcactt ttccaacaac tatttaacta gttaagaacc tccctatctt 3960

agaactgtat ctactttata tttaagaagg ttttatgaat tcaacaacgg tatcatggcc 4020

ttgtatcaag ttgaaaaaca actgaaaata agaaaatttc acagcctcga aagacaacaa 4080

caagtttcta ggatatctca atgacaagag tgatggatac ttaggtaggg aaacgctaat 4140

gcaggaaaaa ctggcaacaa cacaatttat atcaattctc tttgtaggca ggtgataaaa 4200

aattcaagga caaatctcat tatgtcattg tgcatcatat ataatctctt atgagcgaga 4260

atggggggaa tttgtgtttt tactttacac ttcaattcct tacacggtat ttcaaacaaa 4320

cagttttgct gagaggagct tttgtctctc cttaagaaaa tgtttataaa gctgaaagga 4380

aatcaaacag taatcttaaa aatgaaaaca aaacaaccca acaacctaga taactacagt 4440

gatcagggag cacagttcaa ctccttgtta tgttttagtc atatggccta ctcaaacagc 4500

taaataacaa caccagtggc agataaaaat caccatttat ctttcagcta ttaatctttt 4560

gaatgaataa actgtgacaa acaaattaac atttttgaac atgaaaggca acttctgcac 4620

aatcctgtat ccaagcaaac tttaaattat ccacttaatt attacttaat cttaaaaaaa 4680

attagaaccc agaacttttc aatgaagcat ttgaaagttg aagtggaatt taggaaagcc 4740

ataaaaatat aaatactgtt atcacagcac cagcaagcca taatctttat acctatcagt 4800

tctatttcta ttaacagtaa aaacattaag caagatataa gactacctgc ccaagaattc 4860

agtctttttt catttttgtt tttctcagtt ctgaggatgt taatcgtcaa attttctttg 4920

gactgcattc ctcactactt tttgcacaat ggtctcacgt tctcacattt gttctcgcga 4980

ataaattgat aaaaggtgtt aagttctgtg aatgtctttt taattatggg cataattgtg 5040

cttgactgga taaaaactta agtccaccct tatgtttata ataatttctt gagaacagca 5100

aactgcattt accatcgtaa aacaacatct gacttacggg agctgcaggg aagtggtgag 5160

acagttcgaa cggctcctca gaaatccagt gacccaattc taaagaccat agcacctgca 5220

agtgacacaa caagcagatt tattatacat ttattagcct tagcaggcaa taaaccaaga 5280

atcactttga agacacagca aaaagtgata cactccgcag atctgaaata gatgtgttct 5340

cagacaacaa agtcccttca gaatcttcat gttgcataaa tgttatgaat attaataaaa 5400

agttgattga gaaaaa 5416

<210> 38
<211> 548
<212> PRT
<213> Homo sapiens

<400> 38

Met Asp Glu Leu Phe Pro Leu Ile Phe Pro Ala Glu Pro Ala Gln Ala
1               5                   10                  15

Ser Gly Pro Tyr Val Glu Ile Ile Glu Gln Pro Lys Gln Arg Gly Met
            20                  25                  30

Arg Phe Arg Tyr Lys Cys Glu Gly Arg Ser Ala Gly Ser Ile Pro Gly
        35                  40                  45

Glu Arg Ser Thr Asp Thr Thr Lys Thr His Pro Thr Ile Lys Ile Asn

50                  55                  60

Gly Tyr Thr Gly Pro Gly Thr Val Arg Ile Ser Leu Val Thr Lys Asp
65                  70                  75                  80

Pro Pro His Arg Pro His Pro His Glu Leu Val Gly Lys Asp Cys Arg
                85                  90                  95

Asp Gly Phe Tyr Glu Ala Glu Leu Cys Pro Asp Arg Cys Ile His Ser
            100                 105                 110

Phe Gln Asn Leu Gly Ile Gln Cys Val Lys Lys Arg Asp Leu Glu Gln
        115                 120                 125

Ala Ile Ser Gln Arg Ile Gln Thr Asn Asn Asn Pro Phe Gln Glu Glu
    130                 135                 140

Gln Arg Gly Asp Tyr Asp Leu Asn Ala Val Arg Leu Cys Phe Gln Val
145                 150                 155                 160

Thr Val Arg Asp Pro Ser Gly Arg Pro Leu Arg Leu Pro Pro Val Leu
                165                 170                 175

Ser His Pro Ile Phe Asp Asn Arg Ala Pro Asn Thr Ala Glu Leu Lys
            180                 185                 190

Ile Cys Arg Val Asn Arg Asn Ser Gly Ser Cys Leu Gly Gly Asp Glu
        195                 200                 205

Ile Phe Leu Leu Cys Asp Lys Val Gln Lys Glu Asp Ile Glu Val Tyr
    210                 215                 220

Phe Thr Gly Pro Gly Trp Glu Ala Arg Gly Ser Phe Ser Gln Ala Asp
225                 230                 235                 240

Val His Arg Gln Val Ala Ile Val Phe Arg Thr Pro Pro Tyr Ala Asp
                245                 250                 255

Pro Ser Leu Gln Ala Pro Val Arg Val Ser Met Gln Leu Arg Arg Pro
            260                 265                 270

Ser Asp Arg Glu Leu Ser Glu Pro Met Glu Phe Gln Tyr Leu Pro Asp
        275                 280                 285

Thr Asp Asp Arg His Arg Ile Glu Glu Lys Arg Lys Arg Thr Tyr Glu
    290                 295                 300

Thr Phe Lys Ser Ile Met Lys Lys Ser Pro Phe Ser Gly Pro Thr Asp
305                 310                 315                 320

Pro Arg Pro Pro Pro Arg Arg Ile Ala Val Pro Ser Arg Ser Ser Ala
                325                 330                 335

Ser Val Pro Lys Pro Ala Pro Gln Pro Tyr Pro Phe Thr Ser Ser Leu
            340                 345                 350

Ser Thr Ile Asn Tyr Asp Glu Phe Pro Thr Met Val Phe Pro Ser Gly
        355                 360                 365

Gln Ile Ser Gln Ala Ser Ala Leu Ala Pro Ala Pro Pro Gln Val Leu
    370                 375                 380

Pro Gln Ala Pro Ala Pro Ala Pro Ala Pro Ala Met Val Ser Ala Leu
385                 390                 395                 400

Ala Gln Ala Pro Ala Pro Val Pro Val Leu Ala Pro Gly Pro Pro Gln
                405                 410                 415

Ala Val Ala Pro Pro Ala Pro Lys Pro Thr Gln Ala Gly Glu Gly Thr
            420                 425                 430

Leu Ser Glu Ala Leu Leu Gln Leu Gln Phe Asp Asp Glu Asp Leu Gly
        435                 440                 445

Ala Leu Leu Gly Asn Ser Thr Asp Pro Ala Val Phe Thr Asp Leu Ala
    450                 455                 460

Ser Val Asp Asn Ser Glu Phe Gln Gln Leu Leu Asn Gln Gly Ile Pro
465                 470                 475                 480

Val Ala Pro His Thr Thr Glu Pro Met Leu Met Glu Tyr Pro Glu Ala
                485                 490                 495

Ile Thr Arg Leu Val Thr Gly Ala Gln Arg Pro Pro Asp Pro Ala Pro
            500                 505                 510

Ala Pro Leu Gly Ala Pro Gly Leu Pro Asn Gly Leu Leu Ser Gly Asp
        515                 520                 525

Glu Asp Phe Ser Ser Ile Ala Asp Met Asp Phe Ser Ala Leu Leu Ser
    530                 535                 540

Gln Ile Ser Ser
545

<210>  39
<211>  2681
<212>  DNA
<213>  Homo sapiens

<400>  39
cgtcctcggc gaggcgcgca cttggccccg acccccggca gcggctgtgc gtgcagcctc      60

ttcgtcctcc gcgcggcgtg cacttgctcc cggcccctgc gccgggcggc ggcggggcag     120

cgcgcaggcg cggccggatt ccgggcagtg acgcgacggc gggccgcgcg gcgcatttcc     180

gcctctggcg aatggctcgt ctgtagtgca cgccgcgggc ccagctgcga ccccggcccc     240

gcccccggga ccccggccat ggacgaactg ttccccctca tcttcccggc agagccagcc     300

caggcctctg gcccctatgt ggagatcatt gagcagccca agcagcgggg catgcgcttc     360

cgctacaagt gcgaggggcg ctccgcgggc agcatcccag gcgagaggag cacagatacc     420

accaagaccc accccaccat caagatcaat ggctacacag gaccagggac agtgcgcatc     480

tccctggtca ccaaggaccc tcctcaccgg cctcaccccc acgagcttgt aggaaaggac     540

tgccgggatg gcttctatga ggctgagctc tgcccggacc gctgcatcca cagtttccag     600

aacctgggaa tccagtgtgt gaagaagcgg gacctggagc aggctatcag tcagcgcatc     660

cagaccaaca acaacccctt ccaagaagag cagcgtgggg actacgacct gaatgctgtg     720

cggctctgct tccaggtgac agtgcgggac ccatcaggca ggcccctccg cctgccgcct     780

gtcctttctc atcccatctt tgacaatcgt gcccccaaca ctgccgagct caagatctgc     840

cgagtgaacc gaaactctgg cagctgcctc ggtggggatg agatcttcct actgtgtgac     900

aaggtgcaga aagaggacat tgaggtgtat ttcacgggac caggctggga ggcccgaggc     960

tccttttcgc aagctgatgt gcaccgacaa gtggccattg tgttccggac ccctccctac    1020

gcagacccca gcctgcaggc tcctgtgcgt gtctccatgc agctgcggcg gccttccgac    1080

cgggagctca gtgagcccat ggaattccag tacctgccag atacagacga tcgtcaccgg    1140

attgaggaga aacgtaaaag gacatatgag accttcaaga gcatcatgaa gaagagtcct    1200

ttcagcggac ccaccgaccc ccggcctcca cctcgacgca ttgctgtgcc ttcccgcagc    1260

tcagcttctg tccccaagcc agcaccccag ccctatccct ttacgtcatc cctgagcacc    1320

atcaactatg atgagtttcc caccatggtg tttccttctg ggcagatcag ccaggcctcg    1380

gccttggccc cggcccctcc ccaagtcctg ccccaggctc cagcccctgc ccctgctcca    1440

gccatggtat cagctctggc ccaggcccca gcccctgtcc cagtcctagc cccaggccct    1500

cctcaggctg tggccccacc tgcccccaag cccacccagg ctggggaagg aacgctgtca    1560

gaggccctgc tgcagctgca gtttgatgat gaagacctgg gggccttgct tggcaacagc    1620

acagacccag ctgtgttcac agacctggca tccgtcgaca actccgagtt tcagcagctg    1680

ctgaaccagg gcatacctgt ggccccccac acaactgagc ccatgctgat ggagtaccct 1740

gaggctataa ctcgcctagt gacaggggcc cagaggcccc ccgacccagc tcctgctcca 1800

ctgggggccc cggggctccc caatggcctc ctttcaggag atgaagactt ctcctccatt 1860

gcggacatgg acttctcagc cctgctgagt cagatcagct cctaaggggg tgacgcctgc 1920

cctccccaga gcactgggtt gcaggggatt gaagccctcc aaaagcactt acggattctg 1980

gtggggtgtg ttccaactgc ccccaacttt gtggatgtct tccttggagg ggggagccat 2040

attttattct tttattgtca gtatctgtat ctctctctct ttttggaggt gcttaagcag 2100

aagcattaac ttctctggaa aggggggagc tggggaaact caaacttttc ccctgtcctg 2160

atggtcagct cccttctctg tagggaactc tggggtcccc catccccatc ctccagcttc 2220

tggtactctc ctagagacag aagcaggctg gaggtaaggc ctttgagccc acaaagcctt 2280

atcaagtgtc ttccatcatg gattcattac agcttaatca aaataacgcc ccagatacca 2340

gcccctgtat ggcactggca ttgtccctgt gcctaacacc agcgtttgag gggctggcct 2400

tcctgcccta cagaggtctc tgccggctct ttccttgctc aaccatggct gaaggaaacc 2460

agtgcaacag cactggctct ctccaggatc cagaaggggt ttggtctggg acttccttgc 2520

tctccctctt ctcaagtgcc ttaatagtag ggtaagttgt taagagtggg ggagagcagg 2580

ctggcagctc tccagtcagg aggcatagtt tttactgaac aatcaaagca cttggactct 2640

tgctctttct actctgaact aataaatctg ttgccaagct g 2681

<210> 40
<211> 500
<212> DNA
<213> Homo sapiens

<220>
<221> misc_difference
<222> (1)..(500)
<223> V represent the SNP position

<400> 40
aggcgcacct cacacggcac acaggcacat ctcacacatg gcacacatgc acacctcaca 60

cagatggcac acatgcacac ctcacacaca cggcacgcat gcacacctca cacacacggc 120

acgcatgcac acctcacaca cggcacacat gcacacctca cacacgacac acgggcacac 180

ctcacacaca tggcacacgg gcacacctcc cacacacggc acacgggcac acctcccaca 240

cacggcacac vggcacacct caaacgacac acggcacacc tcacacacaa gtctattcag 300

ctgcaagtcc tgcctccact tgctgagaac ctgcatgact gggcaccaag gatacggcac 360

acacacgcac ccaccccaca tacatacagt ccacacacac acaacacata tacaccacac 420

gcaccacaga tgcacaccac acatgccaca cacacataca ctgcacacgc accctacaca 480

caccccccac atgcttacac 500

## Description

The present invention relates to cardiovascular disease, and particularly, although not exclusively, to cardiovascular disease biomarkers and their use in methods of diagnosis and prognosis. The invention also extends to diagnostic and prognostic kits utilising the biomarkers of the invention for diagnosing or prognosing cardiovascular disease.

Due to the complexity of cardiovascular disease (CVD), the ability of single biomarkers to predict cardiovascular outcome (CVO) risk is limited (De Lemos et al 2017), though many genetic loci and protein biomarkers (BMKs) have been discovered. Combining genetic information with other types of omics data should have more predictive power to detect sub-groups of the population and the correspondent molecular signatures related to the clinical outcomes (Vilne et al., 2018). This should point to molecular signatures for CV risk and show their potential to stratify the population in relation to disease progression. A better understanding of the drivers of T2D-associated cardiovascular disease will help to define sub-populations that may differ with regard to disease progression.

The inventors developed and tested a workflow to identify substructure of a trial population by molecular signatures of protein and genetic biomarkers. They discovered signatures defining sub-populations that progressed differently towards CVD, suggesting that these signatures reflect different stages of disease progression. They identified combinations of biomarkers, reflecting differences in CVD progression, will lead to strategies to optimize clinical trials plans, drug efficacy, and so to optimize treatment.

Accordingly, in a first aspect of the invention, there is provided a method of determining, diagnosing and/or prognosing an individual's risk of suffering from cardiovascular disease, the method comprising;
a. detecting, in a sample obtained from an individual, the expression level, amount and/or activity of two or more biomarkers selected from a group consisting of: Tumour Necrosis Factor (TNF)-□□ Glutathione S-Transferase Alpha 1 (GSTA1); N-terminal-pro hormone BNP (NT-proBNP); Retinoic Acid Receptor-Related Orphan Receptor Alpha (RORA); Tenascin C (TNC); Growth Hormone Receptor (GHR); Alpha-2-Macroglobulin (A2M); Insulin Like Growth Factor Binding Protein 2 (IGFBP2); Apolipoprotein B (APOB); Selenoprotein P (SEPP1); Trefoil Factor (TFF3); Interleukin 6 (IL6); Chitinase 3 Like 1 (CHI3L1); Hepatocyte Growth Factor Receptor (MET); Growth Differentiation Factor 15 (GDF15); Chemokine (C-C Motif) Ligand 22 (CCL22); Tumour Necrosis Factor Receptor Superfamily, Member 11 (TNFRSF11); Angiopoietin 2 (ANGPT2); and v -Rel Avian Reticuloendotheliosis Viral Oncogene Homolog A Nuclear Factor-kappa B (ReLA NF-KB);
b. comparing the expression level, amount and/or activity of the biomarker with a reference from a healthy control population; and
c. determining, diagnosing an
d/or prognosing the risk of an individual suffering from cardiovascular disease if the expression level, amount, and/or activity of the biomarker deviates from the reference from a healthy control population.

Advantageously, the method of the invention enables the identification of individuals who are at risk from suffering from a CVD event, and thus enables early intervention to prevent, or reduce the risk of, the individual from suffering from a CVD event. In particular, detection of each biomarker in isolation enables the identification of individuals who are at risk from suffering from a CVD event, and detection of multiple biomarkers, i.e. the biomarker signature provides a particularly effective means of enabling early intervention to prevent, or reduce the risk of, the individual from suffering from a CVD event.

The skilled person would understand that the term prognosis may relate to predicting the rate of progression or improvement and/or the duration of cardiovascular disease with individual suffering from cardiovascular disease.

The method may be performed *in vivo, in vitro* or *ex vivo.* Preferably, the method is performed *in vitro* or *ex vivo.* Most preferably, the method is performed *in vitro.*

The expression level may relate to the level or concentration of a biomarker polynucleotide sequence. The polynucleotide sequence may be DNA or RNA. The DNA may be genomic DNA. The RNA may be mRNA.

The amount of biomarker may relate to the concentration of biomarker polypeptide sequence.

The biomarker activity may relate to the activity of the biomarker protein, preferably activity in relation to interacting biomarkers.

### Categorical Biomarker(s):

LLQQ=2 → inactivation (-1)
LLQQ= from 3 to 4 → no activation (0)
LLQQ=from 5 to higher levels→ activation (+1)

For the quantitative biomarker(s) we used the tertiles of the distribution. Accordingly, the expression level, amount, and/or activity of the biomarker may be detected by: sequencing methods (e.g., Sanger, Next Generation Sequencing, RNA-SEQ), hybridization- based methods, including those employed in biochip arrays, mass spectrometry (e.g., laser desorption/ionization mass spectrometry), fluorescence (e.g., sandwich immunoassay), surface plasmon resonance, ellipsometry and atomic force microscopy. Expression levels of markers (e.g., polynucleotides, polypeptides, or other analytes) may be compared by procedures well known in the art, such as RT-PCR, Northern blotting, Western blotting, flow cytometry, immunocytochemistry, binding to magnetic and/or antibody-coated beads, *in situ* hybridization, fluorescence in situ hybridization (FISH), flow chamber adhesion assay, ELISA, microarray analysis, or colorimetric assays. Methods may further include one or more of electrospray ionization mass spectrometry (ESI-MS), ESI-MS/MS, ESI-MS/(MS)n, matrix-assisted laser desorption ionization time-of-flight mass spectrometry (MALDI-TOF- MS), surface-enhanced laser desorption/ionization time-of-flight mass spectrometry (SELDI- TOF-MS), desorption/ionization on silicon (DIOS), secondary ion mass spectrometry (SFMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n, quadrupole mass spectrometry, fourier transform mass spectrometry (FTMS), and ion trap mass spectrometry, where n is an integer greater than zero.

Thus, the method of detection may relate to a probe that is capable of hybridizing to the biomarker DNA or RNA sequence. The term "probe" may defined to be an oligonucleotide. A probe can be single stranded at the time of hybridization to a target. Probes include but are not limited to primers, i.e., oligonucleotides that can be used to prime a reaction, for example at least in a PCR reaction.

Preferably, a decrease in expression, amount and/or activity of TNF-□, GSTA1, NT-proBNP, RORA and/or TNC, when compared to the reference, is indicative of an individual having a higher risk of suffering from cardiovascular disease or a negative prognosis.

Preferably, an increase in expression, amount and/or activity of GHR, A2M, IGFBP2, APOB, SEPP1, TFF3, IL6 and/or CHI3L1, when compared to the reference, is indicative of an individual having a higher risk of suffering from cardiovascular disease or a negative prognosis.

Preferably, a decrease in expression, amount and/or activity of MET, GDF15, CCL22, TNFRSF11, ANGPT2 and/or RelA NF-KB, when compared to the reference, is indicative of an individual having a lower risk of suffering from cardiovascular disease or a positive prognosis.

Preferably, step a) comprises detecting, in a sample obtained from the individual, the expression levels, amount and/or activities of at least 3 of the biomarkers selected from the group consisting of: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2; and ReIA NF-KB. However, step a) may comprise detecting expression levels, amount and/or activities of at least 4 biomarkers or at least 5 biomarkers.

Step a) may comprise detecting expression levels, amount and/or activities of at least 6 biomarkers or at least 7 biomarkers. Alternatively, step a) may comprise detecting expression levels, amount and/or activities of at least 8 biomarkers or at least 9 biomarkers. In another embodiment, step a) may comprise detecting expression levels, amount and/or activities of at least 10 biomarkers, at least 11 biomarkers, at least 12 biomarkers, at least 13 biomarkers, at least 14 biomarkers or at least 15 biomarkers. In another embodiment, step a) may comprise detecting expression levels, amount and/or activities of at least 16 biomarkers, at least 17 biomarkers or at least 18 biomarkers.

Preferably, step a) comprises detecting, in a sample obtained from the individual, the expression levels, amount and/or activities of the biomarkers: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and ReIA NF-KB.

The cardiovascular disease may be selected from the group consisting of: cardiovascular death; myocardial infarction; stroke; and heart failure.

In one embodiment, RORA is provided by gene bank locus ID: HGNC: 10258; Entrez Gene: 6095; and/or Ensembl: ENSG00000069667. The protein sequence may be represented by the GeneBank ID P35398-2, which is provided herein as SEQ ID No: 1, as follows:












[SEQ ID No:1 ]

Accordingly, preferably RORA comprises or consists of an amino acid sequence substantially as set out in SEQ ID NO: 1, or a fragment or variant thereof.

In one embodiment, RORA is encoded by a nucleotide sequence which is provided herein as SEQ ID No: 2, as follows:

GTACCATAGAGTTGCTCTGAAAACAGAAGATAGAGGGAGTCTCGGAGCTC
GCCATCTCCAGCGATCTCTACATTGGGAAAAAACATGGAGTCAGCTCCGG
CAGCCCCCGACCCCGCCGCCAGCGAGCCAGGCAGCAGCGGCGCGGACG
CGGCCGCCGGCTCCAGGGAGACCCCGCTGAACCAGGAATCCGCCCGCAA
GAGCGAGCCGCCTGCCCCGGTGCGCAGACAGAGCTATTCCAGCACCAGC
AGAGGTATCTCAGTAACGAAGAAGACACATACATCTCAAATTGAAATTATTC
CATGCAAGATCTGTGGAGACAAATCATCAGGAATCCATTATGGTGTCATTA
CATGTGAAGGCTGCAAGGGCTTTTTCAGGAGAAGTCAGCAAAGCAATGCC
ACCTACTCCTGTCCTCGTCAGAAGAACTGTTTGATTGATCGAACCAGTAGA
AACCGCTGCCAACACTGTCGATTACAGAAATGCCTTGCCGTAGGGATGTC
TCGAGATGCTGTAAAATTTGGCCGAATGTCAAAAAAGCAGAGAGACAGCTT
GTATGCAGAAGTACAGAAACACCGGATGCAGCAGCAGCAGCGCGACCAC

CAGCAGCAGCCTGGAGAGGCTGAGCCGCTGACGCCCACCTACAACATCT
CGGCCAACGGGCTGACGGAACTTCACGACGACCTCAGTAACTACATTGAC
GGGCACACCCCTGAGGGGAGTAAGGCAGACTCCGCCGTCAGCAGCTTCT
ACCTGGACATACAGCCTTCCCCAGACCAGTCAGGTCTTGATATCAATGGAA
TCAAACCAGAACCAATATGTGACTACACACCAGCATCAGGCTTCTTTCCCT
ACTGTTCGTTCACCAACGGCGAGACTTCCCCAACTGTGTCCATGGCAGAA
TTAGAACACCTTGCACAGAATATATCTAAATCGCATCTGGAAACCTGCCAA
TACTTGAGAGAAGAGCTCCAGCAGATAACGTGGCAGACCTTTTTACAGGAA
GAAATTGAGAACTATCAAAACAAGCAGCGGGAGGTGATGTGGCAATTGTG
TGCCATCAAAATTACAGAAGCTATACAGTATGTGGTGGAGTTTGCCAAACG
CATTGATGGATTTATGGAACTGTGTCAAAATGATCAAATTGTGCTTCTAAAA
GCAGGTTCTCTAGAGGTGGTGTTTATCAGAATGTGCCGTGCCTTTGACTCT
CAGAACAACACCGTGTACTTTGATGGGAAGTATGCCAGCCCCGACGTCTT
CAAATCCTTAGGTTGTGAAGACTTTATTAGCTTTGTGTTTGAATTTGGAAAG
AGTTTATGTTCTATGCACCTGACTGAAGATGAAATTGCATTATTTTCTGCAT
TTGTACTGATGTCAGCAGATCGCTCATGGCTGCAAGAAAAGGTAAAAATTG
AAAAACTGCAACAGAAAATTCAGCTAGCTCTTCAACACGTCCTACAGAAGA
ATCACCGAGAAGATGGAATACTAACAAAGTTAATATGCAAGGTGTCTACCT
TAAGAGCCTTATGTGGACGACATACAGAAAAGCTAATGGCATTTAAAGCAA
TATACCCAGACATTGTGCGACTTCATTTTCCTCCATTATACAAGGAGTTGTT
CACTTCAGAATTTGAGCCAGCAATGCAAATTGATGGGTAAATGTTATCACC
TAAGCACTTCTAGAATGTCTGAAGTACAAACATGAAAAACAAACAAAAAAAT
TAACCGAGACACTTTATATGGCCCTGCACAGACCTGGAGCGCCACACACT
GCACATCTTTTGGTGATCGGGGTCAGGCAAAGGAGGGGAAACAATGAAAA
CAAATAAAAGTTGAACTTGTTTTTCTCATGCATATGATTTCCATTATGCCTAC
AGATATGGACCCTTTTTCTGTCTTGACTTCTTGATCATTGACCTCTGTTTAC
AACAGGAGGAGGGTACTAAAGTCGGAGGATTTCCTTTTCTTGTAGCTCACT
GCCCACAGACTTTCTACAGAGTCACCAATCTGTCAGTAACAACAGAGAGTC
CAGCAATAATCGGTGACTGGTGTGCATAGCGGAGGTTGCGGCATTACTTT
GCACAACTAGCTCTTTGTTTCATGAAGGAAGTTTTTATTTTTTCACCGATTA
TTGCCAGTCCGCAGGATGGCATGAAAAGGGTCCATAGCAGTAGCAACAAT
AGCATTATAATATATTACAGGGTAAATGGGCATGAAGACTATATATAGCTAA

AAGAGATATTGTTTATATATTGTTTTAAGTAATATAAAATGTAGTTACTGGTG
TAGCTTTTCCTGTTGAATTGATAAGGCACTTTCATTTTGCACCTTTTTCTTTA
AATTAAATGCTAGCGTGTTCACTGTCGTGTCGCATGTGCACCAGAAACACA
AGTTTAACTGAGAAGGCTTGGAAGGTACGTTGGGAGGTATTTATGCTGCTG
TTTACAAAATTATTTTTAAGAGACTGGCTGGTCATATCTAGAAATCACCACG
TTGGATTTTTTTTTTAACATGTGAATTTGGAATTAGAAACGGAACTCTCCCT
AAATTATACTTTGCTTTTTGGTAAGTTTAATGATAGATGTGTTTATGCTTCAT
ACAAAGTTGAATGATTGATTGGCGTGGTGGACATATACCATCATGCTCATT
TTTTTTTTTTAAAGCTTTTTAAAATGCCACCTCATGGAGGCGAGGGGGAGG
AGAAGCTCATTTTACACAATTCAGTAGTTAAATATGGACTCGGTCTCAACTT
GGAATTCTTATGCTTTGAGAACAAATCAACAACCAGAATATTTATTGGAATC
TAGCTTTTATTATAAGAAGGACCCAAAGATTATATCCTGAGCAAATGCACAC
TCCCCATGTGAGGACATGAAGTATTTACTTTGTGAATGTTTATGTTCTTGGT
ATAATCTAGGAACCCTATGAGTTTATCTCAGAGTGAACTAATTCTAGATTTG
TTGTCAATAGATGCTATAATTCAAGAATGTTGCTCTCCATATTTGAAAAACG
ATGGATAGGAGGGTGAGGGAAGCATACAATGTTGAACCAGTTTCTCTTATT
TAAATATTAAATACTTTAAGCCTTTAAAGTGAAGTTGATGCTAGCTGCAAAC
ATTTACTCTTGTATTTATCTTCACTAGGAAACTGTGGACTGTAATTTATTTTA
TTAAATATTTAGAAGATTATTTGGCTTGTGTGTTCAGGTGAGAAATACTGAG
TTGTTTTTGTTTAATTTCATGGTTTTTTTTTTTTTAATGATCCCTAGTGGGGG
AAGGGGAAAGGAATAGTCTGATAAACAGATGTGCATATTTTAAAAACAAGT
GACCTTTTGGGAATGTAGGCATTTAGACGATGATTTTAGTCGCACTAGGGG
TGGGATTCAAACTACTGGTCAAAGACCATTTTGTACAGAAAAGGGAACATC
TCCGATGGGTGTTAAGGTAGGAGTTTCCATGCAGCCCTTTATGTCTGAGAA
ATAGTCTCCTCTGCCATTGGGGTCCCTGCGGAATCTTCTACAGGAATTGCA
GCTCTTCACGTCATGCTAGGTTACCAGCATGGGCTTCCCAGAGCACTTCA
CTCTGTTTCTAACTCCACTGACTTTTCTGACTTGTTTCTTGAGGGACTTGGA
AAAGGGGAAGGTATTATTCACACAGATGTGTGTATGAAGCCTAAATAACAT
CCAACTTTTTTTCCAAAAACATAGTAAGAGTTTAACCATAAATAGAAGAGTA
ATATTCCTTTAAATTTCTTACAGGCATGTAAAATTTTATGTGTTTATAGAGAG
ATGCTAACTGTCAGCATATAGTATTTATATTGGGGCAAGAAGGGTTAAATC
AAATCTTTAATTTAAGTAAGCATAGTTCCTTTAAAGATCAGTAGTATTTATAC

TCTGAAAAGAGTACCAAGCTTAGTTCAGTTATTTATTCATCCATTTTTTTCT
ATTGTTTCTCCTCTGGGGGAAATGGTGTTTGTTTTGTTTGAGTTTTGTG
TTTTAGTTTTTTGGTTTTGGTTTTGTTTTGTTTTTTGTCATTCAGATTCACTA
AATTTGGGGATGGTTTTATTAAAGTATATTAACTTCTTTTTAACCAAAGCTTT
CTGAATATGACCAGCCTCAGGTGCTAGCGCATTAAAGAGCAACTAAACCTA
ATTCCAGTGTCGATTTGTGAAATAATAAAAGCTAATTGAATTTCTCTAAGGG
TGAGAGAGAACATAATGATGAACTTAAAAAGAAATTCCTTTTCCCAGAAGG
GATTCTGCATGTACCTACAAACAATCATGCTCTAACCACAGTAATAGTCATG
CCATGGTGACATTGCTTTTACGGTAAACCAAGATAGGTAATATGATGCTTC
TTCCCAGGTGTTTCTGAAAGAAAAGCAGCGGTGGAGCTTAAGAGCCAAGT
CCACTGATTGACATAATAGGATGGAATTGTAGACAGAGACATGCTCCATGA
AACAAGGAAACAACTGACTACTATTTGGATCTAAGTTGGGATCTGATGTTA
AACAATAAATTCAGTTTAAAAAAAATATGGAGCTCAGAAAAGGATGTGAAAA
ACTTTGCATTTTCCTTTCTTCATTATTACAAAAACACCTATGTGATGACATAA
AATACTTGGGTGATATCAGAACAATTATCCTTAATAGTTTTTATAATTAAAGT
TTCCTAAACTTCAGTCTCCAATAGTCTTTTAAGGATTGGAACCACATCACTG
TCAGCCCCGCTGCTACAATGCCTTTGTACAATTTTTTTACATAAGGCAAATA
AGGCTTTTGTACAAAGCCTGAATACCTTCTGTTCCAATGGTGTCCAAATGG
TTATTATATTGTGAAAAACCTGGCCTTGGTCACAATGCAAACAAAAGTACAG
ATGAAAGTGCTTTTTGGACAGTTTGCAAATTGTGTTAAAGCTACGGATTTTT
TTTAAAGTGTTCAGCATCTTAACGTGTATTAAAGCTATGGATTTTTTTTTAAA
GTCTTCAGCATCCTAATTCACCCTTTCTAACTTAAGAAAAACATGACTTAAG
ACACTGCTTCTAAGTTTGGTTGTTCTTTATAGTGTGGATACCCAGTTATCTG
TGAGCGTATGGGGGTGGGCTGAGGGTCAGGTGAGGAAGGAGTGTGTGTG
TGTGTGTGTGTGTGTGTGTGTGTGTGTGTGTGATTTGCATGTGTATGATGTGT
GTGCGTCGGACCGCTTCTAGGCTACTAAGTGTCAATGGAAAAGAAAATGTA
TTCAAAATACTTAAATCAAAACTAGAAGATGGGGAAAAAAAGATTTATTCTA
TACAAAGCCTTGTCTGGACCACTTTAGAGAGACTTCTATTTTTTTAACCCTT
CTATAAATATTTGATGGCACTTGAAATATTCCTGCAATAAAATGTGATTTGT
GTAAAGAAAAAAAGATTTTGTAATGTGAAACAAAGAAAGAAAGTAATGTAAT
TTTCTAAAAAAAAAAATACAAACAAACAAACTTTGTATTATTTTCTTGATGGA
ATTTGTCTATCTGTCTTTGGAAAACTTTTTATTTCATTGAATGTGCCATAGTA

GAAATGTGTGTTTTTAGTTTTAGACTAAGGAATAGCTAGTTGTTGTGTTCCG
ACATTCCAAAATGCAAAACAACCTAGTAGTATCTTTCATGAAAAGGTTTAAG
TAGTATGTAAGCTTCTCTCATTGTTGCTTTTTTGCACATGTTCTTCATTCCTC
TCTAGTGCAATATGTACATAGAGCACTTGCGGGTGTACCTTGATCCCTCAG
GGAAAAATACATATTTGTACAGTTTTTGGGGTTTTTTGTTTTTGGTTTTT
TTTGTTTTGCCTTTTGTTTTTGGCTAAGGAATGTCGATCGAATCACTTGTTA
TTGTTGAGGGGCAGCCAGATAATAATCCTAAAGCCACTGTTTCCAACATTG
ATTGTTTAAATCATATGTCCTTCCAATGCTATTATTTAAGATAATAATAAAA
AGTTATTTTCTGACAGTTCTTTGTGCTGACTGGTGAAAAACAAGGGTAAATA
AGCACCTTATAATTGACTTACTGTGAATGACAATCCATCTTGGTATCAACGA
TAGAAGCCCTATCATTTTGGAGTTGGGGTTAAGAGTCAGAAACAATGTGCT
CAGGGATCTCCTAAAACTCTTAAAACAGGGTGGCCAGTACTACTGGGACA
AATTGTGTTTTTTATTATTAATAATAATGATAATAATACTATCCCTCCAAGGC
ACAAGTGAACTATATAGAACTGCGTGTGTGTAAACTCTTTACTCTCGTCTCA
TTTTGTTGAGTTTAGAACTTGATGTGCTCGTCAGTCTTGTGTTTCAAAACAC
TGAATAACTTCCAAAGCAAAGTTATGCCAGTGTGTTCAAAAGATAAATTAAT
AATGTACCAGCAAAGAGCATCATTCAAAGTATAGTCCTTGCATATTCCAGTT
ACCATTTCTCTAATAATTAAAATATTCATGATAAATATATATATAGCATCATA
TGTTAAAAACTATTTCAAATTCTACATATTAAGGATGAAAATTTTAAAATCCA
GTAATAAGAGGAGAGACCTGCCTATCAGTACAGTGATATAGGTAAAAGATG
AAATATGTTTTTAAAATACCAGCAATTAACTATTGTTTTCATGGGTTCTCCTC
TAGAAGCAAACCAAAAATTCCTTCATGGAAAACAATTCTTACTTCTACATGT
GTAGTTTATATCTGATGCATTACAAGAGCTAATGTTTAAAGACAAAACAAAA
CCTGCCTGTATGACAGCAGCAACTCGAGCCAACATTTAGTGTTACATGTTA
TATTTTTGAAATACCGTTTTGTTATCATATTCCACATATTACTTTCCATAAAG
TCAGAGAAGTTCAATGTAATTGTTGGCTCTGATTCTTCCACCTTGGGATAC
ACATTCACAAGAAGATTTATATATTTTCTTACTATGATAGAGGAACATAATCT
GGGAAAACTTCCCATGTCTGTAAGATGAAAAGGATACCTTTACCATGTTGT
TTTTGATAATAAAGAATATGGAAAATGGTAGAAATCTCTCCTCTCTATGTGT
ATTTGTATATGTGTGTATACACACACACGTTGACATTTTTACATAACATGTG
ATTGCCACTTCTTATAAAGTTATGATATAGAACCCCATGAAAACAACTTTAT
ATTATAGATCAAATAATGTGCCCAGAAACGCAATTGCAACAGTAAATCTTGA

TCTATTGGTAAGAGTCTATGATACAGGTCTTCATTCTATCCCTAAACATAAT
GTTAGTAAAGAGGTTCCATCAGATTGTATTATAGAGACCTTCCTATTGCTAT
TTATTTTTAAGAGATGAGAAGACTGACTAGCAATGTCTCCACAGTGCAATT
GGTTTCACTTCTGGGTCTGTCTGTCTGTTTGTGTAGGTGAGATCAGTGTTG
TCAGGCTCTCAGAATAATTTTAAAAGAATCCAGAAGCCTGACTTCACACC
AAGTAGCCATCCTAGATGGGCGGGGGGATCTCCATGTTCAACCAAACCC
TCAGAACTTAGAGCAATAAATACATTCAGTCATTTATTTATAAATGAATGAC
AGAATATTCAATCCAAATAGAAACAACCTTTTGTCAGTGATGCAACATAACA
TGGAGTCTTATATTGAGAAAGTGAATATGAATATTTTAAATAGATGCCTAGG
AAATCTGTGTTTGCTGTTTACATTTAATGTACTTTGCCACATTAGCAGTACC
ACACTTCTTTTACTTTCATCCTTCTAAGAACTAATGAAAGATAGCTTGCTATT
AGCTTTGACATGTTGCACATGCCATTATGTTGTTCTCTAAGAACAACTAAAT
TCTCTCCAGTGTTCATGTGTGATTCCTTTTTCATCATTATTACTTTAATGTGG
ATGAATACTATTTCTAGGAACTTGATTATCACTGAACAGATTGCATATGTAA
ATGCAGATAATTCTTGAGCAATATAGTGAAAATGATTTCACAAAAAAAATCC
ATATGTACTGTATACATCTTCCTGAATCCAAAATTCTCCTGTTATCCACACA
GTTTGAAATCTCATATTAAACAGTGGTAAATTTTTAAACATTAGGACATTAG
CTGGCTGCTTCTATAAATGTACCTGTATTGTCTGCCTGCTCCTTTTGTGGA
GATGTGTTTTTGTATTGGATGTTTGGGCCAATGGGAGGCTTCAAGCTACAA
CATATTTTCCCACTTTAAATAATATTTAACATATGACAAACCCCAGACAAGG
CTTTTAATATGTATAAAGAACTGCAGTGTTAGGTGGTTTATTTGCAAACTGT
TTTCAGTGTTGTCCATTTTTATGGCACCTTTAACAATATTCTTGTTTCCAAAG
TACAAAAAAAATAGGTTAAATAATCTAGCCATAACTGAATGTCAAGCAATAA
AACAAATGACTTTTGTGCATAGACTTAAAAAATACAAATTTTAGACATCTGC
ATGTAAATGCAATCTCTTGTTTACTGCTTTCTTCAACTTGAGCAATCGATTC
CTTATTTACTATTAACTTAAGAACTTGTAACGGCCTGTAAACATTTTCTCTCT
TACTCTTCTTTCAAATTTACCTGTCCCTGCTTTTGAGTCATAATATTTAATGT
TGTTCTGCACATCTCTATACAGTTAACTTTTTGGCTTTCATTCTGTATAGATA
AGAAAATGTTATATTATAAACAGCCTACTCAGTGCAAATATTTATCTGTTTAT
CAAATCCACAATATGCTGTATAATACCGGTTTTACTATATAATCTATTTTAGA
CATAGCTGTTTAGAACTAGAGTGTGCTATTTTTGTGTTTTTCTGATGTGTGG
TGCTAGACAAGTTACTTTTGTGAACAACAAAAATTATCCCTTTTATTCCTAG

ACAATACCACCTTTGGGTCTTGTTAATTTCACTGAGTATAACTATATATTTGT
ATATATATACATATATATATATATCTACCTATGCCCAACTGGCAGCTGTATC
AGAGTGCTGGATTTGGGACATGCTTTTCTCTTTAAATACATAATATCATTAT
ATAAATTATTCTAGAGTGTATTTAATTAGGATAAAATTACTTCCTTAGTATGG
ATATTTGACATCTATAGGGTGAATTTGTTTATAAATATGGCTATATGGAAAC
TTATTAGCATTTACTTTATGTTTGCTACTTGGCTTTACAGCATATCTCCTAAG
CTGAAAAATAATTTGCCAGGCCTTCAAGATCCTAAAGAAACTTGTTTAATGG
AGTAATATACTTTTTTTTCTTATTAAGGAATTGTATTACTGGCACCTAACACA
GTTGTATTCTTAGCTCCTATTATAGATAATGGGCATTTACATAAAATATCCTA
GATGGCTTGATGGCAGAATAAACCTTTCCCCTCCTACCTGAGTCATGAGAA
GGATGGAGACGTCCTCTGCCATAACATGGGCCATAAAGCAAATTCGACAT
GGGATGTTCTGTTTCAGTATGACCTCAACCAGTTCCATGAACTGAGTGAAG
GACCTTCATTTTCAAAGTTATTTAATAAGTAGCTTAATTAAGCCTTTCTACCC
ATTCTCCCAAGATCTACTGGCATTATTGAAAAGCAAAGTTTATCAAATATCT
AACTAAGGATGTAGTTAACCTTATTAAATATTGATTAGAATTGTTCTGTAATA
TTACTGAATTTGTAAGATCTTTAGCAAAGATTTTTGAGCAATTTATAAATGTA
GAGCAAATGTTTCTGTTTACTGCACTTTTTGTAACTGAAGGTGATAAATTCT
CAAGCCATGATTATTGGCTTCCATGCACTGCAATATTTATCCACAATTCTAG
ACATTTTCCATTTTTGTGGAAGAGTTGCTGTTACCTTAATTATAAATGCAATT
GTGTGGTTAATGAGAGCTAATGCTAGTAGTTAACCTTTTAAAGTGGATTGG
CTACAGTTGAGGGAGAAATCTCTTTTAATATAAATCACATCATTCCTTAACT
GCCTCTCTTGGAAAGAGATTGAAACCTTTTTTTAAAGCACGATTTAGCATC
CTAAGCTTCCTGAGGGTAGAGATTGTATCTTTTTGCGTCTGCACAATGGCT
AGCACATGTCAGCATTTGACAATTGTTAAATGATAACAAGTGTGCCCCAATT
AAAACGTTTTTCCTGGGTTGTTTTGTTAAATTTACAAAGTAAGCCAAGCCTT
ACGGTTAACATTCTCCTCTACAACCAAGTATTAAAGCCACATTTAAAAAGAC
CACATGAAATGCTGATTCTAATTGTGTGTAGGTCTTGAGGATTAAGCACAC
AAATTTCACAAACTTCTGTTTGAGTAAACAAACTCAGCCTTCTGTAAATATA
CATGCAAGTTTGGAAACAGTAATACTGTACCTATAAATATATGCTGTCTGTT
TTGTGTACAGTATGTAAAAACTCCTTTTCTGCCACACTAAAAATGCAAGCCA
TTTATGGGAATCCTAAAACTAGTATTGAACTAAAACTTTGCTAATGATCTTTA
TTAGAGGATCGTCCAACTTTTCACTTACCTTGGGTTTTCTTTTCAATTCACT

CTTACACTAGTCTGCTTATTTCCAGCTGTTTATTTTATTGAGTCCTGAATTTA
AAAAAAAAATATTTTGATTCATTTTGTAAATACAAGCTGTACAAAAAAGAGA
GATTTAATGTTGTCTTTTAAATACTCCAATTTTCATTCTAATATGAATGTTGT
TATATTGTACTTAGAAACTGTACCTTTAATATTACATTACCTTTATTAAAAGT
GCATTGAACACATCAATTTTAGATGTGCTTTATGTACTGTTATCCTATAATAA
AACTTCAGCTTCTAATGGAA

[SEQ ID No: 2]

Accordingly, preferably RORA comprises or consists of a nucleotide sequence substantially as set out in SEQ ID NO: 2, or a fragment or variant thereof.

→In one embodiment, GHR is provided by gene bank locus ID: HGNC: 4263; Entrez Gene: 2690; and/or Ensembl: ENSG00000112964. The protein sequence is represented by the GeneBank ID P10912, which is provided herein as SEQ ID No: 4, as follows:













[SEQ ID No:4]

Accordingly, preferably GHR comprises or consists of an amino acid sequence substantially as set out in SEQ ID NO: 4, or a fragment or variant thereof.

In one embodiment, GHR is encoded by a nucleotide sequence, which is provided herein as SEQ ID No: 5, as follows:

AACTAGCAATGGTGGTACAGTGGATGAAAAGTGTTTCTCTGTTGATGAAAT
AGGCGGCGGCGGCAGCGGCAGCAGCTGCTACAGTGGCGGTGG
CGGCGGCGGCTGCTGCTGAGCCCGGGCGGCGGGGACCCCGGGCT
GGGGCCACGCGGGCCGGAGGCCCCGGCACCATTGGCCCCAGCGCAGAC
GCGAACCCGCGCTCTCTGATCAGAGGCGAAGCTCGGAGGTCCTACAGGT
ATGGATCTCTGGCAGCTGCTGTTGACCTTGGCACTGGCAGGATCAAGTGA
TGCTTTTTCTGGAAGTGAGGCCACAGCAGCTATCCTTAGCAGAGCACCCT
GGAGTCTGCAAAGTGTTAATCCAGGCCTAAAGACAAATTCTTCTAAGGAGC
CTAAATTCACCAAGTGCCGTTCACCTGAGCGAGAGACTTTTTCATGCCACT
GGACAGATGAGGTTCATCATGGTACAAAGAACCTAGGACCCATACAGCTG
TTCTATACCAGAAGGAACACTCAAGAATGGACTCAAGAATGGAAAGAATGC
CCTGATTATGTTTCTGCTGGGGAAAACAGCTGTTACTTTAATTCATCGTTTA
CCTCCATCTGGATACCTTATTGTATCAAGCTTGCAACCAGATCCACCCATT
GCCCTCAACTGGACTTTACTGAACGTCAGTTTAACTGGGATTCATGCAGAT
ATCCAAGTGAGATGGGAAGCACCACGCAATGCAGATATTCAGAAAGGATG
GATGGTTCTGGAGTATGAACTTCAATACAAAGAAGTAAATGAAACTAAATG
GAAAATGATGGACCCTATATTGACAACATCAGTTCCAGTGTACTCATTGAA
AGTGGATAAGGAATATGAAGTGCGTGTGAGATCCAAACAACGAAACTCTG
GAAATTATGGCGAGTTCAGTGAGGTGCTCTATGTAACACTTCCTCAGATGA
GCCAATTTACATGTGAAGAAGATTTCTACTTTCCATGGCTCTTAATTATTAT
CTTTGGAATATTTGGGCTAACAGTGATGCTATTTGTATTCTTATTTTCTAAAC
AGCAAAGGATTAAAATGCTGATTCTGCCCCCAGTTCCAGTTCCAAAGATTA
AAGGAATCGATCCAGATCTCCTCAAGGAAGGAAAATTAGAGGAGGTGAAC
ACAATCTTAGCCATTCATGATAGCTATAAACCCGAATTCCACAGTGATGACT
CTTGGGTTGAATTTATTGAGCTAGATATTGATGAGCCAGATGAAAAGACTG
AGGAATCAGACACAGACAGACTTCTAAGCAGTGACCATGAGAAATCACATA
GTAACCTAGGGGTGAAGGATGGCGACTCTGGACGTACCAGCTGTTGTGAA
CCTGACATTCTGGAGACTGATTTCAATGCCAATGACATACATGAGGGTACC
TCAGAGGTTGCTCAGCCACAGAGGTTAAAAGGGGAAGCAGATCTCTTATG

CCTTGACCAGAAGAATCAAAATAACTCACCTTATCATGATGCTTGCCCTGC
TACTCAGCAGCCCAGTGTTATCCAAGCAGAGAAAAACAAACCACAACCACT
TCCTACTGAAGGAGCTGAGTCAACTCACCAAGCTGCCCATATTCAGCTAAG
CAATCCAAGTTCACTGTCAAACATCGACTTTTATGCCCAGGTGAGCGACAT
TACACCAGCAGGTAGTGTGGTCCTTTCCCCGGCCAAAAGAATAAGGCAG
GGATGTCCCAATGTGACATGCACCCGGAAATGGTCTCACTCTGCCAAGAA
AACTTCCTTATGGACAATGCCTACTTCTGTGAGGCAGATGCCAAAAAGTGC
ATCCCTGTGGCTCCTCACATCAAGGTTGAATCACACATACAGCCAAGCTTA
AACCAAGAGGACATTTACATCACCACAGAAAGCCTTACCACTGCTGCTGG
GAGGCCTGGGACAGGAGAACATGTTCCAGGTTCTGAGATGCCTGTCCCAG
ACTATACCTCCATTCATATAGTACAGTCCCCACAGGGCCTCATACTCAATG
CGACTGCCTTGCCCTTGCCTGACAAAGAGTTTCTCTCATCATGTGGCTATG
TGAGCACAGACCAACTGAACAAAATCATGCCTTAGCCTTTCTTTGGTTTCC
CAAGAGCTACGTATTTAATAGCAAAGAATTGACTGGGGCAATAACGTTTAA
GCCAAAACAATGTTTAAACCTTTTTTGGGGGAGTGACAGGATGGGGTATG
GATTCTAAAATGCCTTTTCCCAAAATGTTGAAATATGATGTTAAAAAAATAA
GAAGAATGCTTAATCAGATAGATATTCCTATTGTGCAATGTAAATATTTTAA
AGAATTGTGTCAGACTGTTTAGTAGCAGTGATTGTCTTAATATTGTGGGTGT
TAATTTTTGATACTAAGCATTGAATGGCTATGTTTTTAATGTATAGTAAATCA
CGCTTTTTGAAAAAGCGAAAAAATCAGGTGGCTTTTGCGGTTCAGGAAAAT
TGAATGCAAACCATAGCACAGGCTAATTTTTTGTTGTTTCTTAAATAAGAAA
CTTTTTTATTTAAAAAACTAAAAACTAGAGGTGAGAAATTTAAACTATAAGCA
AGAAGGCAAAAATAGTTTGGATATGTAAAACATTTATTTTGACATAAAGTTG
ATAAAGATTTTTTAATAATTTAGACTTCAAGCATGGCTATTTTATATTACACT
ACACACTGTGTACTGCAGTTGGTATGACCCCTCTAAGGAGTGTAGCAACTA
CAGTCTAAAGCTGGTTTAATGTTTTGGCCAATGCACCTAAAGAAAAACAAA
CTCGTTTTTTACAAAGCCCTTTTATACCTCCCCAGACTCCTTCAACAATTCT
AAAATGATTGTAGTAATCTGCATTATTGGAATATAATTGTTTTATCTGAATTT
TTAAACAAGTATTTGTTAATTTAGAAAACTTTAAAGCGTTTGCACAGATCAA
CTTACCAGGCACCAAAAGAAGTAAAAGCAAAAAAGAAAACCTTTCTTCACC
AAATCTTGGTTGATGCCAAAAAAAAATACATGCTAAGAGAAGTAGAAATCAT
AGCTGGTTCACACTGACCAAGATACTTAAGTGCTGCAATTGCACGCGGAG

TGAGTTTTTTAGTGCGTGCAGATGGTGAGAGATAAGATCTATAGCCTCTGC
AGCGGAATCTGTTCACACCCAACTTGGTTTGCTACATAATTATCCAGGAA
GGGAATAAGGTACAAGAAGCATTTTGTAAGTTGAAGCAAATCGAATGAAAT
TAACTGGGTAATGAAACAAAGAGTTCAAGAAATAAGTTTTTGTTTCACAGCC
TATAACCAGACACATACTCATTTTTCATGATAATGAACAGAACATAGACAGA
AGAAACAAGGTTTTCAGTCCCCACAGATAACTGAAAATTATTTAAACCGCTA
AAAGAAACTTTCTTTCTCACTAAATCTTTTATAGGATTTATTTAAAATAGCAA
AAGAAGAAGTTTCATCATTTTTTACTTCCTCTCTGAGTGGACTGGCCTCAAA
GCAAGCATTCAGAAGAAAAAGAAGCAACCTCAGTAATTTAGAAATCATTTT
GCAATCCCTTAATATCCTAAACATCATTCATTTTTGTTGTTGTTGTTGTT
GAGACAGAGTCTCGCTCTGTCGCCAGGCTAGAGTGCGGTGGCGCGATCT
TGACTCACTGCAATCTCCACCTCCCACAGGTTCAGGCGATTCCCGTGCCT
CAGCCTCCTGAGTAGCTGGGACTACAGGCACGCACCACCATGCCAGGCTA
ATTTTTTTGTATTTTAGCAGAGACGGGGTTTCACCATGTTGGCCAGGATGG
TCTCGATCTCCTGACCTCGTGATCCACCCGACTCGGCCTCCCAAAGTGCT
GGGATTACAGGTGTAAGCCACCGTGCCCAGCCCTAAACATCATTCTTGAG
AGCATTGGGATATCTCCTGAAAAGGTTTATGAAAAAGAAGAATCTCATCTC
AGTGAAGAATACTTCTCATTTTTTAAAAAAGCTTAAAACTTTGAAGTTAGCTT
TAACTTAAATAGTATTTCCCATTTATCGCAGACCTTTTTTAGGAAGCAAGCT
TAATGGCTGATAATTTTAAATTCTCTCTCTTGCAGGAAGGACTATGAAAAGC
TAGAATTGAGTGTTTAAAGTTCAACATGTTATTTGTAATAGATGTTTGATAG
ATTTTCTGCTACTTTGCTGCTATGGTTTTCTCCAAGAGCTACATAATTTAGT
TTCATATAAAGTATCATCAGTGTAGAACCTAATTCAATTCAAAGCTGTGTGT
TTGGAAGACTATCTTACTATTTCACAACAGCCTGACAACATTTCTATAGCCA
AAAATAGCTAAATACCTCAATCAGTCTCAGAATGTCATTTTGGTACTTTGGT
GGCCACATAAGCCATTATTCACTAGTATGACTAGTTGTGTCTGGCAGTTTA
TATTTAACTCTCTTTATGTCTGTGGATTTTTTCCTTCAAAGTTTAATAAATTTA
TTTTCTTGGATTCCTGATAGTGTGCTTCTGTTATCAAACACCAACATAAAAA
TGATCTAAACC
[SEQ ID No: 5]

Accordingly, preferably GHR comprises or consists of a nucleotide sequence substantially as set out in SEQ ID NO: 5, or a fragment or variant thereof.

In one embodiment, TNF-□ is provided by gene bank locus ID: HGNC: 11892; Entrez Gene: 7124; and/or Ensembl: ENSG00000232810. The protein sequence may be represented by the GeneBank ID P01375, which is provided herein as SEQ ID No: 6 , as follows:

MSTESMIRDVELAEEALPKKTGGPQGSRRCLFLSLFSFLIVAGATTLFCLLHFG
VIGPQREEFPRDLSLISPLAQAVRSSSRTPSDKPVAHVVANPQAEGQLQWLN
RRANALLANGVELRDNQLVVPSEGLYLIYSQVLFKGQGCPSTHVLLTHTISRIA
VSYQTKVNLLSAIKSPCQRETPEGAEAKPWYEPIYLGGVFQLEKGDRLSAEIN
RPDYLDFAESGQVYFGIIAL

[SEQ ID No: 6]

Accordingly, preferably TNF-□ comprises or consists of an amino acid sequence as substantially as set out in SEQ ID NO: 6, or a fragment or variant thereof.

In one embodiment TNF-□ is encoded by a nucleotide sequence which is provided herein as SEQ ID No: 7, as follows:

AGCAGACGCTCCCTCAGCAAGGACAGCAGAGGACCAGCTAAGAGGGAGA
GAAGCAACTACAGACCCCCCCTGAAAACAACCCTCAGACGCCACATCCCC
TGACAAGCTGCCAGGCAGGTTCTCTTCCTCTCACATACTGACCCACGGCT
CCACCCTCTCTCCCCTGGAAAGGACACCATGAGCACTGAAAGCATGATCC
GGGACGTGGAGCTGGCCGAGGAGGCGCTCCCCAAGAAGACAGGGGGGC
CCCAGGGCTCCAGGCGGTGCTTGTTCCTCAGCCTCTTCTCCTTCCTGATC
GTGGCAGGCGCCACCACGCTCTTCTGCCTGCTGCACTTTGGAGTGATCGG
CCCCCAGAGGGAAGAGTTCCCCAGGGACCTCTCTCTAATCAGCCCTCTGG
CCCAGGCAGTCAGATCATCTTCTCGAACCCCGAGTGACAAGCCTGTAGCC
CATGTTGTAGCAAACCCTCAAGCTGAGGGGCAGCTCCAGTGGCTGAACCG
CCGGGCCAATGCCCTCCTGGCCAATGGCGTGGAGCTGAGAGATAACCAG
CTGGTGGTGCCATCAGAGGGCCTGTACCTCATCTACTCCCAGGTCCTCTT

CAAGGGCCAAGGCTGCCCCTCCACCCATGTGCTCCTCACCCACACCATCA
GCCGCATCGCCGTCTCCTACCAGACCAAGGTCAACCTCCTCTCTGCCATC
AAGAGCCCCTGCCAGAGGGAGACCCCAGAGGGGGCTGAGGCCAAGCCCT
GGTATGAGCCCATCTATCTGGGAGGGGTCTTCCAGCTGGAGAAGGGTGAC
CGACTCAGCGCTGAGATCAATCGGCCCGACTATCTCGACTTTGCCGAGTC
TGGGCAGGTCTACTTTGGGATCATTGCCCTGTGAGGAGGACGAACATCCA
ACCTTCCCAAACGCCTCCCCTGCCCCAATCCCTTTATTACCCCCTCCTTCA
GACACCCTCAACCTCTTCTGGCTCAAAAAGAGAATTGGGGGCTTAGGGTC
GGAACCCAAGCTTAGAACTTTAAGCAACAAGACCACCACTTCGAAACCTGG
GATTCAGGAATGTGTGGCCTGCACAGTGAAGTGCTGGCAACCACTAAGAA
TTCAAACTGGGGCCTCCAGAACTCACTGGGGCCTACAGCTTTGATCCCTG
ACATCTGGAATCTGGAGACCAGGGAGCCTTTGGTTCTGGCCAGAATGCTG
CAGGACTTGAGAAGACCTCACCTAGAAATTGACACAAGTGGACCTTAGGC
CTTCCTCTCTCCAGATGTTTCCAGACTTCCTTGAGACACGGAGCCCAGCCC
TCCCCATGGAGCCAGCTCCCTCTATTTATGTTTGCACTTGTGATTATTTATT
ATTTATTTATTATTTATTTATTTACAGATGAATGTATTTATTTGGGAGACCGG
GGTATCCTGGGGGACCCAATGTAGGAGCTGCCTTGGCTCAGACATGTTTT
CCGTGAAAACGGAGCTGAACAATAGGCTGTTCCCATGTAGCCCCCTGGCC
TCTGTGCCTTCTTTTGATTATGTTTTTTAAAATATTTATCTGATTAAGTTGTC
TAAACAATGCTGATTTGGTGACCAACTGTCACTCATTGCTGAGCCTCTGCT
CCCCAGGGGAGTTGTGTCTGTAATCGCCCTACTATTCAGTGGCGAGAAAT
AAAGTTTGCTTAGAAAAGAAA
[SEQ ID No: 7]

Accordingly, preferably TNF-□ comprises or consists of a nucleotide sequence as substantially as set out in SEQ ID NO: 7, or a fragment or variant thereof.

In one embodiment, GSTA1 is provided by gene bank locus ID: HGNC: 4626; Entrez Gene: 2938; Ensembl: ENSG00000243955; OMIM: 138359; and/or UniProtKB: P08263. The protein sequence may be represented by the GeneBank ID: ENST00000334575.6, which is provided herein as SEQ ID No: 8, as follows:

MAEKPKLHYFNARGRMESTRWLLAAAGVEFEEKFIKSAEDLDKLRNDGYLMF
QQVPMVEIDGMKLVQTRAILNYIASKYNLYGKDIKERALIDMYIEGIADLGEMILL
LPVCPPEEKDAKLALIKEKIKNRYFPAFEKVLKSHGQDYLVGNKLSRADIHLVEL
LYYVEELDSSLISSFPLLKALKTRISNLPTVKKFLQPGSPRKPPMDEKSLEEARK
IFRF

[SEQ ID No: 8]

Accordingly, preferably GSTA1 comprises or consists of an amino acid sequence substantially as set out in SEQ ID NO: 8, or a fragment or variant thereof.

In one embodiment GSTA1 is encoded by a nucleotide sequence which is provided herein as SEQ ID No: 9, as follows:

GTCGAGCCAGGACGGTGACAGCGTTTAACAAAGCTTAGAGAAACCTCCAG
GAGACTGCTATCATGGCAGAGAAGCCCAAGCTCCACTACTTCAATGCACG
GGGCAGAATGGAGTCCACCCGGTGGCTCCTGGCTGCAGCTGGAGTAGAG
TTTGAAGAGAAATTTATAAAATCTGCAGAAGATTTGGACAAGTTAAGAAATG
ATGGATATTTGATGTTCCAGCAAGTGCCAATGGTTGAGATTGATGGGATGA
AGCTGGTGCAGACCAGAGCCATTCTCAACTACATTGCCAGCAAATACAACC
TCTATGGGAAAGACATAAAGGAGAGAGCCCTGATTGATATGTATATAGAAG
GTATAGCAGATTTGGGTGAAATGATCCTCCTTCTGCCCGTATGTCCACCTG
AGGAAAAAGATGCCAAGCTTGCCTTGATCAAAGAGAAAATAAAAAATCGCT
ACTTCCCTGCCTTTGAAAAAGTCTTAAAGAGCCATGGACAAGACTACCTTG
TTGGCAACAAGCTGAGCCGGGCTGACATTCATCTGGTGGAACTTCTCTACT
ACGTCGAGGAGCTTGACTCCAGTCTTATCTCCAGCTTCCCTCTGCTGAAG
GCCCTGAAAACCAGAATCAGCAACCTGCCCACAGTGAAGAAGTTTCTACA
GCCTGGCAGCCCAAGGAAGCCTCCCATGGATGAGAAATCTTTAGAAGAAG
CAAGGAAGATTTTCAGGTTTTAATAACGCAGTCATGGAGGCCAAGAACTTG
CAATACCAATGTTCTAAAGTTTTGCAACAATAAAGTACTTTACCTAAGTGTT
GATTGTGCCTGTTGTGAAGCTAATGAACTCTTTCAAATTATATGCTAATTAA
ATAATACAACTCCTATTCGCTGACTTAGTTAAAATTGATTTGTTTTCATTAGG
ATCTGATGTGAATTCAGATTTCCAATCTTCTCCTAGCCAACCATTTTCCTGG

AATTAAAAATTCAGTAAAAAAGGAAACTATAGATTATGTGGTTTGTTTGACT
TTTCCAAGAATTGTCCCGTAACATACAATTTGTCATACAATCTATTAAAATGT
CAATGTAGAAATGCACTTCTGACATTTTCAGGTATGCACAGGAGAAGAGTT
ACCATCCTGGATAATGGCATAAAGACATTTTCTTCTTTTCCTGGACAGTCAT
TTTATTTCTGATAAAAGCGTTCTTTCTTATGCATTTGCAAAA

[SEQ ID No: 9]

Accordingly, preferably GSTA1 comprises or consists of a nucleotide sequence as substantially as set out in SEQ ID NO: 9, or a fragment or variant thereof.

In one embodiment, NT-proBNP is provided by gene bank locus ID: HGNC: 7940; Entrez Gene: 4879; Ensembl: ENSG00000120937; OMIM: 600295; and/or UniProtKB: P16860.The protein sequence may be represented by the GeneBank ID P16860, which is provided herein as SEQ ID No: 10, as follows:

MDPQTAPSRALLLLLFLHLAFLGGRSHPLGSPGSASDLETSGLQEQRNHLQG
KLSELQVEQTSLEPLQESPRPTGVWKSREVATEGIRGHRKMVLYTLRAPRSP
KMVQGSGCFGRKMDRISSSSGLGCKVLRRH
[SEQ ID No: 10]

Accordingly, preferably NT-proBNP comprises or consists of an amino acid sequence substantially as set out in SEQ ID NO: 10, or a fragment or variant thereof.

In one embodiment NT-proBNP is encoded by a nucleotide sequence which is provided herein as SEQ ID No: 11, as follows:

AGGAGGAGCACCCCGCAGGCTGAGGGCAGGTGGGAAGCAAACCCGGAC
GCATCGCAGCAGCAGCAGCAGCAGCAGAAGCAGCAGCAGCAGCCTCCGC
AGTCCCTCCAGAGACATGGATCCCCAGACAGCACCTTCCCGGCGCTCCT
GCTCCTGCTCTTCTTGCATCTGGCTTTCCTGGGAGGTCGTTCCCACCCGC
TGGGCAGCCCCGGTTCAGCCTCGGACTTGGAAACGTCCGGGTTACAGGA
GCAGCGCAACCATTTGCAGGGCAAACTGTCGGAGCTGCAGGTGGAGCAG
ACATCCCTGGAGCCCCTCCAGGAGAGCCCCCGTCCCACAGGTGTCTGGA

AGTCCCGGGAGGTAGCCACCGAGGGCATCCGTGGGCACCGCAAAATGGT
CCTCTACACCCTGCGGCACCACGAAGCCCCAAGATGGTGCAAGGGTCT
GGCTGCTTTGGGAGGAAGATGGACCGGATCAGCTCCTCCAGTGGCCTGG
GCTGCAAAGTGCTGAGGCGGCATTAAGAGGAAGTCCTGGCTGCAGACAC
CTGCTTCTGATTCCACAAGGGGCTTTTTCCTCAACCCTGTGGCCGCCTTTG
AAGTGACTCATTTTTTTAATGTATTTATGTATTTATTTGATTGTTTTATATAAG
ATGGTTTCTTACCTTTGAGCACAAAATTTCCACGGTGAAATAAAGTCAACAT
TATAAGCTTTA
[SEQ
ID No: 11]

Accordingly, preferably NT-proBNP comprises or consists of a nucleotide sequence substantially as set out in SEQ ID NO: 11, or a fragment or variant thereof.

In one embodiment, TNC is provided by gene bank locus ID: HGNC: 5318; Entrez Gene: 3371; Ensembl: ENSG00000041982; OMIM: 187380; and/or UniProtKB: P24821. The protein sequence may be represented by the GeneBank ID P24821, which is provided herein as SEQ ID No: 12, as follows: 



















 























[SEQ ID No: 12]
 Accordingly, preferably TNC comprises or consists of an amino acid sequence substantially as set out in SEQ ID NO: 12, or a fragment or variant thereof.

In one embodiment TNC is encoded by a nucleotide sequence which is provided herein as SEQ ID No: 13, as follows:

GGCCACAGCCTGCCTACTGTCACCCGCCTCTCCCGCGCAGATACACG
CCCCCGCCTCCGTGGGCACAAAGGCAGCGCTGCTGGGGAACTCGGGGG
AACGCGCACGTGGGAACCGCCGCAGCTCCACACTCCAGGTACTTCTTCCA
AGGACCTAGGTCTCTCGCCCATCGGAAAGAAAATAATTCTTTCAAGAAGAT
CAGGGACAACTGATTTGAAGTCTACTCTGTGCTTCTAAATCCCCAATTCTG
CTGAAAGTGAGATACCCTAGAGCCCTAGAGCCCCAGCAGCACCCAGCCAA
ACCCACCTCCACCATGGGGGCCATGACTCAGCTGTTGGCAGGTGTCTTTC
TTGCTTTCCTTGCCCTCGCTACCGAAGGTGGGGTCCTCAAGAAAGTCATC
CGGCACAAGCGACAGAGTGGGGTGAACGCCACCCTGCCAGAAGAGAACC

AGCCAGTGGTGTTTAACCACGTTTACAACATCAAGCTGCCAGTGGATCC
CAGTGTTCGGTGGATCTGGAGTCAGCCAGTGGGGAGAAAGACCTGGCAC
CGCCTTCAGAGCCCAGCGAAAGCTTTCAGGAGCACACAGTGGATGGGGA
AAACCAGATTGTCTTCACACATCGCATCAACATCCCCCGCCGGGCCTGTG
GCTGTGCCGCAGCCCCTGATGTTAAGGAGCTGCTGAGCAGACTGGAGGA
GCTGGAGAACCTGGTGTCTTCCCTGAGGGAGCAATGTACTGCAGGAGCAG
GCTGCTGTCTCCAGCCTGCCACAGGCCGCTTGGACACCAGGCCCTTCTGT
AGCGGTCGGGGCAACTTCAGCACTGAAGGATGTGGCTGTGTCTGCGAAC
CTGGCTGGAAAGGCCCCAACTGCTCTGAGCCCGAATGTCCAGGCAACTGT
CACCTTCGAGGCCGGTGCATTGATGGGCAGTGCATCTGTGACGACGGCTT
CACGGGCGAGGACTGCAGCCAGCTGGCTTGCCCCAGCGACTGCAATGAC
CAGGGCAAGTGCGTAAATGGAGTCTGCATCTGTTTCGAAGGCTACGCCGG
GGCTGACTGCAGCCGTGAAATCTGCCCAGTGCCCTGCAGTGAGGAGCAC
GGCACATGTGTAGATGGCTTGTGTGTGTGCCACGATGGCTTTGCAGGCGA
TGACTGCAACAAGCCTCTGTGTCTCAACAATTGCTACAACCGTGGACGATG
CGTGGAGAATGAGTGCGTGTGTGATGAGGGTTTCACGGGCGAAGACTGC
AGTGAGCTCATCTGCCCCAATGACTGCTTCGACCGGGGCCGCTGCATCAA
TGGCACCTGCTACTGCGAAGAAGGCTTCACAGGTGAAGACTGCGGGAAAC
CCACCTGCCCACATGCCTGCCACACCCAGGGCCGGTGTGAGGAGGGGCA
GTGTGTATGTGATGAGGGCTTTGCCGGTGTGGACTGCAGCGAGAAGAGGT
GTCCTGCTGACTGTCACAATCGTGGCCGCTGTGTAGACGGGCGGTGTGA
GTGTGATGATGGTTTCACTGGAGCTGACTGTGGGGAGCTCAAGTGTCCCA
ATGGCTGCAGTGGCCATGGCCGCTGTGTCAATGGGCAGTGTGTGTGTGAT
GAGGGCTATACTGGGGAGGACTGCAGCCAGCTACGGTGCCCCAATGACT
GTCACAGTCGGGGCCGCTGTGTCGAGGGCAAATGTGTATGTGAGCAAGG
CTTCAAGGGCTATGACTGCAGTGACATGAGCTGCCCTAATGACTGTCACC
AGCACGGCCGCTGTGTGAATGGCATGTGTGTTTGTGATGACGGCTACACA
GGGGAAGACTGCCGGGATCGCCAATGCCCCAGGGACTGCAGCAACAGGG
GCCTCTGTGTGGACGGACAGTGCGTCTGTGAGGACGGCTTCACCGGCCC
TGACTGTGCAGAACTCTCCTGTCCAAATGACTGCCATGGCCAGGGTCGCT
GTGTGAATGGGCAGTGCGTGTGCCATGAAGGATTTATGGGCAAAGACTGC
AAGGAGCAAAGATGTCCCAGTGACTGTCATGGCCAGGGCCGCTGCGTGG

ACGGCCAGTGCATCTGCCACGAGGGCTTCACAGGCCTGGACTGTGGCCA
GCACTCCTGCCCCAGTGACTGCAACAACTTAGGACAATGCGTCTCGGGCC
GCTGCATCTGCAACGAGGGCTACAGCGGAGAAGACTGCTCAGAGGTGTCT
CCTCCCAAAGACCTCGTTGTGACAGAAGTGACGGAAGAGACGGTCAACCT
GGCCTGGGACAATGAGATGCGGGTCACAGAGTACCTTGTCGTGTACACGC
CCACCCACGAGGGTGGTCTGGAAATGCAGTTCCGTGTGCCTGGGGACCA
GACGTCCACCATCATCCAGGAGCTGGAGCCTGGTGTGGAGTACTTTATCC
GTGTATTTGCCATCCTGGAGAACAAGAAGAGCATTCCTGTCAGCGCCAGG
GTGGCCACGTACTTACCTGCACCTGAAGGCCTGAAATTCAAGTCCATCAA
GGAGACATCTGTGGAAGTGGAGTGGGATCCTCTAGACATTGCTTTTGAAA
CCTGGGAGATCATCTTCCGGAATATGAATAAAGAAGATGAGGGAGAGATC
ACCAAAAGCCTGAGGAGGCCAGAGACCTCTTACCGGCAAACTGGTCTAGC
TCCTGGGCAAGAGTATGAGATATCTCTGCACATAGTGAAAAACAATACCCG
GGGCCCTGGCCTGAAGAGGGTGACCACCACACGCTTGGATGCCCCCAGC
CAGATCGAGGTGAAAGATGTCACAGACACCACTGCCTTGATCACCTGGTT
CAAGCCCCTGGCTGAGATCGATGGCATTGAGCTGACCTACGGCATCAAAG
ACGTGCCAGGAGACCGTACCACCATCGATCTCACAGAGGACGAGAACCAG
TACTCCATCGGGAACCTGAAGCCTGACACTGAGTACGAGGTGTCCCTCAT
CTCCCGCAGAGGTGACATGTCAAGCAACCCAGCCAAAGAGACCTTCACAA
CAGGCCTCGATGCTCCCAGGAATCTTCGACGTGTTTCCCAGACAGATAAC
AGCATCACCCTGGAATGGAGGAATGGCAAGGCAGCTATTGACAGTTACAG
AATTAAGTATGCCCCCATCTCTGGAGGGGACCACGCTGAGGTTGATGTTC
CAAAGAGCCAACAAGCCACAACCAAAACCACACTCACAGGTCTGAGGCCG
GGAACTGAATATGGGATTGGAGTTTCTGCTGTGAAGGAAGACAAGGAGAG
CAATCCAGCGACCATCAACGCAGCCACAGAGTTGGACACGCCCAAGGACC
TTCAGGTTTCTGAAACTGCAGAGACCAGCCTGACCCTGCTCTGGAAGACA
CCGTTGGCCAAATTTGACCGCTACCGCCTCAATTACAGTCTCCCCACAGG
CCAGTGGGTGGGAGTGCAGCTTCCAAGAAACACCACTTCCTATGTCCTGA
GAGGCCTGGAACCAGGACAGGAGTACAATGTCCTCCTGACAGCCGAGAAA
GGCAGACACAAGAGCAAGCCCGCACGTGTGAAGGCATCCACTGAACAAG
CCCCTGAGCTGGAAAACCTCACCGTGACTGAGGTTGGCTGGGATGGCCTC
AGACTCAACTGGACCGCAGCTGACCAGGCCTATGAGCACTTTATCATTCA

GGTGCAGGAGGCCAACAAGGTGGAGGCAGCTCGGAACCTCACCGTGCCT
GGCAGCCTTCGGGCTGTGGACATACCGGGCCTCAAGGCTGCTACGCCTT
ATACAGTCTCCATCTATGGGGTGATCCAGGGCTATAGAACACCAGTGCTCT
CTGCTGAGGCCTCCACAGGGGAAACTCCCAATTTGGGAGAGGTCGTGGT
GGCCGAGGTGGGCTGGGATGCCCTCAAACTCAACTGGACTGCTCCAGAA
GGGGCCTATGAGTACTTTTTCATTCAGGTGCAGGAGGCTGACACAGTAGA
GGCAGCCCAGAACCTCACCGTCCCAGGAGGACTGAGGTCCACAGACCTG
CCTGGGCTCAAAGCAGCCACTCATTATACCATCACCATCCGCGGGGTCAC
TCAGGACTTCAGCACAACCCCTCTCTCTGTTGAAGTCTTGACAGAGGAGGT
TCCAGATATGGGAAACCTCACAGTGACCGAGGTTAGCTGGGATGCTCTCA
GACTGAACTGGACCACGCCAGATGGAACCTATGACCAGTTTACTATTCAG
GTCCAGGAGGCTGACCAGGTGGAAGAGGCTCACAATCTCACGGTTCCTG
GCAGCCTGCGTTCCATGGAAATCCCAGGCCTCAGGGCTGGCACTCCTTAC
ACAGTCACCCTGCACGGCGAGGTCAGGGGCCACAGCACTCGACCCCTTG
CTGTAGAGGTCGTCACAGAGGATCTCCCACAGCTGGGAGATTTAGCCGTG
TCTGAGGTTGGCTGGGATGGCCTCAGACTCAACTGGACCGCAGCTGACAA
TGCCTATGAGCACTTTGTCATTCAGGTGCAGGAGGTCAACAAAGTGGAGG
CAGCCCAGAACCTCACGTTGCCTGGCAGCCTCAGGGCTGTGGACATCCC
GGGCCTCGAGGCTGCCACGCCTTATAGAGTCTCCATCTATGGGGTGATCC
GGGGCTATAGAACACCAGTACTCTCTGCTGAGGCCTCCACAGCCAAAGAA
CCTGAAATTGGAAACTTAAATGTTTCTGACATAACTCCCGAGAGCTTCAATC
TCTCCTGGATGGCTACCGATGGGATCTTCGAGACCTTTACCATTGAAATTA
TTGATTCCAATAGGTTGCTGGAGACTGTGGAATATAATATCTCTGGTGCTG
AACGAACTGCCCATATCTCAGGGCTACCCCCTAGTACTGATTTTATTGTCT
ACCTCTCTGGACTTGCTCCCAGCATCCGGACCAAAACCATCAGTGCCACA
GCCACGACAGAGGCCCTGCCCCTTCTGGAAAACCTAACCATTTCCGACAT
TAATCCCTACGGGTTCACAGTTTCCTGGATGGCATCGGAGAATGCCTTTGA
CAGCTTTCTAGTAACGGTGGTGGATTCTGGGAAGCTGCTGGACCCCCAGG
AATTCACACTTTCAGGAACCCAGAGGAAGCTGGAGCTTAGAGGCCTCATA
ACTGGCATTGGCTATGAGGTTATGGTCTCTGGCTTCACCCAAGGGCATCA
AACCAAGCCCTTGAGGGCTGAGATTGTTACAGAAGCCGAACCGGAAGTTG
ACAACCTTCTGGTTTCAGATGCCACCCCAGACGGTTTCCGTCTGTCCTGGA

CAGCTGATGAAGGGGTCTTCGACAATTTTGTTCTCAAAATCAGAGATACCA
AAAAGCAGTCTGAGCCACTGGAAATAACCCTACTTGCCCCCGAACGTACC
AGGGACATAACAGGTCTCAGAGAGGCTACTGAATACGAAATTGAACTCTAT
GGAATAAGCAAAGGAAGGCGATCCCAGACAGTCAGTGCTATAGCAACAAC
AGCCATGGGCTCCCCAAAGGAAGTCATTTTCTCAGACATCACTGAAAATTC
GGCTACTGTCAGCTGGAGGGCACCCACAGCCCAAGTGGAGAGCTTCCGG
ATTACCTATGTGCCCATTACAGGAGGTACACCCTCCATGGTAACTGTGGAC
GGAACCAAGACTCAGACCAGGCTGGTGAAACTCATACCTGGCGTGGAGTA
CCTTGTCAGCATCATCGCCATGAAGGGCTTTGAGGAAAGTGAACCTGTCT
CAGGGTCATTCACCACAGCTCTGGATGGCCCATCTGGCCTGGTGACAGCC
AACATCACTGACTCAGAAGCCTTGGCCAGGTGGCAGCCAGCCATTGCCAC
TGTGGACAGTTATGTCATCTCCTACACAGGCGAGAAAGTGCCAGAAATTAC
ACGCACGGTGTCCGGGAACACAGTGGAGTATGCTCTGACCGACCTCGAG
CCTGCCACGGAATACACACTGAGAATCTTTGCAGAGAAAGGGCCCCAGAA
GAGCTCAACCATCACTGCCAAGTTCACAACAGACCTCGATTCTCCAAGAGA
CTTGACTGCTACTGAGGTTCAGTCGGAAACTGCCCTCCTTACCTGGCGAC
CCCCCCGGGCATCAGTCACCGGTTACCTGCTGGTCTATGAATCAGTGGAT
GGCACAGTCAAGGAAGTCATTGTGGGTCCAGATACCACCTCCTACAGCCT
GGCAGACCTGAGCCCATCCACCCACTACACAGCCAAGATCCAGGCACTCA
ATGGGCCCCTGAGGAGCAATATGATCCAGACCATCTTCACCACAATTGGA
CTCCTGTACCCCTTCCCCAAGGACTGCTCCCAAGCAATGCTGAATGGAGA
CACGACCTCTGGCCTCTACACCATTTATCTGAATGGTGATAAGGCTGAGGC
GCTGGAAGTCTTCTGTGACATGACCTCTGATGGGGGTGGATGGATTGTGT
TCCTGAGACGCAAAAACGGACGCGAGAACTTCTACCAAAACTGGAAGGCA
TATGCTGCTGGATTTGGGGACCGCAGAGAAGAATTCTGGCTTGGGCTGGA
CAACCTGAACAAAATCACAGCCCAGGGGCAGTACGAGCTCCGGGTGGAC
CTGCGGGACCATGGGGAGACAGCCTTTGCTGTCTATGACAAGTTCAGCGT
GGGAGATGCCAAGACTCGCTACAAGCTGAAGGTGGAGGGGTACAGTGGG
ACAGCAGGTGACTCCATGGCCTACCACAATGGCAGATCCTTCTCCACCTTT
GACAAGGACACAGATTCAGCCATCACCAACTGTGCTCTGTCCTACAAAGG
GGCTTTCTGGTACAGGAACTGTCACCGTGTCAACCTGATGGGGAGATATG
GGGACAATAACCACAGTCAGGGCGTTAACTGGTTCCACTGGAAGGGCCAC

GAACACTCAATCCAGTTTGCTGAGATGAAGCTGAGACCAAGCAACTTCAGA
AATCTTGAAGGCAGGCGCAAACGGGCATAAATTCCAGGGACCACTGGGTG
AGAGAGGAATAAGGCCCAGAGCGAGGAAAGGATTTTACCAAAGCATCAAT
ACAACCAGCCCAACCATCGGTCCACACCTGGGCATTTGGTGAGAGTCAAA
GCTGACCATGGATCCCTGGGGCCAACGGCAACAGCATGGGCCTCACCTC
CTCTGTGATTTCTTTCTTTGCACCAAAGACATCAGTCTCCAACATGTTTCTG
TTTTGTTGTTTGATTCAGCAAAAATCTCCCAGTGACAACATCGCAATAGTTT
TTTACTTCTCTTAGGTGGCTCTGGGAATGGAGAGGGTAGGATGTACAG
GGGTAGTTTGTTTTAGAACCAGCCGTATTTTACATGAAGCTGTATAATTAAT
TGTCATTATTTTTGTTAGCAAAGATTAAATGTGTCATTGGAAGCCATCCCTT
TTTTTACATTTCATACAACAGAACCAGAAAAGCAATACTGTTTCCATTTTAA
GGATATGATTAATATTATTAATATAATAATGATGATGATGATGATGAAAACTA
AGGATTTTTCAAGAGATCTTTCTTTCCAAAACATTTCTGGACAGTACCTGAT
TGTATTTTTTTTTAAATAAAAGCACAAGTACTTTTGAGTTTGTTATTTTGCTT
TGAATTGTTGAGTCTGAATTTCACCAAAGCCAATCATTTGAACAAAGCGGG
GAATGTTGGGATAGGAAAGGTAAGTAGGGATAGTGGTCAAGTGGGAGGG
GTGGAAAGGAGACTAAAGACTGGGAGAGAGGGAAGCACTTTTTTTAAATAA
AGTTGAACACACTTGGGAAAAGCTTACAGGCCAGGCCTGTAATCCCAACA
CTTTGGGAGGCCAAGGTGGGAGGATAGCTTAACCCCAGGAGTTTGAGACC
AGCCTGAGCAACATAGTGAGAACTTGTCTCTACAGAAAAAAAAAAAAAAA
AAATTTAATTAGGCAAGCGTGGTAGTGCGCACCTGTCGTCCCAGCTACTCA
GGAGGCTGAGGTAGGAAAATCACTGGAGCCCAGGAGTTAGAGGTTACAGT
GAGCTATGATCACACTACTGCACTCCAGCCTGGGCAACAGAGGGAGACCC
TGTCTCTAAATAAAAAAAGAAAAGAAAAAAAAAGCTTACAACTTGAGATTCA
GCATCTTGCTCAGTATTTCCAAGACTAATAGATTATGGTTTAAAAGATGCTT
TTATACTCATTTTCTAATGCAACTCCTAGAAACTCTATGATATAGTTGAGGT
AAGTATTGTTACCACACATGGGCTAAGATCCCCAGAGGCAGACTGCCTGA
GTTCAATTCTTGGCTCCACCATTCCCAAGTTCCCTAACCTCTCTATGCCTCA
GTTTCCTCTTCTGTAAAGTAGGGACACTCATACTTCTCATTTCAGAACATTT
TTGTGAAGAATAAATTATGTTATCCATTTGAGGCCCTTAGAATGGTACCCG
GTGTATATTAAGTGCTAGTACATGTTAGCTATCATCATTATCACTTTATATGA
GATGGACTGGGGTTCATAGAAACCCAATGACTTGATTGTGGCTACTACTCA

ATAAATAATAGAATTTGGATTTAAA

[SEQ ID No: 13]

Accordingly, preferably TNC comprises or consists of a nucleotide sequence substantially as set out in SEQ ID NO: 13, or a fragment or variant thereof.

In one embodiment, A2M is provided by gene bank locus ID: HGNC: 7; Entrez Gene: 2; Ensembl: ENSG00000175899; OMIM: 103950; and/or UniProtKB: P01023. The protein sequence may be represented by the GeneBank ID P01023, which is provided herein as SEQ ID No: 14, as follows:

MGKNKLLHPSLVLLLLVLLPTDASVSGKPQYMVLVPSLLHTETTEKGCVLLSYL
NETVTVSASLESVRGNRSLFTDLEAENDVLHCVAFAVPKSSSNEEVMFLTVQV
KGPTQEFKKRTTVMVKNEDSLVFVQTDKSIYKPGQTVKFRVVSMDENFHPLN
ELIPLVYIQDPKGNRIAQWQSFQLEGGLKQFSFPLSSEPFQGSYKVVVQKKSG
GRTEHPFTVEEFVLPKFEVQVTVPKIITILEEEMNVSVCGLYTYGKPVPGHVTV
SICRKYSDASDCHGEDSQAFCEKFSGQLNSHGCFYQQVKTKVFQLKRKEYE
MKLHTEAQIQEEGTVVELTGRQSSEITRTITKLSFVKVDSHFRQGIPFFGQVRL
VDGKGVPIPNKVIFIRGNEANYYSNATTDEHGLVQFSINTTNVMGTSLTVRVNY
KDRSPCYGYQWVSEEHEEAHHTAYLVFSPSKSFVHLEPMSHELPCGHTQTV
QAHYILNGGTLLGLKKLSFYYLIMAKGGIVRTGTHGLLVKQEDMKGHFSISIPVK
SDIAPVARLLIYAVLPTGDVIGDSAKYDVENCLANKVDLSFSPSQSLPASHAHL
RVTAAPQSVCALRAVDQSVLLMKPDAELSASSVYNLLPEKDLTGFPGPLNDQ
DNEDCINRHNVYINGITYTPVSSTNEKDMYSFLEDMGLKAFTNSKIRKPKMCP
QLQQYEMHGPEGLRVGFYESDVMGRGHARLVHVEEPHTETVRKYFPETWIW
DLVVVNSAGVAEVGVTVPDTITEWKAGAFCLSEDAGLGISSTASLRAFQPFFV
ELTMPYSVIRGEAFTLKATVLNYLPKCIRVSVQLEASPAFLAVPVEKEQAPHCI
CANGRQTVSWAVTPKSLGNVNFTVSAEALESQELCGTEVPSVPEHGRKDTVI
KPLLVEPEGLEKETTFNSLLCPSGGEVSEELSLKLPPNVVEESARASVSVLGDI
LGSAMQNTQNLLQMPYGCGEQNMVLFAPNIYVLDYLNETQQLTPEIKSKAIGY
LNTGYQRQLNYKHYDGSYSTFGERYGRNQGNTWLTAFVLKTFAQARAYIFID
EAHITQALIWLSQRQKDNGCFRSSGSLLNNAIKGGVEDEVTLSAYITIALLEIPLT
VTHPVVRNALFCLESAWKTAQEGDHGSHVYTKALLAYAFALAGNQDKRKEVL
KSLNEEAVKKDNSVHWERPQKPKAPVGHFYEPQAPSAEVEMTSYVLLAYLTA
QPAPTSEDLTSATNIVKWITKQQNAQGGFSSTQDTVVALHALSKYGAATFTRT
GKAAQVTIQSSGTFSSKFQVDNNNRLLLQQVSLPELPGEYSMKVTGEGCVYL
QTSLKYNILPEKEEFPFALGVQTLPQTCDEPKAHTSFQISLSVSYTGSRSASNM
AIVDVKMVSGFIPLKPTVKMLERSNHVSRTEVSSNHVLIYLDKVSNQTLSLFFT
VLQDVPVRDLKPAIVKVYDYYETDEFAIAEYNAPCSKDLGNA

[SEQ ID No: 14]

Accordingly, preferably A2M comprises or consists of an amino acid sequence substantially as set out in SEQ ID NO: 14, or a fragment or variant thereof.

In one embodiment A2M is encoded by a nucleotide sequence which is provided herein as SEQ ID No: 15, as follows:

GAAAAGCTTATTAGCTGCTGTACGGTAAAAGTGAGCTCTTACGGGAATGG
GAATGTAGTTTTAGCCCTCCAGGGATTCTATTTAGCCCGCCAGGAATTAAC
CTTGACTATAAATAGGCCATCAATGACCTTTCCAGAGAATGTTCAGAGACC
TCAACTTTGTTTAGAGATCTTGTGTGGGTGGAACTTCCTGTTTGCACACAG
AGCAGCATAAAGCCCAGTTGCTTTGGGAAGTGTTTGGGACCAGATGGATT
GTAGGGAGTAGGGTACAATACAGTCTGTTCTCCTCCAGCTCCTTCTTTCTG
CAACATGGGGAAGAACAAACTCCTTCATCCAAGTCTGGTTCTTCTCCTCTT
GGTCCTCCTGCCCACAGACGCCTCAGTCTCTGGAAAACCGCAGTATATGG
TTCTGGTCCCCTCCCTGCTCCACACTGAGACCACTGAGAAGGGCTGTGTC
CTTCTGAGCTACCTGAATGAGACAGTGACTGTAAGTGCTTCCTTGGAGTCT
GTCAGGGGAAACAGGAGCCTCTTCACTGACCTGGAGGCGGAGAATGACG
TACTCCACTGTGTCGCCTTCGCTGTCCCAAAGTCTTCATCCAATGAGGAGG
TAATGTTCCTCACTGTCCAAGTGAAAGGACCAACCCAAGAATTTAAGAAGC
GGACCACAGTGATGGTTAAGAACGAGGACAGTCTGGTCTTTGTCCAGACA
GACAAATCAATCTACAAACCAGGGCAGACAGTGAAATTTCGTGTTGTCTCC
ATGGATGAAAACTTTCACCCCCTGAATGAGTTGATTCCACTAGTATACATTC
AGGATCCCAAAGGAAATCGCATCGCACAATGGCAGAGTTTCCAGTTAGAG
GGTGGCCTCAAGCAATTTTCTTTTCCCCTCTCATCAGAGCCCTTCCAGGGC
TCCTACAAGGTGGTGGTACAGAAGAAATCAGGTGGAAGGACAGAGCACCC
TTTCACCGTGGAGGAATTTGTTCTTCCCAAGTTTGAAGTACAAGTAACAGT
GCCAAAGATAATCACCATCTTGGAAGAAGAGATGAATGTATCAGTGTGTGG
CCTATACACATATGGGAAGCCTGTCCCTGGACATGTGACTGTGAGCATTTG
CAGAAAGTATAGTGACGCTTCCGACTGCCACGGTGAAGATTCACAGGCTT
TCTGTGAGAAATTCAGTGGACAGCTAAACAGCCATGGCTGCTTCTATCAGC
AAGTAAAAACCAAGGTCTTCCAGCTGAAGAGGAAGGAGTATGAAATGAAAC
TTCACACTGAGGCCCAGATCCAAGAAGAAGGAACAGTGGTGGAATTGACT
GGAAGGCAGTCCAGTGAAATCACAAGAACCATAACCAAACTCTCATTTGTG
AAAGTGGACTCACACTTTCGACAGGGAATTCCCTTCTTTGGGCAGGTGCG

CCTAGTAGATGGGAAAGGCGTCCCTATACCAAATAAAGTCATATTCATCAG
AGGAAATGAAGCAAACTATTACTCCAATGCTACCACGGATGAGCATGGCCT
TGTACAGTTCTCTATCAACACCACCAATGTTATGGGTACCTCTCTTACTGTT
AGGGTCAATTACAAGGATCGTAGTCCCTGTTACGGCTACCAGTGGGTGTC
AGAAGAACACGAAGAGGCACATCACACTGCTTATCTTGTGTTCTCCCCAAG
CAAGAGCTTTGTCCACCTTGAGCCCATGTCTCATGAACTACCCTGTGGCCA
TACTCAGACAGTCCAGGCACATTATATTCTGAATGGAGGCACCCTGCTGG
GGCTGAAGAAGCTCTCCTTCTATTATCTGATAATGGCAAAGGGAGGCATTG
TCCGAACTGGGACTCATGGACTGCTTGTGAAGCAGGAAGACATGAAGGGC
CATTTTTCCATCTCAATCCCTGTGAAGTCAGACATTGCTCCTGTCGCTCGG
TTGCTCATCTATGCTGTTTTACCTACCGGGGACGTGATTGGGGATTCTGCA
AAATATGATGTTGAAAATTGTCTGGCCAACAAGGTGGATTTGAGCTTCAGC
CCATCACAAAGTCTCCCAGCCTCACACGCCCACCTGCGAGTCACAGCGGC
TCCTCAGTCCGTCTGCGCCCTCCGTGCTGTGGACCAAAGCGTGCTGCTCA
TGAAGCCTGATGCTGAGCTCTCGGCGTCCTCGGTTTACAACCTGCTACCA
GAAAAGGACCTCACTGGCTTCCCTGGGCCTTTGAATGACCAGGACAATGA
AGACTGCATCAATCGTCATAATGTCTATATTAATGGAATCACATATACTCCA
GTATCAAGTACAAATGAAAAGGATATGTACAGCTTCCTAGAGGACATGGGC
TTAAAGGCATTCACCAACTCAAAGATTCGTAAACCCAAAATGTGTCCACAG
CTTCAACAGTATGAAATGCATGGACCTGAAGGTCTACGTGTAGGTTTTTAT
GAGTCAGATGTAATGGGAAGAGGCCATGCACGCCTGGTGCATGTTGAAGA
GCCTCACACGGAGACCGTACGAAAGTACTTCCCTGAGACATGGATCTGGG
ATTTGGTGGTGGTAAACTCAGCAGGTGTGGCTGAGGTAGGAGTAACAGTC
CCTGACACCATCACCGAGTGGAAGGCAGGGGCCTTCTGCCTGTCTGAAGA
TGCTGGACTTGGTATCTCTTCCACTGCCTCTCTCCGAGCCTTCCAGCCCTT
CTTTGTGGAGCTCACAATGCCTTACTCTGTGATTCGTGGAGAGGCCTTCAC
ACTCAAGGCCACGGTCCTAAACTACCTTCCCAAATGCATCCGGGTCAGTG
TGCAGCTGGAAGCCTCTCCCGCCTTCCTAGCTGTCCCAGTGGAGAAGGAA
CAAGCGCCTCACTGCATCTGTGCAAACGGGCGGCAAACTGTGTCCTGGGC
AGTAACCCCAAAGTCATTAGGAAATGTGAATTTCACTGTGAGCGCAGAGGC
ACTAGAGTCTCAAGAGCTGTGTGGGACTGAGGTGCCTTCAGTTCCTGAAC
ACGGAAGGAAAGACACAGTCATCAAGCCTCTGTTGGTTGAACCTGAAGGA

CTAGAGAAGGAAACAACATTCAACTCCCTACTTTGTCCATCAGGTGGTGAG
GTTTCTGAAGAATTATCCCTGAAACTGCCACCAAATGTGGTAGAAGAATCT
GCCCGAGCTTCTGTCTCAGTTTTGGGAGACATATTAGGCTCTGCCATGCAA
AACACACAAAATCTTCTCCAGATGCCCTATGGCTGTGGAGAGCAGAATATG
GTCCTCTTTGCTCCTAACATCTATGTACTGGATTATCTAAATGAAACACAGC
AGCTTACTCCAGAGATCAAGTCCAAGGCCATTGGCTATCTCAACACTGGTT
ACCAGAGACAGTTGAACTACAAACACTATGATGGCTCCTACAGCACCTTTG
GGGAGCGATATGGCAGGAACCAGGGCAACACCTGGCTCACAGCCTTTGTT
CTGAAGACTTTTGCCCAAGCTCGAGCCTACATCTTCATCGATGAAGCACAC
ATTACCCAAGCCCTCATATGGCTCTCCCAGAGGCAGAAGGACAATGGCTG
TTTCAGGAGCTCTGGGTCACTGCTCAACAATGCCATAAAGGGAGGAGTAG
AAGATGAAGTGACCCTCTCCGCCTATATCACCATCGCCCTTCTGGAGATTC
CTCTCACAGTCACTCACCCTGTTGTCCGCAATGCCCTGTTTTGCCTGGAGT
CAGCCTGGAAGACAGCACAAGAAGGGGACCATGGCAGCCATGTATATACC
AAAGCACTGCTGGCCTATGCTTTTGCCCTGGCAGGTAACCAGGACAAGAG
GAAGGAAGTACTCAAGTCACTTAATGAGGAAGCTGTGAAGAAAGACAACTC
TGTCCATTGGGAGCGCCCTCAGAAACCCAAGGCACCAGTGGGGCATTTTT
ACGAACCCCAGGCTCCCTCTGCTGAGGTGGAGATGACATCCTATGTGCTC
CTCGCTTATCTCACGGCCCAGCCAGCCCCAACCTCGGAGGACCTGACCTC
TGCAACCAACATCGTGAAGTGGATCACGAAGCAGCAGAATGCCCAGGGCG
GTTTCTCCTCCACCCAGGACACAGTGGTGGCTCTCCATGCTCTGTCCAAAT
ATGGAGCAGCCACATTTACCAGGACTGGGAAGGCTGCACAGGTGACTATC
CAGTCTTCAGGGACATTTTCCAGCAAATTCCAAGTGGACAACAACAACCGC
CTGTTACTGCAGCAGGTCTCATTGCCAGAGCTGCCTGGGGAATACAGCAT
GAAAGTGACAGGAGAAGGATGTGTCTACCTCCAGACATCCTTGAAATACAA
TATTCTCCCAGAAAAGGAAGAGTTCCCCTTTGCTTTAGGAGTGCAGACTCT
GCCTCAAACTTGTGATGAACCCAAAGCCCACACCAGCTTCCAAATCTCCCT
AAGTGTCAGTTACACAGGGAGCCGCTCTGCCTCCAACATGGCGATCGTTG
ATGTGAAGATGGTCTCTGGCTTCATTCCCCTGAAGCCAACAGTGAAAATGC
TTGAAAGATCTAACCATGTGAGCCGGACAGAAGTCAGCAGCAACCATGTC
TTGATTTACCTTGATAAGGTGTCAAATCAGACACTGAGCTTGTTCTTCACG
GTTCTGCAAGATGTCCCAGTAAGAGATCTGAAACCAGCCATAGTGAAAGTC

TATGATTACTACGAGACGGATGAGTTTGCAATTGCTGAGTACAATGCTCCT
TGCAGCAAAGATCTTGGAAATGCTTGAAGACCACAAGGCTGAAAAGTGCTT
TGCTGGAGTCCTGTTCTCAGAGCTCCACAGAAGACACGTGTTTTTGTATCT
TTAAAGACTTGATGAATAAACACTTTTTCTGGTCAAAGACCACAAGGCTGAA
AAGTGCTTTGCTGGAGTCCTGTTCTCAGAGCTCCACAGAAGACACGTGTTT
TTGTATCTTTAAAGACTTGATGAATAAACACTTTTTCTGGTCAA
[SEQ ID No: 15]

Accordingly, preferably A2M comprises or consists of a nucleotide sequence substantially as set out in SEQ ID NO: 15, or a fragment or variant thereof.

In one embodiment, IGFBP2 is provided by gene bank locus ID HGNC: 5471; Entrez Gene: 3485; Ensembl: ENSG00000115457; OMIM: 146731 and/or UniProtKB: P18065.

The protein sequence may be represented by the GeneBank ID P18065, which is provided herein as SEQ ID No: 16, as follows:

MLPRVGCPALPLPPPPLLPLLLLLLGASGGGGGARAEVLFRCPPCTPERLAAC
GPPPVAPPAAVAAVAGGARMPCAELVREPGCGCCSVCARLEGEACGVYTPR
CGQGLRCYPHPGSELPLQALVMGEGTCEKRRDAEYGASPEQVADNGDDHS
EGGLVENHVDSTMNMLGGGGSAGRKPLKSGMKELAVFREKVTEQHRQMGK
GGKHHLGLEEPKKLRPPPARTPCQQELDQVLERISTMRLPDERGPLEHLYSL
HIPNCDKHGLYNLKQCKMSLNGQRGECWCVNPNTGKLIQGAPTIRGDPECHL
FYNEQQEARGVHTQRMQ
[SEQ ID No: 16]

Accordingly, preferably IGFBP2 comprises or consists of an amino acid sequence substantially as set out in SEQ ID NO: 16 or a fragment or variant thereof.

In one embodiment IGFBP2 is encoded by a nucleotide sequence which is provided herein as SEQ ID No: 17, as follows:

GAGGAAGAAGCGGAGGAGGCGGCTCCCGCGCTCGCAGGGCCGTGCCAC
CTGCCCGCCCGCCCGCTCGCTCGCTCGCCCGCCGCGCCGCGCTGCCGA
CCGCCAGCATGCTGCCGAGAGTGGGCTGCCCCGCGCTGCCGCTGCCGC
CGCCGCCGCTGCTGCCGCTGCTGCTGCTGCTACTGGGCGCGAGTGGCGG
CGGCGGCGGGGCGCGCGCGGAGGTGCTGTTCCGCTGCCCGCCCTGCAC
ACCCGAGCGCCTGGCCGCCTGCGGGCCCCCGCCGGTTGCGCCGCCCGC
CGCGGTGGCCGCAGTGGCCGGAGGCGCCCGCATGCCATGCGCGGAGCT
CGTCCGGGAGCCGGGCTGCGGCTGCTGCTCGGTGTGCGCCCGGCTGGA
GGGCGAGGCGTGCGGCGTCTACACCCCGCGCTGCGGCCAGGGGCTGCG
CTGCTATCCCCACCCGGGCTCCGAGCTGCCCCTGCAGGCGCTGGTCATG
GGCGAGGGCACTTGTGAGAAGCGCCGGGACGCCGAGTATGGCGCCAGC
CCGGAGCAGGTTGCAGACAATGGCGATGACCACTCAGAAGGAGGCCTGG
TGGAGAACCACGTGGACAGCACCATGAACATGTTGGGCGGGGGAGGCAG
TGCTGGCCGGAAGCCCCTCAAGTCGGGTATGAAGGAGCTGGCCGTGTTC
CGGGAGAAGGTCACTGAGCAGCACCGGCAGATGGGCAAGGGTGGCAAGC
ATCACCTTGGCCTGGAGGAGCCCAAGAAGCTGCGACCACCCCCTGCCAG
GACTCCCTGCCAACAGGAACTGGACCAGGTCCTGGAGCGGATCTCCACCA
TGCGCCTTCCGGATGAGCGGGGCCCTCTGGAGCACCTCTACTCCCTGCA
CATCCCCAACTGTGACAAGCATGGCCTGTACAACCTCAAACAGTGCAAGAT
GTCTCTGAACGGGCAGCGTGGGGAGTGCTGGTGTGTGAACCCCAACACC
GGGAAGCTGATCCAGGGAGCCCCCACCATCCGGGGGGACCCCGAGTGTC
ATCTCTTCTACAATGAGCAGCAGGAGGCTCGCGGGGTGCACACCCAGCG
GATGCAGTAGACCGCAGCCAGCCGGTGCCTGGCGCCCCTGCCCCCCGCC
CCTCTCCAAACACCGGCAGAAAACGGAGAGTGCTTGGGTGGTGGGTGCT
GGAGGATTTTCCAGTTCTGACACACGTATTTATATTTGGAAAGAGACCAGC
ACCGAGCTCGGCACCTCCCCGGCCTCTCTCTTCCCAGCTGCAGATGCCAC
ACCTGCTCCTTCTTGCTTTCCCCGGGGGAGGAAGGGGGTTGTGGTCGGG
GAGCTGGGGTACAGGTTTGGGGAGGGGGAAGAGAAATTTTTATTTTTGAA
CCCCTGTGTCCCTTTTGCATAAGATTAAAGGAAGGAAAAGTAAA

[SEQ ID No: 17]

Accordingly, preferably IGFBP2 comprises or consists of a nucleotide sequence substantially as set out in SEQ ID NO: 17, or a fragment or variant thereof.

In one embodiment, APOB is provided by gene bank locus ID: HGNC: 603; Entrez Gene: 338; Ensembl: ENSG00000084674; OMIM: 107730; and/or UniProtKB: P04114. The protein sequence may be represented by the GeneBank ID P04114, which is provided herein as SEQ ID No: 18, as follows:

MDPPRPALLALLALPALLLLLLAGARAEEEMLENVSLVCPKDATRFKHLRKYTY
NYEAESSSGVPGTADSRSATRINCKVELEVPQLCSFILKTSQCTLKEVYGFNP
EGKALLKKTKNSEEFAAAMSRYELKLAIPEGKQVFLYPEKDEPTYILNIKRGIISA
LLVPPETEEAKQVLFLDTVYGNCSTHFTVKTRKGNVATEISTERDLGQCDRFK
PIRTGISPLALIKGMTRPLSTLISSSQSCQYTLDAKRKHVAEAICKEQHLFLPFS
YKNKYGMVAQVTQTLKLEDTPKINSRFFGEGTKKMGLAFESTKSTSPPKQAE
AVLKTLQELKKLTISEQNIQRANLFNKLVTELRGLSDEAVTSLLPQLIEVSSPITL
QALVQCGQPQCSTHILQWLKRVHANPLLIDVVTYLVALIPEPSAQQLREIFNMA
RDQRSRATLYALSHAVNNYHKTNPTGTQELLDIANYLMEQIQDDCTGDEDYTY
LILRVIGNMGQTMEQLTPELKSSILKCVQSTKPSLMIQKAAIQALRKMEPKDKD
QEVLLQTFLDDASPGDKRLAAYLMLMRSPSQADINKIVQILPWEQNEQVKNFV
ASHIANILNSEELDIQDLKKLVKEALKESQLPTVMDFRKFSRNYQLYKSVSLPSL
DPASAKIEGNLIFDPNNYLPKESMLKTTLTAFGFASADLIEIGLEGKGFEPTLEA
LFGKQGFFPDSVNKALYWVNGQVPDGVSKVLVDHFGYTKDDKHEQDMVNGI
MLSVEKLIKDLKSKEVPEARAYLRILGEELGFASLHDLQLLGKLLLMGARTLQGI
PQMIGEVIRKGSKNDFFLHYIFMENAFELPTGAGLQLQISSSGVIAPGAKAGVK
LEVANMQAELVAKPSVSVEFVTNMGIIIPDFARSGVQMNTNFFHESGLEAHVA
LKAGKLKFIIPSPKRPVKLLSGGNTLHLVSTTKTEVIPPLIENRQSWSVCKQVFP
GLNYCTSGAYSNASSTDSASYYPLTGDTRLELELRPTGEIEQYSVSATYELQR
EDRALVDTLKFVTQAEGAKQTEATMTFKYNRQSMTLSSEVQIPDFDVDLGTIL
RVNDESTEGKTSYRLTLDIQNKKITEVALMGHLSCDTKEERKIKGVISIPRLQAE
ARSEILAHWSPAKLLLQMDSSATAYGSTVSKRVAWHYDEEKIEFEWNTGTNV
DTKKMTSNFPVDLSDYPKSLHMYANRLLDHRVPQTDMTFRHVGSKLIVAMSS
WLQKASGSLPYTQTLQDHLNSLKEFNLQNMGLPDFHIPENLFLKSDGRVKYTL

NKNSLKIEIPLPFGGKSSRDLKMLETVRTPALHFKSVGFHLPSREFQVPTFTIPK
LYQLQVPLLGVLDLSTNVYSNLYNWSASYSGGNTSTDHFSLRARYHMKADSV
VDLLSYNVQGSGETTYDHKNTFTLSCDGSLRHKFLDSNIKFSHVEKLGNNPVS
KGLLIFDASSSWGPQMSASVHLDSKKKQHLFVKEVKIDGQFRVSSFYAKGTY
GLSCQRDPNTGRLNGESNLRFNSSYLQGTNQITGRYEDGTLSLTSTSDLQSGI
IKNTASLKYENYELTLKSDTNGKYKNFATSNKMDMTFSKQNALLRSEYQADYE
SLRFFSLLSGSLNSHGLELNADILGTDKINSGAHKATLRIGQDGISTSATTNLKC
SLLVLENELNAELGLSGASMKLTTNGRFREHNAKFSLDGKAALTELSLGSAYQ
AMILGVDSKNIFNFKVSQEGLKLSNDMMGSYAEMKFDHTNSLNIAGLSLDFSS
KLDNIYSSDKFYKQTVNLQLQPYSLVTTLNSDLKYNALDLTNNGKLRLEPLKLH
VAGNLKGAYQNNEIKHIYAISSAALSASYKADTVAKVQGVEFSHRLNTDIAGLA
SAIDMSTNYNSDSLHFSNVFRSVMAPFTMTIDAHTNGNGKLALWGEHTGQLY
SKFLLKAEPLAFTFSHDYKGSTSHHLVSRKSISAALEHKVSALLTPAEQTGTWK
LKTQFNNNEYSQDLDAYNTKDKIGVELTGRTLADLTLLDSPIKVPLLLSEPINIID
ALEMRDAVEKPQEFTIVAFVKYDKNQDVHSINLPFFETLQEYFERNRQTIIVVLE
NVQRNLKHINIDQFVRKYRAALGKLPQQANDYLNSFNWERQVSHAKEKLTALT
KKYRITENDIQIALDDAKINFNEKLSQLQTYMIQFDQYIKDSYDLHDLKIAIANIID
EIIEKLKSLDEHYHIRVNLVKTIHDLHLFIENIDFNKSGSSTASWIQNVDTKYQIRI
QIQEKLQQLKRHIQNIDIQHLAGKLKQHIEAIDVRVLLDQLGTTISFERINDILEHV
KHFVINLIGDFEVAEKINAFRAKVHELIERYEVDQQIQVLMDKLVELAHQYKLKE
TIQKLSNVLQQVKIKDYFEKLVGFIDDAVKKLNELSFKTFIEDVNKFLDMLIKKLK
SFDYHQFVDETNDKIREVTQRLNGEIQALELPQKAEALKLFLEETKATVAVYLE
SLQDTKITLIINWLQEALSSASLAHMKAKFRETLEDTRDRMYQMDIQQELQRYL
SLVGQVYSTLVTYISDWWTLAAKNLTDFAEQYSIQDWAKRMKALVEQGFTVP
EIKTILGTMPAFEVSLQALQKATFQTPDFIVPLTDLRIPSVQINFKDLKNIKIPSRF
STPEFTILNTFHIPSFTIDFVEMKVKIIRTIDQMLNSELQWPVPDIYLRDLKVEDIP
LARITLPDFRLPEIAIPEFIIPTLNLNDFQVPDLHIPEFQLPHISHTIEVPTFGKLYSI
LKIQSPLFTLDANADIGNGTTSANEAGIAASITAKGESKLEVLNFDFQANAQLSN
PKINPLALKESVKFSSKYLRTEHGSEMLFFGNAIEGKSNTVASLHTEKNTLELS
NGVIVKINNQLTLDSNTKYFHKLNIPKLDFSSQADLRNEIKTLLKAGHIAWTSSG
KGSWKWACPRFSDEGTHESQISFTIEGPLTSFGLSNKINSKHLRVNQNLVYES
GSLNFSKLEIQSQVDSQHVGHSVLTAKGMALFGEGKAEFTGRHDAHLNGKVI

GTLKNSLFFSAQPFEITASTNNEGNLKVRFPLRLTGKIDFLNNYALFLSPSAQQ
ASWQVSARFNQYKYNQNFSAGNNENIMEAHVGINGEANLDFLNIPLTIPEMRL
PYTIITTPPLKDFSLWEKTGLKEFLKTTKQSFDLSVKAQYKKNKHRHSITNPLAV
LCEFISQSIKSFDRHFEKNRNNALDFVTKSYNETKIKFDKYKAEKSHDELPRTF
QIPGYTVPVVNVEVSPFTIEMSAFGYVFPKAVSMPSFSILGSDVRVPSYTLILPS
LELPVLHVPRNLKLSLPDFKELCTISHIFIPAMGNITYDFSFKSSVITLNTNAELF
NQSDIVAHLLSSSSSVIDALQYKLEGTTRLTRKRGLKLATALSLSNKFVEGSHN
STVSLTTKNMEVSVATTTKAQIPILRMNFKQELNGNTKSKPTVSSSMEFKYDF
NSSMLYSTAKGAVDHKLSLESLTSYFSIESSTKGDVKGSVLSREYSGTIASEAN
TYLNSKSTRSSVKLQGTSKIDDIWNLEVKENFAGEATLQRIYSLWEHSTKNHL
QLEGLFFTNGEHTSKATLELSPWQMSALVQVHASQPSSFHDFPDLGQEVALN
ANTKNQKIRWKNEVRIHSGSFQSQVELSNDQEKAHLDIAGSLEGHLRFLKNIIL
PVYDKSLWDFLKLDVTTSIGRRQHLRVSTAFVYTKNPNGYSFSIPVKVLADKFII
PGLKLNDLNSVLVMPTFHVPFTDLQVPSCKLDFREIQIYKKLRTSSFALNLPTLP
EVKFPEVDVLTKYSQPEDSLIPFFEITVPESQLTVSQFTLPKSVSDGIAALDLNA
VANKIADFELPTIIVPEQTIEIPSIKFSVPAGIVIPSFQALTARFEVDSPVYNATWS
ASLKNKADYVETVLDSTCSSTVQFLEYELNVLGTHKIEDGTLASKTKGTFAHR
DFSAEYEEDGKYEGLQEWEGKAHLNIKSPAFTDLHLRYQKDKKGISTSAASPA
VGTVGMDMDEDDDFSKWNFYYSPQSSPDKKLTIFKTELRVRESDEETQIKVN
WEEEAASGLLTSLKDNVPKATGVLYDYVNKYHWEHTGLTLREVSSKLRRNLQ
NNAEWVYQGAIRQIDDIDVRFQKAASGTTGTYQEWKDKAQNLYQELLTQEGQ
ASFQGLKDNVFDGLVRVTQEFHMKVKHLIDSLIDFLNFPRFQFPGKPGIYTREE
LCTMFIREVGTVLSQVYSKVHNGSEILFSYFQDLVITLPFELRKHKLIDVISMYR
ELLKDLSKEAQEVFKAIQSLKTTEVLRNLQDLLQFIFQLIEDNIKQLKEMKFTYLI
NYIQDEINTIFSDYIPYVFKLLKENLCLNLHKFNEFIQNELQEASQELQQIHQYIM
ALREEYFDPSIVGWTVKYYELEEKIVSLIKNLLVALKDFHSEYIVSASNFTSQLS
SQVEQFLHRNIQEYLSILTDPDGKGKEKIAELSATAQEIIKSQAIATKKIISDYHQ
QFRYKLQDFSDQLSDYYEKFIAESKRLIDLSIQNYHTFLIYITELLKKLQSTTVMN
PYMKLAPGELTIIL
[SEQ ID No: 18]

Accordingly, preferably APOB comprises or consists of an amino acid sequence substantially as set out in SEQ ID NO: 18, or a fragment or variant thereof.

In one embodiment APOB is encoded by a nucleotide sequence which is provided herein as SEQ ID No: 19, as follows:

ATTCCCACCGGGACCTGCGGGGCTGAGTGCCCTTCTCGGTTGCTGCCGC
TGAGGAGCCCGCCCAGCCAGCCAGGGCCGCGAGGCCGAGGCCAGGCCG
CAGCCCAGGAGCCGCCCCACCGCAGCTGGCGATGGACCCGCCGAGGCC
CGCGCTGCTGGCGCTGCTGGCGCTGCCTGCGCTGCTGCTGCTGCTG
GCGGGCGCCAGGGCCGAAGAGGAAATGCTGGAAAATGTCAGCCTGGTCT
GTCCAAAAGATGCGACCCGATTCAAGCACCTCCGGAAGTACACATACAAC
TATGAGGCTGAGAGTTCCAGTGGAGTCCCTGGGACTGCTGATTCAAGAAG
TGCCACCAGGATCAACTGCAAGGTTGAGCTGGAGGTTCCCCAGCTCTGCA
GCTTCATCCTGAAGACCAGCCAGTGCACCCTGAAAGAGGTGTATGGCTTC
AACCCTGAGGGCAAAGCCTTGCTGAAGAAAACCAAGAACTCTGAGGAGTT
TGCTGCAGCCATGTCCAGGTATGAGCTCAAGCTGGCCATTCCAGAAGGGA
AGCAGGTTTTCCTTTACCCGGAGAAAGATGAACCTACTTACATCCTGAACA
TCAAGAGGGGCATCATTTCTGCCCTCCTGGTTCCCCCAGAGACAGAAGAA
GCCAAGCAAGTGTTGTTTCTGGATACCGTGTATGGAAACTGCTCCACTCAC
TTTACCGTCAAGACGAGGAAGGGCAATGTGGCAACAGAAATATCCACTGA
AAGAGACCTGGGGCAGTGTGATCGCTTCAAGCCCATCCGCACAGGCATCA
GCCCACTTGCTCTCATCAAAGGCATGACCCGCCCCTTGTCAACTCTGATCA
GCAGCAGCCAGTCCTGTCAGTACACACTGGACGCTAAGAGGAAGCATGTG
GCAGAAGCCATCTGCAAGGAGCAACACCTCTTCCTGCCTTTCTCCTACAAG
AATAAGTATGGGATGGTAGCACAAGTGACACAGACTTTGAAACTTGAAGAC
ACACCAAAGATCAACAGCCGCTTCTTTGGTGAAGGTACTAAGAAGATGGG
CCTCGCATTTGAGAGCACCAAATCCACATCACCTCCAAAGCAGGCCGAAG
CTGTTTTGAAGACTCTCCAGGAACTGAAAAAACTAACCATCTCTGAGCAAA
ATATCCAGAGAGCTAATCTCTTCAATAAGCTGGTTACTGAGCTGAGAGGCC
TCAGTGATGAAGCAGTCACATCTCTCTTGCCACAGCTGATTGAGGTGTCCA
GCCCCATCACTTTACAAGCCTTGGTTCAGTGTGGACAGCCTCAGTGCTCC

ACTCACATCCTCCAGTGGCTGAAACGTGTGCATGCCAACCCCCTTCTGATA
GATGTGGTCACCTACCTGGTGGCCCTGATCCCCGAGCCCTCAGCACAGCA
GCTGCGAGAGATCTTCAACATGGCGAGGGATCAGCGCAGCCGAGCCACC
TTGTATGCGCTGAGCCACGCGGTCAACAACTATCATAAGACAAACCCTACA
GGGACCCAGGAGCTGCTGGACATTGCTAATTACCTGATGGAACAGATTCA
AGATGACTGCACTGGGGATGAAGATTACACCTATTTGATTCTGCGGGTCAT
TGGAAATATGGGCCAAACCATGGAGCAGTTAACTCCAGAACTCAAGTCTTC
AATCCTGAAATGTGTCCAAAGTACAAAGCCATCACTGATGATCCAGAAAGC
TGCCATCCAGGCTCTGCGGAAAATGGAGCCTAAAGACAAGGACCAGGAG
GTTCTTCTTCAGACTTTCCTTGATGATGCTTCTCCGGGAGATAAGCGACTG
GCTGCCTATCTTATGTTGATGAGGAGTCCTTCACAGGCAGATATTAACAAA
ATTGTCCAAATTCTACCATGGGAACAGAATGAGCAAGTGAAGAACTTTGTG
GCTTCCCATATTGCCAATATCTTGAACTCAGAAGAATTGGATATCCAAGA
[SEQ ID No: 19]

Accordingly, preferably APOB comprises or consists of a nucleotide sequence substantially as set out in SEQ ID NO: 19, or a fragment or variant thereof.

In one embodiment, SEPP1 is provided by gene bank locus ID: HGNC: 10751; Entrez Gene: 6414; Ensembl: ENSG00000250722; OMIM: 601484; and/or UniProtKB: P49908. The protein sequence may be represented by the GeneBank ID P49908, which is provided herein as SEQ ID No: 20, as follows:

MWRSLGLALALCLLPSGGTESQDQSSLCKQPPAWSIRDQDPMLNSNGSVTV
VALLQASUY
LCILQASKLEDLRVKLKKEGYSNISYIVVNHQGISSRLKYTHLKNKVSEHIPVYQ
QEENQ
TDVWTLLNGSKDDFLIYDRCGRLVYHLGLPFSFLTFPYVEEAIKIAYCEKKCGN
CSLTTL
KDEDFCKRVSLATVDKTVETPSPHYHHEHHHNHGHQHLGSSELSENQQPGA
PNAPTHPAP

PGLHHHHKHKGQHRQGHPENRDMPASEDLQDLQKKLCRKRCINQLLCKLPT
DSELAPRSU
CCHCRHLIFEKTGSAITUQCKENLPSLCSUQGLRAEENITESCQURLPPAAUQI
SQQLIP
TEASASURUKNQAKKUEUPSN
[SEQ ID No: 20]

Accordingly, preferably SEPP1 comprises or consists of an amino acid sequence substantially as set out in SEQ ID NO: 20, or a fragment or variant thereof.

In one embodiment SEPP1 is encoded by a nucleotide sequence which is provided herein as SEQ ID No: 21 as follows:

ACAGACAGGCAGGTGCAGGAGACGGGGTGAGCGCTTTTGGGCTCTAGCC
CCATGGCAGCATCTAGGGGTGTGTTACAATTAATGCTCTTTTAGCAGTTGC
TGTTCGCGGATGGCTAAGTGTTAAACCAGCTCAGTGGAGAGTCAGGGTGG
CAGCCTTTTACGCCCTGTCCTCTTGGTACCCGGGTCTTTGTCTAGCATCCA
GGAAGAATCAGGTCCTACAGACTTGAAGGATGGTGAATACAGAGATTTTAT
TGAATGATGGAGGAGGCTTTCAGCAGGATGGATGGGGAGCTGGAAAGCG
GATGGAGTGAGAAGATAATCTTCCCCTGGGGTTTGGCCATCCCATGGCTG
ATCTCTCTGACAGTCCCCAGCCGAACTCCTCTCGATGTTCAGACACTCTTT
CTCCTCTCTCCTCTGCCACACTGCTCTGCTGCTCTTCTGCTCATGGAGCCT
GAGGACAACCCCAGCAATGTGGAGAAGCCTGGGGCTTGCCCTGGCTCTC
TGTCTCCTCCCATCGGGAGGAACAGAGAGCCAGGACCAAAGCTCCTTATG
TAAGCAACCCCCAGCCTGGAGCATAAGAGATCAAGATCCAATGCTAAACTC
CAATGGTTCAGTGACTGTGGTTGCTCTTCTTCAAGCCAGCTGATACCTGTG
CATACTGCAGGCATCTAAATTAGAAGACCTGCGAGTAAAACTGAAGAAAGA
AGGATATTCTAATATTTCTTATATTGTTGTTAATCATCAAGGAATCTCTTCTC
GATTAAAATACACACATCTTAAGAATAAGGTTTCAGAGCATATTCCTGTTTA
TCAACAAGAAGAAAACCAAACAGATGTCTGGACTCTTTTAAATGGAAGCAA
AGATGACTTCCTCATATATGATAGATGTGGCCGTCTTGTATATCATCTTGGT

TTGCCTTTTTCCTTCCTAACTTTCCCATATGTAGAAGAAGCCATTAAGATTG
CTTACTGTGAAAAGAAATGTGGAAACTGCTCTCTCACGACTCTCAAAGATG
AAGACTTTTGTAAACGTGTATCTTTGGCTACTGTGGATAAAACAGTTGAAAC
TCCATCGCCTCATTACCATCATGAGCATCATCACAATCATGGACATCAGCA
CCTTGGCAGCAGTGAGCTTTCAGAGAATCAGCAACCAGGAGCACCAAATG
CTCCTACTCATCCTGCTCCTCCAGGCCTTCATCACCACCATAAGCACAAGG
GTCAGCATAGGCAGGGTCACCCAGAGAACCGAGATATGCCAGCAAGTGAA
GATTTACAAGATTTACAAAAGAAGCTCTGTCGAAAGAGATGTATAAATCAAT
TACTCTGTAAATTGCCCACAGATTCAGAGTTGGCTCCTAGGAGCTGATGCT
GCCATTGTCGACATCTGATATTTGAAAAAACAGGGTCTGCAATCACCTGAC
AGTGTAAAGAAAACCTCCCATCTTTATGTAGCTGACAGGGACTTCGGGCAG
AGGAGAACATAACTGAATCTTGTCAGTGACGTTTGCCTCCAGCTGCCTGAC
AAATAAGTCAGCAGCTTATACCCACAGAAGCCAGTGCCAGTTGACGCTGA
AAGAATCAGGCAAAAAAGTGAGAATGACCTTCAAACTAAATATTTAAAATAG
GACATACTCCCCAATTTAGTCTAGACACAATTTCATTTCCAGCATTTTTATA
AACTACCAAATTAGTGAACCAAAAATAGAAATTAGATTTGTGCAAACATGGA
GAAATCTACTGAATTGGCTTCCAGATTTTAAATTTTATGTCATAGAAATATTG
ACTCAAACCATATTTTTTATGATGGAGCAACTGAAAGGTGATTGCAGCTTTT
GGTTAATATGTCTTTTTTTTTCTTTTTCCAGTGTTCTATTTGCTTTAATGAGA
ATAGAAACGTAAACTATGACCTAGGGGTTTCTGTTGGATAATTAGCAGTTTA
GAATGGAGGAAGAACAACAAAGACATGCTTTCCATTTTTTTCTTTACTTATC
TCTCAAAACAATATTACTTTGTCTTTTCAATCTTCTACTTTTAACTAATAAAAT
AAGTGGATTTTGTATTTTAAGATCCAGAAATACTTAACACGTGAATATTTTG
CTAAAAAAGCATATATAACTATTTTAAATATCCATTTATCTTTTGTATATCTAA
GACTCATCCTGATTTTTACTATCACACATGAATAAAGCCTTTGTATCTT
T [SEQ ID No: 21]

Accordingly, preferably SEPP1 comprises or consists of a nucleotide sequence substantially as set out in SEQ ID NO: 21, or a fragment or variant thereof.

In one embodiment, TFF3 is provided by gene bank locus ID: HGNC: 11757; Entrez Gene: 7033; Ensembl: ENSG00000160180; OMIM: 600633; and/or UniProtKB: Q07654. The protein sequence may be represented by the GeneBank ID Q07654, which is provided herein as SEQ ID No: 22, as follows:

MKRVLSCVPEPTVVMAARALCMLGLVLALLSSSSAEEYVGLSANQCAVPAKD
RVDCGYPH
VTPKECNNRGCCFDSRIPGVPWCFKPLQEAECTF
[SEQ ID No: 22]

Accordingly, preferably TFF3 comprises or consists of an amino acid sequence substantially as set out in SEQ ID NO: 22, or a fragment or variant thereof.

In one embodiment TFF3 is encoded by a nucleotide sequence which is provided herein as SEQ ID No: 23, as follows:

CGCTCCCCAGTAGAGGACCCGGAACCAGAACTGGAATCCGCCCTTACCG
CTTGCTGCCAAAACAGTGGGGGCTGAACTGACCTCTCCCCTTTGGGAGAG
AAAAACTGTCTGGGAGCTTGACAAAGGCATGCAGGAGAGAACAGGAGCAG
CCACAGCCAGGAGGGAGAGCCTTCCCCAAGCAAACAATCCAGAGCAGCT
GTGCAAACAACGGTGCATAAATGAGGCCTCCTGGACCATGAAGCGAGTCC
TGAGCTGCGTCCCGGAGCCCACGGTGGTCATGGCTGCCAGAGCGCTCTG
CATGCTGGGGCTGGTCCTGGCCTTGCTGTCCTCCAGCTCTGCTGAGGAGT
ACGTGGGCCTGTCTGCAAACCAGTGTGCCGTGCCAGCCAAGGACAGGGT
GGACTGCGGCTACCCCCATGTCACCCCCAAGGAGTGCAACAACCGGGGC
TGCTGCTTTGACTCCAGGATCCCTGGAGTGCCTTGGTGTTTCAAGCCCCT
GCAGGAAGCAGAATGCACCTTCTGAGGCACCTCCAGCTGCCCCCGGCCG
GGGGATGCGAGGCTCGGAGCACCCTTGCCCGGCTGTGATTGCTGCCAGG
CACTGTTCATCTCAGCTTTTCTGTCCCTTTGCTCCCGGCAAGCGCTTCTGC
TGAAAGTTCATATCTGGAGCCTGATGTCTTAACGAATAAAGGTCCCATGCT
CCACCCGAGGACAGTTCTTCGTGCCTGAGACTTTCTGAGGTTGTGCTTTAT
TTCTGCTGCGTCGTGGAGAGGGCGGGAGGGTGTCAGGGGAGAGTCTGC
CCAGGCCTCAAGGGCAGGAAAAGACTCCCTAAGGAGCTGCAGTGCATGC
AAGGATATTTTGAATCCAGACTGGCACCCACGTCACAGGAAAGCCTAGGA

ACACTGTAAGTGCCGCTTCCTCGGGAAAGCAGAAAAAATACATTTCAGGTA
GAAGTTTTCAAAAATCACAAGTCTTTCTTGGTGAAGACAGCAAGCCAATAA
AACTGTCTTCCAAAGTGGTCCTTTATTTCACAACCACTCTCGCTACTGTTCA
ATACTTGTACTATTCCTGGGTTTTGTTTCTTTGTACAGTAAACATTATGAACA
AACAGGCA
[SEQ ID No: 23]

Accordingly, preferably TFF3 comprises or consists of a nucleotide sequence substantially as set out in SEQ ID NO: 23, or a fragment or variant thereof.

In one embodiment, IL6 is provided by gene bank locus ID; HGNC: 6018; Entrez Gene: 3569; Ensembl: ENSG00000136244; OMIM: 147620; and/or UniProtKB: P05231. The protein sequence may be represented by the GeneBank ID P05231, which is provided herein as SEQ ID No: 24, as follows:

MNSFSTSAFGPVAFSLGLLLVLPAAFPAPVPPGEDSKDVAAPHRQPLTSSERI
DKQIRYILDGISALRKETCNKSNMCESSKEALAENNLNLPKMAEKDGCFQSGF
NEETCLVKIITGLLEFEVYLEYLQNRFESSEEQARAVQMSTKVLIQFLQKKAKN
LDAITTPDPTTNASLLTKLQAQNQWLQDMTTHLILRSFKEFLQSSLRALRQM

[SEQ ID No: 24]

Accordingly, preferably IL6 comprises or consists of an amino acid sequence substantially as set out in SEQ ID NO: 24 or a fragment or variant thereof.

In one embodiment IL6 is encoded by a nucleotide sequence which is provided herein as SEQ ID No: 25, as follows:

AGGTAACACCATGTTTGGTAAATAAGTGTTTTGGTGTTGTGCAAGGGTCTG
GTTTCAGCCTGAAGCCATCTCAGAGCTGTCTGGGTCTCTGGAGACTGGAG
GGACAACCTAGTCTAGAGCCCATTTGCATGAGACCAAGGATCCTCCTGCA
AGAGACACCATCCTGAGGGAAGAGGGCTTCTGAACCAGCTTGACCCAATA

AGAAATTCTTGGGTGCCGACGCGGAAGCAGATTCAGAGCCTAGAGCCGTG
CCTGCGTCCGTAGTTTCCTTCTAGCTTCTTTTGATTTCAAATCAAGACTTAC
AGGGAGAGGGAGCGATAAACACAAACTCTGCAAGATGCCACAAGGTCCTC
CTTTGACATCCCCAACAAAGAGGACTGGAGATGTCTGAGGCTCATTCTGC
CCTCGAGCCCACCGGGAACGAAAGAGAAGCTCTATCTCCCCTCCAGGAGC
CCAGCTATGAACTCCTTCTCCACAAGCGCCTTCGGTCCAGTTGCCTTCTCC
CTGGGGCTGCTCCTGGTGTTGCCTGCTGCCTTCCCTGCCCCAGTACCCCC
AGGAGAAGATTCCAAAGATGTAGCCGCCCCACACAGACAGCCACTCACCT
CTTCAGAACGAATTGACAAACAAATTCGGTACATCCTCGACGGCATCTCAG
CCCTGAGAAAGGAGACATGTAACAAGAGTAACATGTGTGAAAGCAGCAAA
GAGGCACTGGCAGAAAACAACCTGAACCTTCCAAAGATGGCTGAAAAAGA
TGGATGCTTCCAATCTGGATTCAATGAGGAGACTTGCCTGGTGAAAATCAT
CACTGGTCTTTTGGAGTTTGAGGTATACCTAGAGTACCTCCAGAACAGATT
TGAGAGTAGTGAGGAACAAGCCAGAGCTGTGCAGATGAGTACAAAAGTCC
TGATCCAGTTCCTGCAGAAAAAGGCAAAGAATCTAGATGCAATAACCACCC
CTGACCCAACCACAAATGCCAGCCTGCTGACGAAGCTGCAGGCACAGAAC
CAGTGGCTGCAGGACATGACAACTCATCTCATTCTGCGCAGCTTTAAGGA
GTTCCTGCAGTCCAGCCTGAGGGCTCTTCGGCAAATGTAGCATGGGCACC
TCAGATTGTTGTTGTTAATGGGCATTCCTTCTTCTGGTCAGAAACCTGTCCA
CTGGGCACAGAACTTATGTTGTTCTCTATGGAGAACTAAAAGTATGAGCGT
TAGGACACTATTTTAATTATTTTTAATTTATTAATATTTAAATATGTGAAGCTG
AGTTAATTTATGTAAGTCATATTTATATTTTTAAGAAGTACCACTTGAAACAT
TTTATGTATTAGTTTTGAAATAATAATGGAAAGTGGCTATGCAGTTTGAATA
TCCTTTGTTTCAGAGCCAGATCATTTCTTGGAAAGTGTAGGCTTACCTCAAA
TAAATGGCTAACTTATACATATTTTTAAAGAAATATTTATATTGTATTTATATA
ATGTATAAATGGTTTTTATACCAATAAATGGCATTTTAAAAAATTCAGCA
[SEQ
ID No: 25]

Accordingly, preferably IL6 comprises or consists of a nucleotide sequence substantially as set out in SEQ ID NO: 25, or a fragment or variant thereof.

In one embodiment, CHI3L1 is provided by gene bank locus ID; HGNC: 1932; Entrez Gene: 1116; Ensembl: ENSG00000133048; OMIM: 601525; and/or UniProtKB: P36222. The protein sequence may be represented by the GeneBank ID P36222, which is provided herein as SEQ ID No: 26 as follows:

MGVKASQTGFVVLVLLQCCSAYKLVCYYTSWSQYREGDGSCFPDALDRFLC
THIIYSFANISNDHIDTWEWNDVTLYGMLNTLKNRNPNLKTLLSVGGWNFGSQ
RFSKIASNTQSRRTFIKSVPPFLRTHGFDGLDLAWLYPGRRDKQHFTTLIKEMK
AEFIKEAQPGKKQLLLSAALSAGKVTIDSSYDIAKISQHLDFISIMTYDFHGAWR
GTTGHHSPLFRGQEDASPDRFSNTDYAVGYMLRLGAPASKLVMGIPTFGRSF
TLASSETGVGAPISGPGIPGRFTKEAGTLAYYEICDFLRGATVHRILGQQVPYA
TKGNQWVGYDDQESVKSKVQYLKDRQLAGAMVWALDLDDFQGSFCGQDLR
FPLTNAIKDALAAT
[SEQ ID No: 26]

Accordingly, preferably CHI3L1 comprises or consist of an amino acid sequence substantially as set out in SEQ ID NO: 26, or a fragment or variant thereof.

In one embodiment CHI3L1 is encoded by a nucleotide sequence which is provided herein as SEQ ID No: 27, as follows:

GAGGCCCTGTCTAGGTAGCTGGCACCAGGAGCCGTGGGCAAGGAAGAG
GCCACACCCTGCCCTGCTCTGCTGCAGCCAGAATGGGTGTGAAGGCGTCT
CAAACAGGCTTTGTGGTCCTGGTGCTGCTCCAGTGCTGCTCTGCATACAA
ACTGGTCTGCTACTACACCAGCTGGTCCCAGTACCGGGAAGGCGATGGGA
GCTGCTTCCCAGATGCCCTTGACCGCTTCCTCTGTACCCACATCATCTACA
GCTTTGCCAATATAAGCAACGATCACATCGACACCTGGGAGTGGAATGAT
GTGACGCTCTACGGCATGCTCAACACACTCAAGAACAGGAACCCCAACCT
GAAGACTCTCTTGTCTGTCGGAGGATGGAACTTTGGGTCTCAAAGATTTTC
CAAGATAGCCTCCAACACCCAGAGTCGCCGGACTTTCATCAAGTCAGTAC
CGCCATTTCTGCGCACCCATGGCTTTGATGGGCTGGACCTTGCCTGGCTC
TACCCTGGACGGAGAGACAAACAGCATTTTACCACCCTAATCAAGGAAATG
AAGGCCGAATTTATAAAGGAAGCCCAGCCAGGGAAAAAGCAGCTCCTGCT
CAGCGCAGCACTGTCTGCGGGGAAGGTCACCATTGACAGCAGCTATGACA
TTGCCAAGATATCCCAACACCTGGATTTCATTAGCATCATGACCTACGATTT

TCATGGAGCCTGGCGTGGGACCACAGGCCATCACAGTCCCCTGTTCCGA
GGTCAGGAGGATGCAAGTCCTGACAGATTCAGCAACACTGACTATGCTGT
GGGGTACATGTTGAGGCTGGGGGCTCCTGCCAGTAAGCTGGTGATGGGC
ATCCCCACCTTCGGGAGGAGCTTCACTCTGGCTTCTTCTGAGACTGGTGTT
GGAGCCCCAATCTCAGGACCGGGAATTCCAGGCCGGTTCACCAAGGAGG
CAGGGACCCTTGCCTACTATGAGATCTGTGACTTCCTCCGCGGAGCCACA
GTCCATAGAATCCTCGGCCAGCAGGTCCCCTATGCCACCAAGGGCAACCA
GTGGGTAGGATACGACGACCAGGAAAGCGTCAAAAGCAAGGTGCAGTAC
CTGAAGGACAGGCAGCTGGCGGGCGCCATGGTATGGGCCCTGGACCTGG
ATGACTTCCAGGGCTCCTTCTGTGGCCAGGATCTGCGCTTCCCTCTCACC
AATGCCATCAAGGATGCACTCGCTGCAACGTAGCCCTCTGTTCTGCACAC
AGCACGGGGGCCAAGGATGCCCCGTCCCCCTCTGGCTCCAGCTGGCCGG
GAGCCTGATCACCTGCCCTGCTGAGTCCCAGGCTGAGCCTCAGTCTCCCT
CCCTTGGGGCCTATGCAGAGGTCCACAACACACAGATTTGAGCTCAGCCC
TGGTGGGCAGAGAGGTAGGGATGGGGCTGTGGGGATAGTGAGGCATCGC
AATGTAAGACTCGGGATTAGTACACACTTGTTGATTAATGGAAATGTTTACA
GATCCCCAAGCCTGGCAAGGGAATTTCTTCAACTCCCTGCCCCCCAGCCC
TCCTTATCAAAGGACACCATTTTGGCAAGCTCTATCACCAAGGAGCCAAAC
ATCCTACAAGACACAGTGACCATACTAATTATACCCCCTGCAAAGCCCAGC
TTGAAACCTTCACTTAGGAACGTAATCGTGTCCCCTATCCTACTTCCCCTTC
CTAATTCCACAGCTGCTCAATAAAGTACAAGAGCTTAACAGTGGTATCTGG
GCTAGCCAAGGTTAATCCATCAGAGTTGTGGGTTTTCAGGCCCAGACAGC
CCGCAGAGCCATCTGCCTGCTGGGTGAGGGACTAAGGGAGTGGCAGAG
GGGGAGGAGAAGCAGAGCCAGGGGAGGGACTGAGGCTGCAACCAGGAG
GTGGGGGTGGGGGAGGTGGGTCTCAGTTGCTTGGGGGAGGGAGCAGGG
CGGAAGGGCAGGATGCACTTGCAGGGGTCTCATCCTGGATTTCTCTTCAG
GTGAGTAATCCCTCCACCTCCACTTTTAAGTCCAGAGGCGTGGCGAGGGC
ACAGGGCAGGTGTGGAGGAGGTCTTAGCTCCAAGGGAACACTTTGCCAG
GTTCTTCTGTGCTTCCAATGACTTCGAAATAGTCACGTTTGCTAAACTGGA
GTGAGGAGTATATGGATGTTTATTTTGCTATACTCTTTGGTATATGTGAAAA
TTCTCAAAATAAGAAACTTTAAAAGGTCCTGGTTCAGTGAAGGCTTTGACTA
ACAGCCCCTGGGAGCCGCTAGTACAGTTGCCCAGTAGCTGCTTGGATGAG

TGATGCCCACATTGTTTGCAGCAGGAGAAACAGAATTAGAGGCGAGGAAG
GATTTCCTGGTCCATTGTGATATTGTGCCCCAGCCTGGGGACATGCCGA
GGGAGCACAGGACTGCCATTCCGGGTGGGTTACAAGTTAGAGGCACTCTC
ACCAGGAGGCAGAGAAAGGTGGGCCAGAGTCCCTCATGGATGAAGGACC
TCACTGCAGGTCACTAAAGGTGCCAGCCTGAAAGCCAGGGCCATCTGTGA
CACAGGCTATCTTGGGCCTTCCCCTCCCACAAAGCCACTGCTTCCAGGAA
GCCTGCTGTGCTGGCACCAAGTCCCATTTCATCCTTATTCCCCAGCTCCTA
CCCTTCCCCCACACAGTGCCTGTAGCGTATTCATCCCCTGCATTGGATCTT
TTCTAAGCATTTTCAGAATAGGCCTTATTTTCACCAGTCAGGGAACTCCCC
AGAGGCAGGGGGACCTGAACCAGCTCCTTTCATTTAAGAATTCTGACTTTG
CTCACAATCTGCACCCCACTCCTCCCTACCCAACCCCACACCCCATTGGT
GCTCAGCTCTGGGCTCAGCCTGAGGTCTCTTGCCGAATCCTGGCCCGGG
CCCCATCCCAGACTTCTCCTTCAGGTTGGGCCAGGGCTGGCCAGGAGAG
GAGGGACCAGCTGGGCTGGTCCCAGGGCAGGGCAACCCCACCCAGGAG
GAAGGGCGAGGCCCCACCCTGCTCACCTATGTTTGGAGAGTTAGAGGGG
CTTGGCTTAAATGGGTGGAGGGGATGACTGTAGGTGCTGGGCAATATTTG
GGGTGATGAGAGACTGTAGACGGAAGCGCAGCTGAGCCCCTCTGGGAGA
GGGGAACCCTCTCCTGCCCCTAACGTGCTCTCCAGGGAGCATGGAAAATG
AGCAAGACTCTACTTTGCCATACTCTGTCTTCTCCACTGGGGAAACAAAA
ATGGGCAGCAGACCTCAGAGTAACTCCCATGAAGCCTGTGACCCCTCAGA
GATCCACCACTATGAATCTCTATGGGATTCCCATGATGTCCTATGGGAGGA
CATCAGCGGCCTAACCCAGCCTCTCACCCAAAGCAGGAAACCTTCTATGG
CCTCTCAGACATGGGGCCACCCAGTGTCATGACAATGTCATTCCACTCCT
GCCCTCCCCACCTCCCTGTGCTCCACAGGTAGGAGCCTCTCCCCAGGGG
CAGGACGGCAGAGGATGATGGCATAGGAGTGAGGAGCTTGGGTCTCCCG
CATCCACTGTATGGATGTTCCAGGGGCTCCAGACTAGATAGGTACAAGGC
CTACCTGTTTGTCAAGGGCCTGCCTGATGTGTAGCAAAGAAAGCAGAGCC
CAGAGAGAGAGCAGGACTTGCCAAGAGTCACACAGCAAGTTAACAGTGGA
TCTCCCAATTCCCTGCCAATGCTCTATTTACTACCTCACAGGCCCGAAAAT
ATGGGACTTCTGGGGCTACCACCATTAGGGCTGGAAAGAGAGAGATGGAA
ACCAATGGGGACATTGAGAAGTGGGGAGACCCTGGGAGGAGTCTTTGGAT
TAGGTGGGGTTGGAGCAGGGCTTGAAATGGGGGTGGTTATGGCCATCGTT

GGCACCCATGTGCTGGGTACTCTTTCTGTGTCCAGCACCCTGTCTCCCCA
TGATCCCATGTTGTCCTCACAACAGCAAGGTGAGCTGTTATATTTGTCCCC
ATTTTACAGATGATGAAACTGAGACTCAGGTTATAACCTTTCACAGTGGCT
GGCTAGTAAGTGATAGAGCCAGACTTGGAAACCTGGAATGCCTGGCTCTA
AGGCACATGCATGCCTGGAGGCGACCCCTGTCCCAATCATGCCCTCCCAG
AGCTGTGTGGCCTCAGGATCCCAGCTCTGCAGGTCCTGGAAACCCCACCA
GAGGCCCAAGGCACCTAGCATATCAGTGCTGAGCATGCTACAGGGCTGAT
TTTGGTCCTAGGTGCTGGTCAGGTCAGGGACAGGGAGGGAGGTGGGCAG
GCATGAGGGAATGGGGTGGGCTAGAAGCCGGCGTCAGCTGCTGTCCTCC
TGAGGACAGGTAAAGAGGGACTTCAGCCTCAGGGCAGTGTCCTGGGACC
CTGTGCCCTGAAGATCTCACATAGCAAGGAAGGCTTCTTGTGACCATGGT
GGGAGGTGGGAATGGGGTTCTAAGAGGTGGAAGGTTGTGACTGAGCAGA
GCACCCACTTATAACTACCCACTTAGTGCATTGCCCATTGCCCACCCTTCA
ATCCCATACTGATGCCACCCATACCCAGCATGCACTGTGTCCAGCAGCTCT
CAGCTCTGCCTGGGGGCAGCCTAAGTATCTCCAGTTACCAGGGGCAGAAG
GAGAGTCTAAACAAATTGTTCACAATACCAGCAGCAATCACTTTAACTTTGG
GATTTCATGCACCAGCTAGAACAAAGCACAGACCGAAAGGCAAATGTCTC
CCAAAGTCATCTGTGGGCCAATAGGGGTCACCCACTGTCCGATGCTCCCT
CCAGGACAGGGAGTAATTGAGTGCTGATGGGTGTGCATGGGTTTGGGGA
GAAGATTAGTCAGTTTTTTAGGAAAAGGATGGGTGACTTGAGGATGGGAC
CACTGATGAGCCACTTTATCACTTCCACTAGGCCCCAGGCAGGTTGGGGA
GTACAGCAAATGGGTTGCGCAGAGACCTAGTCCGCCCTCGATGAGTCTAC
CTCTCATGCCACTTGGGACCCTTTCTCTCACCTGCCTGTCTCCCATCTAGG
TGAGTCTCCTGCCCCAGGCAGCCTCCCCAGAACCTCTGCCAACACTCCTG
TTTCCCCGCCACCCCCAGCCCTAGCACTCCATGTAGACTGAGGACACTGG
GCTTCCAGTCCCAGTTCTTCCAAAGCCTTGATGTGTGACCTTGAGCAAGTC
ACTTCCCTGCCCTGTGCCTCAGTTTCCCCATCTGTCAAATGAGGCATAACA
ATCCTTGCCTTTCCTTTTTCACCTGGGCTGTTGGCTGAGCAGTTGAGAGAG
CTGCTATGAAAGTATTTTGGAAAGGAAGTGGAAAAAGCTATTCAAACCCT
CCAGCTATTGCAGAGTATTTTCTCCACACCAGGCAGAGCAGAGTGCTGGG
CCCTGGAGAGGCCACACAGCAGCCTCTTTCTAGAGTGCTGGGGAGTCCTC
AAGGCTCTCTCATACACACAGGCCTCCCCCTTTCCTGTTGGCCCCACGCC

TCATCCTCACCCCACCCCTCATGCCTGTGAGGAACAAGGAACAGCCAAGC
AGCCTCATCTCTCCTGAGAGCAGAGCCATGGGTCTCGGGAAACCCAGGAG
TAAGGAACAAAGACTCTTCAAAATGACTTCAGAGCTTTCTTTAGGATCCCA
GGGAGGTGTAAACTCAGTGCTTAATTAAATGGATTCTTTAGAGGGTGGGAA
ACAGGTGGATGTCAACCATTTGCCCCACATACCCGTATTCATGCAATCCAC
CCCCAGTGGGCTCACCGGTGCGGGTGTGCAAAACCTCCTCCCACCCCAG
TCATCTTAGGTTCAGGTCATCCTTTGGTCCTGCTCTTTCCCCGCCAGGCTG
TCTGTTGATGCTACTTTTAATCTGCTTTCACTAAGATAAGCCTGGCAGAAGT
GGTGGGGGTAAGGTGGGCTGTAGGCCAGCTCCCAAATGTGTGCTGGGCA
TAACAGAAGACCCATTCTTGACTGAAGTGCCCTTGTGGACCCTGAGCCC
GTGCCCTGGAGTGGCACAGGGAGGTGTGCCAGCAATGGGGACCGAGGTC
ACTGGGGACTGCTGGGGTTCGGGCTAGTGGCCTGTCTGGCTGCTGTCTG
CTTCCCTCGTTCACATCCTGCTGGAGCCTTAAATAGGAGCCCAAAAGCTTT
TCTTTCTTACTTTTTTTTGAGACAAAATCTCACTCTGTTGCCCAGGCCGGAG
TGCAGTGGCACAACCTCTGCCGCCTGGGTTCAAGCGATTCTCCTGCCTCA
GCCTCCCGAGTAGCTGGGATTACAGGTGCCTGCCACCATGCCTGGCTAAT
TTTCGTAGTTAGAGTAGAGACGGGGTTTCACCATCTTGGCCAGGCTGGTC
TTGAACTCCTCACCTCATGATCCACCTGCCTCGGCCTCCCAAAGTGCTGG
GATTCCAGGTATGAGCCACCACGCCCGGCCTAAAGCTTTTCTATTAATAAT
TTCCTGCCTCACCCTCCATCCCCTTCCTCCTCAGGTGAGTTCCCAGAAGGA
GGGAGGGCCAGGAGGGGGCCGCAAGACCTGCCATCTGCCAGTGCTCACA
CACCAATCTCTCTAGCCCTCAGTACAGTCCTGCAAGAGGGGGGTCATGAG
GCCCATTCCACAGATAAGGAAACTGAGGCCCAAAGTCTGGGCATGCTGCG
TTGCTCTGGGAAGGTGATCTGCAGGGTAAATGGAGTGAGGGCAGGGGGC
CGAATGGGGAGAGGCTGGGAGCCGAGGAGGTAGGAGTCATTGTGCCCTC
AGAGCCAACCACCTGATTTCTGCATCTGTCAAATAGTAATAGCCCCTTCCT
ATGCCTCAAAGGATTTTTTTCAAGAATGAAACTGTAAAATTCACTTTAAAGT
GACATGATCCGTTCCCGGAGGGACAGGGGAATCCCCAGTGCACCATACAC
CAATAACCCCTGCTAAGGCAGCAGTATTAATTGCTCAACCTTCCGCACCTG
TGCACTAACTGCTCACGTTGTTCCCACCCTACCCCACCCAGGTAATGGAGT
TGGGGAGAAGGTAGGATTCCCCCACACCACCAGCAGCCCTGGGGAAGGT
GATTCCCCACCACGTTCTTGCTTTTTCTCCTTTGGGAATAAAGAAAATGTCC

GGTTGCCCCAGCATGCCTGAGGAAGTGGAAGGGAGAGGTTAGGACATTT
GTTGCTGAAAATCTCCAGCAGGTACAGGCACATGGGCCTGCACCACTAGG
CACCTGGGATAGCCCTCTGGCTATGGGGCTGAGGTCTTCCTTCCAGCCCA
GGAAAGAGCAGAGGTCAAGAGGCAGATTTTTTGTTTCACTCTAGCCTCTGC
TACTCTGTGTGGCCTTGGGCCTGTCCTCAGTGTCAACCAGCAGGCCTCAC
ATCTCTGTTTAAATGGAAGAAGCTAAGCAGGGCCAAGGCAGCCACCATCT
CTGGGTCATTTGCCTCTGGTTTGTATAAACTTGTGTGATGCATGCAGCCTG
CAGACCCTGCAGAGAGTGAGGCTGCAGGATGGAGCAGGAGCTAAAAGAG
ATTTGGAGAGTGGCGTCTCCTGGTGACCTGCAAGGTCTCGGCACGACTCC
CCACACTGCCTTTTCCCTGTTATCTGCTCAGGTAGGTTTCCCCAAGGCCAC
ACCTCAGGACAAAGAGAAAGAAGGAGGCCCCGCTCCCAGCAGCCAGATT
CCTGTCCCTTGCACTGAGGTCTGGGCTGGGCTCACAGAGCACATGTGCCC
TGTACACACTCTGGGTCAGGGAACCCAGCCCTGCTCCTCTGGGCCTCCCC
TGCCAGCCCTCTGTTCTGCACACAGCACGGGGGCCAAGGATGCCCCGTC
CCCCTCTGGCTCCAGCTGGCCGGGAGCCTGATCACCTGCCCTGCTGAGT
CCCAGGCTGAGCCTCAGTCTCCCTCCCTTGGGGCCTATGCAGAGGTCCAC
AACACACAGATTTGAGCTCAGCCCTGGTGGGCAGAGAGGTAGGGATGGG
GCTGTGGGGATAGTGAGGCATCGCAATGTAAGACTCGGGATTAGTACACA
CTTGTTGATTAATGGAAATGTTTACAGATCCCCAAGCCTGGCAAGGGAATT
TCTTCAACTCCCTGCCCCCCAGCCCTCCTTATCAAAGGACACCATTTTGGC
AAGCTCTATCACCAAGGAGCCAAACATCCTACAAGACACAGTGACCATACT
AATTATACCCCCTGCAAAGCCCAGCTTGAAACCTTCACTTAGGAACGTAAT
CGTGTCCCCTATCCTACTTCCCCTTCCTAATTCCACAGCTGCTCAATAAAG
TACAAGAGCTTAACAGTGGAGGCCCTGTCTAGGTAGCTGGCACCAGGAGC
CGTGGGCAAGGGAAGAGGCCACACCCTGCCCTGCTCTGCTGCAGCCAGA
GAGGCCCTGTCTAGGTAGCTGGCACCAGGAGCCGTGGGCAAGGGAAGAG
GCCACACCCTGCCCTGCTCTGCTGCAGCCAGAATGGGTGTGAAGGCGTCT
CAAACAGGTATCTGGGCTAGCCAAGGTTAATCCATCAGAGTTGTGGGTTTT
CAGGCCCAGACAGCCCGCAGAGCCATCTGCCTGCTGGGTGAGGGACTAA
GGGAGTGGGCAGAGGGGGAGGAGAAGCAGAGCCAGGGGAGGGACTGAG
GCTGCAACCAGGAGGTGGGGGTGGGGGAGGTGGGTCTCAGTTGCTTGGG
GGAGGGAGCAGGGCGGAAGGGCAGGATGCACTTGCAGGGGTCTCATCCT

GGATTTCTCTTCAGGCTTTGTGGTCCTGGTGCTGCTCCAGTGCTGTGAGTA
ATCCCTCCACCTCCACTTTTAAGTCCAGAGGCGTGGCGAGGGCACAGGGC
AGGTGTGGAGGAGGTCTTAGCTCCAAGGGAACACTTTGCCAGGTTCTTCT
GTGCTTCCAATGACTTCGAAATAGTCACGTTTGCTAAACTGGAGTGAGGAG
TATATGGATGTTTATTTTGCTATACTCTTTGGTATATGTGAAAATTCTCAAAA
TAAGAAACTTTAAAAGGTCCTGGTTCAGTGAAGGCTTTGACTAACAGCCCC
TGGGAGCCGCTAGTACAGTTGCCCAGTAGCTGCTTGGATGAGTGATGCCC
ACATTGTTTGCAGCAGGAGAAACAGAATTAGAGGCGAGGAAGGATTTCCT
GGTCCATTGTGATATTGTGCCCCAGCCTGGGGGACATGCCGAGGGAGCA
CAGGACTGCCATTCCGGGTGGGTTACAAGTTAGAGGCACTCTCACCAGGA
GGCAGAGAAAGGTGGGCCAGAGTCCCTCATGGATGAAGGACCTCACTGC
AGGTCACTAAAGGTGCCAGCCTGAAAGCCAGGGCCATCTGTGACACAGGC
TATCTTGGGCCTTCCCCTCCCACAAAGCCACTGCTTCCAGGAAGCCTGCT
GTGCTGGCACCAAGTCCCATTTCATCCTTATTCCCCAGCTCCTACCCTTCC
CCCACACAGTGCCTGTAGCGTATTCATCCCCTGCATTGGATCTTTTCTAAG
CATTTTCAGAATAGGCCTTATTTTCACCAGTCAGGGAACTCCCCAGAGGCA
GGGGGACCTGAACCAGCTCCTTTCATTTAAGAATTCTGACTTTGCTCACAA
TCTGCACCCCACTCCTCCCTACCCAACCCCACACCCCATTGGTGCTCAGC
TCTGGGCTCAGCCTGAGGTCTCTTGCCGAATCCTGGCCCGGGCCCCATCC
CAGACTTCTCCTTCAGGCTCTGCATACAAACTGGTCTGCTACTACACCAGC
TGGTCCCAGTACCGGAAGGCGATGGAGCTGCTTCCCAGATGCCCTTG
ACCGCTTCCTCTGTACCCACATCATCTACAGCTTTGCCAATATAAGCAACG
ATCACATCGACACCTGGGAGTGGAATGATGTGACGCTCTACGGCATGCTC
AACACACTCAAGAACAGGTTGGGCCAGGGCTGGCCAGGAGAGGAGGGAC
CAGCTGGGCTGGTCCCAGGGCAGGGCAACCCCACCCAGGAGGAAGGGC
GAGGCCCCACCCTGCTCACCTATGTTTGGAGAGTTAGAGGGGCTTGGCTT
AAATGGGTGGAGGGGATGACTGTAGGTGCTGGGCAATATTTGGGGTGATG
AGAGACTGTAGACGGAAGCGCAGCTGAGCCCCTCTGGGAGAGGGGAACC
CTCTCCTGCCCCTAACGTGCTCTCCAGGGAGCATGGAAAATGAGCAAGAC
TCTACTTTGCCATACTCTGTCTTCTCCACTGGGGAAAACAAAAATGGGCAG
CAGACCTCAGAGTAACTCCCATGAAGCCTGTGACCCCTCAGAGATCCACC
ACTATGAATCTCTATGGGATTCCCATGATGTCCTATGGGAGGACATCAGCG

GCCTAACCCAGCCTCTCACCCAAAGCAGGAAACCTTCTATGGCCTCTCAG
ACATGGGGCCACCCAGTGTCATGACAATGTCATTCCACTCCTGCCCTCCC
CACCTCCCTGTGCTCCACAGGAACCCCAACCTGAAGACTCTCTTGTCTGTC
GGAGGATGGAACTTTGGGTCTCAAAGGTAGGAGCCTCTCCCAGGGGCA
GGACGGCAGAGGATGATGGCATAGGAGTGAGGAGCTTGGGTCTCCCGCA
TCCACTGTATGGATGTTCCAGGGGCTCCAGACTAGATAGGTACAAGGCCT
ACCTGTTTGTCAAGGGCCTGCCTGATGTGTAGCAAAGAAAGCAGAGCCCA
GAGAGAGAGCAGGACTTGCCAAGAGTCACACAGCAAGTTAACAGTGGATC
TCCCAATTCCCTGCCAATGCTCTATTTACTACCTCACAGGCCCGAAAATAT
GGGACTTCTGGGGCTACCACCATTAGGGCTGGAAAGAGAGATGGAAAC
CAATGGGGACATTGAGAAGTGGGGAGACCCTGGGAGGAGTCTTTGGATTA
GGTGGGGTTGGAGCAGGGCTTGAAATGGGGGTGGTTATGGCCATCGTTG
GCACCCATGTGCTGGGTACTCTTTCTGTGTCCAGCACCCTGTCTCCCCAT
GATCCCATGTTGTCCTCACAACAGCAAGGTGAGCTGTTATATTTGTCCCCA
TTTTACAGATGATGAAACTGAGACTCAGGTTATAACCTTTCACAGTGGCTG
GCTAGTAAGTGATAGAGCCAGACTTGGAAACCTGGAATGCCTGGCTCTAA
GGCACATGCATGCCTGGAGGCGACCCCTGTCCCAATCATGCCCTCCCAGA
GCTGTGTGGCCTCAGGATCCCAGCTCTGCAGGTCCTGGAAACCCCACCAG
AGGCCCAAGGCACCTAGCATATCAGTGCTGAGCATGCTACAGGGCTGATT
TTGGTCCTAGATTTTCCAAGATAGCCTCCAACACCCAGAGTCGCCGGACTT
TCATCAAGTCAGTACCGCCATTTCTGCGCACCCATGGCTTTGATGGGCTG
GACCTTGCCTGGCTCTACCCTGGACGGAGAGACAAACAGCATTTTACCAC
CCTAATCAAGGTGCTGGTCAGGTCAGGGACAGGGAGGGAGGTGGGCAGG
CATGAGGGAATGGGGTGGGCTAGAAGCCGGCGTCAGCTGCTGTCCTCCT
GAGGACAGGTAAAGAGGGACTTCAGCCTCAGGGCAGTGTCCTGGGACCC
TGTGCCCTGAAGATCTCACATAGCAAGGAAGGCTTCTTGTGACCATGGTG
GGAGGTGGGAATGGGGTTCTAAGAGGTGGAAGGTTGTGACTGAGCAGAG
CACCCACTTATAACTACCCACTTAGTGCATTGCCCATTGCCCACCCTTCAA
TCCCATACTGATGCCACCCATACCCAGCATGCACTGTGTCCAGCAGCTCT
CAGCTCTGCCTGGGGGCAGCCTAAGTATCTCCAGTTACCAGGGGCAGAAG
GAGAGTCTAAACAAATTGTTCACAATACCAGCAGCAATCACTTTAACTTTGG
GATTTCATGCACCAGCTAGAACAAAGCACAGACCGAAAGGCAAATGTCTC

CCAAAGTCATCTGTGGGCCAATAGGGGTCACCCACTGTCCGATGCTCCCT
CCAGGACAGGGAGTAATTGAGTGCTGATGGGTGTGCATGGGTTTGGGGA
GAAGATTAGTCAGTTTTTTAGGAAAAGGATGGGTGACTTGAGGATGGGAC
CACTGATGAGCCACTTTATCACTTCCACTAGGCCCCAGGCAGGTTGGGGA
GTACAGCAAATGGGTTGCGCAGAGACCTAGTCCGCCCTCGATGAGTCTAC
CTCTCATGCCACTTGGGACCCTTTCTCTCACCTGCCTGTCTCCCATCTAGG
AAATGAAGGCCGAATTTATAAAGGAAGCCCAGCCAGGGAAAAAGCAGCTC
CTGCTCAGCGCAGCACTGTCTGCGGGGAAGGTCACCATTGACAGCAGCTA
TGACATTGCCAAGATATCCCAGTGAGTCTCCTGCCCCAGGCAGCCTCCCC
AGAACCTCTGCCAACACTCCTGTTTCCCCGCCACCCCCAGCCCTAGCACT
CCATGTAGACTGAGGACACTGGGCTTCCAGTCCCAGTTCTTCCAAAGCCTT
GATGTGTGACCTTGAGCAAGTCACTTCCCTGCCCTGTGCCTCAGTTTCCCC
ATCTGTCAAATGAGGCATAACAATCCTTGCCTTTCCTTTTCACCTGGGCT
GTTGGCTGAGCAGTTGAGAGAGCTGCTATGAAAGTATTTTGGAAAGGGAA
GTGGAAAAAGCTATTCAAACCCTCCAGCTATTGCAGAGTATTTTCTCCACA
CCAGGCAGAGCAGAGTGCTGGGCCCTGGAGAGGCCACACAGCAGCCTCT
TTCTAGAGTGCTGGGGAGTCCTCAAGGCTCTCTCATACACACAGGCCTCC
CCCTTTCCTGTTGGCCCCACGCCTCATCCTCACCCCACCCCTCATGCCTG
TGAGGAACAAGGAACAGCCAAGCAGCCTCATCTCTCCTGAGAGCAGAGCC
ATGGGTCTCGGGAAACCCAGGAGTAAGGAACAAAGACTCTTCAAAATGAC
TTCAGAGCTTTCTTTAGGATCCCAGGGAGGTGTAAACTCAGTGCTTAATTA
AATGGATTCTTTAGAGGGTGGGAAACAGGTGGATGTCAACCATTTGCCCC
ACATACCCGTATTCATGCAATCCACCCCCAGTGGGCTCACCGGTGCGGGT
GTGCAAAACCTCCTCCCACCCCAGTCATCTTAGGTTCAGGTCATCCTTTGG
TCCTGCTCTTTCCCCGCCAGGCTGTCTGTTGATGCTACTTTTAATCTGCTTT
CACTAAGATAAGCCTGGCAGAAGTGGTGGGGGTAAGGTGGGCTGTAGGC
CAGCTCCCAAATGTGTGCTGGGCATAACAGAAGACCCATTCTTGACTGAA
GTGCCCTTGTGGGACCCTGAGCCCGTGCCCTGGAGTGGCACAGGGAGGT
GTGCCAGCAATGGGGACCGAGGTCACTGGGGACTGCTGGGGTTCGGGCT
AGTGGCCTGTCTGGCTGCTGTCTGCTTCCCTCGTTCACATCCTGCTGGAG
CCTTAAATAGGAGCCCAAAAGCTTTTCTTTCTTACTTTTTTTGAGACAAAAT
CTCACTCTGTTGCCCAGGCCGGAGTGCAGTGGCACAACCTCTGCCGCCTG

GGTTCAAGCGATTCTCCTGCCTCAGCCTCCCGAGTAGCTGGGATTACAGG
TGCCTGCCACCATGCCTGGCTAATTTTCGTAGTTAGAGTAGAGACGGGGT
TTCACCATCTTGGCCAGGCTGGTCTTGAACTCCTCACCTCATGATCCACCT
GCCTCGGCCTCCCAAAGTGCTGGGATTCCAGGTATGAGCCACCACGCCC
GGCCTAAAGCTTTTCTATTAATAATTTCCTGCCTCACCCTCCATCCCCTTCC
TCCTCAGACACCTGGATTTCATTAGCATCATGACCTACGATTTTCATGGAG
CCTGGCGTGGGACCACAGGCCATCACAGTCCCCTGTTCCGAGGTCAGGA
GGATGCAAGTCCTGACAGATTCAGCAACACTGTGAGTTCCCAGAAGGAGG
GAGGGCCAGGAGGGGGCCGCAAGACCTGCCATCTGCCAGTGCTCACACA
CCAATCTCTCTAGCCCTCAGTACAGTCCTGCAAGAGGGGGTCATGAGGC
CCATTCCACAGATAAGGAAACTGAGGCCCAAAGTCTGGGCATGCTGCGTT
GCTCTGGGAAGGTGATCTGCAGGGTAAATGGAGTGAGGGCAGGGGGCCG
AATGGGGAGAGGCTGGGAGCCGAGGAGGTAGGAGTCATTGTGCCCTCAG
AGCCAACCACCTGATTTCTGCATCTGTCAAATAGTAATAGCCCCTTCCTAT
GCCTCAAAGGATTTTTTCAAGAATGAAACTGTAAAATTCACTTTAAAGTGA
CATGATCCGTTCCCGGAGGGACAGGGGAATCCCCAGTGCACCATACACCA
ATAACCCCTGCTAAGGCAGCAGTATTAATTGCTCAACCTTCCGCACCTGTG
CACTAACTGCTCACGTTGTTCCCACCCTACCCCACCCAGGACTATGCTGTG
GGGTACATGTTGAGGCTGGGGGCTCCTGCCAGTAAGCTGGTGATGGGCA
TCCCCACCTTCGGGAGGAGCTTCACTCTGGCTTCTTCTGAGACTGGTGTT
GGAGCCCCAATCTCAGGACCGGGAATTCCAGGCCGGTTCACCAAGGAGG
CAGGGACCCTTGCCTACTATGAGGTAATGGAGTTGGGGAGAAGGTAGGAT
TCCCCCACACCACCAGCAGCCCTGGGGAAGGTGATTCCCCACCACGTTCT
TGCTTTTTCTCCTTTGGGAATAAAGAAAATGTCCGGTTGCCCCAGCATGCC
TGAGGAAGTGGAAGGGAGAGGTTAGGACATTTGTTGCTGAAAATCTCCAG
CAGGTACAGGCACATGGGCCTGCACCACTAGGCACCTGGGATAGCCCTCT
GGCTATGGGGCTGAGGTCTTCCTTCCAGCCCAGGAAAGAGCAGAGGTCAA
GAGGCAGATTTTTTGTTTCACTCTAGCCTCTGCTACTCTGTGTGGCCTTGG
GCCTGTCCTCAGTGTCAACCAGCAGGCCTCACATCTCTGTTTAAATGGAAG
AAGCTAAGCAGGGCCAAGGCAGCCACCATCTCTGGGTCATTTGCCTCTGG
TTTGTATAAACTTGTGTGATGCATGCAGCCTGCAGACCCTGCAGAGAGTGA
GGCTGCAGGATGGAGCAGGAGCTAAAAGAGATTTGGAGAGTGGCGTCTC

CTGGTGACCTGCAAGGTCTCGGCACGACTCCCCACACTGCCTTTTCCCTG
TTATCTGCTCAGATCTGTGACTTCCTCCGCGGAGCCACAGTCCATAGAATC
CTCGGCCAGCAGGTCCCCTATGCCACCAAGGGCAACCAGTGGGTAGGAT
ACGACGACCAGGAAAGCGTCAAAAGCAAGGTAGGTTTCCCCAAGGCCACA
CCTCAGGACAAAGAGAAAGAAGGAGGCCCCGCTCCCAGCAGCCAGATTC
CTGTCCCTTGCACTGAGGTCTGGGCTGGGCTCACAGAGCACATGTGCCCT
GTACACACTCTGGGTCAGGGAACCCAGCCCTGCTCCTCTGGGCCTCCCCT
GCCAGGTGCAGTACCTGAAGGACAGGCAGCTGGCGGGCGCCATGGTATG
GGCCCTGGACCTGGATGACTTCCAGGGCTCCTTCTGTGGCCAGGATCTGC
GCTTCCCTCTCACCAATGCCATCAAGGATGCACTCGCTGCAACGTAGCCC
TCTGTTCTGCACACAGCACGGGGGCCAAGGATGCCCCGTCCCCCTCTGG
CTCCAGCTGGCCGGGAGCCTGATCACCTGCCCTGCTGAGTCCCAGGCTG
AGCCTCAGTCTCCCTCCCTTGGGGCCTATGCAGAGGTCCACAACACACAG
ATTTGAGCTCAGCCCTGGTGGGCAGAGAGGTAGGGATGGGGCTGTGGGG
ATAGTGAGGCATCGCAATGTAAGACTCGGGATTAGTACACACTTGTTGATT
AATGGAAATGTTTACAGATCCCCAAGCCTGGCAAGGGAATTTCTTCAACTC
CCTGCCCCCCAGCCCTCCTTATCAAAGGACACCATTTTGGCAAGCTCTATC
ACCAAGGAGCCAAACATCCTACAAGACACAGTGACCATACTAATTATACCC
CCTGCAAAGCCCAGCTTGAAACCTTCACTTAGGAACGTAATCGTGTCCCCT
ATCCTACTTCCCCTTCCTAATTCCACAGCTGCTCAATAAAGTACAAGAGCTT
AACAGTG
[SEQ ID No: 27]

Accordingly, preferably CHI3L1 comprises or consists of a nucleotide sequence substantially as set out in SEQ ID NO: 27, or a fragment or variant thereof.

In one embodiment, MET is provided by gene bank locus ID: HGNC: 7029; Entrez Gene: 4233; Ensembl: ENSG00000105976; OMIM: 164860; and/or UniProtKB: P08581. The protein sequence may be represented by the GeneBank ID P08581, which is provided herein as SEQ ID No: 28, as follows:




























[SEQ ID No: 28]

Accordingly, preferably MET comprises or consist of an amino acid sequence substantially as set out in SEQ ID NO: 28, or a fragment or variant thereof.

In one embodiment MET is encoded by a nucleotide sequence which is provided herein as SEQ ID No: 29, as follows:

AGACACGTGCTGGGGCGGGCAGGCGAGCGCCTCAGTCTGGTCGCCTGG
CGGTGCCTCCGGCCCCAACGCGCCCGGCCGCCGCGGCCGCGCGC
CGATGCCCGGCTGAGTCACTGGCAGGGCAGCGCGTGTGGAAGGGG
CGGAGGGAGTGCGGCCGGCGGGCGGGGCGCTGGGCTCAGCCCG
GCCGCAGGTGACCCGGAGGCCCTCGCCGCCCGCGGCGCCCCGAGCGCT
TTGTGAGCAGATGCGGAGCCGAGTGGAGGGCGCGAGCCAGATGCGGGG
CGACAGCTGACTTGCTGAGAGGAGGCGGGGAGGCGCGGAGCGCGCGTG
TGGTCCTTGCGCCGCTGACTTCTCCACTGGTTCCTGGGCACCGAAAGATA
AACCTCTCATAATGAAGGCCCCCGCTGTGCTTGCACCTGGCATCCTCGTG
CTCCTGTTTACCTTGGTGCAGAGGAGCAATGGGGAGTGTAAAGAGGCACT
AGCAAAGTCCGAGATGAATGTGAATATGAAGTATCAGCTTCCCAACTTCAC
CGCGGAAACACCCATCCAGAATGTCATTCTACATGAGCATCACATTTTCCT
TGGTGCCACTAACTACATTTATGTTTTAAATGAGGAAGACCTTCAGAAGGTT
GCTGAGTACAAGACTGGGCCTGTGCTGGAACACCCAGATTGTTTCCCATG
TCAGGACTGCAGCAGCAAAGCCAATTTATCAGGAGGTGTTTGGAAAGATAA
CATCAACATGGCTCTAGTTGTCGACACCTACTATGATGATCAACTCATTAG
CTGTGGCAGCGTCAACAGAGGGACCTGCCAGCGACATGTCTTTCCCCACA
ATCATACTGCTGACATACAGTCGGAGGTTCACTGCATATTCTCCCCACAGA
TAGAAGAGCCCAGCCAGTGTCCTGACTGTGTGGTGAGCGCCCTGGGAGC
CAAAGTCCTTTCATCTGTAAAGGACCGGTTCATCAACTTCTTTGTAGGCAAT
ACCATAAATTCTTCTTATTTCCCAGATCATCCATTGCATTCGATATCAGTGA
GAAGGCTAAAGGAAACGAAAGATGGTTTTATGTTTTTGACGGACCAGTCCT
ACATTGATGTTTTACCTGAGTTCAGAGATTCTTACCCCATTAAGTATGTCCA
TGCCTTTGAAAGCAACAATTTTATTTACTTCTTGACGGTCCAAAGGGAAACT
CTAGATGCTCAGACTTTTCACACAAGAATAATCAGGTTCTGTTCCATAAACT
CTGGATTGCATTCCTACATGGAAATGCCTCTGGAGTGTATTCTCACAGAAA
AGAGAAAAAAGAGATCCACAAAGAAGGAAGTGTTTAATATACTTCAGGCTG
CGTATGTCAGCAAGCCTGGGGCCCAGCTTGCTAGACAAATAGGAGCCAGC
CTGAATGATGACATTCTTTTCGGGGTGTTCGCACAAAGCAAGCCAGATTCT

GCCGAACCAATGGATCGATCTGCCATGTGTGCATTCCCTATCAAATATGTC
AACGACTTCTTCAACAAGATCGTCAACAAAAACAATGTGAGATGTCTCCAG
CATTTTTACGGACCCAATCATGAGCACTGCTTTAATAGGACACTTCTGAGA
AATTCATCAGGCTGTGAAGCGCGCCGTGATGAATATCGAACAGAGTTTACC
ACAGCTTTGCAGCGCGTTGACTTATTCATGGGTCAATTCAGCGAAGTCCTC
TTAACATCTATATCCACCTTCATTAAAGGAGACCTCACCATAGCTAATCTTG
GGACATCAGAGGGTCGCTTCATGCAGGTTGTGGTTTCTCGATCAGGACCA
TCAACCCCTCATGTGAATTTTCTCCTGGACTCCCATCCAGTGTCTCCAGAA
GTGATTGTGGAGCATACATTAAACCAAAATGGCTACACACTGGTTATCACT
GGGAAGAAGATCACGAAGATCCCATTGAATGGCTTGGGCTGCAGACATTT
CCAGTCCTGCAGTCAATGCCTCTCTGCCCCACCCTTTGTTCAGTGTGGCT
GGTGCCACGACAAATGTGTGCGATCGGAGGAATGCCTGAGCGGGACATG
GACTCAACAGATCTGTCTGCCTGCAATCTACAAGGTTTTCCCAAATAGTGC
ACCCCTTGAAGGAGGGACAAGGCTGACCATATGTGGCTGGGACTTTGGAT
TTCGGAGGAATAATAAATTTGATTTAAAGAAAACTAGAGTTCTCCTTGGAAA
TGAGAGCTGCACCTTGACTTTAAGTGAGAGCACGATGAATACATTGAAATG
CACAGTTGGTCCTGCCATGAATAAGCATTTCAATATGTCCATAATTATTTCA
AATGGCCACGGGACAACACAATACAGTACATTCTCCTATGTGGATCCTGTA
ATAACAAGTATTTCGCCGAAATACGGTCCTATGGCTGGTGGCACTTTACTT
ACTTTAACTGGAAATTACCTAAACAGTGGGAATTCTAGACACATTTCAATTG
GTGGAAAAACATGTACTTTAAAAAGTGTGTCAAACAGTATTCTTGAATGTTA
TACCCCAGCCCAAACCATTTCAACTGAGTTTGCTGTTAAATTGAAAATTGAC
TTAGCCAACCGAGAGACAAGCATCTTCAGTTACCGTGAAGATCCCATTGTC
TATGAAATTCATCCAACCAAATCTTTTATTAGTACTTGGTGGAAAGAACCTC
TCAACATTGTCAGTTTTCTATTTTGCTTTGCCAGTGGTGGGAGCACAATAAC
AGGTGTTGGGAAAAACCTGAATTCAGTTAGTGTCCCGAGAATGGTCATAAA
TGTGCATGAAGCAGGAAGGAACTTTACAGTGGCATGTCAACATCGCTCTAA
TTCAGAGATAATCTGTTGTACCACTCCTTCCCTGCAACAGCTGAATCTGCA
ACTCCCCCTGAAAACCAAAGCCTTTTTCATGTTAGATGGGATCCTTTCCAA
ATACTTTGATCTCATTTATGTACATAATCCTGTGTTTAAGCCTTTTGAAAAGC
CAGTGATGATCTCAATGGGCAATGAAAATGTACTGGAAATTAAGGGAAATG
ATATTGACCCTGAAGCAGTTAAAGGTGAAGTGTTAAAAGTTGGAAATAAGA

GCTGTGAGAATATACACTTACATTCTGAAGCCGTTTTATGCACGGTCCCCA
ATGACCTGCTGAAATTGAACAGCGAGCTAAATATAGAGTGGAAGCAAGCAA
TTTCTTCAACCGTCCTTGGAAAAGTAATAGTTCAACCAGATCAGAATTTCAC
AGGATTGATTGCTGGTGTTGTCTCAATATCAACAGCACTGTTATTACTACTT
GGGTTTTTCCTGTGGCTGAAAAAGAGAAAGCAAATTAAAGATCTGGGCAGT
GAATTAGTTCGCTACGATGCAAGAGTACACACTCCTCATTTGGATAGGCTT
GTAAGTGCCCGAAGTGTAAGCCCAACTACAGAAATGGTTTCAAATGAATCT
GTAGACTACCGAGCTACTTTTCCAGAAGATCAGTTTCCTAATTCATCTCAGA
ACGGTTCATGCCGACAAGTGCAGTATCCTCTGACAGACATGTCCCCCATC
CTAACTAGTGGGGACTCTGATATATCCAGTCCATTACTGCAAAATACTGTC
CACATTGACCTCAGTGCTCTAAATCCAGAGCTGGTCCAGGCAGTGCAGCA
TGTAGTGATTGGGCCCAGTAGCCTGATTGTGCATTTCAATGAAGTCATAGG
AAGAGGGCATTTTGGTTGTGTATATCATGGGACTTTGTTGGACAATGATGG
CAAGAAAATTCACTGTGCTGTGAAATCCTTGAACAGAATCACTGACATAGG
AGAAGTTTCCCAATTTCTGACCGAGGGAATCATCATGAAAGATTTTAGTCAT
CCCAATGTCCTCTCGCTCCTGGGAATCTGCCTGCGAAGTGAAGGGTCTCC
GCTGGTGGTCCTACCATACATGAAACATGGAGATCTTCGAAATTTCATTCG
AAATGAGACTCATAATCCAACTGTAAAAGATCTTATTGGCTTTGGTCTTCAA
GTAGCCAAAGGCATGAAATATCTTGCAAGCAAAAAGTTTGTCCACAGAGAC
TTGGCTGCAAGAAACTGTATGCTGGATGAAAAATTCACAGTCAAGGTTGCT
GATTTTGGTCTTGCCAGAGACATGTATGATAAAGAATACTATAGTGTACACA
ACAAAACAGGTGCAAAGCTGCCAGTGAAGTGGATGGCTTTGGAAAGTCTG
CAAACTCAAAAGTTTACCACCAAGTCAGATGTGTGGTCCTTTGGCGTGCTC
CTCTGGGAGCTGATGACAAGAGGAGCCCCACCTTATCCTGACGTAAACAC
CTTTGATATAACTGTTTACTTGTTGCAAGGGAGAAGACTCCTACAACCCGA
ATACTGCCCAGACCCCTTATATGAAGTAATGCTAAAATGCTGGCACCCTAA
AGCCGAAATGCGCCCATCCTTTTCTGAACTGGTGTCCCGGATATCAGCGA
TCTTCTCTACTTTCATTGGGGAGCACTATGTCCATGTGAACGCTACTTATGT
GAACGTAAAATGTGTCGCTCCGTATCCTTCTCTGTTGTCATCAGAAGATAA
CGCTGATGATGAGGTGGACACACGACCAGCCTCCTTCTGGGAGACATCAT
AGTGCTAGTACTATGTCAAAGCAACAGTCCACACTTTGTCCAATGGTTTTTT
CACTGCCTGACCTTTAAAAGGCCATCGATATTCTTTGCTCTTGCCAAAATTG

CACTATTATAGGACTTGTATTGTTATTTAAATTACTGGATTCTAAGGAATTTC
TTATCTGACAGAGCATCAGAACCAGAGGCTTGGTCCCACAGGCCACGGAC
CAATGGCCTGCAGCCGTGACAACACTCCTGTCATATTGGAGTCCAAAACTT
GAATTCTGGGTTGAATTTTTAAAAATCAGGTACCACTTGATTTCATATGGG
AAATTGAAGCAGGAAATATTGAGGGCTTCTTGATCACAGAAAACTCAGAAG
AGATAGTAATGCTCAGGACAGGAGCGGCAGCCCCAGAACAGGCCACTCAT
TTAGAATTCTAGTGTTTCAAAACACTTTTGTGTGTTGTATGGTCAATAACATT
TTTCATTACTGATGGTGTCATTCACCCATTAGGTAAACATTCCCTTTTAAAT
GTTTGTTTGTTTTTTGAGACAGGATCTCACTCTGTTGCCAGGGCTGTAGTG
CAGTGGTGTGATCATAGCTCACTGCAACCTCCACCTCCCAGGCTCAAGCC
TCCCGAATAGCTGGGACTACAGGCGCACCACCATCCCCGGCTAATTTT
TGTATTTTTTGTAGAGACGGGGTTTTGCCATGTTGCCAAGGCTGGTTTCAA
ACTCCTGGACTCAAGAAATCCACCCACCTCAGCCTCCCAAAGTGCTAGGA
TTACAGGCATGAGCCACTGCGCCCAGCCCTTATAAATTTTTGTATAGACAT
TCCTTTGGTTGGAAGAATATTTATAGGCAATACAGTCAAAGTTTCAAAATAG
CATCACACAAAACATGTTTATAAATGAACAGGATGTAATGTACATAGATGAC
ATTAAGAAAATTTGTATGAAATAATTTAGTCATCATGAAATATTTAGTTGTCA
TATAAAAACCCACTGTTTGAGAATGATGCTACTCTGATCTAATGAATGTGAA
CATGTAGATGTTTTGTGTGTATTTTTTTAAATGAAAACTCAAAATAAGACAA
GTAATTTGTTGATAAATATTTTTAAAGATAACTCAGCATGTTTGTAAAGCAG
GATACATTTTACTAAAGGTTCATTGGTTCCAATCACAGCTCATAGGTAGAG
CAAAGAAAGGGTGGATGGATTGAAAAGATTAGCCTCTGTCTCGGTGGCAG
GTTCCCACCTCGCAAGCAATTGGAAACAAAACTTTTGGGGAGTTTTATTTT
GCATTAGGGTGTGTTTTATGTTAAGCAAAACATACTTTAGAAACAAATGAAA
AAGGCAATTGAAAATCCCAGCTATTTCACCTAGATGGAATAGCCACCCTGA
GCAGAACTTTGTGATGCTTCATTCTGTGGAATTTTGTGCTTGCTACTGTATA
GTGCATGTGGTGTAGGTTACTCTAACTGGTTTTGTCGACGTAAACATTTAA
AGTGTTATATTTTTTATAAAAATGTTTATTTTTAATGATATGAGAAAAATTTTG
TTAGGCCACAAAAACACTGCACTGTGAACATTTTAGAAAAGGTATGTCAGA
CTGGGATTAATGACAGCATGATTTTCAATGACTGTAAATTGCGATAAGGAA
ATGTACTGATTGCCAATACACCCCACCCTCATTACATCATCAGGACTTGAA
GCCAAGGGTTAACCCAGCAAGCTACAAAGAGGGTGTGTCACACTGAAACT

CAATAGTTGAGTTTGGCTGTTGTTGCAGGAAAATGATTATAACTAAAAGCTC
TCTGATAGTGCAGAGACTTACCAGAAGACACAAGGAATTGTACTGAAGAGC
TATTACAATCCAAATATTGCCGTTTCATAAATGTAATAAGTAATACTAATTCA
CAGAGTATTGTAAATGGTGGATGACAAAAGAAAATCTGCTCTGTGGAAAGA
AAGAACTGTCTCTACCAGGGTCAAGAGCATGAACGCATCAATAGAAAGAAC
TCGGGGAAACATCCCATCAACAGGACTACACACTTGTATATACATTCTTGA
GAACACTGCAATGTGAAAATCACGTTTGCTATTTATAAACTTGTCCTTAGAT
TAATGTGTCTGGACAGATTGTGGGAGTAAGTGATTCTTCTAAGAATTAGAT
ACTTGTCACTGCCTATACCTGCAGCTGAACTGAATGGTACTTCGTATGTTA
ATAGTTGTTCTGATAAATCATGCAATTAAAGTAAAGTGATGCAA
[SEQ ID No: 29]

Accordingly, preferably MET comprises or consist of a nucleotide sequence substantially as set out in SEQ ID NO: 29, or a fragment or variant thereof.

In one embodiment, GDF15 is provided by gene bank locus ID: HGNC: 30142; Entrez Gene: 9518; Ensembl: ENSG00000130513; OMIM: 605312; and/or UniProtKB: Q99988. The protein sequence may be represented by the GeneBank ID Q99988, which is provided herein as SEQ ID No: 30, as follows:

MPGQELRTVNGSQMLLVLLVLSWLPHGGALSLAEASRASFPGPSELHSEDSR
FRELRKRYEDLLTRLRANQSWEDSNTDLVPAPAVRILTPEVRLGSGGHLHLRI
SRAALPEGLPEASRLHRALFRLSPTASRSWDVTRPLRRQLSLARPQAPALHLR
LSPPPSQSDQLLAESSSARPQLELHLRPQAARGRRRARARNGDHCPLGPGR
CCRLHTVRASLEDLGWADWVLSPREVQVTMCIGACPSQFRAANMHAQIKTSL
HRLKPDTVPAPCCVPASYNPMVLIQKTDTGVSLQTYDDLLAKDCHCI
[SEQ ID No:30]

Accordingly, preferably GDF15 comprises or consists of an amino acid sequence substantially as set out in SEQ ID NO: 30, or a fragment or variant thereof.

In one embodiment GDF15 is encoded by a nucleotide sequence which is provided herein as SEQ ID No: 31, as follows:

AGTCCCAGCTCAGAGCCGCAACCTGCACAGCCATGCCCGGGCAAGAACT
CAGGACGGTGAATGGCTCTCAGATGCTCCTGGTGTTGCTGGTGCTCTCGT
GGCTGCCGCATGGGGGCGCCCTGTCTCTGGCCGAGGCGAGCCGCGCAA
GTTTCCCGGGACCCTCAGAGTTGCACTCCGAAGACTCCAGATTCCGAGAG
TTGCGGAAACGCTACGAGGACCTGCTAACCAGGCTGCGGGCCAACCAGA
GCTGGGAAGATTCGAACACCGACCTCGTCCCGGCCCCTGCAGTCCGGAT
ACTCACGCCAGAAGTGCGGCTGGGATCCGGCGGCCACCTGCACCTGCGT
ATCTCTCGGGCCGCCCTTCCCGAGGGGCTCCCCGAGGCCTCCCGCCTTC
ACCGGGCTCTGTTCCGGCTGTCCCCGACGGCGTCAAGGTCGTGGACGT
GACACGACCGCTGCGGCGTCAGCTCAGCCTTGCAAGACCCCAGGCGCCC
GCGCTGCACCTGCGACTGTCGCCGCCGCCGTCGCAGTCGGACCAACTGC
TGGCAGAATCTTCGTCCGCACGGCCCCAGCTGGAGTTGCACTTGCGGCC
GCAAGCCGCCAGGGGGCGCCGCAGAGCGCGTGCGCGCAACGGGGACCA
CTGTCCGCTCGGGCCCGGGCGTTGCTGCCGTCTGCACACGGTCCGCGCG
TCGCTGGAAGACCTGGGCTGGGCCGATTGGGTGCTGTCGCCACGGGAGG
TGCAAGTGACCATGTGCATCGGCGCGTGCCCGAGCCAGTTCCGGGCGGC
AAACATGCACGCGCAGATCAAGACGAGCCTGCACCGCCTGAAGCCCGAC
ACGGTGCCAGCGCCCTGCTGCGTGCCCGCCAGCTACAATCCCATGGTGC
TCATTCAAAAGACCGACACCGGGGTGTCGCTCCAGACCTATGATGACTTG
TTAGCCAAAGACTGCCACTGCATATGAGCAGTCCTGGTCCTTCCACTGTGC
ACCTGCGCGGAGGACGCGACCTCAGTTGTCCTGCCCTGTGGAATGGGCT
CAAGGTTCCTGAGACACCCGATTCCTGCCCAAACAGCTGTATTTATATAAG
TCTGTTATTTATTATTAATTTATTGGGGTGACCTTCTTGGGGACTCGGGGG
CTGGTCTGATGGAACTGTGTATTTATTTAAAACTCTGGTGATAAAAATAAAG
CTGTCTGAACTGTT

[SEQ ID No: 31]

Accordingly, preferably GDF15comprises or consists of a nucleotide sequence substantially as set out in SEQ ID NO: 31, or a fragment or variant thereof.

In one embodiment, CCL22 is provided by gene bank locus ID: HGNC: 10621; Entrez Gene: 6367; Ensembl: ENSG00000102962; OMIM: 602957; and/or UniProtKB: O00626. The protein sequence may be represented by the GeneBank ID O00626, which is provided herein as SEQ ID No: 32, as follows:

MDRLQTALLVVLVLLAVALQATEAGPYGANMEDSVCCRDYVRYRLPLRVVKH
FYWTSDSC
PRPGVVLLTFRDKEICADPRVPWVKMILNKLSQ
[SEQ ID No: 32]

Accordingly, preferably CCL22 comprises or consists of an amino acid sequence substantially as set out in SEQ ID NO: 32, or a fragment or variant thereof.

In one embodiment CCL22 is encoded by a nucleotide sequence which is provided herein as SEQ ID No: 33, as follows:

GCAGACACCTGGGCTGAGACATACAGGACAGAGCATGGATCGCCTACAGA
CTGCACTCCTGGTTGTCCTCGTCCTCCTTGCTGTGGCGCTTCAAGCAACT
GAGGCAGGCCCCTACGGCGCCAACATGGAAGACAGCGTCTGCTGCCGTG
ATTACGTCCGTTACCGTCTGCCCCTGCGCGTGGTGAAACACTTCTACTGG
ACCTCAGACTCCTGCCCGAGGCCTGGCGTGGTGTTGCTAACCTTCAGGGA
TAAGGAGATCTGTGCCGATCCCAGAGTGCCCTGGGTGAAGATGATTCTCA
ATAAGCTGAGCCAATGAAGAGCCTACTCTGATGACCGTGGCCTTGGCTCC
TCCAGGAAGGCTCAGGAGCCCTACCTCCCTGCCATTATAGCTGCTCCCCG
CCAGAAGCCTGTGCCAACTCTCTGCATTCCCTGATCTCCATCCCTGTGGCT
GTCACCCTTGGTCACCTCCGTGCTGTCACTGCCATCTCCCCCCTGACCCC
TCTAACCCATCCTCTGCCTCCCTCCCTGCAGTCAGAGGGTCCTGTTCCCAT
CAGCGATTCCCCTGCTTAAACCCTTCCATGACTCCCCACTGCCCTAAGCTG
AGGTCAGTCTCCCAAGCCTGGCATGTGGCCCTCTGGATCTGGGTTCCATC
TCTGTCTCCAGCCTGCCCACTTCCCTTCATGAATGTTGGGTTCTAGCTCCC
TGTTCTCCAAACCCATACTACACATCCCACTTCTGGGTCTTTGCCTGGGAT
GTTGCTGACACCCAGAAAGTCCCACCACCTGCACATGTGTAGCCCCACCA
GCCCTCCAAGGCATTGCTCGCCCAAGCAGCTGGTAATTCCATTTCATGTAT
TAGATGTCCCCTGGCCCTCTGTCCCCTCTTAATAACCCTAGTCACAGTCTC
CGCAGATTCTTGGGATTTGGGGGTTTTCTCCCCCACCTCTCCACTAGTTGG
ACCAAGGTTTCTAGCTAAGTTACTCTAGTCTCCAAGCCTCTAGCATAGAGC
ACTGCAGACAGGCCCTGGCTCAGAATCAGAGCCCAGAAAGTGGCTGCAG
ACAAAATCAATAAAACTAATGTCCCTCCCCTCTCCCTGCCAAAAGGCAGTT
ACATATCAATACAGAGACTCAAGGTCACTAGAAATGGGCCAGCTGGGTCA
ATGTGAAGCCCCAAATTTGCCCAGATTCACCTTTCTTCCCCCACTCCCTTTT
TTTTTTTTTTTTTGAGATGGAGTTTCGCTCTTGTCACCCACGCTGGAGTGCA

ATGGTGTGGTCTTGGCTTATTGAAGCCTCTGCCTCCTGGGTTCAAGTGATT
CTCTTGCCTCAGCCTCCTGAGTAGCTGGGATTACAGGTTCCTGCTACCAC
GCCCAGCTAATTTTTGTATTTTTAGTAGAGACGAGGCTTCACCATGTTGGC
CAGGCTGGTCTCGAACTCCTGTCCTCAGGTAATCCGCCCACCTCAGCCTC
CCAAAGTGCTGGGATTACAGGCGTGAGCCACAGTGCCTGGCCTCTTCCCT
CTCCCCACCCCCCCCCAACTTTTTTTTTTTTTATGGCAGGGTCTCACTCT
GTCGCCCAGGCTGGAGTGCAGTGGCGTGATCTCGGCTCACTACAACCTC
GACCTCCTGGGTTCAAGCGATTCTCCCACCCCAGCCTCCCAAGTAGCTGG
GATTACAGGTGTGTGCCACTACGGCTGGCTAATTTTTGTATTTTTAGTAGA
GACAGGTTTCACCATATTGGCCAGGCTGGTCTTGAACTCCTGACCTCAAGT
GATCCACCTTCCTTGTGCTCCCAAAGTGCTGAGATTACAGGCGTGAGCTAT
CACACCCAGCCTCCCCCTTTTTTCCTAATAGGAGACTCCTGTACCTTTCTT
CGTTTTACCTATGTGTCGTGTCTGCTTACATTTCCTTCTCCCCTCAGGCTTT
TTTTGGGTGGTCCTCCAACCTCCAATACCCAGGCCTGGCCTCTTCAGAGTA
CCCCCCATTCCACTTTCCCTGCCTCCTTCCTTAAATAGCTGACAATCAAATT
CATGCTATGGTGTGAAAGACTACCTTTGACTTGGTATTATAAGCTGGAGTT
ATATATGTATTTGAAAACAGAGTAAATACTTAAGAGGCCAAATAGATGAATG
GAAGAATTTTAGGAACTGTGAGAGGGGACAAGGTGGAGCTTTCCTGGCC
CTGGGAGGAAGCTGGCTGTGGTAGCGTAGCGCTCTCTCTCTGTCTGTG
GCAGGAGGCAAAGAGTAGGGTGTAATTGAGTGAAGGAATCCTGGGTAGAG
ACCATTCTCAGGTGGTTGGGCCAGGCTAAAGACTGGGATTTGGGTCTATC
TATGCCTTTCTGGCTGATTTTTGTAGAGACGGGGTTTTGCCATGTTACCCA
GGCTGGTCTCAAACTCCTGGGCTCAAGCGATCCTCCTGGCTCAGCCTCCC
AAAGTGCTGGGATTACAGGCGTGAGTCACTGCGCCTGGCTTCCTCTTCCT
CTTGAGAAATATTCTTTTCATACAGCAAGTATGGACAGCAGTGTCCCAGG
TAAAGGACATAAATGTTACAAGTGTCTGGTCCTTTCTGAGGGAGGCTGGTG
CCGCTCTGCAGGGTATTTGAACCTGTGGAATTGGAGGAGGCCATTTCACT
CCCTGAACCCAGCCTGACAATCACAGTGAGAATGTTCACCTTATAGGCTT
GCTGTGGGGCTCAGGTTGAAAGTGTGGGGAGTGACACTGCCTAGGCATC
CAGCTCAGTGTCATCCAGGGCCTGTGTCCCTCCCGAACCCAGGGTCAACC
TGCCTACCACAGGCACTAGAAGGACGAATCTGCCTACTGCCCATGAACGG

GGCCCTCAAGCGTCCTGGGATCTCCTTCTCCCTCCTGTCCTGTCCTTGCC
CCTCAGGACTGCTGGAAAATAAATCCTTTAAAATAG
[SEQ ID No: 33]

Accordingly, preferably CCL22 comprises or consists of a nucleotide sequence substantially as set out in SEQ ID NO: 33 or a fragment or variant thereof.

In one embodiment, TNFRSF11 is provided by gene bank locus ID: HGNC: 11909; Entrez Gene: 4982; Ensembl: ENSG00000164761; OMIM: 602643; and/or UniProtKB: O00300. The protein sequence may be represented by the GeneBank ID O00300, which is provided herein as SEQ ID No: 34, as follows:

MNNLLCCALVFLDISIKWTTQETFPPKYLHYDEETSHQLLCDKCPPGTYLKQH
CTAKWKTVCAPCPDHYYTDSWHTSDECLYCSPVCKELQYVKQECNRTHNRV
CECKEGRYLEIEFCLKHRSCPPGFGVVQAGTPERNTVCKRCPDGFFSNETSS
KAPCRKHTNCSVFGLLLTQKGNATHDNICSGNSESTQKCGIDVTLCEEAFFRF
AVPTKFTPNWLSVLVDNLPGTKVNAESVERIKRQHSSQEQTFQLLKLWKHQN
KDQDIVKKIIQDIDLCENSVQRHIGHANLTFEQLRSLMESLPGKKVGAEDIEKTI
KACKPSDQILKLLSLWRIKNGDQDTLKGLMHALKHSKTYHFPKTVTQSLKKTIR
FLHSFTMYKLYQKLFLEMIGNQVQSVKISCL
[SEQ ID No: 34]

Accordingly, preferably TNFRSF11 comprises or consists of an amino acid sequence substantially as set out in SEQ ID NO: 34, or a fragment or variant thereof.

In one embodiment TNFRSF11 is be encoded by a nucleotide sequence which is provided herein as SEQ ID No: 35, as follows:

GGAGACGCACCGGAGCGCTCGCCCAGCCGCCGCCTCCAAGCCCCTGAG
GTTTCCGGGGACCACAATGAACAACTTGCTGTGCTGCGCGCTCGTGTTTC
TGGACATCTCCATTAAGTGGACCACCCAGGAAACGTTTCCTCCAAAGTACC
TTCATTATGACGAAGAAACCTCTCATCAGCTGTTGTGTGACAAATGTCCTC

CTGGTACCTACCTAAAACAACACTGTACAGCAAAGTGGAAGACCGTGTGC
GCCCCTTGCCCTGACCACTACTACACAGACAGCTGGCACACCAGTGACGA
GTGTCTATACTGCAGCCCCGTGTGCAAGGAGCTGCAGTACGTCAAGCAGG
AGTGCAATCGCACCCACAACCGCGTGTGCGAATGCAAGGAAGGGCGCTA
CCTTGAGATAGAGTTCTGCTTGAAACATAGGAGCTGCCCTCCTGGATTTGG
AGTGGTGCAAGCTGGAACCCCAGAGCGAAATACAGTTTGCAAAAGATGTC
CAGATGGGTTCTTCTCAAATGAGACGTCATCTAAAGCACCCTGTAGAAAAC
ACACAAATTGCAGTGTCTTTGGTCTCCTGCTAACTCAGAAAGGAAATGCAA
CACACGACAACATATGTTCCGGAAACAGTGAATCAACTCAAAAATGTGGAA
TAGATGTTACCCTGTGTGAGGAGGCATTCTTCAGGTTTGCTGTTCCTACAA
AGTTTACGCCTAACTGGCTTAGTGTCTTGGTAGACAATTTGCCTGGCACCA
AAGTAAACGCAGAGAGTGTAGAGAGGATAAAACGGCAACACAGCTCACAA
GAACAGACTTTCCAGCTGCTGAAGTTATGGAAACATCAAAACAAAGACCAA
GATATAGTCAAGAAGATCATCCAAGATATTGACCTCTGTGAAAACAGCGTG
CAGCGGCACATTGGACATGCTAACCTCACCTTCGAGCAGCTTCGTAGCTT
GATGGAAAGCTTACCGGGAAAGAAAGTGGGAGCAGAAGACATTGAAAAA
CAATAAAGGCATGCAAACCCAGTGACCAGATCCTGAAGCTGCTCAGTTTGT
GGCGAATAAAAAATGGCGACCAAGACACCTTGAAGGGCCTAATGCACGCA
CTAAAGCACTCAAAGACGTACCACTTTCCCAAAACTGTCACTCAGAGTCTA
AAGAAGACCATCAGGTTCCTTCACAGCTTCACAATGTACAAATTGTATCAG
AAGTTATTTTTAGAAATGATAGGTAACCAGGTCCAATCAGTAAAAATAAGCT
GCTTATAACTGGAAATGGCCATTGAGCTGTTTCCTCACAATTGGCGAGATC
CCATGGATGAGTAAACTGTTTCTCAGGCACTTGAGGCTTTCAGTGATATCT
TTCTCATTACCAGTGACTAATTTTGCCACAGGGTACTAAAAGAAACTATGAT
GTGGAGAAAGGACTAACATCTCCTCCAATAAACCCCAAATGGTTAATCCAA
CTGTCAGATCTGGATCGTTATCTACTGACTATATTTTCCCTTATTACTGCTT
GCAGTAATTCAACTGGAAATTAAAAAAAAAAAACTAGACTCCATTGTGCCTT
ACTAAATATGGGAATGTCTAACTTAAATAGCTTTGAGATTTCAGCTATGCTA
GAGGCTTTTATTAGAAAGCCATATTTTTTTCTGTAAAAGTTACTAATATATCT
GTAACACTATTACAGTATTGCTATTTATATTCATTCAGATATAAGATTTGTAC
ATATTATCATCCTATAAAGAAACGGTATGACTTAATTTTAGAAAGAAAATTAT
ATTCTGTTTATTATGACAAATGAAAGAGAAAATATATATTTTTAATGGAAAGT

TTGTAGCATTTTTCTAATAGGTACTGCCATATTTTTCTGTGTGGAGTATTTTT
ATAATTTTATCTGTATAAGCTGTAATATCATTTTATAGAAAATGCATTATTTA
GTCAATTGTTTAATGTTGGAAAACATATGAAATATAAATTATCTGAATATTAG
ATGCTCTGAGAAATTGAATGTACCTTATTTAAAAGATTTTATGGTTTTATAAC
TATATAAATGACATTATTAAAGTTTTCAAATTATTTTTTA

[SEQ ID No: 35]

Accordingly, preferably TNFRSF11 comprises or consists of a nucleotide sequence substantially as set out in SEQ ID NO: 35, or a fragment or variant thereof.

In one embodiment, ANGPT2 is provided by gene bank locus ID is HGNC: 485; Entrez Gene: 285; Ensembl: ENSG00000091879; OMIM: 601922; and/or UniProtKB: O15123. The protein sequence may be represented by the GeneBank ID O15123, which is provided herein as SEQ ID No: 36, as follows:

MWQIVFFTLSCDLVLAAAYNNFRKSMDSIGKKQYQVQHGSCSYTFLLPEMDN
CRSSSSPYVSNAVQRDAPLEYDDSVQRLQVLENIMENNTQWLMKLENYIQDN
MKKEMVEIQQNAVQNQTAVMIEIGTNLLNQTAEQTRKLTDVEAQVLNQTTRLE
LQLLEHSLSTNKLEKQILDQTSEINKLQDKNSFLEKKVLAMEDKHIIQLQSIKEEK
DQLQVLVSKQNSIIEELEKKIVTATVNNSVLQKQQHDLMETVNNLLTMMSTSN
SAKDPTVAKEEQISFRDCAEVFKSGHTTNGIYTLTFPNSTEEIKAYCDMEAGG
GGWTIIQRREDGSVDFQRTWKEYKVGFGNPSGEYWLGNEFVSQLTNQQRYV
LKIHLKDWEGNEAYSLYEHFYLSSEELNYRIHLKGLTGTAGKISSISQPGNDFS
TKDGDNDKCICKCSQMLTGGWWFDACGPSNLNGMYYPQRQNTNKFNGIKW
YYWKGSGYSLKATTMMIRPADF

[SEQ ID No: 36]

Accordingly, preferably ANGPT2 comprises or consists of an amino acid sequence substantially as set out in SEQ ID NO: 36, or a fragment or variant thereof.

In one embodiment ANGPT2 is encoded by a nucleotide sequence which is provided herein as SEQ ID No: 37, as follows:

CTGGTTGGAGGGCAGGCATTCTGCTCTGATTTTTCCTGTTGCCTGGCTAGT
GACCCCCTACAGGAAGATAACGGCTAAGCCAGGAGGGCGGAGCAGCCCA
CTACACATGTCTGGCTGCTCTTATCAACTTATCATATAAGGAAAGGAAAGT
GATTGATTCGGATACTGACACTGTAGGATCTGGGGAGAGAGGAACAAAGG
ACCGTGAAAGCTGCTCTGTAAAAGCTGACACAGCCCTCCCAAGTGAGCAG
GACTGTTCTTCCCACTGCAATCTGACAGTTTACTGCATGCCTGGAGAGAAC
ACAGCAGTAAAAACCAGGTTTGCTACTGGAAAAAGAGGAAAGAGAAGACTT
TCATTGACGGACCCAGCCATGGCAGCGTAGCAGCCCTGCGTTTTAGACGG
CAGCAGCTCGGGACTCTGGACGTGTGTTTGCCCTCAAGTTTGCTAAGCTG
CTGGTTTATTACTGAAGAAAGAATGTGGCAGATTGTTTCTTTACTCTGAGC
TGTGATCTTGTCTTGGCCGCAGCCTATAACAACTTTCGGAAGAGCATGGAC
AGCATAGGAAAGAAGCAATATCAGGTCCAGCATGGGTCCTGCAGCTACAC
TTTCCTCCTGCCAGAGATGGACAACTGCCGCTCTTCCTCCAGCCCCTACG
TGTCCAATGCTGTGCAGAGGGACGCGCCGCTCGAATACGATGACTCGGTG
CAGAGGCTGCAAGTGCTGGAGAACATCATGGAAAACAACACTCAGTGGCT
AATGAAGCTTGAGAATTATATCCAGGACAACATGAAGAAAGAAATGGTAGA
GATACAGCAGAATGCAGTACAGAACCAGACGGCTGTGATGATAGAAATAG
GGACAAACCTGTTGAACCAAACAGCGGAGCAAACGCGGAAGTTAACTGAT
GTGGAAGCCCAAGTATTAAATCAGACCACGAGACTTGAACTTCAGCTCTTG
GAACACTCCCTCTCGACAAACAAATTGGAAAAACAGATTTTGGACCAGACC
AGTGAAATAAACAAATTGCAAGATAAGAACAGTTTCCTAGAAAAGAAGGTG
CTAGCTATGGAAGACAAGCACATCATCCAACTACAGTCAATAAAAGAAGAG
AAAGATCAGCTACAGGTGTTAGTATCCAAGCAAAATTCCATCATTGAAGAA
CTAGAAAAAAAAATAGTGACTGCCACGGTGAATAATTCAGTTCTTCAGAAG
CAGCAACATGATCTCATGGAGACAGTTAATAACTTACTGACTATGATGTCC
ACATCAAACTCAGCTAAGGACCCCACTGTTGCTAAAGAAGAACAAATCAGC
TTCAGAGACTGTGCTGAAGTATTCAAATCAGGACACACCACGAATGGCATC
TACACGTTAACATTCCCTAATTCTACAGAAGAGATCAAGGCCTACTGTGAC

ATGGAAGCTGGAGGAGGCGGGTGGACAATTATTCAGCGACGTGAGGATG
GCAGCGTTGATTTTCAGAGGACTTGGAAAGAATATAAAGTGGGATTTGGTA
ACCCTTCAGGAGAATATTGGCTGGGAAATGAGTTTGTTTCGCAACTGACTA
ATCAGCAACGCTATGTGCTTAAAATACACCTTAAAGACTGGGAAGGGAATG
AGGCTTACTCATTGTATGAACATTTCTATCTCTCAAGTGAAGAACTCAATTA
TAGGATTCACCTTAAAGGACTTACAGGGACAGCCGGCAAAATAAGCAGCA
TCAGCCAACCAGGAAATGATTTTAGCACAAAGGATGGAGACAACGACAAAT
GTATTTGCAAATGTTCACAAATGCTAACAGGAGGCTGGTGGTTTGATGCAT
GTGGTCCTTCCAACTTGAACGGAATGTACTATCCACAGAGGCAGAACACAA
ATAAGTTCAACGGCATTAAATGGTACTACTGGAAAGGCTCAGGCTATTCGC
TCAAGGCCACAACCATGATGATCCGACCAGCAGATTTCTAAACATCCCAGT
CCACCTGAGGAACTGTCTCGAACTATTTTCAAAGACTTAAGCCCAGTGCAC
TGAAAGTCACGGCTGCGCACTGTGTCCTCTTCCACCACAGAGGGCGTGTG
CTCGGTGCTGACGGGACCCACATGCTCCAGATTAGAGCCTGTAAACTTTAT
CACTTAAACTTGCATCACTTAACGGACCAAAGCAAGACCCTAAACATCCAT
AATTGTGATTAGACAGAACACCTATGCAAAGATGAACCCGAGGCTGAGAAT
CAGACTGACAGTTTACAGACGCTGCTGTCACAACCAAGAATGTTATGTGCA
AGTTTATCAGTAAATAACTGGAAAACAGAACACTTATGTTATACAATACAGA
TCATCTTGGAACTGCATTCTTCTGAGCACTGTTTATACACTGTGTAAATACC
CATATGTCCTGAATTCACCATCACTATCACAATTAAAAGGAAGAAAAAACT
CTCTAAGCCATAAAAGACATATTCAGGGATATTCTGAGAAGGGGTTACTA
GAAGTTTAATATTTGGAAAAACAGTTAGTGCATTTTTACTCCATCTCTTAGG
TGCTTTAAATTTTTATTTCAAAAACAGCGTATTTACATTTATGTTGACAGCTT
AGTTATAAGTTAATGCTCAAATACGTATTTCAAATTTATATGGTAGAAACTTC
CAGAATCTCTGAAATTATCAACAGAAACGTGCCATTTTAGTTTATATGCAGA
CCGTACTATTTTTTTCTGCCTGATTGTTAAATATGAAGGTATTTTTAGTAATT
AAATATAACTTATTAGGGGATATGCCTATGTTTAACTTTTATGATAATATTTA
CAATTTTATAATTTGTTTCCAAAAGACCTAATTGTGCCTTGTGATAAGGAAA
CTTCTTACTTTTAATGATGAGGAAAATTATACATTTCATTCTATGACAAAGAA
ACTTTACTATCTTCTCACTATTCTAAAACAGAGGTCTGTTTTCTTTCCTAGTA
AGATATATTTTTATAGAACTAGACTACAATTTAATTTCTGGTTGAGAAAAGC
CTTCTATTTAAGAAATTTACAAAGCTATATGTCTCAAGATTCACCCTTAAATT

TACTTAAGGAAAAAATAATTGACACTAGTAAGTTTTTTATGTCAATCAGC
AAACTGAAAAAAAAAAAGGGTTTCAAAGTGCAAAAACAAAATCTGATGTTC
ATAATATATTTAAATATTTACCAAAAATTTGAGAACACAGGGCTGGGCGCAG
TGGCTCACACCTATAATCCCAGTACATTGGTAGGCAAGGTGGGCAGATCA
CCTGAGGTCAGGAGTTCAAGACCAGCCTGGACAACATGGTGAAACCCTGT
CTCTACTAAATAATACAAAAATTAGCCAGGCGTGCTGGCGGGCACCTGTAA
TCCCAGCTACTCGGGAGGCTGAGGCAGGGAGAATTGCTTGCACCAGGGA
GGTAGAGGTTGCAGTGAGCCAAGATCGCACCACTGCACTCCAGCCGGGG
CAACAGAGCAAGACTCCATCTCAAAAAAAAAAAAAAAAAAAAGAAAGAAAAG
AAAATTTGAGAACACAGCTTTATACTCGGGACTACAAAACCATAAACTCCT
GGAGTTTTAACTCCTTTTGAAATTTTCATAGTACAATTAATACTAATGAACAT
TTGTGTAAAGCTTTATAATTTAAAGGCAATTTCTCATATATTCTTTTCTGAAT
CATTTGCAAGGAAGTTCAGAGTCCAGTCTGTAACTAGCATCTACTATATGT
CTGTCTTCACCTTACAGTGTTCTACCATTATTTTTTCTTTATTCCATTTCAAA
ATCTAATTTATTTTACCCCAACTTCTCCCCACCACTTGACGTAGTTTTAGAA
CACACAGGTGTTGCTACATATTTGGAGTCAATGATGGACTCTGGCAAAGTC
AAGGCTCTGTTTTATTTCCACCAAGGTGCACTTTTCCAACAACTATTTAACT
AGTTAAGAACCTCCCTATCTTAGAACTGTATCTACTTTATATTTAAGAAGGT
TTTATGAATTCAACAACGGTATCATGGCCTTGTATCAAGTTGAAAAACAACT
GAAAATAAGAAAATTTCACAGCCTCGAAAGACAACAACAAGTTTCTAGGAT
ATCTCAATGACAAGAGTGATGGATACTTAGGTAGGGAAACGCTAATGCAG
GAAAAACTGGCAACAACACAATTTATATCAATTCTCTTTGTAGGCAGGTGAT
AAAAAATTCAAGGACAAATCTCATTATGTCATTGTGCATCATATATAATCTCT
TATGAGCGAGAATGGGGGGAATTTGTGTTTTTACTTTACACTTCAATTCCTT
ACACGGTATTTCAAACAAACAGTTTTGCTGAGAGGAGCTTTTGTCTCTCCTT
AAGAAAATGTTTATAAAGCTGAAAGGAAATCAAACAGTAATCTTAAAAATGA
AAACAAAACAACCCAACAACCTAGATAACTACAGTGATCAGGGAGCACAGT
TCAACTCCTTGTTATGTTTTAGTCATATGGCCTACTCAAACAGCTAAATAAC
AACACCAGTGGCAGATAAAAATCACCATTTATCTTTCAGCTATTAATCTTTT
GAATGAATAAACTGTGACAAACAAATTAACATTTTTGAACATGAAAGGCAAC
TTCTGCACAATCCTGTATCCAAGCAAACTTTAAATTATCCACTTAATTATTAC
TTAATCTTAAAAAAAATTAGAACCCAGAACTTTTCAATGAAGCATTTGAAAG

TTGAAGTGGAATTTAGGAAAGCCATAAAAATATAAATACTGTTATCACAGCA
CCAGCAAGCCATAATCTTTATACCTATCAGTTCTATTTCTATTAACAGTAAA
AACATTAAGCAAGATATAAGACTACCTGCCCAAGAATTCAGTCTTTTTCAT
TTTTGTTTTTCTCAGTTCTGAGGATGTTAATCGTCAAATTTTCTTTGGACTG
CATTCCTCACTACTTTTTGCACAATGGTCTCACGTTCTCACATTTGTTCTCG
CGAATAAATTGATAAAAGGTGTTAAGTTCTGTGAATGTCTTTTTAATTATGG
GCATAATTGTGCTTGACTGGATAAAAACTTAAGTCCACCCTTATGTTTATAA
TAATTTCTTGAGAACAGCAAACTGCATTTACCATCGTAAAACAACATCTGAC
TTACGGGAGCTGCAGGGAAGTGGTGAGACAGTTCGAACGGCTCCTCAGAA
ATCCAGTGACCCAATTCTAAAGACCATAGCACCTGCAAGTGACACAACAAG
CAGATTTATTATACATTTATTAGCCTTAGCAGGCAATAAACCAAGAATCACT
TTGAAGACACAGCAAAAAGTGATACACTCCGCAGATCTGAAATAGATGTGT
TCTCAGACAACAAAGTCCCTTCAGAATCTTCATGTTGCATAAATGTTATGAA
TATTAATAAAAAGTTGATTGAGAAAAA

[SEQ ID No: 37]

Accordingly, preferably ANGPT2 comprises or consists of a nucleotide sequence substantially as set out in SEQ ID NO: 37, or a fragment or variant thereof.

In one embodiment, RelA NF-KB is provided by gene bank locus ID: HGNC: 9955; Entrez Gene: 5970; Ensembl: ENSG00000173039; OMIM: 164014; and/or UniProtKB: Q04206. The protein sequence may be represented by the GeneBank ID Q04206, which is provided herein as SEQ ID No: 38, as follows:

MDELFPLIFPAEPAQASGPYVEIIEQPKQRGMRFRYKCEGRSAGSIPGERSTD
TTKTHPTIKINGYTGPGTVRISLVTKDPPHRPHPHELVGKDCRDGFYEAELCPD
RCIHSFQNLGIQCVKKRDLEQAISQRIQTNNNPFQEEQRGDYDLNAVRLCFQV
TVRDPSGRPLRLPPVLSHPIFDNRAPNTAELKICRVNRNSGSCLGGDEIFLLCD
KVQKEDIEVYFTGPGWEARGSFSQADVHRQVAIVFRTPPYADPSLQAPVRVS
MQLRRPSDRELSEPMEFQYLPDTDDRHRIEEKRKRTYETFKSIMKKSPFSGPT

DPRPPPRRIAVPSRSSASVPKPAPQPYPFTSSLSTINYDEFPTMVFPSGQISQA
SALAPAPPQVLPQAPAPAPAPAMVSALAQAPAPVPVLAPGPPQAVAPPAPKP
TQAGEGTLSEALLQLQFDDEDLGALLGNSTDPAVFTDLASVDNSEFQQLLNQ
GIPVAPHTTEPMLMEYPEAITRLVTGAQRPPDPAPAPLGAPGLPNGLLSGDED
FSSIADMDFSALLSQISS

[SEQ ID No: 38]

Accordingly, preferably RelA NF-KB comprises or consists of an amino acid sequence substantially as set out in SEQ ID NO: 38, or a fragment or variant thereof.

In one embodiment RelA NF-KB is be encoded by a nucleotide sequence which is provided herein as SEQ ID No: 39, as follows:

CGTCCTCGGCGAGGCGCACTTGGCCCCGACCCCCGGCAGCGGCTGT
GCGTGCAGCCTCTTCGTCCTCCGCGGCGTGCACTTGCTCCCGGCCCC
TGCGCCGGGCGGCGGCGGGGCAGCGCGCAGGCGCGGCCGGATTCCGG
GCAGTGACGCGACGGCGGGCCGCGGCGCATTTCCGCCTCTGGCGAAT
GGCTCGTCTGTAGTGCACGCCGCGGGCCCAGCTGCGACCCCGGCCCCG
CCCCCGGGACCCCGGCCATGGACGAACTGTTCCCCCTCATCTTCCCGGC
AGAGCCAGCCCAGGCCTCTGGCCCCTATGTGGAGATCATTGAGCAGCCCA
AGCAGCGGGGCATGCGCTTCCGCTACAAGTGCGAGGGGCGCTCCGCGG
GCAGCATCCCAGGCGAGAGGAGCACAGATACCACCAAGACCCACCCCAC
CATCAAGATCAATGGCTACACAGGACCAGGGACAGTGCGCATCTCCCTGG
TCACCAAGGACCCTCCTCACCGGCCTCACCCCCACGAGCTTGTAGGAAAG
GACTGCCGGGATGGCTTCTATGAGGCTGAGCTCTGCCCGGACCGCTGCA
TCCACAGTTTCCAGAACCTGGGAATCCAGTGTGTGAAGAAGCGGGACCTG
GAGCAGGCTATCAGTCAGCGCATCCAGACCAACAACAACCCCTTCCAAGA
AGAGCAGCGTGGGGACTACGACCTGAATGCTGTGCGGCTCTGCTTCCAG
GTGACAGTGCGGGACCCATCAGGCAGGCCCCTCCGCCTGCCGCCTGTCC
TTTCTCATCCCATCTTTGACAATCGTGCCCCCAACACTGCCGAGCTCAAGA

TCTGCCGAGTGAACCGAAACTCTGGCAGCTGCCTCGGTGGGATGAGATC
TTCCTACTGTGTGACAAGGTGCAGAAAGAGGACATTGAGGTGTATTTCACG
GGACCAGGCTGGGAGGCCCGAGGCTCCTTTTCGCAAGCTGATGTGCACC
GACAAGTGGCCATTGTGTTCCGGACCCCTCCCTACGCAGACCCCAGCCTG
CAGGCTCCTGTGCGTGTCTCCATGCAGCTGCGGCGGCCTTCCGACCGGG
AGCTCAGTGAGCCCATGGAATTCCAGTACCTGCCAGATACAGACGATCGT
CACCGGATTGAGGAGAAACGTAAAAGGACATATGAGACCTTCAAGAGCAT
CATGAAGAAGAGTCCTTTCAGCGGACCCACCGACCCCCGGCCTCCACCTC
GACGCATTGCTGTGCCTTCCCGCAGCTCAGCTTCTGTCCCCAAGCCAGCA
CCCCAGCCCTATCCCTTTACGTCATCCCTGAGCACCATCAACTATGATGAG
TTTCCCACCATGGTGTTTCCTTCTGGGCAGATCAGCCAGGCCTCGGCCTT
GGCCCCGGCCCCTCCCCAAGTCCTGCCCCAGGCTCCAGCCCCTGCCCCT
GCTCCAGCCATGGTATCAGCTCTGGCCCAGGCCCCAGCCCCTGTCCCAGT
CCTAGCCCCAGGCCCTCCTCAGGCTGTGGCCCCACCTGCCCCCAAGCCC
ACCCAGGCTGGGGAAGGAACGCTGTCAGAGGCCCTGCTGCAGCTGCAGT
TTGATGATGAAGACCTGGGGGCCTTGCTTGGCAACAGCACAGACCCAGCT
GTGTTCACAGACCTGGCATCCGTCGACAACTCCGAGTTTCAGCAGCTGCT
GAACCAGGGCATACCTGTGGCCCCCCACACAACTGAGCCCATGCTGATGG
AGTACCCTGAGGCTATAACTCGCCTAGTGACAGGGGCCCAGAGGCCCCC
CGACCCAGCTCCTGCTCCACTGGGGGCCCCGGGGCTCCCCAATGGCCTC
CTTTCAGGAGATGAAGACTTCTCCTCCATTGCGGACATGGACTTCTCAGCC
CTGCTGAGTCAGATCAGCTCCTAAGGGGGTGACGCCTGCCCTCCCCAGA
GCACTGGGTTGCAGGGGATTGAAGCCCTCCAAAAGCACTTACGGATTCTG
GTGGGGTGTGTTCCAACTGCCCCCAACTTTGTGGATGTCTTCCTTGGAGG
GGGGAGCCATATTTTATTCTTTTATTGTCAGTATCTGTATCTCTCTCTCTTTT
TGGAGGTGCTTAAGCAGAAGCATTAACTTCTCTGGAAAGGGGGGAGCTGG
GGAAACTCAAACTTTTCCCCTGTCCTGATGGTCAGCTCCCTTCTCTGTAGG
GAACTCTGGGGTCCCCCATCCCCATCCTCCAGCTTCTGGTACTCTCCTAG
AGACAGAAGCAGGCTGGAGGTAAGGCCTTTGAGCCCACAAAGCCTTATCA
AGTGTCTTCCATCATGGATTCATTACAGCTTAATCAAAATAACGCCCCAGAT
ACCAGCCCCTGTATGGCACTGGCATTGTCCCTGTGCCTAACACCAGCGTT
TGAGGGGCTGGCCTTCCTGCCCTACAGAGGTCTCTGCCGGCTCTTTCCTT

GCTCAACCATGGCTGAAGGAAACCAGTGCAACAGCACTGGCTCTCTCCAG
GATCCAGAAGGGGTTTGGTCTGGGACTTCCTTGCTCTCCCTCTTCTCAAGT
GCCTTAATAGTAGGGTAAGTTGTTAAGAGTGGGGGAGAGCAGGCTGGCAG
CTCTCCAGTCAGGAGGCATAGTTTTTACTGAACAATCAAAGCACTTGGACT
CTTGCTCTTTCTACTCTGAACTAATAAATCTGTTGCCAAGCTG
[SEQ ID No: 39]

Accordingly, preferably RelA NF-KB comprises or consists of a nucleotide sequence substantially as set out in SEQ ID NO: 39, or a fragment or variant thereof.

In one embodiment, the biomarker is RORA, and a decrease in the expression, amount and/or activity of RORA when compared to a reference is indicative of an individual having a higher risk of suffering from cardiovascular disease.

In one embodiment, the biomarker is GHR, and an increase in the expression, amount and/or activity of GHR when compared to a reference is indicative of an individual having a higher risk of suffering from cardiovascular disease.

Preferably, the sample comprises a biological sample. The sample may be any material that is obtainable from the subject from which protein, RNA and/or DNA is obtainable. Furthermore, the sample may be blood, plasma, serum, spinal fluid, urine, sweat, saliva, tears, breast aspirate, prostate fluid, seminal fluid, vaginal fluid, stool, cervical scraping, cytes, amniotic fluid, intraocular fluid, mucous, moisture in breath, animal tissue, cell lysates, tumour tissue, hair, skin, buccal scrapings, lymph, interstitial fluid, nails, bone marrow, cartilage, prions, bone powder, ear wax, or combinations thereof.

Preferably, however, the sample comprises blood, urine or tissue.

In one embodiment, the sample comprises a blood sample. The blood may be venous or arterial blood. Blood samples may be assayed immediately.

Alternatively, the blood sample may be stored at low temperatures, for example in a fridge or even frozen before the method is conducted. Detection may be carried out on whole blood. Preferably, however, the blood sample comprises blood serum. Preferably, the blood sample comprises blood plasma.

The blood may be further processed before the method is performed. For instance, an anticoagulant, such as citrate (such as sodium citrate), hirudin, heparin, PPACK, or sodium fluoride may be added. Thus, the sample collection container may contain an anticoagulant in order to prevent the blood sample from clotting. Alternatively, the blood sample may be centrifuged or filtered to prepare a plasma or serum fraction, which may be used for analysis. Hence, it is preferred that the method is performed in a blood plasma or a blood serum sample. It is preferred that the expression level, amount and/or activity of the biomarker is measured *in vitro* from a blood serum sample or a plasma sample taken from the individual.

The invention also provides for a kit for determining, diagnosing and/or prognosing CVD risk.

Accordingly, in a second aspect, there is provided a kit for determining, diagnosing and/or prognosing the risk of an individual suffering from cardiovascular disease, the kit comprising:
a. detection means for detecting, in a sample obtained from a test subject, the expression level, amount and/or activity of two or more biomarkers selected from the group consisting of TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and ReIA NF-KB; and
b. a reference value from a healthy control population for expression level, amount and/or activity of two or more a biomarkers selected from the group consisting of TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and ReIA NF-KB,
wherein the kit is used to identify:
i) a decrease in expression, amount and/or activity of TNF-a; GSTA1; NT-proBNP; RORA and/or TNC when compared to the reference; and /or an increase in expression, amount and/or activity of GHR; A2M; IGFBP2; APOB; SEPP1; TFF3; IL6 and/or CHI3L1;when compared to the reference to determine, diagnose and/or prognose that an individual has a higher risk of suffering from cardiovascular disease; and/or
ii) a decrease expression, amount and/or activity of MET; GDF15; CCL22; TNFRSF11; ANGPT2 and/or RelA NF-KB when compared to the reference to determine, diagnose and/or prognose that an individual has a lower risk of suffering from cardiovascular disease.

The cardiovascular disease, the biomarker, detection and the sample may be as defined in the first aspect.

Preferably, a decrease in expression, amount and/or activity of TNF-□, GSTA1, NT-proBNP, RORA and/or TNC, when compared to the reference, is indicative of an individual having a higher risk of suffering from cardiovascular disease or a negative prognosis.

Preferably, an increase in expression, amount and/or activity of GHR, A2M, IGFBP2, APOB, SEPP1, TFF3, IL6 and/or CHI3L1, when compared to the reference, is indicative of an individual having a higher risk of suffering from cardiovascular disease or a negative prognosis.

Preferably, a decrease in expression, amount and/or activity of MET, GDF15, CCL22, TNFRSF11, ANGPT2 and/or RelA NF-KB, when compared to the reference, is indicative of an individual having a lower risk of suffering from cardiovascular disease or a positive prognosis.

The expression levels, amount and/or activities of the biomarkers may be as defined in the first aspect.

The kit may comprise detection means for detecting the expression levels, amount and/or activities of at least 3 biomarkers or at least 4 biomarkers. The kit may comprise detection means for detecting the expression levels, amount and/or activities of at least 5 biomarkers. The kit may comprise detection means for detecting the expression levels, amount and/or activities of at least 6 biomarkers or at least 7 biomarkers. Alternatively, the kit may comprise detection means for detecting the expression levels, amount and/or activities of at least 8 biomarkers or at least 9 biomarkers. In another embodiment, the kit may comprise detection means for detecting the expression levels, amount and/or activities of at least 10 biomarkers, at least 11 biomarkers, at least 12 biomarkers, at least 13 biomarkers, at least 14 biomarkers or at least 15 biomarkers. In another embodiment, the kit may comprise detection means for detecting the expression levels, amount and/or activities of at least 16 biomarkers, at least 17 biomarkers or at least 18 biomarkers.

Preferably, the kit of the second aspect may be for determining, diagnosing and prognosing the risk of an individual suffering from cardiovascular disease, the kit comprising:
a. detection means for detecting, in a sample obtained from a test subject, the expression level, amount and/or activity of: TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB; and
b. a reference value from a healthy control population for expression level, amount and/or activity of TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB, wherein the kit is used to identify:
   i) a decrease in expression, amount and/or activity of TNF-a, GSTA1, NT-proBNP, RORA and TNC when compared to the reference; and an increase in expression, amount and/or activity of GHR, A2M, IGFBP2, APOB, SEPP1, TFF3, IL6 and CHI3L1; when compared to the reference to determine, diagnose and/or prognose that an individual has a higher risk of suffering from cardiovascular disease; and
   ii) a decrease expression, amount and/or activity of MET, GDF15, CCL22, TNFRSF11, ANGPT2 and ReIA NF-KB when compared to the reference to determine, diagnose and/or prognose that an individual has a lower risk of suffering from cardiovascular disease.

The detection means may detect the expression level, for example the level or concentration, of a biomarker polynucleotide sequence, for example DNA or RNA. The DNA may be genomic DNA. The RNA may be mRNA. Alternatively, the detection means may detect polypeptide concentration and/or activity of the biomarker.

Accordingly, the detection means may include: sequencing methods (e.g., Sanger, Next Generation Sequencing, RNA-SEQ), hybridization- based methods, including those employed in biochip arrays, mass spectrometry (e.g., laser desorption/ionization mass spectrometry), fluorescence (e.g., sandwich immunoassay), surface plasmon resonance, ellipsometry and atomic force microscopy. Expression levels of markers (e.g., polynucleotides, polypeptides, or other analytes) may be compared by procedures well known in the art, such as RT-PCR, Northern blotting, Western blotting, flow cytometry, immunocytochemistry, binding to magnetic and/or antibody-coated beads, *in situ* hybridization, fluorescence in situ hybridization (FISH), flow chamber adhesion assay, ELISA, microarray analysis, or colorimetric assays. Methods may further include one or more of electrospray ionization mass spectrometry (ESI-MS), ESI-MS/MS, ESI-MS/(MS)n, matrix-assisted laser desorption ionization time-of-flight mass spectrometry (MALDI-TOF- MS), surface-enhanced laser desorption/ionization time-of-flight mass spectrometry (SELDI-TOF-MS), desorption/ionization on silicon (DIOS), secondary ion mass spectrometry (SFMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n, quadrupole mass spectrometry, fourier transform mass spectrometry (FTMS), and ion trap mass spectrometry, where n is an integer greater than zero.

Preferably, the kit comprises detection means for detecting RORA present in a sample from a test subject, wherein a decrease in the expression, amount and/or activity of RORA indicative of an individual having a higher risk of suffering from cardiovascular disease.

Preferably, the kit comprises detection means for detecting GHR present in a sample from a test subject, wherein an increase in the expression, amount and/or activity of GHR indicative of an individual having a higher risk of suffering from cardiovascular disease.

Using the methods described herein, the inventors have been able to identify SNPs within RORA and GHR that may be used in diagnosis and prognosis, and in particular gene variants that are associated with CVD risk.

Accordingly, in a third aspect of the invention, there is provided a method of determining, diagnosing and/or prognosing an individual's risk of suffering from cardiovascular disease, the method comprising detecting, in a sample obtained from a individual, a single nucleotide polymorphism (SNP) in the RORA gene, wherein the presence of the SNP is indicative of an individual having an increased risk of suffering from cardiovascular disease.

Preferably, RORA, the sample, detection and the cardiovascular disease is as defined in the first aspect.

The method may be performed *in vivo, in vitro* or *ex vivo.* Preferably, the method is performed *in vitro* or *ex vivo.* Most preferably, the method is performed *in vitro.*

Preferably, the SNP is present in a region of chromosome 15, preferably at nucleic acid position 60542728 of the reference sequence NC_000015.10.

Preferably, the SNP comprises a substitution of Adenine (A) to Guanine (G) or Adenine (A) to Cytosine (C).

Preferably, the SNP comprises a substitution of Adenine (A) to Guanine (G)

Thus, preferably, the SNP may be referred to by the sequence variant GRCh38.p12 chr 15; NC 000015.10:g.60542728A>G.

Preferably, the SNP comprises a substitution of Adenine (A) to Cytosine (C).

Thus, preferably, the SNP may be referred to by the sequence variant GRCh38.p12 chr 15; NC 000015.10:g.60542728A>C.

Preferably, the SNP may be Reference SNP cluster ID: rs73420079.

Thus, in one embodiment, SNP is present in the sequence represented by Reference SNP cluster ID rs73420079, referred to herein as SEQ ID No: 3, as follows:

AGGCGCACCT CACACGGCAC ACAGGCACAT CTCACACATG
GCACACATGC ACACCTCACA CAGATGGCAC ACATGCACAC
CTCACACACA CGGCACGCAT GCACACCTCA CACACACGGC
ACGCATGCAC ACCTCACACA CGGCACACAT GCACACCTCA
CACACGACAC ACGGGCACAC CTCACACACA TGGCACACGG
GCACACCTCC CACACACGGC ACACGGGCAC ACCTCCCACA
CACGGCACAC
V GGCACACCTC AAACGACACA CGGCACACC TCACACACAA
GTCTATTCAG CTGCAAGTCC TGCCTCCACT TGCTGAGAAC

CTGCATGACT GGGCACCAAG GATACGGCAC ACACACGCAC
CCACCCCACA TACATACAGT CCACACACAC ACAACACATA
TACACCACAC GCACCACAGA  TGCACACCAC ACATGCCACA
CACACATACA CTGCACACGC ACCCTACACA CACCCCCCAC
ATGCTTACAC
[SEQ ID No: 3]

Where "V" represent the SNP position. Accordingly, in one embodiment, SNP may comprise or consist of the sequence as substantially set out in SEQ ID No: 3, or a fragment or variant thereof.

Preferably, the SNP comprises a substitution of nucleic acid position X in SEQ D No: 3.

Thus, preferably RORA comprises a single nucleotide polymorphism (SNP), the presence of which is associated with an individual having an increased risk of suffering from CVD.

Preferably, the method of detecting the presence of the SNP comprises a probe that is capable of hybridizing to the biomarker sequence. Preferably, the probe is capable of hybridizing to SEQ ID No 3 such that the SNP is detected.

In a fourth aspect of the invention, there is provided a method of determining, diagnosing and/or prognosing a individual's risk of suffering from cardiovascular disease, the method comprising detecting, in a sample obtained from an individual, a single nucleotide polymorphism (SNP) in the GHR gene, wherein the presence of the SNP is indicative of an individual having an increased risk of suffering from cardiovascular disease.

Preferably, GHR, the sample, detection and the cardiovascular disease is as defined in the first aspect.

Preferably, detecting the SNP in a subject is indicative of an increased risk of suffering from cardiovascular disease.

The method may be performed *in vivo, in vitro* or *ex vivo.* Preferably, the method is performed *in vitro* or *ex vivo.* Most preferably, the method is performed *in vitro.*

Preferably, the SNP is present in a region of chromosome 5, preferably at nucleic acid position 42546623 of the reference sequence NC_000005.10.

Preferably, the SNP comprises a substitution of Guanine (G) to Adenine (A).

Preferably, the SNP may be Reference SNP cluster ID rs4314405.

Thus, in one embodiment, SNP is present in the sequence represented by Reference SNP cluster ID: rs73420079, referred to herein as SEQ ID No: 40, as follows:

AGGCGCACCT CACACGGCAC ACAGGCACAT CTCACACATG
GCACACATGC ACACCTCACA CAGATGGCAC ACATGCACAC
CTCACACACA CGGCACGCAT GCACACCTCA CACACACGGC
ACGCATGCAC ACCTCACACA CGGCACACAT GCACACCTCA
CACACGACAC ACGGGCACAC CTCACACACA TGGCACACGG
GCACACCTCC CACACACGGC ACACGGGCAC ACCTCCCACA
CACGGCACAC

V GGCACACCTC AAACGACACA CGGCACACC TCACACACAA
GTCTATTCAG CTGCAAGTCC TGCCTCCACT TGCTGAGAAC
CTGCATGACT GGGCACCAAG GATACGGCAC ACACACGCAC
CCACCCCACA TACATACAGT CCACACACAC ACAACACATA
TACACCACAC GCACCACAGA TGCACACCAC ACATGCCACA

CACACATACA CTGCACACGC ACCCTACACA CACCCCCCAC
ATGCTTACAC
[SEQ ID No: 40]

Where "V" represent the SNP position. Accordingly, in one embodiment, SNP may comprise or consist of the sequence as substantially set out in SEQ ID No: 40, or a fragment or variant thereof.

Preferably, the SNP comprises a substitution of nucleic acid position X in SEQ ID No: 3.

Thus, preferably GHR comprises a single nucleotide polymorphism (SNP), the presence of which is associated with an individual having an increased risk of suffering from CVD. →

In a fifth aspect, there is provided GHR and/or RORA, for use in diagnosis or prognosis.

Preferably, RORA and GHR may comprise a SNP as defined in the third and fourth aspects. The cardiovascular disease may be as defined in the first aspect.

In a sixth aspect, there is provided GHR and/or RORA, for use in diagnosing or prognosing an individual's risk of suffering from cardiovascular disease.

Preferably, RORA and GHR may comprise a SNP as defined in the third and fourth aspects. The cardiovascular disease may be as defined in the first aspect.

Preferably, the method of detecting the presence of the SNP comprises a probe that is capable of hybridizing to the biomarker sequence. Preferably, the probe is capable of hybridizing to SEQ ID No 40 such that the SNP is detected.

In a seventh aspect, there is provided a kit for determining, diagnosing and/or prognosing an individual's risk of suffering from cardiovascular disease, the kit comprising a detection means for detecting, in a sample obtained from a test subject, a single nucleotide polymorphism (SNP) in the RORA gene and/or GHR gene, wherein the presence of the SNP is used to determine, diagnose and/or prognose that an individual has a higher risk of suffering from cardiovascular disease.

The RORA gene, the GHR gene, the sample, detection and the cardiovascular disease may be as defined in the first aspect.

The detection means may be as defined in the second aspect. The single nucleotide polymorphism (SNP) in the RORA gene and/or GHR gene may be as defined in the third and fourth aspects.

In an eighth aspect, there is provided a method of treating an individual having a higher risk of suffering from cardiovascular disease, the method comprising:-
(a) analysing, in a sample obtained from the subject, the expression level, amount and/or activity of two or more biomarkers selected from the group consisting of: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB;
(b) comparing the expression level, amount and/or activity of the biomarker with a reference from a healthy control population, where a decrease in expression, amount and/or activity of TNF-□, GSTA1, NT-proBNP, RORA and/or TNC when compared to the reference and an increase in expression, amount and/or activity of GHR, A2M, IGFBP2, APOB, SEPP1, TFF3, IL6 and/or CHI3L1 when compared to the reference is suggests that the individual has a higher risk of suffering from cardiovascular disease; and
(c) administering, or having administered, to the individual, a therapeutic agent that prevents, or reduces the likelihood of, the individual suffering from cardiovascular disease.

Preferably, the biomarkers, detection of the biomarkers, the cardiovascular disease, the expression levels, amount and/or activities of the biomarkers and the sample are as defined in the first aspect.

Preferably, the method of treatment comprises analysing and comparing the expression levels, amount and/or activities of 3, 4, 5, 6, 7, 8, 9, 10, 11, 12, 13, 14, 15, 16, 17, 18 or more of the biomarkers: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and ReIA NF-KB.

The method of treatment may comprise analysing and comparing the expression levels, amount and/or activities of at least 6 biomarkers or at least 7 biomarkers. Alternatively, the method of treatment may comprise analysing and comparing the expression levels, amount and/or activities of at least 8 biomarkers or at least 9 biomarkers. In another embodiment, the method of treatment may comprise analysing and comparing the expression levels, amount and/or activities of at least 10 biomarkers, at least 11 biomarkers, at least 12 biomarkers, at least 13 biomarkers, at least 14 biomarkers or at least 15 biomarkers. In another embodiment, the method of treatment may comprise analysing and comparing the expression levels, amount and/or activities of at least 16 biomarkers, at least 17 biomarkers or at least 18 biomarkers.

Preferably, the method of treatment comprises analysing and comparing the expression levels, amount and/or activities of the biomarkers: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB

A clinician would be able to make a decision as to the preferred course of treatment required, for example, the type and dosage of the therapeutic agent according to the eighth and ninth aspects to be administered.

Suitable therapeutic agents may include: statins, including the statins selected from the group consisting of: atorvastatin; simvastatin; rosuvastatin; and pravastatin, beta blockers, blood thinning agents including the blood thinning agents selected from the group consisting of: low-dose aspirin; clopidogrel; rivaroxaban; ticagrelor and prasugrel, nitrates, angiotensin-converting enzyme (ACE) inhibitors, angiotensin II receptor antagonists, calcium channel blockers, including the calcium channel blockers selected from the group consisting of: amlodipine; verapamil and diltiazem and/or diuretics.

Treatment may include enacting lifestyle changes.

In an ninth aspect, there is also provided a method of treating an individual having a higher risk of suffering from cardiovascular disease, the method comprising:-
(a) detecting, in a sample obtained from the subject, a single nucleotide polymorphism (SNP) in the RORA gene and/or the GHR gene, wherein the presence of the SNP suggests that the individual has a higher risk of suffering from cardiovascular disease; and
(b) administering, or having administered, to the individual, a therapeutic agent that prevents, or reduces the likelihood of, the individual suffering from cardiovascular disease.

Preferably, the biomarkers, detection of the biomarkers, the cardiovascular disease, the expression levels, amount and/or activities of the biomarkers and the sample are as defined in the first aspect.

Preferably, the single nucleotide polymorphism (SNP) RORA and/or GHR is as defined in the third and/or fourth aspect.

A clinician would be able to make a decision as to the preferred course of treatment required, for example, the type and dosage of the therapeutic agent according to the eighth and ninth aspects to be administered.

Suitable therapeutic agents may include: statins, including the statins selected from the group consisting of: atorvastatin; simvastatin; rosuvastatin; and pravastatin, beta blockers, blood thinning agents including the blood thinning agents selected from the group consisting of: low-dose aspirin; clopidogrel; rivaroxaban; ticagrelor and prasugrel, nitrates, angiotensin-converting enzyme (ACE) inhibitors, angiotensin II receptor antagonists, calcium channel blockers, including the calcium channel blockers selected from the group consisting of: amlodipine; verapamil and diltiazem and/or diuretics.

Treatment may include enacting lifestyle changes.

It will be appreciated that the invention extends to any nucleic acid or peptide or variant, derivative or analogue thereof, which comprises substantially the amino acid or nucleic acid sequences of any of the sequences referred to herein, including variants or fragments thereof. The terms "substantially the amino acid/nucleotide/peptide sequence", "variant" and "fragment", can be a sequence that has at least 40% sequence identity with the amino acid/nucleotide/peptide sequences of any one of the sequences referred to herein, for example 40% identity with the sequence identified as SEQ ID Nos: 1 to 40 and so on.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (v) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22, 4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps and either including or excluding overhangs. Preferably, overhangs are included in the calculation. Hence, a most preferred method for calculating percentage identity between two sequences comprises (i) preparing a sequence alignment using the ClustalW program using a suitable set of parameters, for example, as set out above; and (ii) inserting the values of N and T into the following formula:- Sequence Identity = (N/T)*100.

Alternative methods for identifying similar sequences will be known to those skilled in the art. For example, a substantially similar nucleotide sequence will be encoded by a sequence which hybridizes to DNA sequences or their complements under stringent conditions. By stringent conditions, the inventors mean the nucleotide hybridises to filter-bound DNA or RNA in 3x sodium chloride/sodium citrate (SSC) at approximately 45ºC followed by at least one wash in 0.2× SSC/0.1% SDS at approximately 20-65ºC. Alternatively, a substantially similar polypeptide may differ by at least 1, but less than 5, 10, 20, 50 or 100 amino acids from the sequences shown in, for example, SEQ ID Nos: 1 to 40 and so on.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent (synonymous) change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-

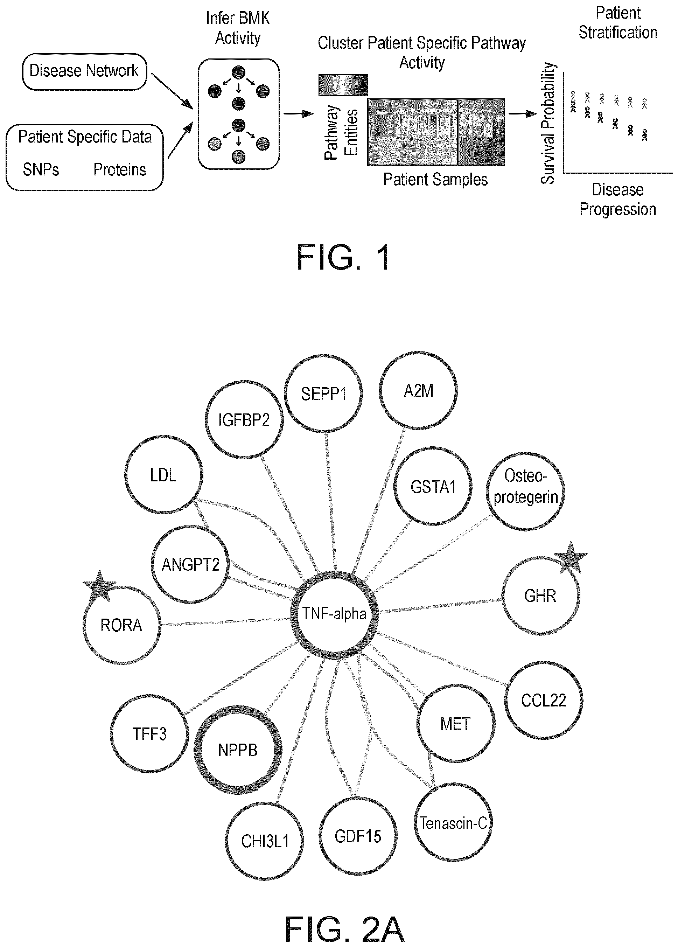

**Figure 1** shows after the disease network identification (upper left panel), single level patient data is integrated based on Bayesian probabilistic graphical models to inferred patient specific pathway activity for each of the BMKs entered in the model. Calculated activities per patient and molecule are them clustered by hierarchical clustering. Survival analysis is done in an independent step to define the relevance of the identified clusters for disease progression (patient stratification). BMKs = biomarkers; TF = transcription factor.

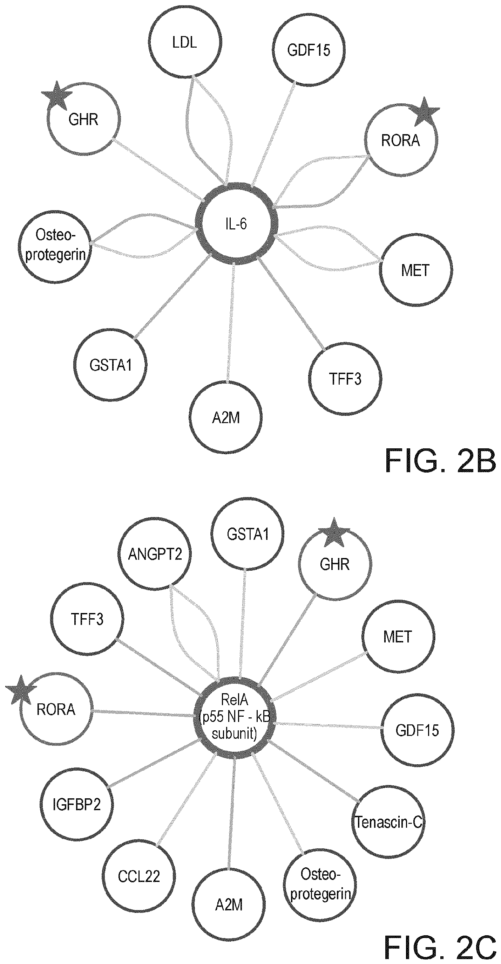

**Figure 2 a-c** shows the networks of molecular entities that were prioritized in the overconnectivity analyses. To infer patient specific activity (Workflow Figure 1), the inventors used patient data from: i) 13 proteins (nodes highlighted in blue), and ii) genotypes of 2 genes (nodes with stars). The activity of 4 nodes (red circles), was inferred by Bayesian statistics based on molecular interactions (connecting edges, red = inhibition, or green = activation, if bidirectional, right edge = from central node to neighbouring molecule, and left edge = from neighbouring molecule to central node) known from the literature (Supplementary Table S5).

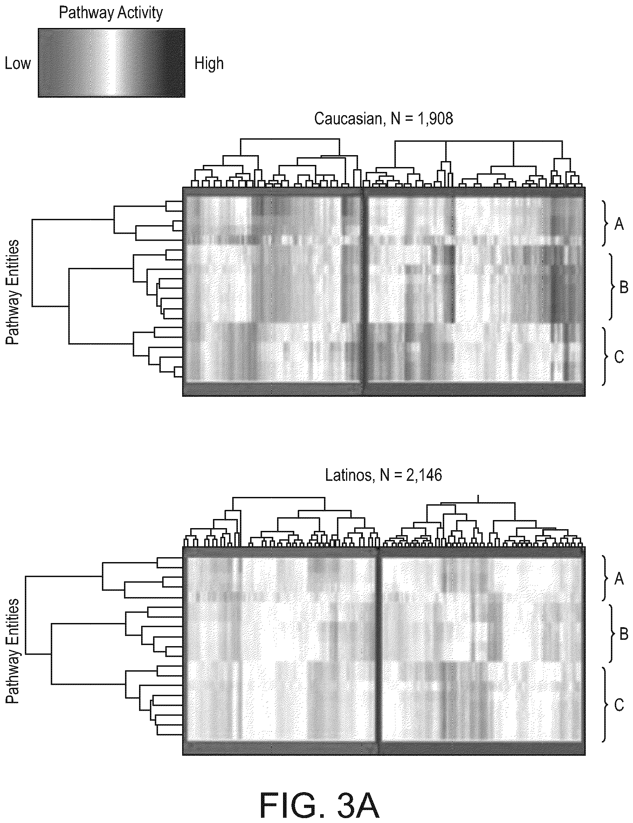

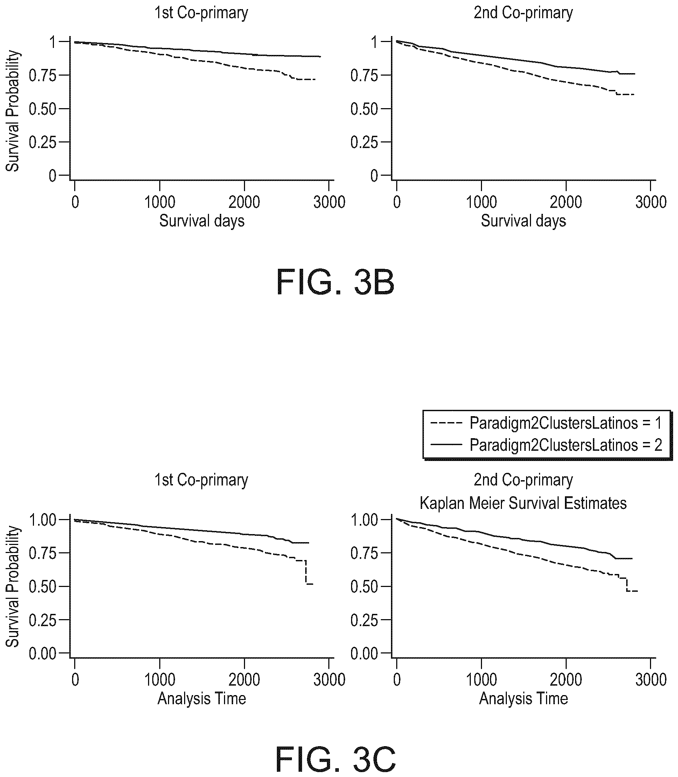

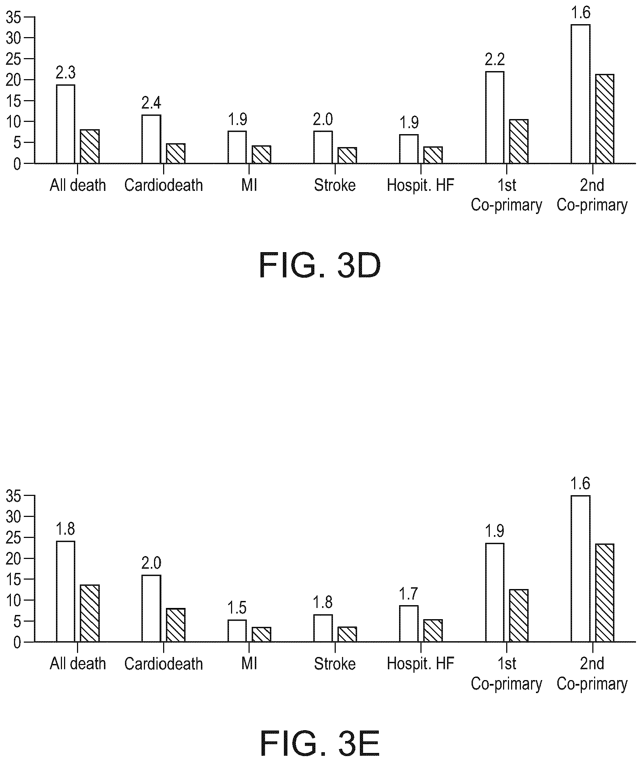

**Figure 3** shows that the molecular signatures of population sub-types relate to CVD progression. a, Two main patient clusters (highlighted in red and blue frames) were identified based on molecular signatures in Caucasians (left) and Latinos (right). b-c,
Kaplan-Meier analyses comparing survival probabilities for the identified clusters (a, red and blue) related to 1st and 2^{nd} co-primary expanded CV composites in Caucasians (b) and Latinos (c). d,e, Proportion of CVO events or all death per cluster (blue = higher and blue = lower CVO risk clusters) with the respective calculated relative risk of event for patients in the blue versus red highlighted cluster (a). There were significant differences in survival between clusters (Log-rank P ¡ 0.0000 in b-c) for each of the outcomes adjusted for CVO risk factors (Cox regression models P ¡ 0.0000 in b-c). A, C, B represent the main clusters of pathway entities (Table 1). PA = pathway activity; MI = myocardial infarction; Hospit. HF = hospitalization by heart failure.

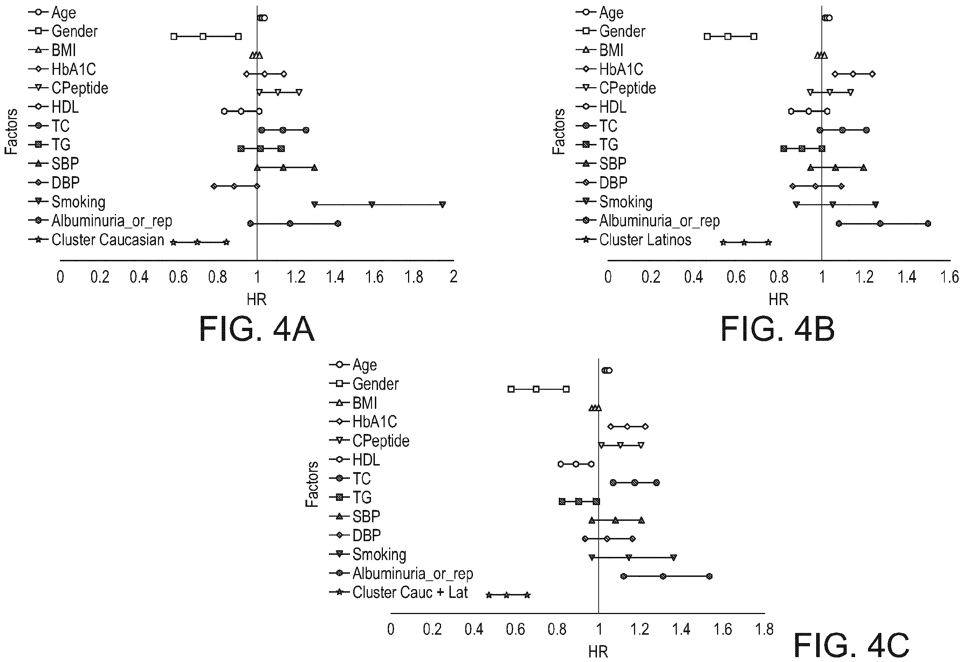

**Figure 4** shows cox-regression results presented as Hazard rations (HR) with confidence intervals. a-b, HR for the 2nd co-primary composite of CVO in Caucasians (a) and Latinos (b) for known CVO risk factors and cluster identity (as defined in Figure 3, panel a-b). c, HR for the 1st co-primary composite of CVO for the same factors as above, considering the entire population (Caucasians plus Latinos). Cox regression models were significant P below 0.0000).

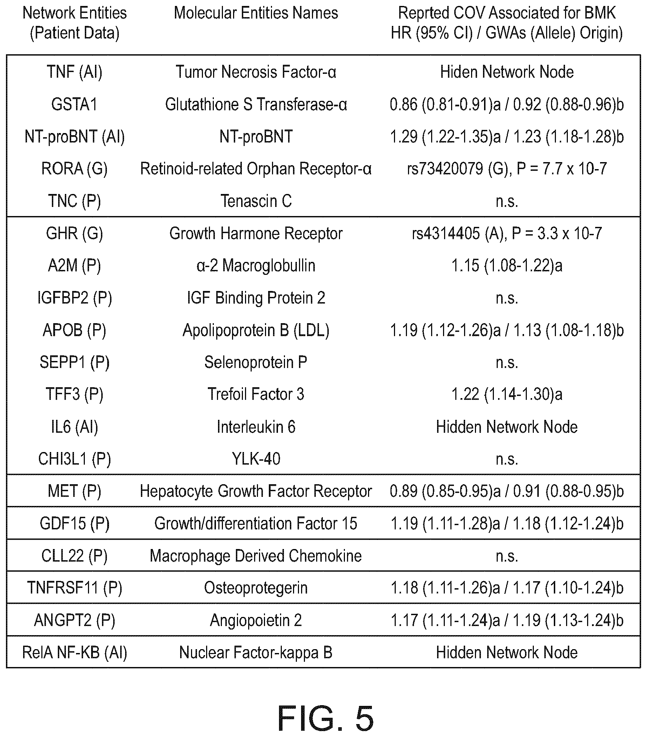

**Figure 5** shows the list of gene names from the identified disease network. Three main clusters (A, B, C in Figure 3) of genes were identified (highlighted in red = lower activity, and shades of blue = higher activity of molecules in patients at higher risk vs.
those at lower risk to CVO events). Individual patient data for these genes (G = genotype and P = protein levels) was used for the patient stratification workflow (figure 1). Al = molecular activity was inferred by Bayesian statistics. RORA and GHR variants were not previously reported to be associated with CVD, with the effect allele being rare in Europeans, but frequent for in Africans (MAF = 0.41) and for the GHR variant in Asians (MAF = 0.13). Results from standard BMK analyses for CVO in the ORIGIN cohort are reported for (a) 1st and (b) second co-primary cardiovascular composites, as previously published (CITE PUBLICATION). 1st co-primary endpoint = composite of CV death, or nonfatal MI or nonfatal stroke, and 2^{nd} co-primary endpoint = composite of 1st co-primary or revascularization procedure or hospitalization for heart failure). n.s. = biomarkers that were not significantly associated with CVO, but associated with death in the ORIGIN CVO trial.

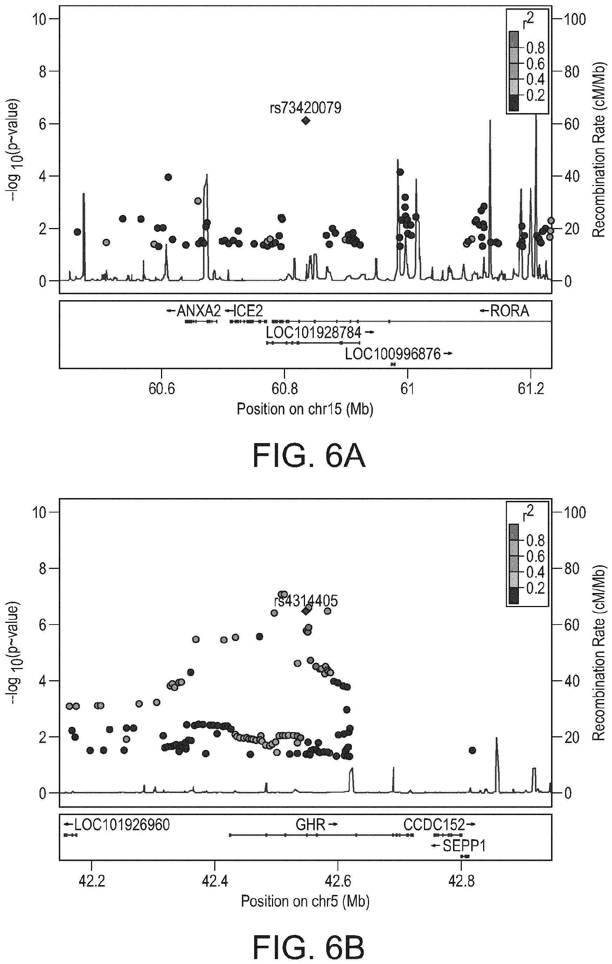

**Figures 6a and 6b** (s2) show locus zoom plots of the GWAs results from the ORIGIN study. SNPs are plotted on the x-axis according to their position on each chromosome against associations with CAD on the y-axis (shown as - log10 P). The loci used for patient stratification (rs73420079, rs4314405; P < 5 × 10-8) are indicated in the locus zoom plots b and c, respectively. MAF for these loci was around 1% in Europeans, except for MIR3169, which had a MAF = 18%.

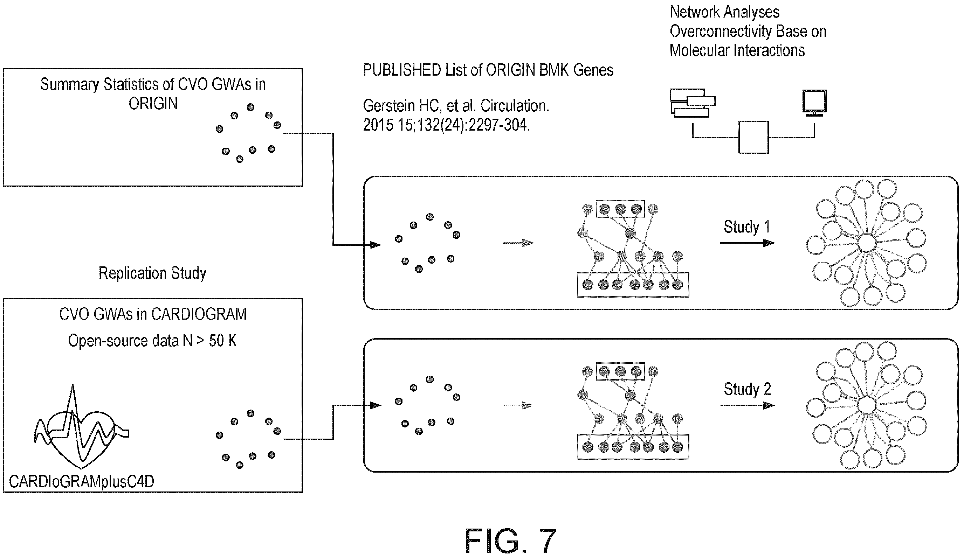

**Figure 7** shows the network analyses design. Summary statistics from ORIGIN CAD GWAs was used to identify most relevant genes association with CVO in this cohort. In a posterio analysis these gene plus a list of biomarkers (BMKs) encoding genes were used to produce networks of overconnected genes. This procedure was replicated using GWAs summary statistics for CAD outcome from the CARDIOGRAM consortium (CARDIoGRAMplusC4D, Nikpey et al 2015) plus the identical list of BMKs associate with CAD outcome.

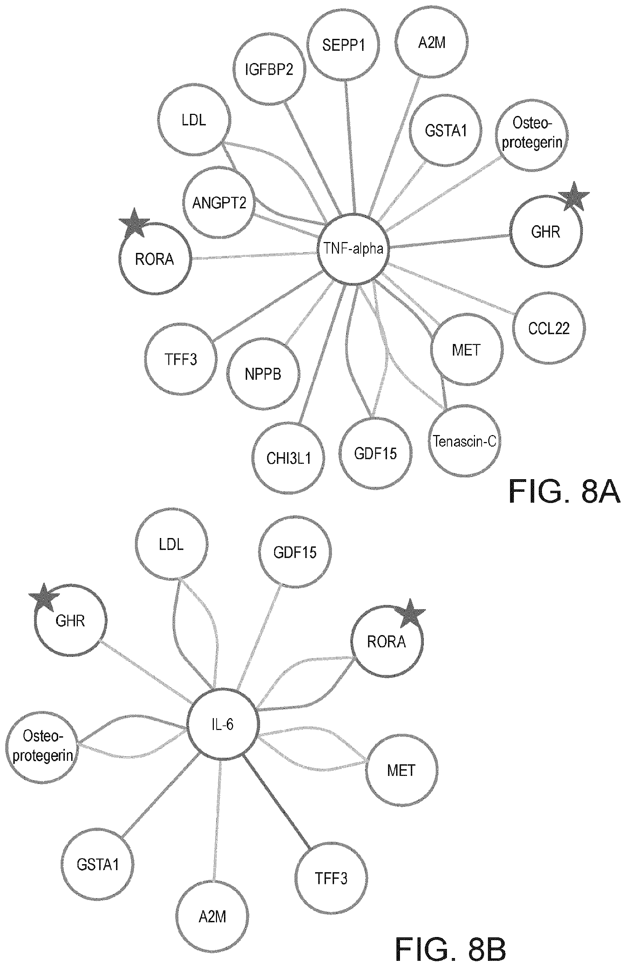

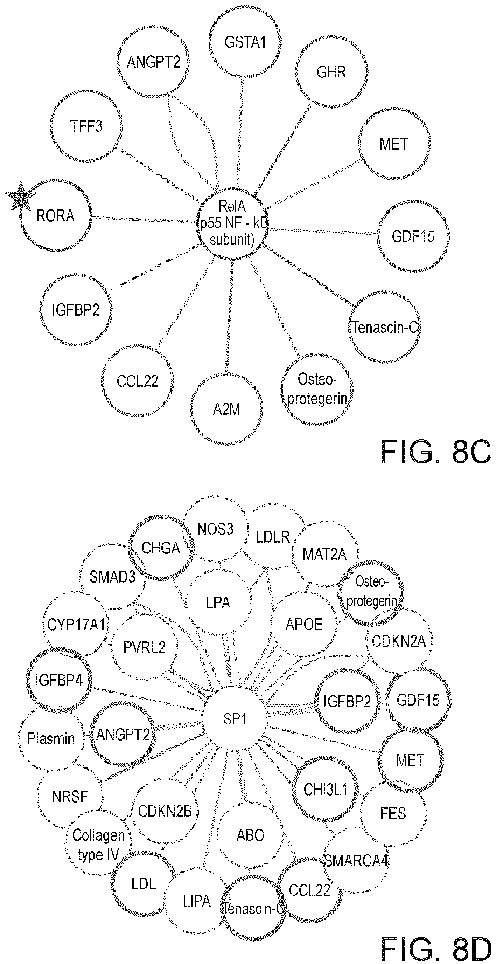

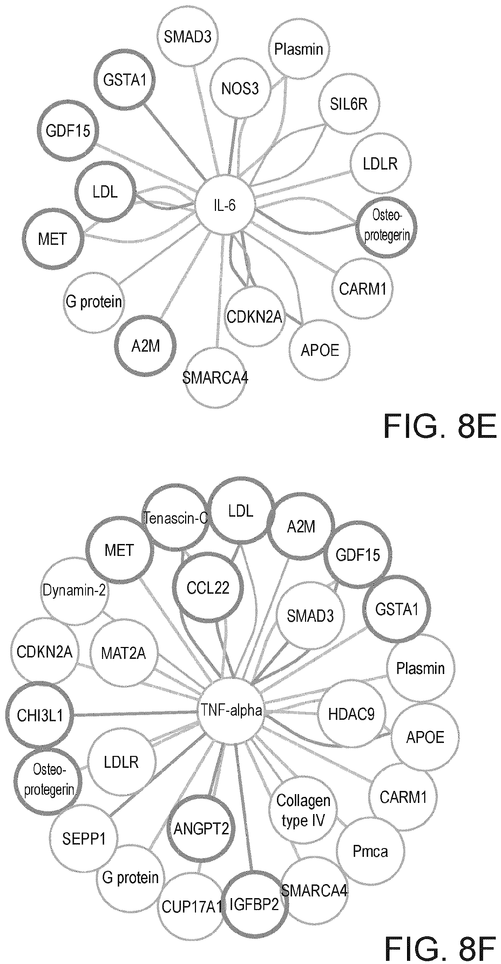

**Figure 8** shows networks of molecular entities that were prioritized in the overconnectivity analyses. a, b, c, First 3 top ranking networks in the discovery network analyses using candidates from GWAs and BMK analyses from the ORIGIN datasets. c, d, e Replication network analyses showing top sub-networks (ranking 1st, 4th and 8th) identified when using the CARDIOGRAM GWAs results plus BMK analyses results from ORIGIN. These most resembled the networks identified in a-c (replicated BMK in blue). Stars = genes associated with CVO in the ORIGIN GWAs.

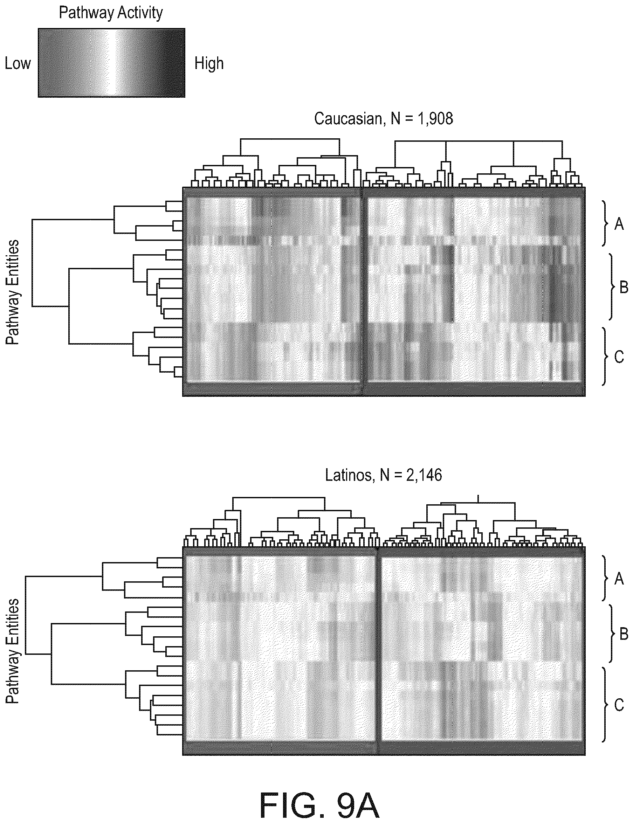

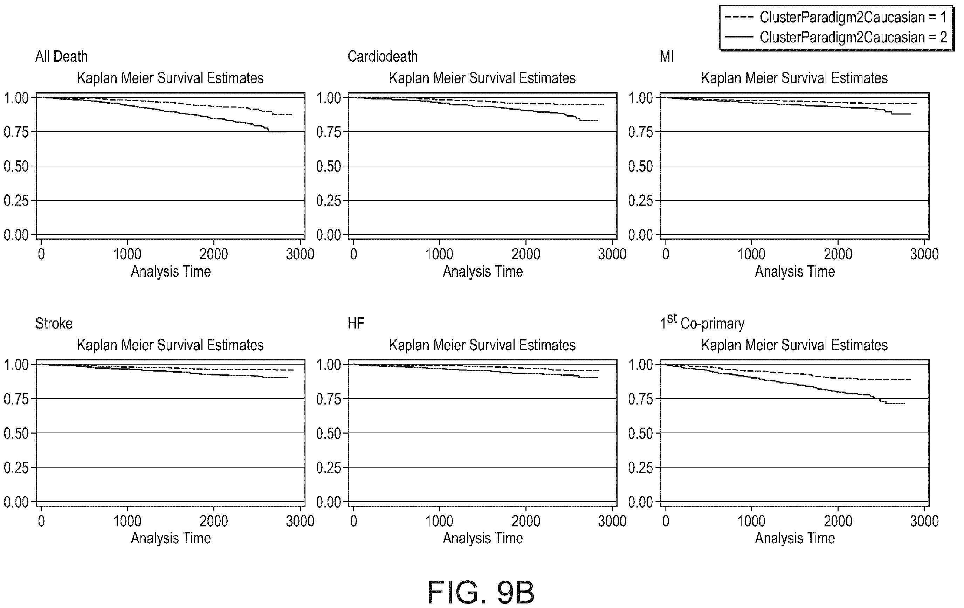

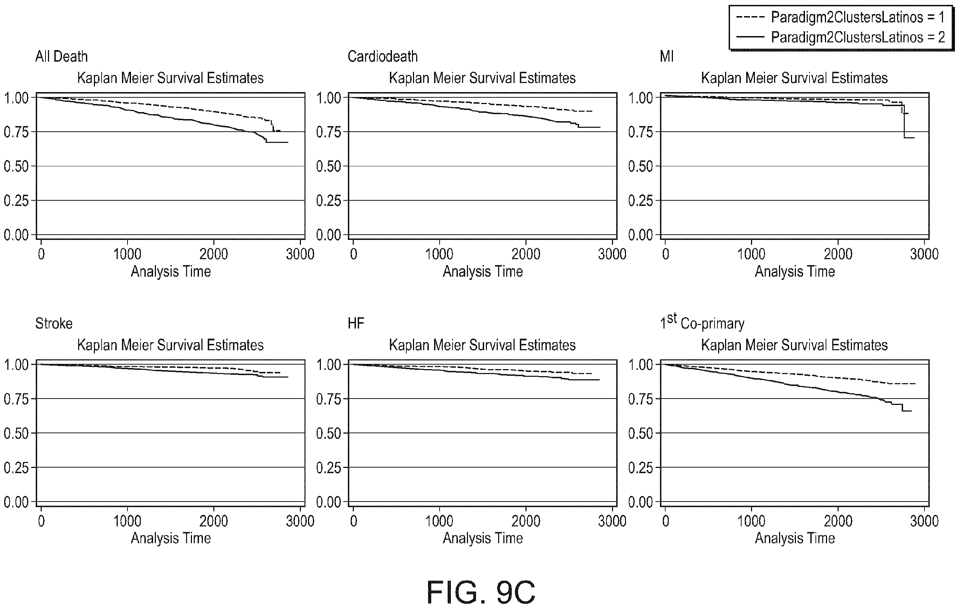

**Figure 9** shows Kaplan-Meier survival estimates by patient clusters for the measured CV outcomes in Caucasian and Latinos. (a), clusters identified and b-c, survival curves (red = cluster highlighted in red, blue = cluster highlighted in blue in the upper panels). (b) Caucasians and Latinos (c). There were significant differences in survival between clusters in all cases (Log-rank P < 0.000 was the less significant difference).

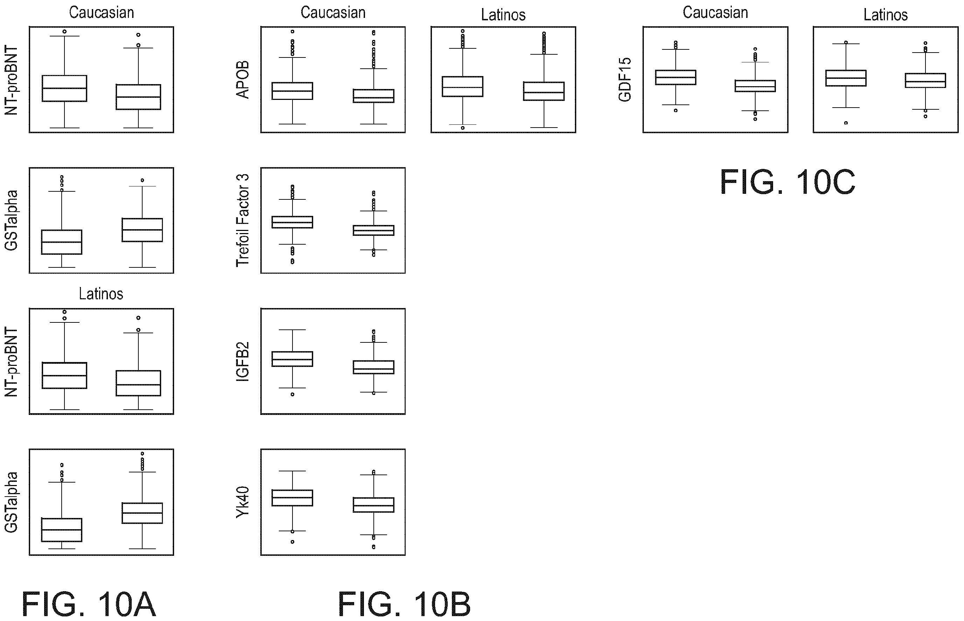

**Figure 10** Box-plots for levels of biomarkers comprising the clusters of genes (A, B, C) identified when clustering patient specific BMK activity (Figure 2). Patients in the cluster at higher risk for CVO (left box-plot in each graphic) had higher BMKs levels, except for GSTalpha, which was lower in the higher risk group.

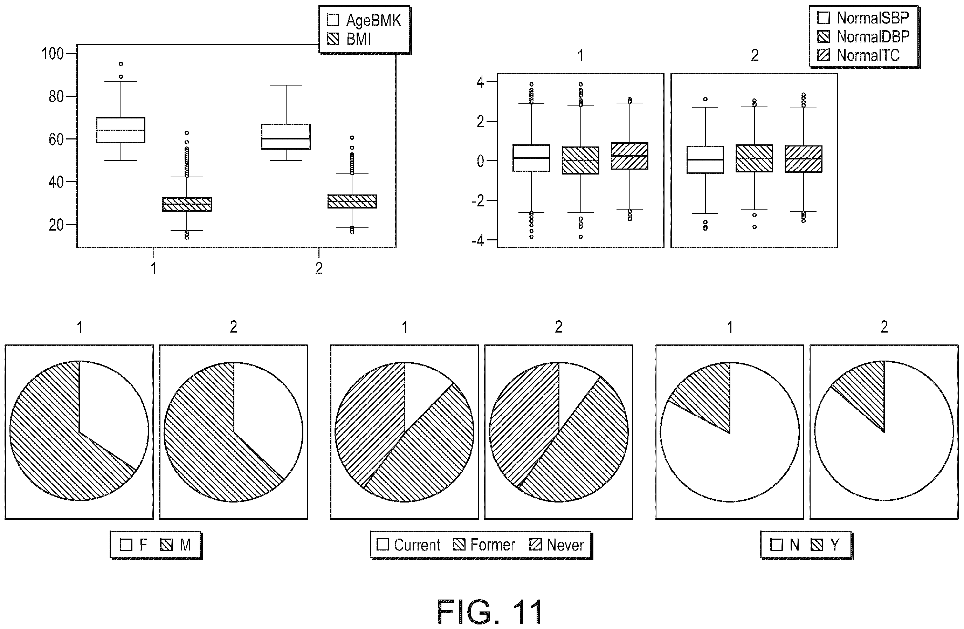

**Figure 11** shows visual inspections of cardiovascular risk factors in the high (1) versus low (2) CVO risk clusters (Caucasian plus Latino sub-populations). Upper panels, Box-plots for age, BMI and levels of BMK routinely measured in the clinic. Patients in the cluster at higher risk for CVO (left box-plots in each graphic) were in average similar to patients in the cluster with lower CVO risk. Lower panel, Categorical CVO risk variables such as sex (left lower panel), smoking (mid lower panel), and albuminuria (right lower panel) or reported albuminuria were slightly different between clusters. More details for these and other risk factors are given in Table 1. Statistical analyses for CVO risk (logistic regression) and survival (cox regression) included these as co-variates to calculate the risk and Hazard ratios per cluster as presented in the MS body. NormalTC = normalized Total Cholesterol; normalSBP / DBP = normalized systolic blood pressure / diastolic blood pressure.

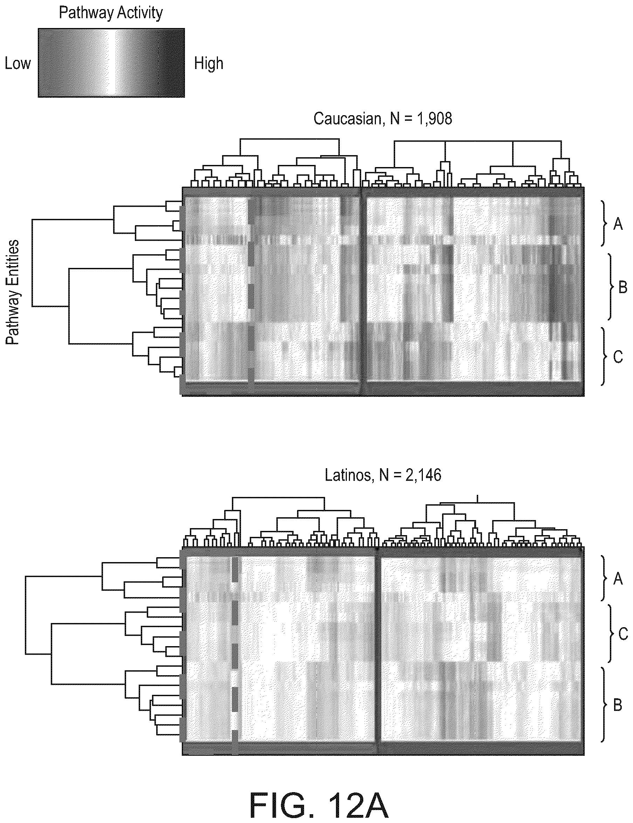

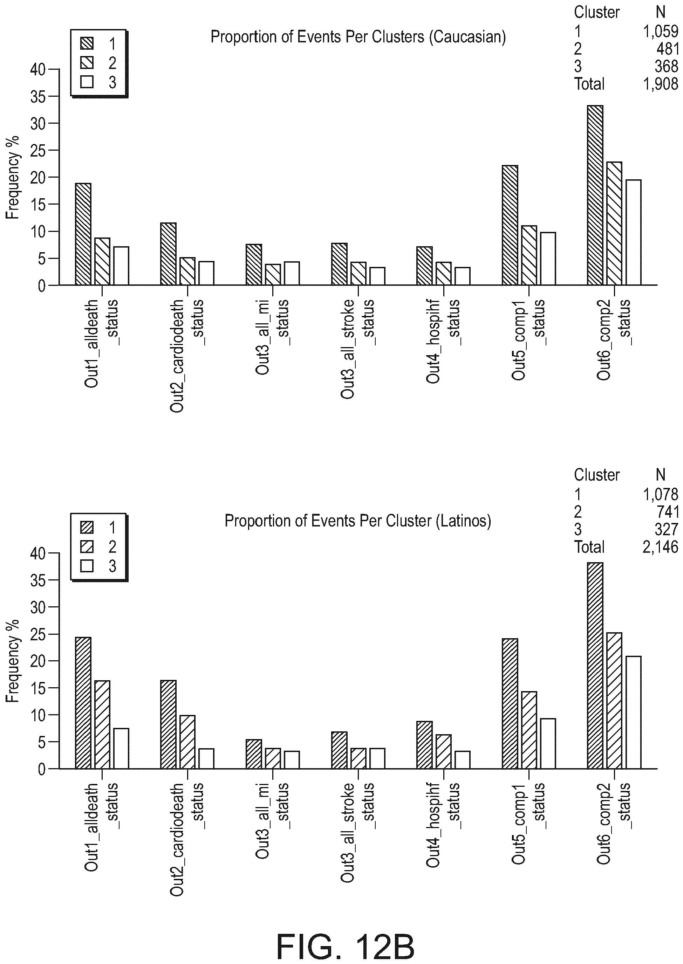

**Figure 12** shows sub-cluster analyses. (a) Sub-clusters within clusters (cluster 2 is now divided in 2 and 3) in both Caucasian and Latinos. (b) Proportion of CVO events per clusters 1 to 3 and the corresponding N of individuals.

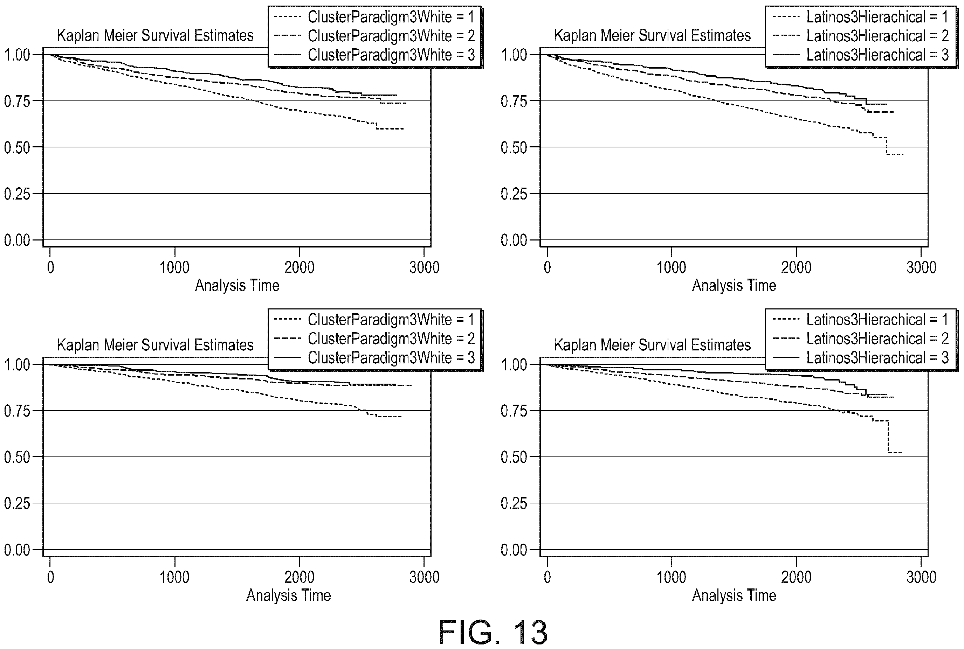

**Figure 13** shows Kaplan-Meier survival estimates for measured outcomes in Caucasian (left panels) and Latinos (right panels) for the 3 identified sub-clusters (as shown in Figure 13). There were significant differences in survival between clusters in all cases (Log-rank P < 0.0000).

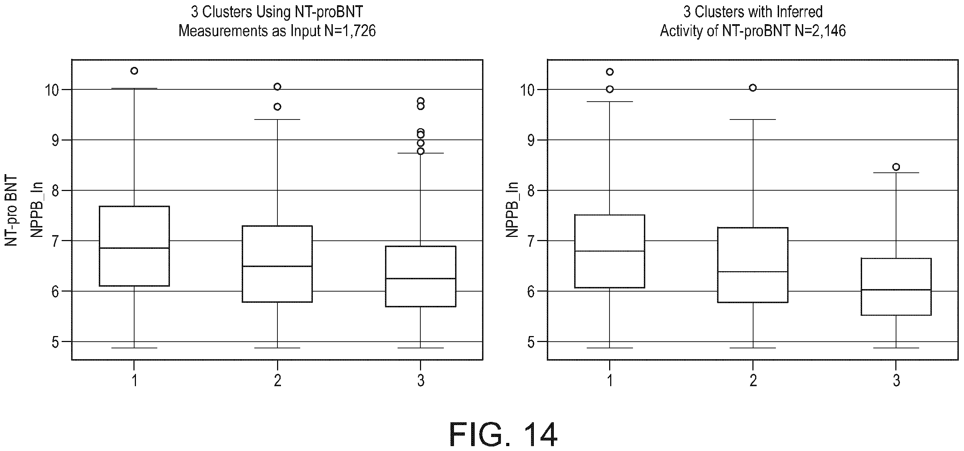

**Figure 14** (S10) shows NTproBNP biomarker levels in 3 clusters of patients obtained by either using NT-proBNT levels (left panel) or without using it (right panel) as input for the network calculations that generated these clusters in Latinos. There were no significant differences NT-proBNT levels when using the first or the later analyses results as classifier of patients (clusters 1 to 3).

### Examples

The inventors set out to identify biomarkers, and combinations of biomarkers, associated with CVD progression, with the aim of developing strategies to enable identification of individuals who are at risk from suffering from a CVD event, and thus enable early intervention to prevent, or reduce the risk of, the individual from suffering from a CVD event. Identification of suitable biomarkers and biomarker networks will also optimize clinical trials plans, drug efficacy, and optimize treatment.

Example 1 - identification of biomarkers and biomarker networks.

### Materials and Methods

### ORIGIN cohort

The participants of the ORIGIN biomarker sub-study (N = 8,401, Supplementary Table S1) were chosen among the randomized patients who were treated by Lantus or placebo. A smaller sub-population of this sample was genotyped (5,078 samples). After quality control (see below), sample size was 4,390 individuals. Only these genotyped subjects entered the analyses described in this protocol for patient stratification (demographic characteristics in Supplementary Table S1).

### Biomarkers quality control and normalization

The quality control for the protein biomarkers measured in the ORIGIN cohort was previously described (Gerstein et al., 2015). Normalization = biomarkers that were not normally distributed were log transformed. Standard biomarker analyses for CVO prediction in this cohort were described elsewhere (Gerstein et al., 2015).

Quantitative BMK measurements were also transformed to categorical variables (-1, 0, +1) based on percentiles of the distributions for each BMK, separately for Caucasian, Latinos and subjects of African origin. The algorithm PARADIGM cannot deal with continuous traits for clustering.

### Genotyping quality control and population stratification analysis

Genotyping of the ORIGIN cohort, N = 5,078 samples, was performed using Illumina HumanCore Exome DNA Analysis Bead Chip (Illumina Omni2.5). Over 540,000 genetic variants were called, including extensive coverage of coding variants, both common and rare. Single nucleotide polymorphisms (SNPs) were excluded with call rate < 0.99, minor allele frequency < 0.01, or deviation from Hardy-Weinberg equilibrium (P<1×10-6). Individuals were excluded if their self-reported sex, ethnicity and relatedness were not in concordance with their genetic information. After quality control, sample size was 4,390 individuals and 284,024 SNPs (note: SNPs excluded because of low allele frequency were accurately genotyped for the most part and will be included in future analyses). Genome-wide genotype imputation was performed using Impute v2.3.0. The inventors used the NCBI build 37 of Phase I integrated variant 1000 Genomes Haplotypes (SHAPEIT2) as the reference panel. Imputed SNPs were excluded with imputation certainty score < 0.3. Final number of Imputed SNPs was 10,501,330.

Principal components were generated based on whole genome genotyping separately for Caucasian and Latinos. These were used as co-variates in genetic analysis for the genome-wide association study (GWAs) from the ORIGIN study.

Due to population ethnic substructure, subpopulations were defined by ethnic groups (Caucasian and Latinos) and analyzed separately. HWE (P > 0.001) for the SNPs entering the analyses was tested as part of the quality control to define if the population subgroups met expected genotypes distributions. Subpopulations were meta-analyzed and association results were visualized in Manhattan and QQ plots (Figure 6).

### Genome-wide association analyses

GWA analyses were conducted separately for Caucasian (N = 1,931) and Latinos (N = 2,216). Genotypes consisting of both directly typed and imputed SNPs (N = 4.9-9 Mio) entered the GWA analyses. To avoid over-inflation of test statistics due to population structure or relatedness, the inventors applied genomic controls for the GWA analysis. Principal components were generated based on whole genome genotyping separately for Caucasian and Latinos. These were used as co-variates in the genome-wide association analyses from the ORIGIN study.

Linear regression (PLINK) for associations with normalization was performed under an additive model, with SNP allele dosage as predictor and with age, gender.

Meta-analysis was performed. Corresponding to Bonferroni adjustment for one million independent tests, the inventors specified a threshold of P for genome-wide significance. The CARDIOGRAM consortium dataset used was the CARDIoGRAMplusC4D 1000 Genomes-based GWAS meta-analysis summary statistics. It comprised GWAS studies of mainly European, South Asian, and East Asian, descents imputed using the 1000 Genomes phase 1 v3 training set with 38 million variants. The study interrogated 9.4 million variants and involved 60,801 CVD cases and 123,504 controls (Nikpey et al., 2015). To assess the number of independent loci associated with CVD, correlated SNPs were grouped using a LD-based result clumping procedure (PLINK, Purcell et al, 2007). This procedure was used for gene mapping of loci (Supplementary Table S2) entering the overconnectivity network analysis. Variants associated with CVD at a p-value below 10-6 with proxies at a p-value below or equal 10-5 in ORIGIN cohort and p-value below 10-7 with proxies at a p-value below equal 10-6 in the CARDIOGRAM cohort were mapped to genes, so that these could be considered in the network analyses. The inventors excluded alleles with a MAF below 1 percent and poor imputation quality (Info below 0.4) from the clumping procedure.

### Workflow

All steps of the workflow (Figure 1) are as follows:

### (1) Candidate molecules selection

A list of entities included in the discovery and replication studies is presented in Table S3.

### (2) Gene mapping

SNPs associated with CAD in ORIGIN and in CARDIOGRAM were mapped to genes using the clustering procedure available in PLINK (Purcell et al, 2007) based on empirical estimates of linkage disequilibrium (LD) between single nucleotide polymorphisms (SNPs). The inventors used 1000 Genomes (phase 1 release v3) as the reference dataset to estimate the LD between variants; the clumping analysis was performed using LD r2 > 0.8 (--clump-r2 0.8) to clump variants to an index SNP within a range of 250kb (--clump-kb 250). To identify clumped genomic regions corresponding to genes The inventors used the --clump-range function with a gene list (hg19).(Supplementary Table S2);

### (3) Network analysis for disease network identification

The inventors used overconnectivity analyses to build a network representative of cardiovascular disease, as described in more detail below.

### (4) Single level patient data curation

To initiate the patient stratification workflow (Figure 1), the inventors generated first the disease network and used the patient specific data for the prioritized molecules (Table 1). Patient specific data quality control is described above.

### (5) Probabilistic graphical model analysis for identification of patient specific pathway activity

The method is explained in the manuscript and below in more detail.

### (6) Clustering

The inventors used an R package as described below.

### (7) Linking the identified patient clusters to CVO

More details about the analyses, outcomes and co-variates are described below.

### (8) Single biomarkers comparisons

These were done by visual inspection of box-plots and median comparisons.

### (9) Characterization of clustered populations.

### Disease network identification

A disease network was identified using the overconnectivity algorithm, as implemented in the R based Computational Biology for Drug discovery (CDDD) package developed by Clarivate Analytics. The specificity of the network for the disease relies on the disease linked molecules chosen to produce the network (Figure 7) and on the underneath libraries of protein-protein interactions in humans. For this purpose, the inventors extracted high trust interaction manually curated systems biology knowledge bases from Ingenuity (IPA from QIAGEN Inc.) and Metabase (Metacore from Clarivate Analytics) to be used as libraries for the CBDD package. To create the CVD disease network, the inventors: i) made use of topological characteristics of human protein interaction networks to identify one-step away direct regulators of the dataset that are statistically overconnected with the objects from the data set (hypergeometric distribution), ii) used as input datasets, names of genes having evidence of association to CVD (see Baysian network analyses below and in Figure 7). As input data for the overconnectivity analyses, the inventors used in the discovery analyses, names of genes corresponding to 16 protein BMKs and, from 8 loci associated with CVO in the Origin cohort GWAs (Table S3). To validate the initially identified network, the inventors re-ran these analyses replacing genetic associated loci from the ORIGIN study, by other 90 genes from an independent large GWAs meta-analysis (CARDIoGRAMplusC4D, cite Nikpey et al 20115) from the CARDIOGRAM consortium (Figure 7, Table S3).

### Bayesian network analyses for data integration

PARADIGM is a data integration approach based on probabilistic graphical models. It renders a pathway or network as a probabilistic graphical model (PGM), learning its parameters from supplied omics data sets. The model allows inference of true activity score for each node in the pathway given the different omics measurements for the nodes. PARADIGM allows prediction on the level of individual patients and is capable of accommodating such data types as gene / protein expression, copy number changes, metabolomics, direct protein activity assays such as kinase activity measurements. PARADIGM combines multiple genome-scale measurements at the sample level to infer the activities of genes, products and abstract process within a pathway or subnetwork. Edges of original network connect hidden variables of different nodes (e.g. activity hidden variable of node A affects protein or DNA hidden variable of node B, depending on mechanism of A-B link).

Each node is assigned a conditional probability distribution when the model is created. The distribution tells how likely it is to observe a node in particular state given states of its parents in the model Three states are allowed for each node (activated, repressed, unchanged). The distributions for hidden variables are defined at the first step. Distributions for observed variables of each molecular level are learned by EM algorithm using the input data. After model is complete, inference can be made about probabilities of observing hidden nodes in a particular state - either without observed data (prior probability) or taking data into account (posterior probability).

The main output is a matrix of integrated pathway activities (IPAs) A where A_ij represents the inferred activity of entity i in patient sample j. The values in A are signed and are non-zero if the patient data makes the activation or inhibition of the hidden node more likely compared to prior. The A is supposed to be used instead of original data sets for purposes such as patient stratification or association analysis to reveal biological entities with activity associated to clinical traits.

The output is a matrix of activity scores for each node in the network and each sample. The activity score represents signed log likelihood ratio (positive when the node is predicted to be active, negative when node is predicted to be repressed). A pathway is converted into a probabilistic graphical model that includes both hidden states for each node and observed states for the nodes which can correspond to the input data sets. There are two possible modes to assess IPA significance. Both involve permutation - calculation of IPA scores on many randomized samples.

For the 'within' permutation, a permuted data sample is created by creating new set of evidence (i.e. states for observed variables at gene expression and gene copy number) by assigning a value of the random node in pathway / subnetwork and random sample to each observed node.

For the 'any' permutation, the procedure is the same, but the random node selection step could choose a node from anywhere in the input data (regardless of whether a particular pathway / subnetwork contains such a node).

For both permutation types, iterations permuted samples are created, and the IPA scores for each permuted sample is calculated. The distribution of scores from permuted data is used as a null distribution to estimate the significance of IPA scores in real data set. SCRIPT: Paradigm (Vaske et al., 2010) in R with CBDD package developed by CLARIVATES.

Clusters were produced separately for the discovery (Caucasian; N = 1908) and replication (Latinos; N = 2146 sub-populations.
1. Network: Interactome (Sanofi network from IPA and Metabase) human high quality.
2. Matrix: Patient specific information form 13 protein biomarkers and genotypes from 2 GWAs genes were used to run paradigm. These molecular entities appeared in the first 3 subnetworks obtained from the network analysis with the overconnectivity algorithm. Proteins coded as -1, 0, 1 (accordingly with the distribution) per patient. A matrix containing the 3 subnetworks obtained with the OVERCONNECTIVITY algorithm was also used as input for paradigm (though it contained molecular entities from which the inventors did not provide BMK measurements as input).
3. Levels: DNA and protein

### List of proteins/genes included in the analysis:

### Phenotypic Matrix (proteins)

1) Alpha 2 macroglobulin (1st co-primary and death)
2) Angiopoietin 2 (1st, 2nd CVO and death)
3) Apolipoprotein B (1st, 2nd CVO and death)
4) YLK-40 (CHI3L1) (death)
5) Glutathione S transferase alpha (1st, 2nd CVO and death)
6) Tenascin c (death)
7) IGF binding protein 2 (death)
8) Hepatocyte growth factor receptor (MET) (1st, 2nd CVO and death)
9) Osteoprotegerin (TNFRSF11) (1st, 2nd CVO and death)
10) Macrophage derived chemokine (CCL22) (death)
11) Selenoprotein P (death)
12) Trefoil factor 3 (1st CVO and death)
13) GDF15 (1st, 2nd CVO and death)

### Genotypes from genes:

1) RORA (rs73420079 CAD effect allele G -AA=-1, AG=0, GG=1),
2) GHR (rs4314405 CAD effect allele A - GG=-1, AG=0, AA=1).

### Biomarkers to be predicted as key members of the input networks:

TNF-alpha, IL6, RelA NF-KB Subunit, NT-proBNP (NPPB)

### Patient Stratification

The inventors initiated the patient stratification analyses based on the disease sub-networks identified in the ORIGIN cohort (Figure 2) and using the respective patient specific protein or genotypes data (workflow Figure 1; Figure 2 and Table 1). Due to population ethnic background. Two main subpopulation with protein biomarker and genotypes available were considered (Caucasian and Latinos), with similar sample sizes (N = 1,908 and N = 2,146; respectively) and sample characteristics for CVO risk factors (Supplementary Table S1). These were thereby considered as discovery and validation samples for i) Bayesian network analyses, ii) hierarchical clustering, and iii) CVO risk and survival analyses. The Bayesian network analyses were conducted using the PARADIGM algorithm (Vaske et al), implemented in R as part of the CDDD package developed by Clarivate Analytics. The data integration approach is based on probabilistic graphical models. It renders a pathway or network as a probabilistic graphical model (PGM), learning its parameters from supplied omics data sets (Figure 2. The model allows inference of true activity score for each node (molecule) in the pathway given the different omics measurements (per patient) for the nodes (Figure 2; Table 1). Each node is assigned a conditional probability distribution when the model is created. The distribution tells how likely it is to observe a node in particular state given states of its parents in the model. Three states are allowed for each node (activated, repressed, unchanged). The distributions for hidden nodes are defined at the first step (e.g., a transcription factor regulating a network of genes, see Figure 1). Distributions for observed variables of each molecular level are learned by EM algorithm using the input data. After model is complete, inference can be made about probabilities of observing hidden nodes in a particular state - either without observed data (prior probability) or taking data into account (posterior probability). The output is a matrix of activity scores for each node in the network and each sample. The activity score represents signed log likelihood ratio (positive when the node is predicted to be active, negative when node is predicted to be repressed). A pathway is converted into a probabilistic graphical model that includes both hidden states for each node and observed states for the nodes which can correspond to the input data sets. This function takes a matrix of activity scores and calculates p-values for each value using permutation approach. Hierarchical clustering of the calculated pathway activity per patient and molecule was done using the R hclust function. The dissimilarities between clusters was computed using squared Euclidean distance.

### Linking the identified patient clusters to CVO

To verify the relevance of the identified patient clusters in both the Caucasian and Latino populations (Figure 3) to the CV outcomes measured in the ORIGIN cohort, the inventors used logistic regression, Cox proportional hazard models, and survival analyses. To confirm consistency of results, these analyses were repeated for sub-clusters within the initially identified clusters (Figures 12 and 13). Logistic regression was used to estimate if known CVO risk factors could alone distinguish patients from the higher versus lower CVO risk clusters identified by molecular signatures. Cox proportional hazard models were used to estimate the association between clusters and time-to-event, as variation in cumulative disease incidence could be accessed in the prospective study. Kaplan-Meier survival estimates were used to visualize the relevance of the identified clusters to disease progression and log-rank test was used to test the equality of survivor functions. CV outcomes used for the analyses were: myocardial infarction (MI), stroke, cardiovascular death and heart failure (HF) with hospitalization, beyond death for all causes (Figures 9 and 12). Two additional MACEs were used, (i) 1st co-primary endpoint (= CV composite): composite of CV death, or nonfatal MI or nonfatal stroke, and (ii) 2nd co-primary endpoint (= expanded CV composite): composite of 1st co-primary or revascularization procedure or hospitalization for heart failure (Figure 3). Co-variates used in the models were: age, sex, BMI (kg/m2), HbA1c (%), c-Peptide, HDL-C (mmol/L), LDL-C (mmol/L), TG (mmol/L), TC (mmol/L), SBP (mm Hg), DBP (mm Hg), smoking status, albuminuria or reported albuminuria. All continuous variants were normalized by inverse normal transformation. For sample characteristics for the higher versus lower CVO risk identified clusters in the Caucasian and Latino see Supplementary Table S1 and Figure 10. Box-plots of the original biomarker levels were produced for visual inspection of differences in BMK levels between clusters (Figure 9). Analyses were done using STATA Version 15.

### Results

### ORIGIN GWAs results

In the GWAs the inventors conducted with the ORIGIN cohort, the inventors identified a few variants associated or borderline associated to CVD (Figure 11). These were mostly rare variants (MAF = 0.01 in EUR), or did not map to a gene region (Supplementary Table S2). These variants were not present in the CARDIOGRAM dataset, and the inventors could not validate these findings using the Cardiovascular Disease Knowledge Portal (http://broadcvdi.org/home/portalHome). The inventors mapped GWAs results to nearby loci (Supplementary Tables S2) to inform the network analysis, identifying biological interactions of these loci to other CVD biomarkers (Supplementary Table S3). Results from the GWA analyses are shown in Table 1 for loci that were prioritized in the network analyses, all other findings are reported in Figure 6 and Supplementary Table S2.

### CVD disease network prioritization

The inventors built a CVD network (Figure 7) based on proteins reported to be associated with CVO or death and the loci associated with CVD in the ORIGIN CVO trial (Supplementary Table S3), with the purpose to identify direct regulators of disease that are statistically overconnected. From the 8 top ranking loci (GHR, RORA, CLINT1, GRM8, LOC101928784, MYH16, SPOCK1, and TMTC2) from the CVD GWAs in the ORIGIN cohort (Supplementary Table S2), only 2 (RORA and GHR; Figure 6 were prioritized in the overconnectivity analyses (Figure 2). To validate the main networks identified (Figure 2), the inventors re-ran these analyses with an independent GWAs dataset from the CARDIOGRAM consortium (Supplementary Table S2 and Figure 7 for study design). Overconnectivity analysis pointed to 14 (12 in the replication study; (Figure 8) out of the 16 protein BMKs (Supplementary Table S3) that were earlier identified to be associated with CVO in the ORIGIN cohort (Table 1). These were part of the top ranking significant networks (Supplementary Table S4) in the discovery and replication analyses (Figures 7 and 8). To note, though 90 other candidate genes were fed as input to the network calculations in the replication study (Supplementary Table S3), only 2 protein BMKs (NPPB and TFF3) and two genetically associated genes (RORA and GHR) were specific to the sub-network identified in the discovery analyses (Figure 8).

### Patient stratification linked to CVO progression

The prioritized sub-networks and their molecular directional interactions (Figure 2; Supplementary Table S5 for interactions with the respective supporting references) were used to inform the subsequent patient stratification analyses, as specified in the
workflow (Figure 1; see Methods). The clustering of the calculated molecular activities was agnostic to CVO: 3 main gene clusters (A, B, C in Figure 3, and panel a) and 2 main patient clusters were identified (Figure 3, panel a) in Caucasians (with 1,059 and 849 patients) and replicated in Latinos (with 1,078 and 1,068 patients). The pathway activity for the higher CVO risk patients was clearly repressed in gene cluster A, and activated in gene cluster B, as for most of the patients in cluster C (Figure 3 and Table 1). The protein BMKs were previously reported to be associated with CVO or death for all causes in the ORIGIN cohort (Gerstein et al., 2015).

A relation of the clusters to 1st and 2nd co-primary composites of CVO was found in a second step through survival analyses (Kaplan-Maier survival estimates and Log rank analyses to test for equality of survival function) and Cox-regression models adjusted for CVO risk factors (Figure 3, panels b-c; and Figure 4, panels a-c). Similarly, patient clusters also associated to myocardial infarction, stroke, cardiovascular death, heart failure with hospitalization, beyond death for all causes (Figure 9 for Kaplan-Maier survival estimates), with the higher risk cluster having in average about 2 times greater chances of event occurrence than the lower risk cluster (Figure 3, panels d-e).

The distribution of CVO risk factors (Supplementary Table 1 and Figure 11) was mostly similar between the higher versus lower CVO risk patients cluster in both Caucasian and Latinos (Supplementary Table 1). In a logistic regression model including age, sex, BMI, HbA1c, c-Peptide, HDL-C, LDL-C, TG, TC, SBP, DBP, smoking status, albuminuria or reported albuminuria; only albuminuria, total cholesterol and age were relevant to separate lower versus higher CVO risk clusters in both Caucasians and Latinos (Supplementary Table 6). Smoking habits were just distinct for clusters identified in Caucasians, while Gender and BMI only contributed to separate clusters in Latinos (Supplementary Table 6). To test how relevant the clusters were to predict CVO after adjusting for these known CVO risk factors, the inventors used Cox-regression models (Figure 3 and in more detail in Figure 4). Hazard ratios (HR) to the 2nd co-primary composites of CVO were largest for gender (women had a reduced HR in relation to man) and cluster (lower CVO risk cluster was protective) in both the Caucasian and Latino group (Figure 4, a-b). Smoking had a large effect in Caucasians, but a modest one in Latinos. Consistently across populations, HDL, TC, SBP and albuminuria were also relevant, but contributed to a lesser extent than Gender and Cluster. Above 80 percent of the population was diabetic (Supplementary Table 1); HbA1C was more relevant for the HR in Latinos, while was C-Peptide in Caucasians (Figure 4, a-b). The inventors repeated this analysis for all outcomes (Figure 3, d-e) for the combined Caucasian and Latino populations, as presented in Figure 4 (panel c) for the 1st co-primary composites of CVO - which results were similar to analyses with the 2nd co-primary composites of CVO. To test if the clustering was not randomly associated with CVO survival, the inventors repeated the analyses shown in Figure 3 for sub-clusters of the initially identified clusters, with consistent results (Supplementary Figures 12 and 13). Average levels of protein BMKs in the higher versus lower CVO risk cluster (Figure 10) were as expected, corresponding to the direction of associations of these proteins to CVO in the ORIGIN cohort (Table 1). Also, measured levels of protein BMKs in the higher versus lower CVO risk cluster mostly corresponded to higher versus lower calculated activity for the respective molecules (Figure 3, panel a; Table 1), except for NT-proBNP. Thereby, calculated activities does not necessary reflects levels of BMKs, as activity will be inferred (e.g., how likely the protein x activity is de-activated?) based on known molecular interactions. Although NT-proBNP levels were higher in the higher CVO risk patient cluster compared to the lower CVO risk patients (Figure 10), the inferred activity for this gene in the network was lower in the higher CVO risk patient group, than in the lower risk patient group (Table 1). When recalculating the network activities using NT-proBNP levels as input for a sub-set of patients who had its levels measured, levels of the protein per cluster were equivalent to its levels in similar clusters when calculating the network activity without using NTproBNP levels (Figure 14). Similarly, in the absence of TNFalpha measurements for the ORIGIN trial, the network activity was calculated taking into consideration only the neighboring molecules, which indicated that TNFalpha was less activity in higher CVO risk patients, although levels of the actual protein could have been high. The molecular activities of IL6 and RelA NF-KB, also upstream regulators of the networks, were estimated to be higher in the higher CVO risk group (Figure 3, Table 1). These networks were less informative than TNFalpha, which contained already all interacting molecules (Figure 2), but as IL6 and RelA NF-KB were central to those their activities could be calculated.

### Discussion

Here, the inventors developed a workflow that goes from the identification of a CVD network to the proof of concept that the molecular interactions identified can be used to stratify patients with regard to disease progression. The inventor's computational biology approach has identified the group of biomarkers associated with CVD outcomes and has associated, for the first time, SNPs associated with CVD risk. The inventor's work has identified molecular interactions and shows interdependencies of molecular activities that associate with different stages of CVD progression.

These results may enable the identification of individuals who are at risk from suffering from a CVD event, and thus enables early intervention to prevent, or reduce the risk of, the individual from suffering from a CVD event. In particular, detection of each biomarker in isolation enables the identification of individuals who are at risk from suffering from a CVD event, and detection of multiple biomarkers, i.e. the biomarker signature provides a particularly effective means of enabling early intervention to prevent, or reduce the risk of, the individual from suffering from a CVD event.

This is the first evidence for the reported GHR and RORA variants to be associated with CVD, and Example 2 sets out the method of determining a subject's risk of cardiovascular disease based on detections of SNPs in GHR and RORA genes. Though the genetic evidence of association with CVD is not strong, RORA is known to regulate a number of genes involved in lipid metabolism such as apolipoproteins Al, APOA5, CIII, CYP71 and PPARgamma, possibly working as a receptor for cholesterol or one of its derivatives cite Uniprot). Additionally, overexpression of RORA isoforms suppresses TNFalpha induced expression of adhesion molecules in human umbilical vein endothelial cells, regulating inflammatory response (Migita et al., 2004). The inventor's network analyses shows that RORA and GHR interact with main regulatory hubs of the identified CVD network of BMKs. Consistent with that, their genotypes contributed to calculations of the network activity and to the clustering of patients, which were in turn related to CVD progression.

RORA and GHR connected to the main networks through upstream regulators (TNFalpha and IL6) common to the prioritized molecules. Though, TNFalpha and IL6 were not part of the input information to create the network, the inventors could uncover these as hidden main regulators of the input molecules. Links of TNFalpha and IL6 to CVD are known (Lopez-Candales et al., 2017) - but, the network allows the visualization of the directions of the molecular interactions (Figure 2). These were used to inform the Bayesian statistics method to calculate the patient specific pathway activity, providing thereby, a summarization of different omics layers, as more robust signatures for elucidating meaningful patient subgroups and understanding mechanistic interactions.

One of the advantages of the Bayesian network analyses over single BMK analyses, is that the activity of BMKs that were not measured or had poor measurement quality in the patient sample (for instance TNFalpha, and IL6 in the ORIGIN trial) can be estimated, in special if these are central to the disease network. This can lead to the discovery of new BMKs of disease progression. TNFalpha and IL6 have been intensively studies in CVD models, but in humans their protein detection is somewhat cumbersome and their levels are also influenced by physical activity (Vijayaraghava et al., 2017).

Here, the inventors could infer their activities based on downstream interacting protein, from which measured levels were fed into the Bayesian model (Figure 2). The calculated activities may be interpreted as activation of the molecule, rather than actually a measure of levels of the protein, i.e., a transcription factor is calculated to be active or not based on downstream targets, not only of the effective measured protein levels. For instance, the TNFalpha pathway was less active in patients at higher risk to CV events. It may appear at first contradictory, as in functional studies inhibition of TNFalpha had beneficial effects on cardiac function and outcome. Nevertheless, most prospective studies with TNFalpha inhibitors bind, which to TNFalpha directly, reported disappointing and inconsistent results for CVO risk. For instance, in rheumatoid arthritis (RA) patients, it is rather the control of the disease itself (e.g., inflammatory pathway) than TNFalpha levels reduction per se that is necessary for CVO prevention (Coblyn et al., 2016). Thereby, specific protein interactions should be relevant to the inflammatory process and TNFalpha is just a peace of the puzzle. In this context, IL6, which is also an inflammatory marker, was predicted to be more active in the higher CVO risk cluster in the inventors study. The transcription factor nuclear factor kappa-B, which was predicted to be more active in the higher CVO risk cluster in the inventors study, regulates expression of many proinflammatory cytokines and is cardioprotective during acute hypoxia and reperfusion injury. As for TNFalpha, the activity of NTproBNT, a cardiac hormone that may function as a paracrine antifibrotic factor in the heart, was predicted to be lower in patients at higher CVO risk. Here, the inventors could confirm that the majority of these patients had actually higher levels of protein, than those less prone to CVO. BNP and NTproBNP peptides are released into the blood circulation in response to pressure and volume overload of the cardiac chambers. Cleavage of pre-proBNP precursor within cardiomyocytes leads to the formation of proBNP, which is subsequently cleaved into Nterminal (NTproBNP) and C-terminal (BNP) fragments. Most biological effects of BNP are the result of its binding to the natriuretic peptide receptor (Dhingra 2002). Thereby, NTproBNP calculated activity reflects rather molecular interactions that indicate it is active than the actual protein levels. The inventors, without wishing to be bound to any particularly theory, hypothesize that though NTproBNP levels are high in patients at higher CVO risk, it is being not effective as it should be.

NPPB and TFF3 were the top ranking associated BMKs by conventional statistics in the ORIGIN cohort, but did not appear in the network constructed with the CARDIOGRAM gene set. As the overconnectivity algorithm identifies the shortest path connecting genes, the inventors, without wishing to be bound to any particular theory, can conclude that: i) there is an active biological network that connects GWAs associated genes to protein biomarkers that had been associated to CVD, to which TNFalpha plays a central role; ii) though NPPB and TFF3 clearly are relevant biomarkers of CVD, these appear to be more downstream to the cascade of events related to the TNFalpha than the gene set identified in the CARDIOGRAM study. Nevertheless, for the purpose of identifying a disease related network for BMK discovery, protein BMKs should be more relevant than loci associated with CVD, as GWA results often map to genes that do not encode circulating proteins. In the patient stratification analyses, the inventors used mainly circulating proteins (N = 13 plus 2 genetic markers), making it feasible for translational application, as tissue specific samples are often difficult or not feasible to obtain in standard clinical practice.

Though the complexity of this computational approach does not make it a straight forward process to be used in the clinic, the obtained patient strata can be further investigated to identify ideal BMKs (including known clinical parameters) combinations and ratios that would represent different stages of disease progression.

Identifying clusters of sub-populations progressing differently towards CVO will be informative to: i) define how the molecular signatures of each cluster translate into combinations of biomarkers with prognostic value, and iii) to define how these markers will respond to treatment in an additional cohort. Thereby, molecular signatures of disease progression should lead to strategies to optimize clinical trials plans, and drug efficacy, and so to optimize treatment.

**Supplementary TABLE S1.** Study sample characteristics for the total sample, and high versus low CVO risk clusters for the Caucasian and Latino sub-populations.

**Supplementary TABLE S2**. List of molecular entities used in the overconnectivity analyses. Results from the GWA analyses conducted in ORIGIN and in the CARDIOGRAM consortium were assigned to loci (see Methods) and protein biomarkers to their respective genes.

**Supplementary TABLE S3**. List of associated loci included in the overconnectivity network analyses. Identified loci at P < 10-6, in the ORIGIN cohort, and P < 10-8, in the CARDIOGRAM consortium, were entered in the analyses. Attached excel file.

**Supplementary TABLE S4**. List of sub-networks identified in the overconnectivity analyses with their respective p-values.

**Supplementary TABLE S5.** Overconnectivity network result table. Nodes and relationships of entities comprising the networks used for the Bayesian statistics based network analyses.

**Supplementary TABLE S6.** Logistic regression model to identify standard CVO risk factors (age, sex, BMI, HbA1c, c-Peptide, HDL-C, LDL-C, TG, TC, SBP, DBP, smoking status, albuminuria or reported albuminuria) contributing to clusters separation in Caucasian and Latinos.

### Example 2 - detection of SNPs associated with CVD risk

Having identified two SNPs associated with an individual having an increased risk in cardiovascular disease (CVD), .i.e. Reference SNP cluster ID: rs73420079 (SEQ ID No: 3) and Reference SNP cluster ID: rs4314405 (SEQ ID No: 40), the inventors work enables the identification of individuals with an increased risk of CVD using oligonucleotide probes designed to detected the SNPs.

Oligonucleotide probes for detecting the presence of the SNPs can be produced and synthesized by any available oligonucleotide probe design tool, based on the SNP rs number. For example, probes of the SNPs can be sent to Illumina's^{®} Illumina Assay Design Tool for scoring, based on the rs number format, to produce an assay ready probe.

A sample may be isolated from the patient, the sample can be a blood sample. The individual's nucleic acid is isolated from the sample. The isolation may occur by any means convenient to the practitioner. For instance, the isolation may occur by first lysing the cell using detergents, enzymatic digestion or physical disruption. The contaminating material is then removed from the nucleic acids by use of, for example, enzymatic digestion, organic solvent extraction, or chromatographic methods.

The individual's nucleic acid may be purified and/or concentrated by any means, including precipitation with alcohol, centrifugation and/or dialysis. The individual's nucleic acid is then assayed for presence or absence of one or more of the SNPs using the oligonucleotide probes that are capable of hybridizing to a nucleic acid sequence comprising one or more of the SNPs.

The detection of Reference SNP cluster ID: rs73420079 (SEQ ID No: 3) and/or Reference SNP cluster ID: rs4314405 (SEQ ID No: 40) in the sample indicates that the individual is at in increased risk of CVD.

## Claims

1. A method of determining, diagnosing and/or prognosing an individual's risk of suffering from cardiovascular disease, the method comprising;
a. detecting, in a sample obtained from an individual, the expression level, amount and/or activity of two or more biomarkers selected from a group consisting of: Tumour Necrosis Factor (TNF)-□□ Glutathione S-Transferase Alpha 1 (GSTA1); N-terminal-pro hormone BNP (NT-proBNP); Retinoic Acid Receptor-Related Orphan Receptor Alpha (RORA); Tenascin C (TNC); Growth Hormone Receptor (GHR); Alpha-2-Macroglobulin (A2M); Insulin Like Growth Factor Binding Protein 2 (IGFBP2); Apolipoprotein B (APOB); Selenoprotein P (SEPP1); Trefoil Factor (TFF3); Interleukin 6 (IL6); Chitinase 3 Like 1 (CHI3L1); Hepatocyte Growth Factor Receptor (MET); Growth Differentiation Factor 15 (GDF15); Chemokine (C-C Motif) Ligand 22 (CCL22); Tumour Necrosis Factor Receptor Superfamily, Member 11 (TNFRSF11); Angiopoietin 2 (ANGPT2); and v -Rel Avian Reticuloendotheliosis Viral Oncogene Homolog A Nuclear Factor-kappa B (ReLA NF-KB);
b. comparing the expression level, amount and/or activity of the biomarker with a reference from a healthy control population; and
c. determining, diagnosing and/or prognosing the risk of an individual suffering from cardiovascular disease if the expression level, amount, and/or activity of the biomarker deviates from the reference from a healthy control population.

2. The method of claim 1, wherein a decrease in expression, amount and/or activity of TNF-□, GSTA1, NT-proBNP, RORA and/or TNC, when compared to the reference, is indicative of an individual having a higher risk of suffering from cardiovascular disease or a negative prognosis.

3. The method of either claim 1 or claim 2, wherein an increase in expression, amount and/or activity of GHR, A2M, IGFBP2, APOB, SEPP1, TFF3, IL6 and/or CHI3L1, when compared to the reference, is indicative of an individual having a higher risk of suffering from cardiovascular disease or a negative prognosis.

4. The method of any preceding claim, wherein a decrease in expression, amount and/or activity of MET, GDF15, CCL22, TNFRSF11, ANGPT2 and/or RelA NF-KB, when compared to the reference, is indicative of an individual having a lower risk of suffering from cardiovascular disease or a positive prognosis.

5. The method according to any preceding claim, wherein step a) comprises detecting, in a sample obtained from the individual, the expression levels, amount and/or activities of at least 3, at least 4, at least 5, at least 6, at least 7, at least 8, at least 9, at least 10, at least 11, at least 12, at least 13, at least 14, at least 15, at least 16, at least 17 or at least 18 of the biomarkers selected from the group consisting of: TNF-□; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2; and RelA NF-KB.

6. The method according to any proceeding claim, wherein the cardiovascular disease is selected from the group consisting of: cardiovascular death; myocardial infarction; stroke; and heart failure.

7. The method according to any proceeding claim, wherein the sample comprises blood, urine or tissue.

8. A kit for determining, diagnosing and/or prognosing the risk of an individual suffering from cardiovascular disease, the kit comprising:
a. detection means for detecting, in a sample obtained from a test subject, the expression level, amount and/or activity of two or more biomarkers selected from the group consisting of TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB; and
b. a reference value from a healthy control population for expression level, amount and/or activity of two or more a biomarkers selected from the group consisting of TNF-a; GSTA1; NT-proBNP; RORA; TNC; GHR; A2M; IGFBP2; APOB; SEPP1; TFF3; IL6; CHI3L1; MET; GDF15; CCL22; TNFRSF11; ANGPT2 and RelA NF-KB,
wherein the kit is used to identify:
i) a decrease in expression, amount and/or activity of TNF-a; GSTA1; NT-proBNP; RORA and/or TNC when compared to the reference; and /or an increase in expression, amount and/or activity of GHR; A2M; IGFBP2; APOB; SEPP1; TFF3; IL6 and/or CHI3L1; when compared to the reference to determine, diagnose and/or prognose that an individual has a higher risk of suffering from cardiovascular disease; and/or
ii) a decrease expression, amount and/or activity of MET; GDF15; CCL22; TNFRSF11; ANGPT2 and/or RelA NF-KB when compared to the reference to determine, diagnose and/or prognose that an individual has a lower risk of suffering from cardiovascular disease.

9. A method of determining, diagnosing and/or prognosing an individual's risk of suffering from cardiovascular disease, the method comprising detecting, in a sample obtained from an individual, a single nucleotide polymorphism (SNP) in the RORA gene, wherein the presence of the SNP is indicative of an individual having an increased risk of suffering from cardiovascular disease.

10. The method according to claim 9, wherein the SNP is Reference SNP cluster ID: rs73420079.

11. A method of determining, diagnosing and/or prognosing an individual's risk of suffering from cardiovascular disease, the method comprising detecting, in a sample obtained from an individual, a single nucleotide polymorphism (SNP) in the GHR gene, wherein the presence of the SNP is indicative of an individual having an increased risk of suffering from cardiovascular disease.

12. The method according to claim 11, wherein the SNP is Reference SNP cluster ID: rs4314405.

13. GHR and/or RORA, for use in diagnosis or prognosis.

14. GHR and/or RORA, for use in diagnosing or prognosing cardiovascular disease.

15. GHR and/or RORA for use according to claim 13 or claim 14, wherein RORA comprises an SNP as defined in claim 10 and/or GHR comprise an SNP as defined in claim 12.
